(19) Europäisches Patentamt
European Patent Office
Office européen des brevets

(11) EP 4 585 598 A1

(12) **EUROPEAN PATENT APPLICATION**
published in accordance with Art. 153(4) EPC

(43) Date of publication:
**16.07.2025 Bulletin 2025/29**

(21) Application number: **23862325.0**

(22) Date of filing: **04.09.2023**

(51) International Patent Classification (IPC):
*C07D 498/22* (2006.01) *C07D 471/22* (2006.01)
*C07D 487/22* (2006.01) *C07D 515/22* (2006.01)
*A61K 31/5025* (2006.01) *A61P 25/00* (2006.01)
*A61P 29/00* (2006.01) *A61P 35/00* (2006.01)

(52) Cooperative Patent Classification (CPC):
**A61K 31/5025; A61K 31/504; A61K 31/529; A61P 25/00; A61P 25/28; A61P 29/00; A61P 35/00; A61P 35/02; C07D 471/22; C07D 487/22; C07D 498/22; C07D 515/22**

(86) International application number:
**PCT/CN2023/116671**

(87) International publication number:
**WO 2024/051631 (14.03.2024 Gazette 2024/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.09.2022 CN 202211090622**

(71) Applicant: **Suzhou Langrui Biopharmaceutical Co., Ltd.**
**Suzhou, Jiangsu 215000 (CN)**

(72) Inventors:
- **XING, Li**
  **Suzhou, Jiangsu 215000 (CN)**
- **ZHU, Wei**
  **Suzhou, Jiangsu 215000 (CN)**
- **LIU, Haitao**
  **Suzhou, Jiangsu 215000 (CN)**
- **WU, Fan**
  **Suzhou, Jiangsu 215000 (CN)**

(74) Representative: **Mewburn Ellis LLP**
**Aurora Building**
**Counterslip**
**Bristol BS1 6BX (GB)**

(54) **MACROCYCLIC IMIDAZO[1,2-B]PYRIDAZINE DERIVATIVE, PREPARATION METHOD THEREFOR, AND USE THEREOF**

(57) Disclosed are a macrocyclic imidazo[1,2-b]pyridazine compound represented by formula (I) or an isotopically labeled compound thereof, or an optical isomer thereof, a geometric isomer, a tautomer thereof or an isomer mixture thereof, or a pharmaceutically acceptable salt thereof, or a prodrug thereof, or a metabolite thereof, and a pharmaceutical composition of the derivative. The macrocyclic imidazo[1,2-b]pyridazine compound represented by formula I or the pharmaceutical composition can be used as a TRK kinase inhibitor for treating or preventing diseases or symptoms mediated by TRK or TRK mutation.

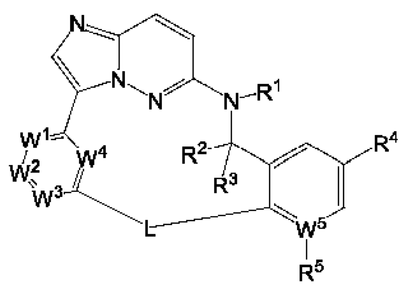

Formula I

EP 4 585 598 A1

## Description

This application claims the priority interests of application number 202211090622.9, titled "macrocyclic imidazo [1,2-b] pyridazine derivative, preparation method therefor, and use thereof" submitted to the China National Intellectual Property Administration on September 7, 2022, and the content of which is incorporated herein by reference in its entirety.

## Technical field

The application belongs to the field of medicine, specifically, relates to a macrocyclic imidazo [1,2-b] pyridazine derivative and a pharmaceutically acceptable salt thereof, preparation method therefor, as well as its use as a TRK kinase inhibitor, and a pharmaceutical composition comprising the derivative.

## Background

The tropomyosin receptor kinase (TRK) protein is a family of tyrosine receptor kinases consisting of three members: TRKA, TRKB, and TRKC. It is widely expressed in the nervous system and many non neuronal tissue types. TRKA, B, and C are encoded by the neurotrophic tyrosine receptor kinase (NTRK) genes NTRK1, NRTK2, and NTRK3, respectively. These three subtypes have sequence and structural homology, consisting of extracellular, transmembrane, and intracellular domains, and differ in ligand specific and tissue-specific expression. The TRK receptor is activated by the high affinity binding of its extracellular domain to its homologous ligand, leading to receptor dimerization and subsequent autophosphorylation of tyrosine residues in the intracellular domain. Due to the fact that most early studies on the functional role of TRK have focused on neural development, homologous ligands are known as neurotrophic factors, namely nerve growth factor (NGF), brain-derived neurotrophic factor (BDNF), neurotrophin-3 (NT-3), and neurotrophin-4 (NT-4, also known as NT-5). During neuronal development, TRK protein mediates neuronal survival and synaptic plasticity in the central and peripheral nervous systems. The TRK receptor is physiologically activated through interaction with its primary neurotrophic factor ligand, which binds to the extracellular domain of the receptor. The binding specificity of neurotrophinic ligand is TRK receptor type dependent, and subsequent interactions induce receptor homodimerization, phosphorylation of tyrosine residues on cytoplasmic domains, and activation of downstream signaling pathways known to be associated with growth and development. TRKA mediates signal transduction through MAPK and RAS/ERK pathways, promoting neuronal proliferation and differentiation. TRKB activates RAS/ERK and PI3K signaling pathways, which is crucial for neuronal survival. TRKC activates PI3K/AKT pathway to prevent cell apoptosis and increase cell survival.

The fusion involving all three NTRK genes has been reported in various types of cancer, which is caused by chromosomal gene rearrangements or chromosomal translocations. NTRK gene fusion events occur between NTRK1, 2, or 3 and various unrelated gene partners, representing major genomic changes with carcinogenic potential. Compared to less common oncogenic mechanisms, the latter include intra frame deletions and alternative splicing variants of NTRK1. Carcinogenic rearrangements involving NTRK genes typically originate from the fusion of the 3' region of NTRK genes and the 5' region of unrelated genes. The resulting NTRK gene fusion retains the kinase domain of the TRK receptor, which fuses with its partner in the framework to form a new oncoprotein. Carcinogenic fusion proteins are typically constitutive activated or overexpressed kinases, resulting in abnormal expression and activation of fusion oncoproteins, which subsequently promote the development of cancer cells and tumor. For example, the frequency of fusion genes of NTRK1 and TPM3 is about 0.04%, ranking first among various fusion genes, which is closely related to breast cancer, cervical cancer, cholangiocarcinoma, colorectal cancer, glioma, infantile fibrosarcoma, lung, soft tissue sarcoma, thyroid cancer, uterine sarcoma, etc. For another example, the frequency of fusion genes of NTRK3 and ETV6 is about 0.09%, which is closely related to acute lymphoblastic leukemia, acute myeloid leukemia, breast cancer, colorectal cancer, congenital mesodermal nephroma, gastrointestinal stromal tumor, glioma, infantile fibrosarcoma, inflammatory myofibroblastic tumor, lung, melanoma, neuroendocrine, secretory breast cancer, salivary gland secretory cancer, paranasal sinus adenocarcinoma, soft tissue sarcoma, Spitzoid melanoma, and thyroid cancer.

NTRK gene fusion in human cancer: after the discovery of kinase inhibitors with therapeutic effects in this field, there has been renewed interest in NTRK gene fusion as an oncogene. Although NTRK gene fusion is rare (occurring only in about 1% of all malignant tumors), they have been identified in many different tumor types in children and adults. In more common types of tumors, such as lung cancer, sarcoma, thyroid cancer, and colorectal cancer, NTRK gene fusion occurs at a low frequency. On the contrary, in several rare cancers, including secretory breast cancer, breast analog secretory cancer, infantile fibrosarcoma and congenital mesenchymal kidney, the prevalence of NTRK gene fusion is very high. NTRK fusion is a rare carcinogenic driver, occurring in a series of pediatric cancers. These include infantile fibrosarcoma and secretory breast cancer, in which this fusion is almost characteristic, and a series of more common pediatric cancers, in which NTRK fusion occurs at a lower frequency. In the past few years, two TRK inhibitors, Lalotinib and Entrotinib, have demonstrated histological activity against NTRK fusion driven cancer and have received FDA approval.

Larotinib and Entrotinib have shown significant efficacy in cancer patients carrying TRK fusion, however, like

most kinase inhibitors, acquired resistance mediated by kinase domain mutations has occurred. The acquired resistance mutations of TRK, such as solvent-front mutations, gatekeeper mutations, and DFG motif mutations, has a median response duration of 8.3 months for larotinib and 10.5 months for Entrotinib in the NTRK fusion subgroup based on summary analysis of early trials. Subsequent targeted sequencing in patients with initial response indicates that up to 90% of patients have secondary mutations in the kinase domain, which are expected to lead to drug resistance. Most of the reported mutations involve amino acid substitutions in the solvent-front (NTRK1 p.G595R, NTRK2 p.G639R, NTRK3 p.G623R), goalkeeper residues (NTRK1 p.F589 L, NTRK3 p.F617L), or activated ring X-aspartate-phenylalanine-glycine, "xDFG" motif (NTRK1 p.G667C, NTRK2 p.G709C, NTRK3 p.G696A). The mutation sites are mainly located in TRKCG623R (homologous TRKAG595R) and TRKAG667C. Computational modeling and X-ray crystallography indicate that most of these mutations result in steric hindrance conflicts between bulky side chains involved in amino acids (such as arginine) and hydroxypyrrolidine or difluorophenyl groups of the first generation TRK inhibitors. Some of these mutations are also predicted to increase the ATP affinity of kinase domains. Some identified mutations are homologous to the reported recurrent solvent-front and gatekeeper resistance mutations in ALK and ROS1 rearranged cancers, which often occurs and counteracts the inhibitory effect of the first generation inhibitors.

To overcome acquired resistance, the second-generation TRK inhibitors TPX-0005 and LOXO-195 targeting wild-type and mutant TRK fusion are currently under clinical development. TPX-0005 and LOXO-195 have been proven effective in preclinical animal models and patient clinical studies. The potential drawback of TRK inhibitors TPX-0005 and LOXO-195 is their limited ability to penetrate the blood-brain barrier (BBB). Therefore, more research is needed to effectively target TRK fusion related tumors in the brain. For example, some newly developed TRK small molecule inhibitors have been disclosed in Chinese patent applications such as CN102264736A and CN104520300A. Therefore, the development of new TRK inhibitors that can effectively overcome drug resistance mutations can provide huge clinical benefits for patients.

## Summary

According to one aspect of the present invention, a purpose of the present invention is to provide a macrocyclic imidazo [1,2-b] pyridazine compound represented by Formula I, or an isotopically labeled compound, an optical isomer, a geometric isomer, a tautomer, an isomers mixture, a pharmaceutically acceptable salt, a prodrug, or a metabolite thereof,

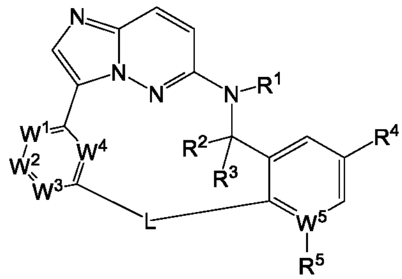

Formula I

wherein,

$W^1$, $W^2$, $W^3$, $W^4$ and $W^5$ are each independently selected from carbon or nitrogen, and at least one of $W^1$, $W^2$, $W^3$, $W^4$ and $W^5$ is nitrogen;

$R^1$, $R^2$ and $R^3$ are each independently selected from hydrogen, deuterium, halogen, cyano, hydroxyl, amino, substituted or unsubstituted saturated or unsaturated $C_1$-$C_6$ alkyl, substituted or unsubstituted saturated or unsaturated $C_3$-$C_6$ cycloalkyl, substituted or unsubstituted saturated or unsaturated $C_1$-$C_6$ alkoxy, substituted or unsubstituted saturated or unsaturated $C_3$-$C_6$ cycloalkoxy, or $R^1$ and $R^2$ together with the N and C atoms to which they are connected form a substituted or unsubstituted 4- to 10- membered heterocycloalkyl containing 1 to 3 heteroatoms selected from N, O and S, or a substituted or unsubstituted 5-to 14- membered heteroaryl containing 1 to 3 heteroatoms selected from N, O and S, or $R^2$ and $R^3$ together with the C atom to which they are connected form a substituted or unsubstituted saturated or unsaturated $C_3$-$C_6$ cycloalkyl; wherein the "substituted" refers to selectively having 1 to 4 substituents selected from deuterium, hydroxyl, halogen, cyano, sulfonyl, amino, $C_1$-$C_4$ alkyl, $C_3$-$C_6$ cycloalkyl, $C_1$-$C_4$ alkoxy and $C_3$-$C_6$ cycloalkoxy;

$R^4$ is selected from hydrogen, deuterium, halogen, cyano, hydroxyl, amino, saturated or unsaturated $C_1$-$C_6$ alkyl, saturated or unsaturated $C_3$-$C_6$ cycloalkyl, saturated or unsaturated $C_1$-$C_6$ alkoxy, and saturated or unsaturated $C_3$-$C_6$ cycloalkoxy;

$R^5$ is selected from hydrogen, deuterium, halogen, cyano, hydroxyl, amino, saturated or unsaturated $C_1$-$C_6$ alkyl,

saturated or unsaturated $C_3$-$C_6$ cycloalkyl, substituted or unsaturated $C_1$-$C_6$ alkoxy, and saturated or unsaturated $C_3$-$C_6$ cycloalkoxy, or $R^5$ is absent;

L is selected from -O-, -NH-, substituted or unsubstituted branched or straight chain $C_1$-$C_6$ alkylene, substituted or unsubstituted branched or straight chain $C_1$-$C_6$ alkyleneoxy, substituted or unsubstituted saturated or unsaturated $C_3$-$C_6$ cycloalkylene, substituted or unsubstituted saturated or unsaturated $C_3$-$C_6$ cycloalkyleneoxy, substituted or unsubstituted branched or straight chain $C_1$-$C_6$ alkylenethio, substituted or unsubstituted saturated or unsaturated 3 to 6-membered oxacycloalkylene, substituted or unsubstituted saturated or unsaturated 3 to 6-membered azacycloalkylene, substituted or unsubstituted saturated or unsaturated 3 to 6-membered thiacycloalkylene, substituted or unsubstituted unsubstituted saturated or unsaturated 3- to 6-membered oxacycloalkyleneoxy, substituted or unsubstituted saturated or unsaturated 3- to 6-membered azacycloalkyleneoxy, substituted or unsubstituted saturated or unsaturated 3- to 6-membered thiacycloalkyleneoxy; wherein the "substituted" refers to selectively having 1 to 4 substituents selected from deuterium, halogen, cyano, hydroxyl, carboxyl, carbonyl, sulfonyl, amino, $C_1$-$C_4$ alkyl, $C_1$-$C_4$ hydroxyalkyl, $C_3$-$C_6$ cycloalkyl, $C_1$-$C_4$ alkoxy, $C_3$-$C_6$ cycloalkoxy, 4 to 8-membered heterocycloalkyl containing 1 to 3 heteroatoms selected from N, O and S, and 5 to 10 membered heteroaryl containing 1 to 3 heteroatoms selected from N, O and S.

Preferably, $R^1$, $R^2$ and $R^3$ are each independently selected from hydrogen, deuterium, halogen, cyano, hydroxyl, amino, substituted or unsubstituted saturated or unsaturated $C_1$-$C_4$ alkyl, substituted or unsubstituted saturated or unsaturated $C_3$-$C_6$ cycloalkyl, substituted or unsubstituted saturated or unsaturated $C_1$-$C_4$ alkoxy, substituted or unsubstituted saturated or unsaturated $C_3$-$C_6$ cycloalkoxy, or $R^1$ and $R^2$ together with the N and C atoms to which they are connected form a substituted or unsubstituted 4-to 8- membered heterocycloalkyl containing 1 to 3 heteroatoms selected from N, O and S, or a substituted or unsubstituted 5-to 10- membered heteroaryl containing 1 to 3 heteroatoms selected from N, O and S, or $R^2$ and $R^3$ together with the C atom to which they are connected form a substituted or unsubstituted saturated or unsaturated $C_3$-$C_6$ cycloalkyl; wherein the "substituted" refers to selectively having 1 to 3 substituents selected from deuterium, hydroxyl, halogen, cyano, sulfonyl, amino, $C_1$-$C_3$ alkyl, $C_3$-$C_6$ cycloalkyl, $C_1$-$C_3$ alkoxy and $C_3$-$C_6$ cycloalkoxy;

More preferably, $R^1$, $R^2$ and $R^3$ are each independently selected from hydrogen, deuterium, halogen, cyano, hydroxyl, amino, substituted or unsubstituted saturated or unsaturated $C_1$-$C_3$ alkyl, substituted or unsubstituted saturated or unsaturated $C_5$-$C_6$ cycloalkyl, or $R^1$ and $R^2$ together with the N and C atoms to which they are connected form a substituted or unsubstituted 4-to 7-membered heterocycloalkyl containing 1 to 3 heteroatoms selected from N, O and S, or $R^2$ and $R^3$ together with the C atom to which they are connected form a substituted or unsubstituted saturated or unsaturated $C_5$-$C_6$ cycloalkyl; wherein the "substituted" refers to selectively having 1 to 3 substituents selected from deuterium, hydroxyl, halogen, cyano, sulfonyl, amino, $C_1$-$C_3$ alkyl and $C_5$-$C_6$ cycloalkyl.

More preferably, $R^1$, $R^2$ and $R^3$ are each independently selected from hydrogen, deuterium, halogen, substituted or unsubstituted saturated or unsaturated $C_1$-$C_3$ alkyl, or $R^1$ and $R^2$ together with the N and C atoms to which they are connected form a substituted or unsubstituted 4- to 6-membered heterocycloalkyl containing 1 or 2 heteroatoms selected from N, O and S, or $R^2$ and $R^3$ together with the C atom to which they are connected form a substituted or unsubstituted saturated or unsaturated $C_5$-$C_6$ cycloalkyl; wherein the "substituted" refers to selectively having 1 or 2 substituents selected from deuterium, hydroxyl and halogen.

Preferably, $R^4$ is selected from hydrogen, deuterium, halogen, cyano, hydroxyl, amino, saturated or unsaturated $C_1$-$C_4$ alkyl, saturated or unsaturated $C_5$-$C_6$ cycloalkyl, saturated or unsaturated $C_1$-$C_4$ alkoxy, and saturated or unsaturated $C_5$-$C_6$ cycloalkoxy.

More preferably, $R^4$ is selected from hydrogen, deuterium, halogen, cyano, hydroxyl, amino, saturated or unsaturated $C_1$-$C_3$ alkyl, and saturated or unsaturated $C_1$-$C_3$ alkoxy.

More preferably, $R^4$ is selected from hydrogen, deuterium, halogen, saturated or unsaturated $C_1$-$C_3$ alkyl, and saturated or unsaturated $C_1$-$C_3$ alkoxy.

More preferably, $R^4$ is selected from hydrogen, deuterium and halogen.

Preferably, $R^5$ is selected from hydrogen, deuterium, halogen, cyano, hydroxyl, amino, saturated or unsaturated $C_1$-$C_4$ alkyl, saturated or unsaturated $C_5$-$C_6$ cycloalkyl, substituted or unsaturated $C_1$-$C_4$ alkoxy, and saturated or unsaturated $C_5$-$C_6$ cycloalkoxy, or $R^5$ is absent.

More preferably, $R^5$ is selected from hydrogen, deuterium, halogen, cyano, hydroxyl, amino, saturated or unsaturated $C_1$-$C_3$ alkyl, and substituted or unsaturated $C_1$-$C_3$ alkoxy, or $R^5$ is absent.

More preferably, $R^5$ is selected from hydrogen, deuterium, halogen, saturated or unsaturated $C_1$-$C_3$ alkyl, and substituted or unsaturated $C_1$-$C_3$ alkoxy, or $R^5$ is absent.

More preferably, $R^5$ is selected from hydrogen, deuterium and halogen, or $R^5$ is absent.

Preferably, L is selected from -O-, -NH-, substituted or unsubstituted branched or straight chain $C_1$-$C_4$ alkylene, substituted or unsubstituted branched or straight chain $C_1$-$C_4$ alkyleneoxy, substituted or unsubstituted saturated or unsaturated $C_4$-$C_6$ cycloalkylene, substituted or unsubstituted saturated or unsaturated $C_4$-$C_6$ cycloalkyleneoxy, sub-

stituted or unsubstituted branched or straight chain $C_1$-$C_4$ alkylenethio, substituted or unsubstituted saturated or unsaturated 4 to 6-membered oxacycloalkylene, substituted or unsubstituted saturated or unsaturated 4 to 6-membered azacycloalkylene, substituted or unsubstituted saturated or unsaturated 4 to 6-membered thiacycloalkylene, substituted or unsubstituted unsubstituted saturated or unsaturated 4- to 6-membered oxacycloalkyleneoxy, substituted or unsubstituted saturated or unsaturated 4- to 6-membered azacycloalkyleneoxy, substituted or unsubstituted saturated or unsaturated 4- to 6-memberedthiacycloalkyleneoxy; wherein the "substituted" refers to selectively having 1 to 3 substituents selected from deuterium, halogen, cyano, hydroxyl, carbonyl, amino, $C_1$-$C_4$ alkyl, $C_1$-$C_4$ hydroxyalkyl and $C_3$-$C_6$ cycloalkyl.

More preferably, L is selected from -O-, -NH-, substituted or unsubstituted branched or straight chain $C_1$-$C_3$ alkylene, substituted or unsubstituted branched or straight chain $C_1$-$C_4$ alkyleneoxy, substituted or unsubstituted saturated or unsaturated $C_5$-$C_6$ cycloalkylene, substituted or unsubstituted saturated or unsaturated $C_5$-$C_6$ cycloalkyleneoxy, substituted or unsubstituted saturated or unsaturated 5 to 6-membered oxacycloalkylene, substituted or unsubstituted saturated or unsaturated 5 to 6-membered azacycloalkylene, substituted or unsubstituted unsubstituted saturated or unsaturated 5 to 6-membered oxacycloalkyleneoxy, substituted or unsubstituted saturated or unsaturated 5 to 6-membered azacycloalkyleneoxy; wherein the "substituted" refers to selectively having 1 to 3 substituents selected from deuterium and halogen

More preferably, L is selected from substituted or unsubstituted branched or straight chain $C_1$-$C_3$ alkylene, substituted or unsubstituted branched or straight chain $C_1$-$C_3$ alkyleneoxy, substituted or unsubstituted saturated or unsaturated $C_5$-$C_6$ cycloalkylene, substituted or unsubstituted saturated or unsaturated $C_5$-$C_6$ cycloalkyleneoxy, substituted or unsubstituted saturated or unsaturated 5 to 6-membered oxacycloalkylene, substituted or unsubstituted saturated or unsaturated 5 to 6-membered azacycloalkylene, substituted or unsubstituted unsubstituted saturated or unsaturated 5 to 6-membered oxacycloalkyleneoxy, substituted or unsubstituted saturated or unsaturated 5 to 6-membered azacycloalkyleneoxy; wherein the "substituted" refers to selectively having 1 to 2 substituents selected from deuterium and halogen.

More preferably, L is selected from $-CH_2O-$, $-CH_2CH_2O-$, $-CH_2CH_2CH_2O-$, $-CH_2CH(CH_3)O-$, $-CH(CH_3)CH_2O-$,

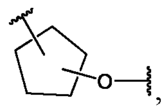, 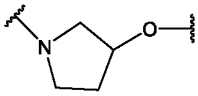, 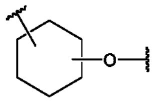 and 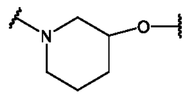.

Preferably, the compound represented by Formula I, or an isotopically labeled compound, an optical isomer, a geometric isomer, a tautomer, an isomers mixture, a pharmaceutically acceptable salt, a prodrug, or a metabolite thereof, is represented by the following Formula I-1, Formula I-2, Formula I-3, Formula I-4, Formula I-5 or Formula I-6:

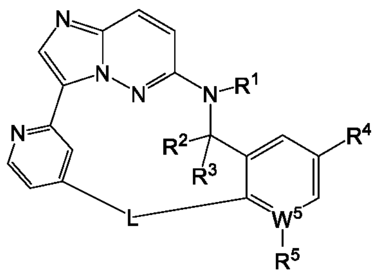
Formula I-1

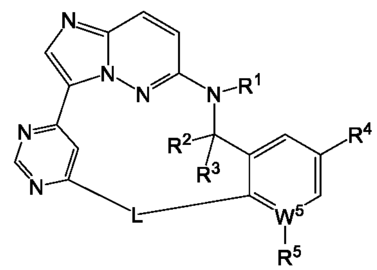
Formula I-2

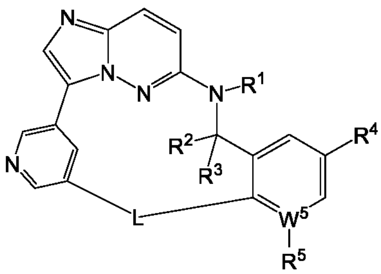
Formula I-3

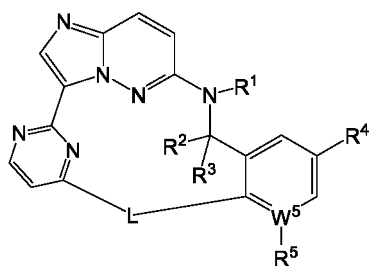
Formula I-4

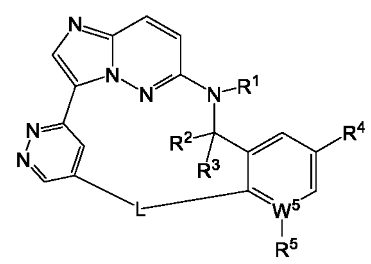
Formula I-5

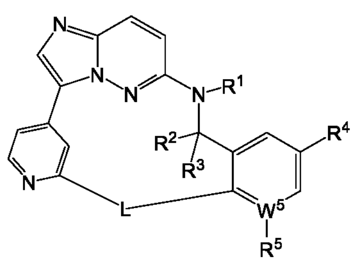
Formula I-6

wherein, the substituents $W^5$, $R^1$, $R^2$, $R^3$, $R^4$, $R^5$ and L are as defined in the above Formula I.

Preferably, the compound represented by Formula I, or an isotopically labeled compound, an optical isomer, a geometric isomer, a tautomer, an isomers mixture, a pharmaceutically acceptable salt, a prodrug, or a metabolite thereof, is represented by the following Formula I-1-1, Formula I-2-1, Formula I-3-1, Formula I-4-1, Formula I-5-1 or Formula I-6-1:

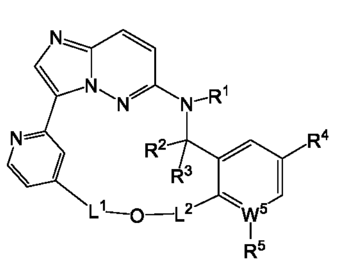

Formula I-1-1

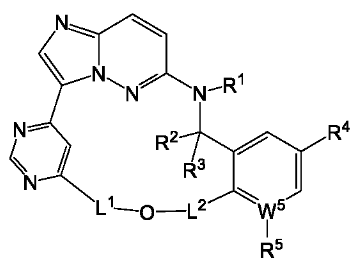

Formula I-2-1

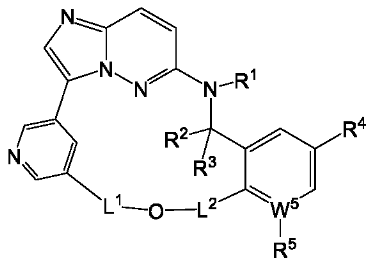

Formula I-3-1

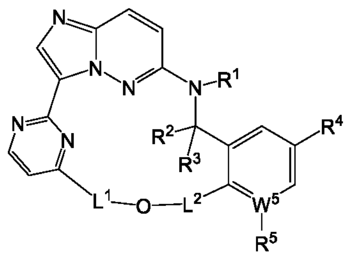

Formula I-4-1

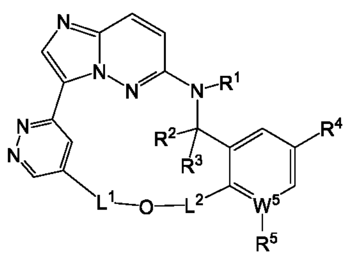

Formula I-5-1

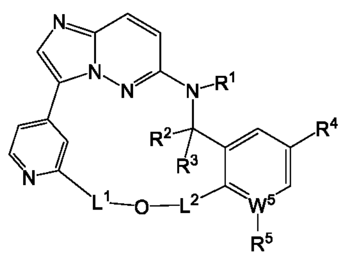

Formula I-6-1

wherein, the substituents $W^5$, $R^1$, $R^2$, $R^3$, $R^4$ and $R^5$ are as defined in the above Formula I;
$L^1$ and $L^2$ are independently selected from a chemical bond, substituted or unsubstituted branched or straight chain $C_1$-$C_3$ alkylene, substituted or unsubstituted saturated or unsaturated -$C_3$-$C_6$ - cycloalkylene, substituted or unsubstituted saturated or unsaturated 3- to 6-membered heterocyclic alkylene ring containing 1 or 2 heteroatoms selected from O and N; wherein the "substituted" refers to selectively having 1 to 2 substituents selected from deuterium and halogen, and $L^1$ and $L^2$ are not simultaneously chemical bonds.

Preferably, $L^1$ and $L^2$ are independently selected from chemical bond, methylene, ethylene, propylene, isopropylene, cyclopropylene, cyclobutylene, cyclopentylene, cyclohexylene, halogenated methylene, halogenated ethylene, halogenated propylene, halogenated isopropylene, halogenated cyclopropylene, halogenated cyclobutylene, halogenated cyclopentylene, halogenated cyclohexylene, oxiranylene, oxetanylene, tetrahydrofuranylene, tetrahydropyranylene, pyrrolidinylene, piperidinylene, halogenated oxiranylene, halogenated oxetanylene, halogenated tetrahydrofuranylene, halogenated tetrahydropyranylene, halogenated pyrrolidinylene, halogenated piperidinylene, and $L^1$ and $L^2$ are not simultaneously chemical bonds.

Preferably, the compound represented by Formula I, Formula I-1, Formula I-2, Formula I-3, Formula I-4, Formula I-5, Formula I-6, Formula I-1-1, Formula I-2-1, Formula I-3-1, Formula I-4-1, Formula I-5-1 or Formula I-6-1, or an isotopically labeled compound, an optical isomer, a geometric isomer, a tautomer, an isomers mixture, a pharmaceutically acceptable salt, a prodrug, or a metabolite thereof, is selected from the following compounds:

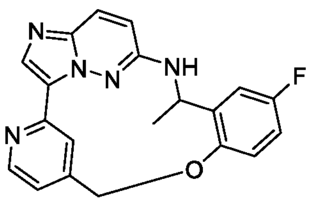

I-1

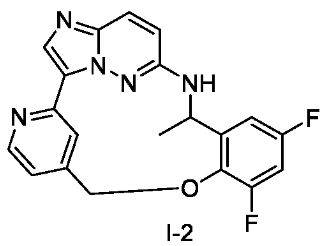

I-2

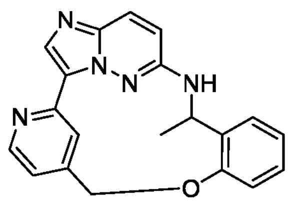

I-3

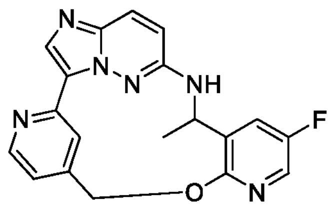

I-4

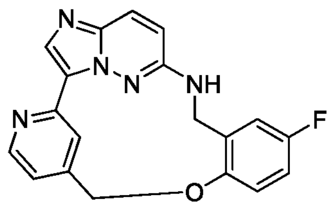

I-5

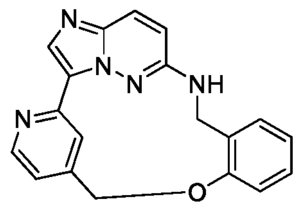

I-6

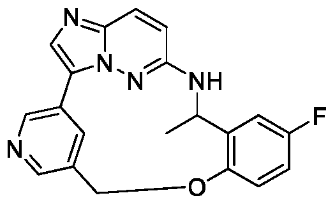

I-7

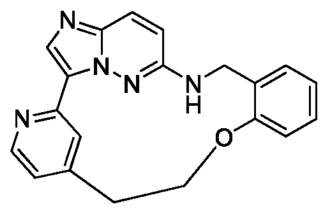

I-8

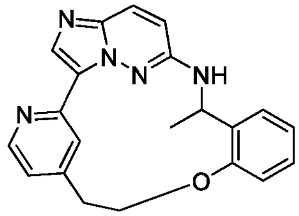

I-9

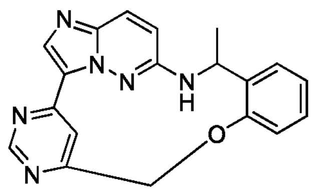

I-10

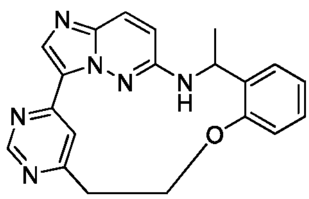

I-11

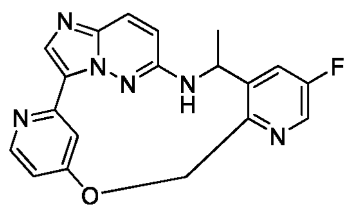

I-12

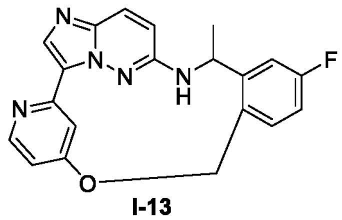

I-13

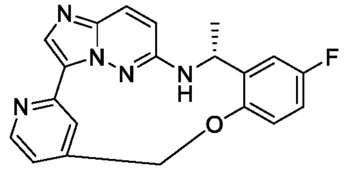

I-14

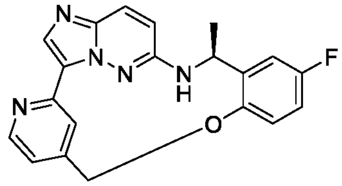

I-15

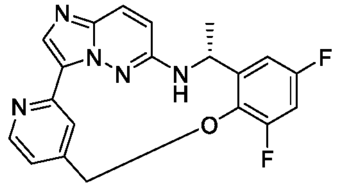

I-16

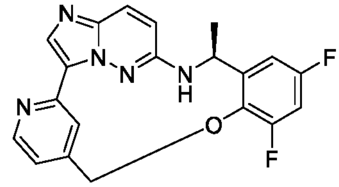

I-17

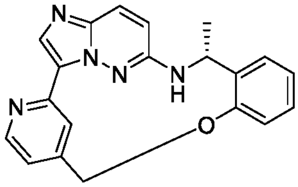

I-18

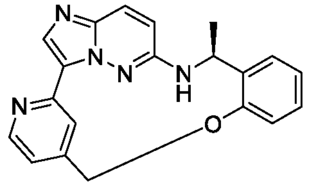

I-19

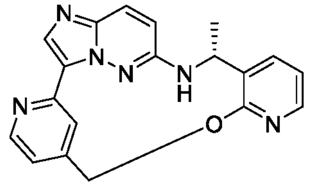

I-20

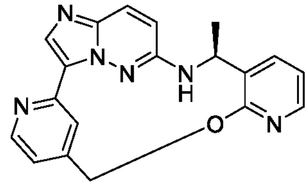

I-21

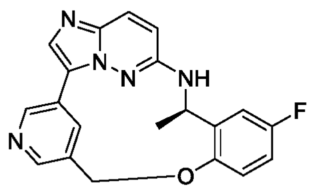

I-22

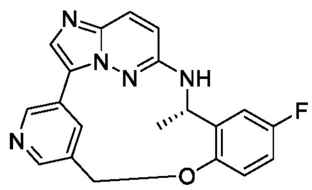

I-23

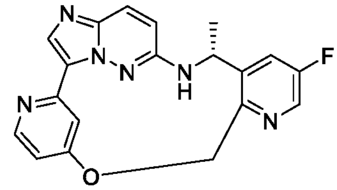

I-24

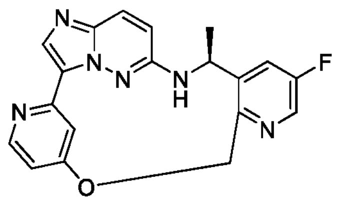

I-25

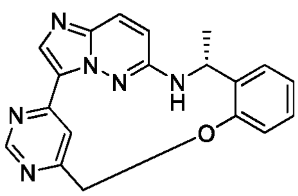

I-26

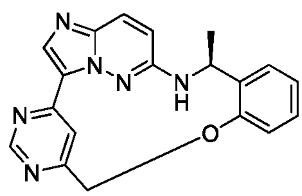

I-27

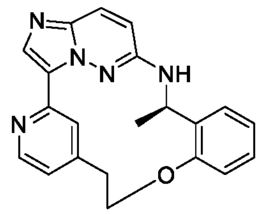

I-28

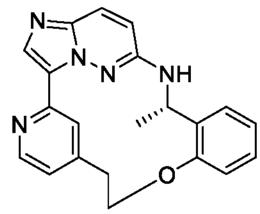

I-29

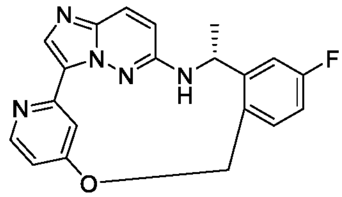

I-30

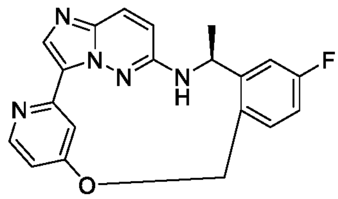

I-31

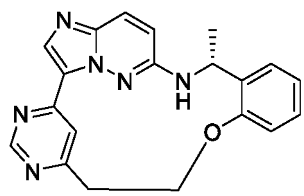

I-32

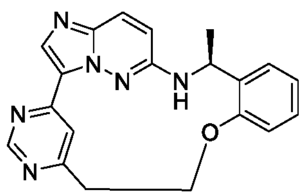

I-33

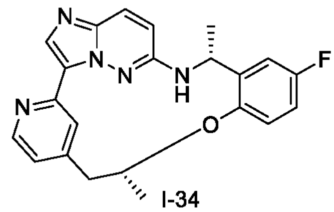

I-34

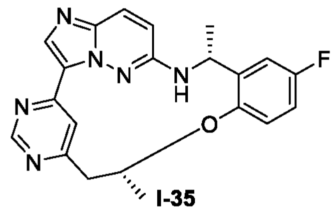

I-35

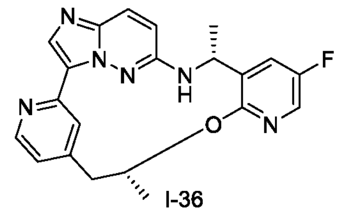

I-36

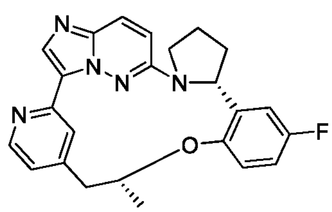

I-37

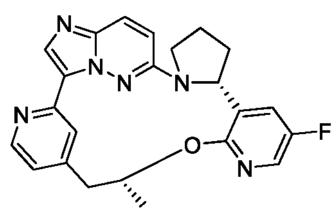

I-38

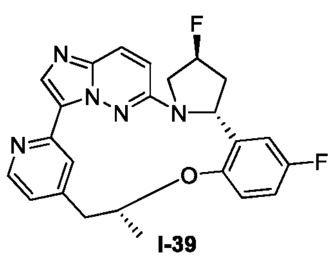

I-39

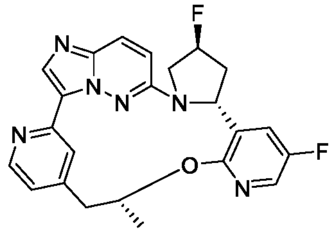

I-40

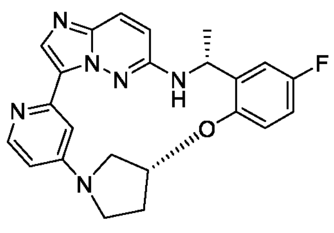

I-41

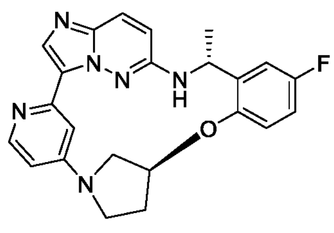

I-42

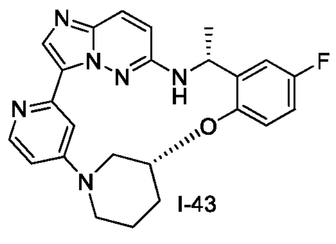

I-43

I-43

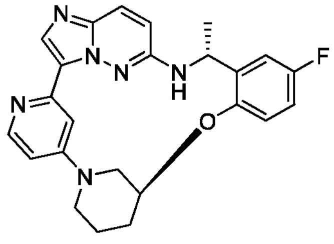

I-44

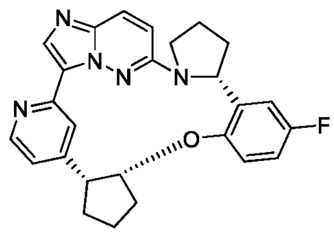

I-45

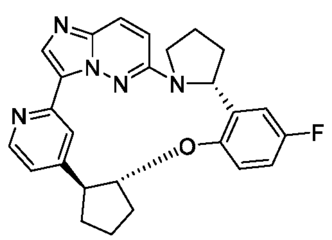

I-46

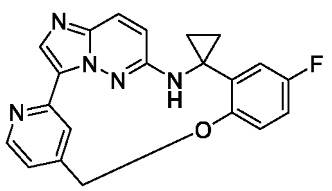

I-47

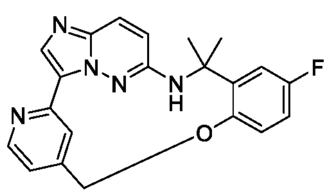

I-48

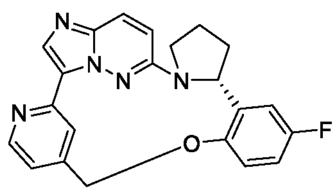

I-49

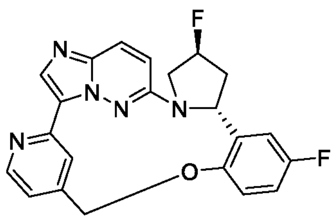

I-50

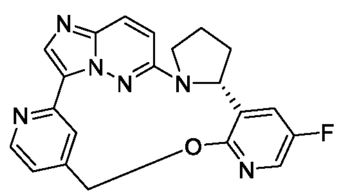

I-51

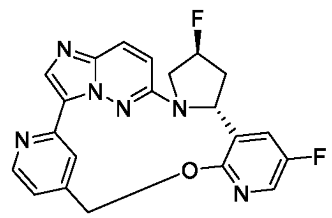

I-52

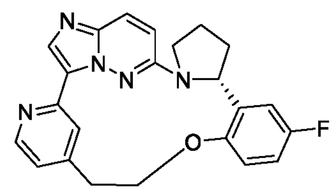

I-53

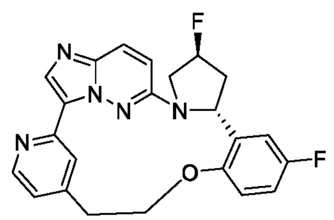

I-54

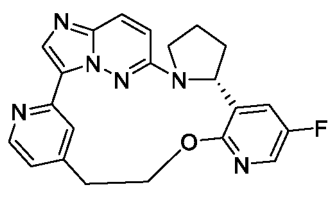

I-55

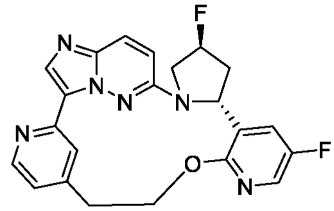

I-56

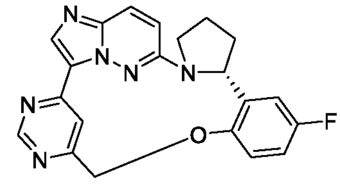

I-57

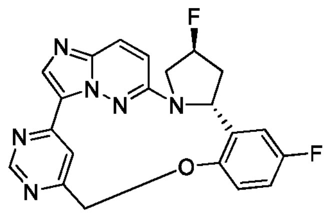

I-58

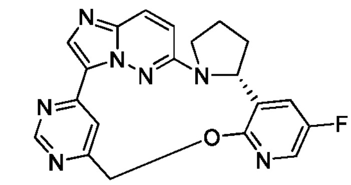

I-59

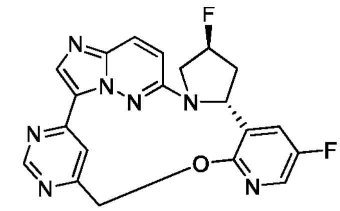

I-60

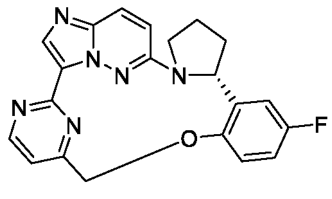

I-61

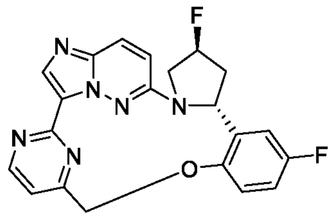

I-62

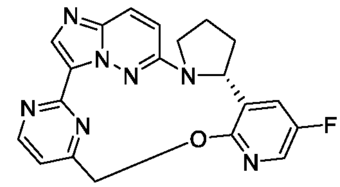

I-63

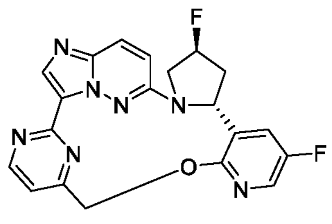

I-64

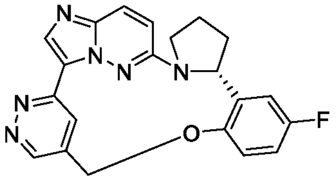

I-65

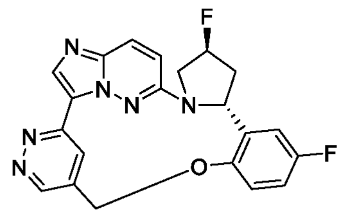

I-66

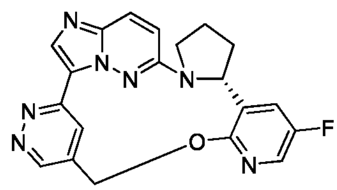

I-67

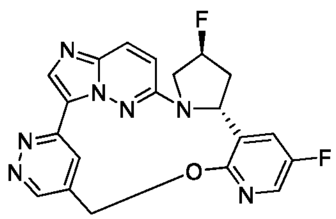

I-68

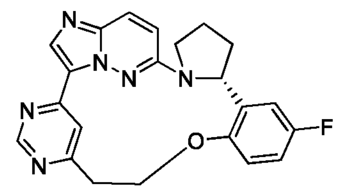

I-69

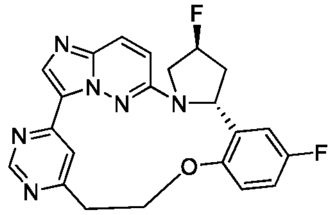

I-70

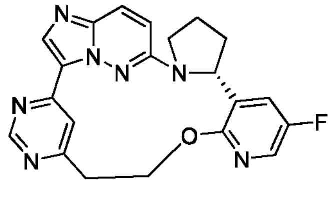

I-71

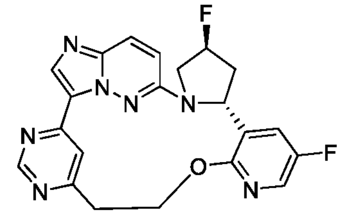

I-72

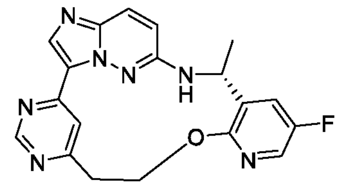

I-73

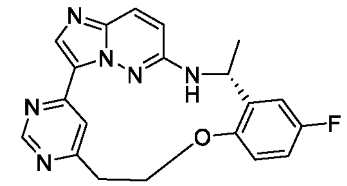

I-74

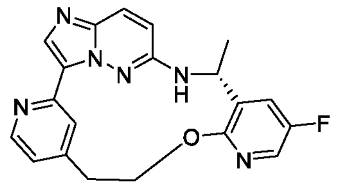

I-75

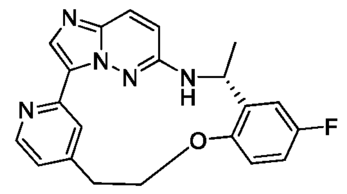

I-76

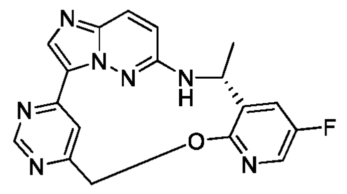

I-77

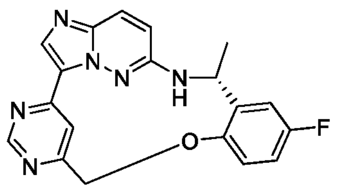

I-78

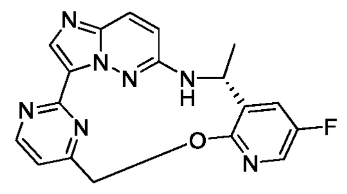

I-79

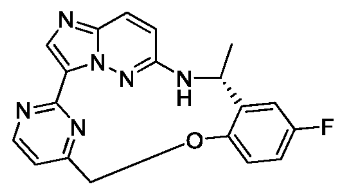

I-80

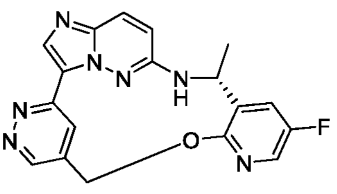

I-81

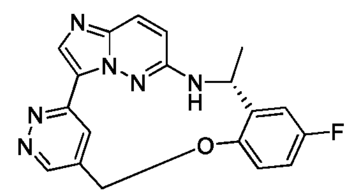

I-82

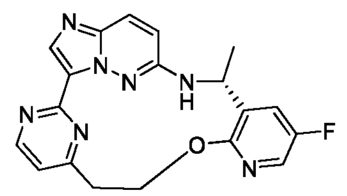

I-83

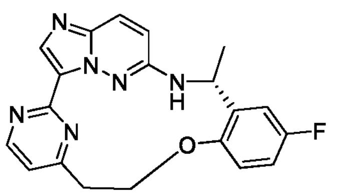

I-84

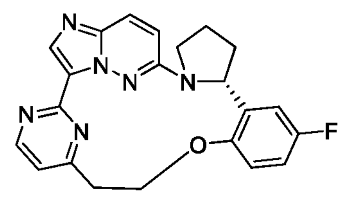

I-85

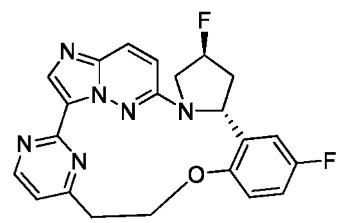

I-86

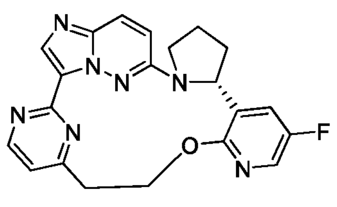

I-87

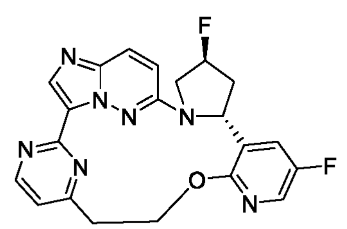

I-88

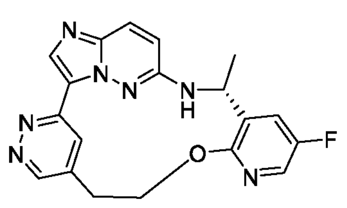

I-89

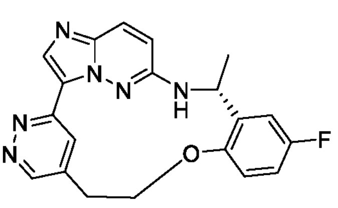

I-90

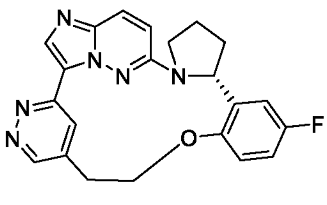

I-91

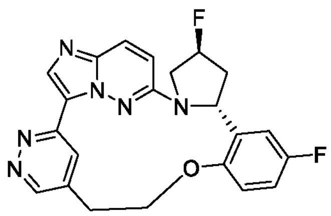

I-92

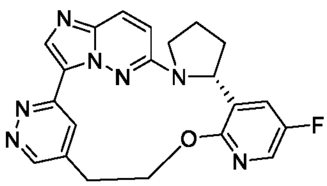

I-93

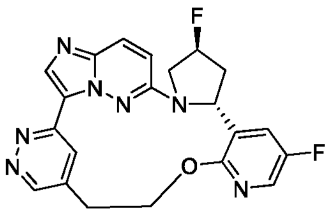

I-94

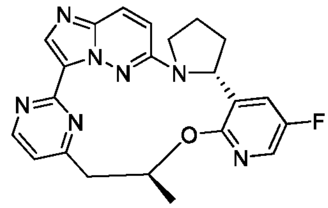

I-95

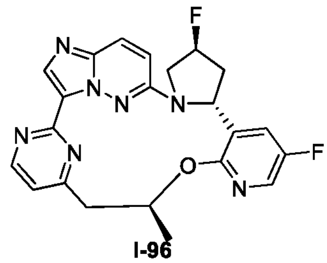

I-96

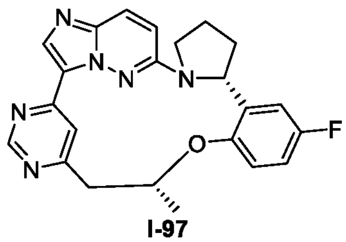

I-97

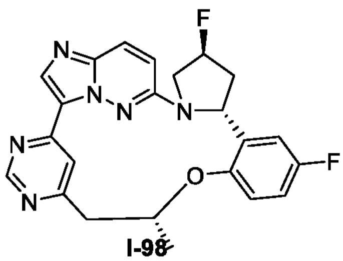

I-98.

According to the second aspect of the present invention, another purpose of the present invention is to provide a pharmaceutical composition comprising a therapeutically effective amount of the compound represented by formula I according to the present invention, or an isotopically labeled compound, an optical isomer, a geometric isomer, a tautomer, an isomers mixture, a pharmaceutically acceptable salt, a prodrug, or a metabolite thereof, and pharmaceutically acceptable carriers.

According to the third aspect of the present invention, another purpose of the present invention is to provide a use of the compound represented by formula I, or an isotopically labeled compound, an optical isomer, a geometric isomer, a tautomer, an isomers mixture, a pharmaceutically acceptable salt, a prodrug, or a metabolite thereof, in the preparation of a drug as a TRK kinase inhibitor for the treatment or prevention of diseases or disorders mediated by TRK or TRK mutations in a subject in need thereof.

Preferably, the disease or disorder mediated by TRK or TRK mutations is selected from one or more of cancers, neurodegenerative diseases, inflammation, and pain.

More preferably, the disease or disorder mediated by TRK or TRK mutations is selected from surgical pain, inflammatory pain, neuropathic pain, Alzheimer's disease, Parkinson's disease, multiple sclerosis, colon cancer, thyroid cancer, lung cancer, prostate cancer, ovarian cancer, breast cancer, salivary gland cancer, pancreatic cancer, melanoma, salivary tumor, cholangiocarcinoma, stromal tumor, brain tumor and malignant blood disease.

According to the fourth aspect of the present invention, another purpose of the present invention is to provide a kit, comprising the compound represented by Formula I of the present invention, or an isotopically labeled compound, an optical isomer, a geometric isomer, a tautomer, an isomers mixture, a pharmaceutically acceptable salt, a prodrug, or a metabolite thereof, or the pharmaceutical composition according to the present invention, as well as a container and instructions for use.

According to the fifth aspect of the present invention, another purpose of the present invention is to provide a method for treating diseases or disorders mediated by TRK or TRK mutations, comprising administering to a subject in need thereof an effective amount of the compound represented by formula I according to the present invention, or an isotopically labeled compound, an optical isomer, a geometric isomer, a tautomer, an isomers mixture, a pharmaceutically acceptable salt, a prodrug, or a metabolite thereof, or the pharmaceutical composition according to the present invention.

## Detailed Description of the Invention

The present invention will be described in detail below. Before proceeding with the description, it should be understood that the terms used in this specification and the appended claims should not be interpreted as limited to the general and dictionary meanings, but should be interpreted based on the principle of allowing the inventor to define the terms appropriately for the best interpretation, according to the meanings and concepts corresponding to the technical aspects of the present invention. Therefore, the description presented here is only a preferred embodiment for illustrative purposes and is not intended to limit the scope of the invention. It should be understood that other equivalents or improved ways may be obtained from it without departing from the spirit and scope of the invention.

As used herein, the terms "comprise", "include", "have", "contain" or any other similar terms are open-ended transitional phrases, which intended to cover non-exclusive inclusions. For example, a composition or article containing a plurality of elements is not limited to only the elements listed herein, but can also include other elements not expressly listed, but which are generally inherent to the composition or article. In addition, unless expressly stated to the contrary, the term "or" refers to an inclusive "or" rather than an exclusive "or". For example, the condition "A or B" is satisfied by any of the followings: A is true (or present) and B is false (or absent), A is false (or absent) and B is true (or present), both A and B are true (or present). In addition, the terms "comprise", "include", "have", "contain" herein intend to be interpreted as having specifically disclosed and encompassing both "consisting of" and "substantially consisting of" and other closed or semi-closed transitional phrase.

All features or conditions herein defined as numerical ranges or percentage ranges are for brevity and convenience only. Accordingly, the description of numerical ranges or percentage ranges should be considered to encompass and specifically disclose all possible sub-ranges and individual numerical values within the range, particularly integer numerical values. For example, a range description of "1 to 8" should be deemed to have specifically disclosed all subranges, such as 1 to 7, 2 to 8, 2 to 6, 3 to 6, 4 to 8, 3 to 8, etc., particularly are sub-ranges bounded by all integer values, and should be deemed to have disclosed the individual values within the specifically disclosed range, such as 1, 2, 3, 4, 5, 6, 7, 8, etc. Unless otherwise indicated, the foregoing method of interpretation applies to all content throughout this disclosure, whether broad or not.

If a quantity or other value or parameter is expressed as a range, a preferred range, or a series of upper and lower limits, it should be understood that all the ranges constituted by any upper limit of the range or preferred value and the lower limit of the range or preferred value have been specifically disclosed herein, whether or not those ranges are disclosed separately. Furthermore, unless otherwise indicated, when a numerical range is referred to herein, the range shall include its endpoints and all integers and fractions within the range.

As used herein, numerical values should be understood to have an accuracy of significant digits of the numerical value, provided that the purpose of the invention can be achieved. For example, the number 40.0 should be understood to cover the range from 39.50 to 40.49.

Where Markush group or alternative terminology is used herein to describe features or examples of the invention, those skilled in the art will understand that all subgroups of elements or any individual element within the Markush group or list of options may also be used to describe the invention. For example, if X is described as "selected from the group consisting of $X_1$, $X_2$ and $X_3$", it also means that the claim that X is $X_1$ and that X is $X_1$ and/or $X_2$ have been fully described. Further for the case that Markush group or alternative terminology is used herein to describe features or examples of the invention, those skilled in is the art will understand that all subgroups of elements or any individual element within the Markush group or list of options may also be used to describe the invention. Accordingly, for example, if X is described as "selected from the group consisting of $X_1$, $X_2$ and $X_3$" and Y is described as "selected from the group consisting of $Y_1$, $Y_2$ and $Y_3$" means that the claim that X is $X_1$ or $X_2$ or $X_3$ and Y is $Y_1$ or $Y_2$ or $Y_3$ has been fully described.

## Definition of relevant terms

"Alkyl" refers to a linear or branched saturated hydrocarbon group having 1 to 8 carbon atoms (" C1-8 alkyl "). In some embodiments, the alkyl has 1 to 7 carbon atoms ("C1-7 alkyl"). In some embodiments, the alkyl has 1 to 6 carbon atoms ("C1-6 alkyl"). In some embodiments, the alkyl has 1 to 5 carbon atoms ("C1-5 alkyl"). In some embodiments, the alkyl has 1 to 4 carbon atoms ("C1-4 alkyl"). In some embodiments, the alkyl has 1 to 3 carbon atoms ("C1-3 alkyl"). In some embodiments, the alkyl has 1 to 2 carbon atoms ("C1-2 alkyl"). In some embodiments, the alkyl has 1 carbon atom ("C1 alkyl"). In some embodiments, the alkyl has 1 to 6 carbon atoms ("C1-6 alkyl"). Examples of C1-6 alkyl include methyl (C1), ethyl (C2), propyl (C3) (e.g. n-propyl, isopropyl), butyl (C4) (e.g. n-butyl, tert-butyl, sec-butyl, isobutyl), pentyl (C5) (e.g. n-pentyl, 3-pentyl, neopentyl, 3-methyl-2-butyl, tert-pentyl), and hexyl (C6) (e.g. n-hexyl). Other examples of alkyl include n-heptyl (C7), n-octyl (C8), etc. Unless otherwise specified, each embodiment of alkyl is independently unsubstituted ("unsubstituted alkyl") or substituted with one or more substituents (e.g. halogen, such as F) ("substituted alkyl"). In some embodiments, the alkyl is an unsubstituted C1-8 alkyl (such as an unsubstituted C1 alkyl, such as -CH3). In some embodiments, the alkyl is a substituted C1-8 alkyl (such as a substituted C1 alkyl, such as -CF3).

"Alkoxy" represents a monovalent -O-alkyl, wherein the alkyl moiety has a specified number of carbon atoms. The alkoxy in this disclosure typically contains 1-6 carbon atoms ("C1-C6 alkoxy") or 1-4 carbon atoms ("C1-C4 alkoxy"). For example, C1-C4 alkoxy include methoxy, ethoxy, isopropoxy, tert-butyloxy, etc. Unless otherwise specified, each embodiment of alkoxy is independently optionally substituted, i.e., unsubstituted ("unsubstituted alkoxy ") or substituted with one or more substituents ("substituted alkoxy "). In some embodiments, the alkoxy is an unsubstituted C1-C6 alkoxy. In some embodiments, the alkoxy is a substituted C1-C6 alkoxy.

"Cycloalkyl" refers to a non-aromatic cyclic hydrocarbyl having 3 to 8 ring carbon atoms (C3-8 cycloalkyl) and zero heteroatoms in the non-aromatic ring system. In some embodiments, the cycloalkyl has 3 to 8 ring carbon atoms ("C3-8 cycloalkyl"). In some embodiments, the cycloalkyl has 3 to 6 ring carbon atoms ("C3-6 cycloalkyl"). In some embodiments, the cycloalkyl has 5 to 8 ring carbon atoms ("C5-8 cycloalkyl"). Exemplary C3-6 cycloalkyl include but are not limited to cyclopropyl (C3), cyclopropenyl (C3), cyclobutyl (C4), cyclobutenyl (C4), cyclopentyl (C5), cyclopentenyl (C5), cyclohexyl (C6), cyclohexenyl (C6), cyclohexadienyl (C6), etc. Exemplary C3-8 cycloalkyl include but are not limited to the C3-6 cycloalkyl mentioned above, as well as cycloheptyl (C7), cycloheptenyl (C7), cycloheptadienyl (C7), Cycloheptatrienyl (C7), cyclooctyl (C8), cyclooctenyl (C8), etc. Unless otherwise specified, each embodiment of a cycloalkyl is independently optionally substituted, i.e., unsubstituted ("unsubstituted cycloalkyl") or substituted with one or more substituents ("substituted cycloalkyl"). In some embodiments, the cycloalkyl is an unsubstituted C3-8 cycloalkyl; in some embodiments, the cycloalkyl is a substituted C3-8 cycloalkyl.

"Heterocycloalkyl" refers to a group of 5 to 14 membered non aromatic ring system with ring carbon atoms and 1 to 4 ring heteroatoms, where each heteroatom is independently selected from nitrogen, oxygen, and sulfur ("5-14 membered heterocyclyl"). In heterocyclyl containing one or more nitrogen atoms, as long as the valence allows, the connecting point can be a carbon atom or a nitrogen atom. The heterocyclyl can be a monocyclic ("monocyclic heterocyclyl") or a fused, bridged, or spiro ring system, such as a bicyclic system ("bicyclic heterocyclyl"), and can be saturated or partially unsaturated. The term "heterocyclyl" also includes a ring system in which a heterocycle as defined above is fused with one or more cycloalkyls (with the connecting point on the cycloalkyl or heterocycle), or a ring system in which a heterocycle as defined above is fused with one or more aryl or heteroaryl (with the connecting point on the heterocycle), and in this case, the number of ring members continues to refer to the number of ring members in the heterocycle system. Unless otherwise specified, each embodiment of the heterocyclyl is independently and optionally substituted, i.e., unsubstituted ("unsubstituted heterocycloalkyl") or substituted with one or more substituents ("substituted heterocycloalkyl"). In some embodiments, the heterocycloalkyl is an unsubstituted 5-14 membered heterocycloalkyl. In some embodiments, the heterocycloalkyl is a substituted 5-14 membered heterocycloalkyl.

"Aryl" or "aromatic ring" or "aromatic cyclic group" refers to a group have single or multi (e.g., bicyclic or tricyclic) 4n+2 aromatic ring system (e.g., having 6, 10, or 14 $\pi$ electrons shared in a cyclic array) with 6-14 ring carbon atoms and zero heteroatoms provided in the aromatic ring system ("C6-14 aryl"). In some embodiments, the aryl group has 6 ring carbon atoms ("C6 aryl"; for example, phenyl). In some embodiments, the aryl group has 10 ring carbon atoms ("C10 aryl"; for example, naphthyl groups such as 1-naphthyl and 2-naphthyl). In some embodiments, the aryl group has 14 ring carbon atoms ("C14 aryl"; for example, anthryl). "Aryl" also includes ring systems in which the aryl ring, as defined above, is fused with one or more cycloalkyl or heterocyclyl (with the connecting point on the aromatic ring), and in this case, the number of carbon atoms continues to refer to the number of carbon atoms in the aromatic ring system. Unless otherwise specified, each example of aryl is independently and optionally substituted, i.e., unsubstituted ("unsubstituted aryl") or substituted with one or more substituents ("substituted aryl"). In some embodiments, the aryl is an unsubstituted C6-14 aryl. In some embodiments, the aryl is a substituted C6-14 aryl.

"Heteroaryl" is a 5-14 membered aromatic ring system with ring carbon atoms and 1-4 ring heteroatoms provided in the aromatic ring system, wherein each heteroatom is independently selected from nitrogen, oxygen, and sulfur (" 5-14 membered heteroaryl"). In some embodiments, heteroaryl is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, heteroaryl is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen and sulfur. In some embodiments, the 5-6 membered heteroaryl has 1-2 ring heteroatoms selected from nitrogen, oxygen and sulfur. In some embodiments, the 5-6 membered heteroaryl has 1 ring heteroatom selected from nitrogen, oxygen and sulfur. Unless otherwise specified, each example of heteroaryl is independently and optionally substituted, i.e., unsubstituted ("unsubstituted heteroaryl") or substituted with one or more substituents ("substituted heteroaryl"). In some embodiments, the heteroaryl is an unsubstituted 5-14 membered heteroaryl. In some embodiments, the heteroaryl is a substituted 5-14 membered heteroaryl.

Structures such as "alkylene", "-alkoxy(ene)-", "-heterocycloalkyl(ene)-", "-aryl(ene)-", "-aromatic ring-", "-aromatic ring group-", and "-heteroaryl(ene)-" refer to the portion of a molecule that serves as an intermediate linking group that simultaneously connects two or more other functional groups. For example, the alkylene refers to a bonding group

formed by an alkyl defined above simultaneously connecting two or more other groups, with the structural formula being expressed as $-C_nH_{2n}-$, which connects to at least two different other groups through at least two separate bonds. For example, in some embodiments, methylene represents $-CH_2-$, in some embodiments, -ethoxy- represents $-CH_2CH_2-O-$, and in some embodiments, -phenylene- represents,

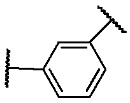, 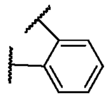 or 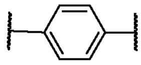.

"Halogen" or "halogenated" refers to fluorine (fluorine, -F), chlorine (chlorine, -Cl), bromine (bromine, -Br) or iodine (iodine, -I).

"Substituted" or "optionally substituted" refers to the substitution of atoms in a functional group, such as hydrogen atoms. In some embodiments, alkyl, cycloalkyl, heterocyclyl, aryl, and heteroaryl are substituted (such as "substituted" alkyl, "substituted" cycloalkyl, "substituted" heterocyclyl, "substituted" aryl, or "substituted" heteroaryl). Typically, the term "substituted', regardless of the definition of the term "optionally", refers to at least one hydrogen present on a functional group (such as a carbon or nitrogen atom) being substituted by an acceptable substituent, for example, a stable compound can be formed after being substituted by a substituent, for example, a compound that does not spontaneously undergo transformation (such as through rearrangement, cyclization, elimination, or other reactions). Unless otherwise specified, a "substituted" group has substituents at one or more substitutable positions on the group, and when more than one position is substituted in any given structure, the substituents are the same or different at each position. The expected term "substituted" includes all permissible substituents of organic compounds and any substituents described herein that result in the formation of stable compounds. This disclosure expects any and all of these combinations to obtain stable compounds. For the purpose of this disclosure, heteroatoms such as nitrogen may have hydrogen substituents and/or any suitable substituents as described herein that satisfy the valence of the heteroatoms and result in the formation of stable moieties. In some embodiments, the substituent is a substituent on carbon atom. In some embodiments, the substituent is a substituent on nitrogen atom. In some embodiments, the substituent is a substituent on oxygen atom. In some embodiments, the substituent is a substituent on sulfur atom.

"Unsaturated" or "partially unsaturated" refers to a functional group containing at least one double or triple bond. The "partially unsaturated" ring system is also intended to encompass rings with multiple unsaturated sites, but is not intended to include aromatic groups (such as aryl or heteroaryl). Similarly, "saturated" refers to functional groups that do not contain double or triple bonds, meaning they all contain single bonds.

The term "pharmaceutically acceptable" as used herein refers to compounds, materials, compositions, and/or dosage forms that are suitable for use in contact with human and animal tissues within reliable medical judgment, without excessive toxicity, irritation, allergic reactions, or other issues or complications, and are commensurate with a reasonable benefit/risk ratio.

The term "pharmaceutically acceptable salts" refers to the salts of the compounds of the present invention, prepared from compounds with specific substituents discovered in the present invention and relatively non-toxic acids or bases. When the compound of the present invention contains relatively acidic functional group, base addition salts can be obtained by contacting a sufficient amount of base with the neutral form of such compound in a pure solution or suitable inert solvent. Pharmaceutically acceptable base addition salts include sodium, potassium, calcium, ammonium, organic ammonia, magnesium salts, or similar salts. When the compound of the present invention contains relatively basic functional group, acid addition salts (i.e., pharmaceutically acceptable salt) can be obtained by contacting a sufficient amount of acid with the neutral form of such compound in a pure solution or suitable inert solvent. Examples include inorganic acid salts and organic acid salts, the inorganic acids include acids such as hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodate, phosphorous acid, etc; the organic acids include acids such as benzoic acid, 2-hydroxyethanesulfonic acid, aminosulfonic acid, benzenesulfonic acid, phenylacetic acid, mandelic acid, malonic acid, propionic acid, oxalic acid, p-aminobenzenesulfonic acid, p-toluenesulfonic acid, polygalacturonic acid, pantothenic acid, fumaric acid, glutamic acid, succinic acid, methanesulfonic acid, tartaric acid, ascorbic acid, phthalic acid, maleic acid, citric acid, malic acid, glucoheptonic acid, gluconic acid, hydroxyethyl sulfonic acid, lactic acid, lactobionic acid, dodecyl sulfonic acid, dihydroxynaphthalene acid, salicylic acid, octanedioic acid, folinic acid, edetic acid, glycolic acid, acetic acid, ethanesulfonic acid, isobutyric acid, stearic acid, and similar acids; it also includes salts of amino acids such as arginine, as well as salts of organic acids such as glucuronic acid. Some specific compounds of the present invention contain basic and acidic functional groups, which can be converted into any base or acid addition salts. The difference between the parent form of a compound and its various salt forms lies in certain physical properties, such as different solubility in polar solvents.

As used herein, the modifier term "about" refers to possible numerical changes that may occur, such as through

routine testing and processing; unintentional errors in such testing and handling; differences in the manufacturing, source, or purity of the ingredients used in the present invention; etc. As used herein, "about" a specific value also includes that specific value, for example, about 10% includes 10%. Whether or not modified by the term "about', the claims include equivalent forms of the listed quantities. In one embodiment, the term "about" refers to within 20% of the reported value.

As used herein, the term "treatment" refers to the elimination, alleviation, or improvement of a disease or disorder and/or its associated symptoms. Although not ruled out, treating a disease or disorder does not require completely eliminating the related diseases, disorders, or symptoms. As used herein, the term "treatment" may include "preventive treatment", which refers to reducing the possibility of disease or disorder recurrence or previously controlled disease or disorder recurrence in subjects who do not have or are at risk of developing or being susceptible to disease or disorder, or experiencing a recurrence of disease or disorder. The term "treatment" and its synonyms consider administering a therapeutically effective amount of the compound described herein to subjects in need of such treatment.

For drugs or pharmacologically active agents, the term "effective amount" or "therapeutically effective amout" refers to a sufficient amount of non-toxic drugs or agents that can achieve the expected effect. For the oral dosage form of the present invention, the "effective amount" of one active substance in the composition refers to the amount required to achieve the desired effect when used in combination with another active substance in the composition. The determination of effective dosage varies from person to person, depending on the age and general situation of the receptor, as well as the specific active substance. The appropriate effective dosage in each case can be determined by those skilled in the art based on routine experiments.

The macrocyclic triazole compound represented by formula I, or an isotopically labeled compound, an optical isomer, a geometric isomer, a tautomer, an isomers mixture, a pharmaceutically acceptable salt, a prodrug, or a metabolite thereof, and the pharmaceutical composition containing the compound can be in various forms, such as tablets, capsules, powders, syrups, solutions, suspensions and aerosols, and can be present in suitable solid or liquid carriers or diluents, as well as suitable disinfection equipments for injection or drip.

Various dosage forms of the pharmaceutical compositions of the present invention may be prepared according to conventional methods of preparation in the field of pharmacy. For example, the unit dose of the formulation contains 0.05-2000mg of the macrocyclic triazole compound of formula I or a pharmaceutically acceptable salt thereof, and preferably, the unit dose of the formulation contains 0.1mg to 1000mg of the compound of formula I.

The compound of formula I and pharmaceutical composition of the present invention can be used clinically in mammals, including human and animals, and can be administered by routes of administration such as the mouth, nose, skin, lung or gastrointestinal tract, etc. Most preferably, they are administered orally. The most preferred daily dose is 0.01-200 mg/kg body weight in a single dose, or 0.01-100 mg/kg body weight in divided doses. Regardless of the method of administration, the optimal dose for an individual should be based on the specific treatment. It is common to start with a small dose and gradually increase until the most appropriate dose is found.

In the present invention, the term "effective amount" may refer to the effective amount of dosage and time period required to achieve the desired effect. This effective dose may vary depending on certain factors, such as the type of disease or the symptoms of the disease during treatment, the structure of the specific target organ being administered, the individual size of the patient, or the severity of the disease or symptoms. In this field, it is common for knowledgeable individuals to determine the effective amount of a specific compound based on experience without excessive experimentation.

A typical formula is prepared by mixing the compound represented by formula I of the present invention with a carrier, diluent, or excipient. Suitable carriers, diluents, or excipients are well-known to those skilled in the art, including substances such as carbohydrates, waxes, water-soluble and/or swellable polymers, hydrophilic or hydrophobic substances, gelatin, oils, solvents, water, etc.

The specific carrier, diluent, or excipient used will depend on the usage and purpose of the compound of the present invention. Generally, solvents are selected based on the principle that the solvent is considered by those skilled in the art to be safe and effective for delivery to mammals Generally speaking, safe solvents are non-toxic aqueous solvents such as water, as well as other non-toxic solvents that are soluble or miscible with water. Suitable aqueous solvents include one or more of water, ethanol, propylene glycol, polyethylene glycol (such as PEG400, PEG300), etc. The formula may also include one or more buffering agents, stabilizers, surfactants, wetting agents, lubricants, emulsifiers, suspensions, preservatives, antioxidants, light blocking agents, flow aids, processing aids, coloring agents, sweeteners, flavorings, seasonings, or other known additives to manufacture or use the drug in an acceptable form.

When the compound of formula I described in the present invention is used in combination with at least one other drug, the two or more drugs can be used separately or in combination, preferably used in the form of a pharmaceutical composition. The compound of formula (I) or pharmaceutical composition of the present invention can be administered separately or together to a subject in any known form of oral, intravenous, rectal, vaginal, transdermal, or other local or systemic administration.

The pharmaceutical composition may also include one or more buffering agents, stabilizers, surfactants, wetting agents, lubricants, emulsifiers, suspensions, preservatives, antioxidants, light blocking agents, flow aids, processing aids,

coloring agents, sweeteners, flavorings, seasonings, or other known additives to allow the pharmaceutical composition to be manufactured or used in an acceptable form.

The drug of the invention is preferably suitable for oral administration. Solid dosage forms for oral administration may include capsules, tablets, powders, and granules. In solid dosage forms, the compounds or pharmaceutical compositions of the present invention are mixed with at least one inert excipient, diluent, or carrier. Suitable excipients, diluents, or carriers include substances such as sodium citrate or dicalcium phosphate, or starch, lactose, sucrose, mannitol, silicic acid, etc; adhesives such as carboxymethyl cellulose, alginate, gelatin, polyvinylpyrrolidone, sucrose, arabic gum, etc; wetting agents such as glycerol, etc; disintegrating agents such as agar, calcium carbonate, potato or cassava starch, alginic acid, specific chelating silicates, sodium carbonate, etc; solution blockers such as paraffin wax, etc; absorption enhancers such as quaternary ammonium compounds; adsorbents such as kaolin and bentonite; lubricants such as talc, calcium stearate, magnesium stearate, solid polyethylene glycol, sodium lauroyl sulfate, etc. In the cases of capsules and tablets, these dosage form may also include buffering agents. Similar types of solid compositions can also be used as fillers in soft and hard filled gelatin capsules, using lactose and high molecular weight polyethylene glycol as excipients.

Liquid dosage forms suitable for oral administration include pharmaceutically acceptable emulsions, solutions, suspensions, syrups, and elixirs. In addition to the compound or pharmaceutical composition of the present invention, the liquid dosage form may contain inert diluents commonly used in the art, such as water and other solvents; Solubilizers and emulsifiers such as ethanol, isopropyl alcohol, ethyl carbonate, ethyl acetate, benzyl alcohol, benzyl benzoate, propylene glycol, 1,3-butanediol, dimethylformamide; Oils (such as cottonseed oil, peanut oil, corn germ oil, olive oil, castor oil, sesame oil, etc.); Glycerol; Tetrahydrofurfurfuryl alcohol; Fatty acid esters formed by polyethylene glycol and dehydrated sorbitol; Or a mixture of several of these substances, etc.

In addition to these inert diluents, the composition may also include excipients such as one or more of wetting agents, emulsifiers, suspensions, sweeteners, seasonings, and flavorings.

As for suspensions, in addition to the compound represented by formula I of the present invention, or a pharmaceutically acceptable salt thereof, or a pharmaceutical composition comprising the same, it may further contain a carrier such as a suspension agent, such as ethoxylated isostearol, polyoxyethylene sorbitol, dehydrated sorbitol ester, microcrystalline cellulose, aluminum hydroxide(boehmite), bentonite, agar and astragalus gum, or a mixture of several of these substances.

The compound represented by formula I of the present invention, or a pharmaceutically acceptable salt thereof, or a pharmaceutical composition comprising the same, can be administered in other local administration forms, including cream, powder, spray, and inhaler. The drug can be mixed under sterile conditions with pharmaceutically acceptable excipients, diluents, or carriers, as well as any required preservatives, buffering agents, or propellants. Eye formulas, eye ointments, powders and solutions are also intended to be included within the scope of the present invention.

In addition, this disclosure also covers kits (such as pharmaceutical packaging). The provided kit may contain the pharmaceutical composition or compound described herein and a container (such as a vial, ampoule, bottle, syringe, and/or packaging or other suitable container). In some embodiments, the provided kit may optionally further include a second container containing a pharmaceutical excipient for diluting or suspending the pharmaceutical composition or compound described herein. In some embodiments, the pharmaceutical composition or compound combination described herein set in the first container and the second container forms a unit dose form.

In some embodiments, the kit described herein further includes instructions for use of the compound or pharmaceutical composition contained within the kit. The kit described herein may also include information required by regulatory agencies such as the United States Food and Drug Administration (FDA). In some embodiments, the information included in the kit is prescription information. In some embodiments, the kit and instructions for use provides the TRK mutation mediated diseases to be treated and/or prevented in subjects in need thereof. The kit described herein may contain one or more additional drug formulations as individual compositions.

The present invention will be further described in detail with specific examples, but the present invention is not limited to the following examples. The examples are intended to better illustrate certain specific embodiments of the present invention and cannot be interpreted as limiting the scope of the present invention in any way. The conditions not specified in the examples are conventional conditions. Unless otherwise specified, the reagents and instruments used in the following examples are commercially available products.

The compound of formula I in this application can be synthesized using various methods familiar to those skilled in the field of organic synthesis. The following specific examples provide some exemplary synthesis methods for compounds of formula I, which are well-known in the field of synthetic chemistry. Obviously, referring to the exemplary scheme in this application, those skilled in the art can easily design synthetic routes for other compounds of formula I by adjusting the reactants, reaction conditions, and protective groups appropriately.

The present invention will be further explained in conjunction with examples; however, these examples do not limit the scope of the present invention. Unless otherwise stated, all reactants used in each example are obtained commercially; the instruments and equipments used in synthesis experiments and product analysis and detection are

conventional instruments and equipments commonly used in organic synthesis.

In the following embodiment, the structure of the exemplary compound of the present invention is determined by nuclear magnetic resonance (NMR) and/or liquid-mass chromatography (LC-MS). NMR chemical shifts ($\delta$) are given in parts per million (ppm) units. The solvents were deuterated dimethyl sulfoxide (DMSO-d6), deuterated methanol ($CD_3OD$) and deuterated chloroform ($CDCl_3$), and the internal standard was tetramethylsilane (TMS).

Agilent 1200 Infinity Series mass spectrometer was used for the determination of LC-MS. HPLC was performed using Agilent 1200DAD High Performance Liquid Chromatography. Thin layer chromatography silica gel plate uses Yantai Huanghai HSGF254 or Qingdao GF254 silica gel plate. Column chromatography generally uses 200~300 mesh silica gel from Yantai Huanghai as the carrier. In addition, in the absence of special instructions, all reactions of the invention are carried out under continuous magnetic agitation under dry nitrogen or argon atmosphere, the solvent is the dry solvent, and the reaction temperature unit is Celsius.

ABBVIATIONS: NIS: N-Iodosuccinimide; TMS: Trimethylsily; $PdCl_2(PPh_3)_2$ : Bis(triphenylphosphine)palladium(II) chloride; $Pd(PPh_3)_4$ : Tetrakis(triphenylphosphine) palladium; CuI: Cuprous iodide; KF: Potassium fluoride; $CuSO_4$: Copper(II) sulfate; VCNa: Sodium ascorbate; $Pd(dba)_2$: Bis(dibenzylideneacetone)palladium; Xantphos: 4,5-Bis(diphenyl phosphino)-9,9-dimethylxanthene; BINAP: 1.1'-Binaphthyl-2.2'-diphemyl phosphine; RuPhos Pd G4: Methanesulfonato(2-dicyclohexylphosphino-2',6'-di-i-propoxy-1,1'-biphenyl)(2'-methyl amino-1,1'-biphenyl-2-yl)palladium(II); DIAD: Diisopropyl azodicarboxylate; TBSCl: tert-Butyldimethylsilyl chloride; $Cs_2CO_3$: Cesium carbonate; $Na_2CO_3$: Sodium carbonate; $NaBH_4$: Sodium borohydride; $BBr_3$: Boron tribromide; LAH: Lithium Aluminum Hydride; NaOH: Sodium hydroxide; BOC: t-Butyloxy carbonyl; EA: Ethyl acetate; PE: Petroleum ether; MeOH: Methonal; EtOH: Ethanol; DIEA: N,N-Diisopropylethylamine; DMSO: Dimethyl sulfoxide; TEA: Triethylamine; DMAP: 4-Dimethylaminopyridine; Dioxane: 1,4-Dioxane; DMF: N,N-Dimethylformamide; NMP: N-Methyl-2-pyrrolidone; THF: Tetrahydrofuran; DCM: Dichloromethane; TFA: Trifluoroacetic acid; TLC: thin-layer chromatography.

**Example 1: (*E*)-5$^4$-fluoro-6-methyl-4-oxa-7-aza-1(3,6)-imidazo[1,2-*b*]pyridazina-2(2,4) -pyridina-5(1,2)-benzenacycloheptaphane (I-1)**

**Step A: 2-bromo-4-(((tert-butyldimethylsilyl)oxy)methyl)pyridine (2)**

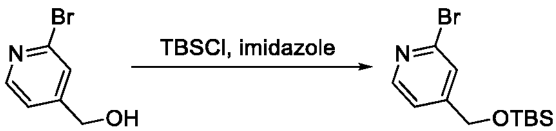


1 2

To a solution of (2-bromo-4-pyridyl)-methanol (Compound **1,** 50 g, 265.93 mmol) and imidazole (23.53 g, 345.70 mmol) in DCM (750 mL) was addedTBSCl (52.11 g, 345.70 mmol) at 0 °C. The mixture was stirred at roomtemperature for 16 h. The reaction was diluted with water (1.5 L). The mixture was extracted with EA (500 mL ) three times. The combined organic layer was washed with brine, dried over anhydrous $Na_2SO_4$, and concentrated. The residue was purified by the chromatography column to give 2-bromo-4-(((tert-butyldimethylsilyl)oxy)methyl)pyridine (64.95 g, 81%). **LCMS:** m/z (ESI), 302[M+H]$^+$.

**Step B: 4-(((tert-butyldimethylsilyl)oxy)methyl)-2-(tributylstannyl)pyridine (3)**

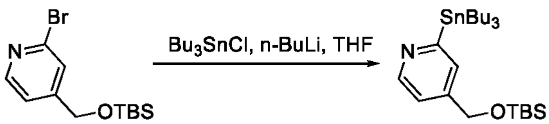


2 3

To a solution of 2-bromo-4-(((tert-butyldimethylsilyl)oxy)methyl)pyridine (Compound **2,** 30 g, 99.25 mmol) in THF (750 mL) was added n-BuLi (2.5 M, 39.70 mL) dropwise at -70 °C and stirred for 0.5 h, and then tributyl(chloro) stannane (38.77 g, 119.09 mmol) was added. The reaction was stirred at -70 °C for 2 h. The reaction was quenched by the addition of aqueous saturated $NH_4Cl$ solution (1.5 L). The mixture was extracted by EtOAc (500 mL) three times. The combined organic layers were washed with brine, dried over anhydrous sodium sulfate, and concentrated. The residue was purified

by the chromatography column to give 4-(((tert-butyldimethylsilyl)oxy)methyl)-2-(tributylstannyl)pyridine (52 g). **LCMS:** m/z (ESI), 514.3[M+H]+.

**Step C: 3-(4-(((tert-butyldimethylsilyl)oxy)methyl)pyridin-2-yl)-6-chloroimidazo[1,2 -*b*]pyridazine (5)**

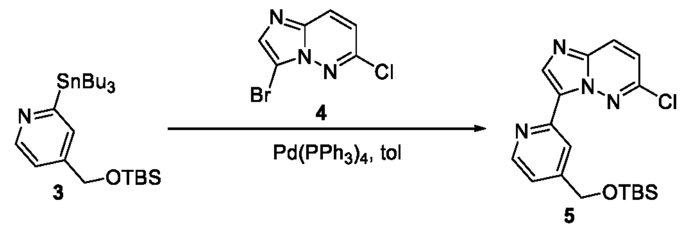


To a solution of 4-(((tert-butyldimethylsilyl)oxy)methyl)-2-(tributylstannyl)pyridine (50 g, 97.57 mmol) in toluene (500 mL) was added 3-bromo-6-chloro-imidazo[1,2-***b***]pyridazine (Compound **4,** 22.68 g, 97.57 mmol) and $Pd(PPh_3)_4$ (11.28 g, 9.76 mmol) in sequence. The mixture was stirred at 100 °C for 16 h. The reaction was diluted with water (1.5 L). The mixture was extracted with EA (500 mL) three times. The combined organic layer was washed with brine, dried over anhydrous $Na_2SO_4$, and concentrated. The residue was purified by the chromatography column to give 3-(4-(((tert-butyldimethylsilyl)oxy)methyl)pyridin-2-yl)-6-chloroimidazo[1,2 -***b***]pyridazine (1.94 g, 5.3%).**$^1$H NMR:** (400 MHz, DMSO-d6) δ 8.48 (d, *J* = 5.0 Hz, 1H), 8.35 (d, *J* = 17.6 Hz, 2H), 8.21 (d, *J* = 9.5 Hz, 1H), 7.34 (d, *J* = 9.5 Hz, 1H), 7.13 (br d, *J* = 4.9 Hz, 1H), 4.71 (s, 2H), 0.80 (s, 9H), 0.00 (s, 6H).

**Step D: 1-(5-fluoro-2-((4-methoxybenzyl)oxy)phenyl)ethan-1-one (7)**

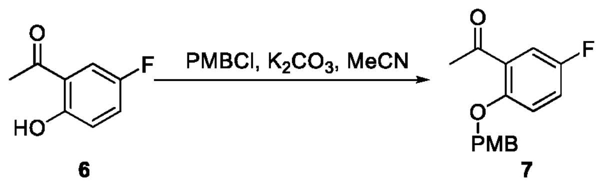


To a solution of 1-(5-fluoro-2-hydroxy-phenyl)ethanone (Compound **6,** 10 g, 64.88 mmol) in $CH_3CN$ (200 mL) was added 4-methoxybenzylchloride (12.19 g, 77.85 mmol), $Cs_2CO_3$ (63.41 g, 194.63 mmol), and stirred at 90 °C for 12 h, then diluted with water (300 mL), extracted with EA (200 mL) three times. The combined organic layer was washed with brine, dried over anhydrous $Na_2SO_4$, and concentrated. The residue was purified by the chromatography column to give the title compound 1-(5-fluoro-2-((4-methoxybenzyl)oxy)phenyl)ethan-1-one (14.1 g, 79%).**$^1$H NMR:** (400 MHz, CHLOROFORM-*d*) δ = 7.43 - 7.34 (m, 1H), 7.29 - 7.21 (m, 2H), 7.10 - 7.00 (m, 1H), 6.94 - 6.78 (m, 3H), 4.97 (s, 2H), 3.74 (s, 3H), 2.48 (s, 3H).

**Step E: 1-(5-fluoro-2-((4-methoxybenzyl)oxy)phenyl)ethan-1-one oxime (8)**

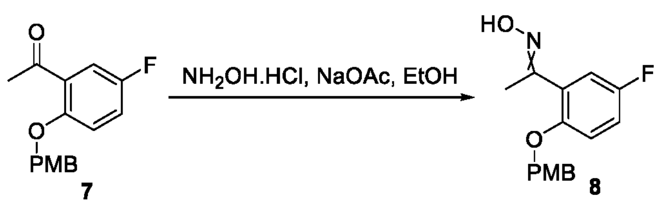


To a solution of 1-(5-fluoro-2-((4-methoxybenzyl)oxy)phenyl)ethan-1-one (Compound **7**, 14.1 g, 51.41 mmol) in EtOH (180 mL) was added NaOAc (12.65 g, 154.22 mmol), $NH_2OH.HCl$ (4.29 g, 61.69 mmol), stirred at 70 °C for 2 h, then diluted with water (600 mL). The mixture was extracted with EA (200 mL) three times. The combined organic layer was washed with brine, dried over anhydrous $Na_2SO_4$, and concentrated. The residue was purified by the chromatography

column to give 1-(5-fluoro-2-((4-methoxybenzyl)oxy)phenyl)ethan-1-one oxime (6.5 g, 44%). **$^1$H NMR:** (400 MHz, DMSO-*d*6) δ = 11.15 (s, 1H), 7.37 (d, *J* = 8.5 Hz, 2H), 7.21 - 7.13 (m, 2H), 7.03 (dd, *J* = 2.4, 8.9 Hz, 1H), 6.98 - 6.91 (m, 2H), 5.04 (s, 2H), 3.81 - 3.72 (m, 3H), 2.08 - 2.02 (m, 3H).

**Step F: 1-(5-fluoro-2-((4-methoxybenzyl)oxy)phenyl)ethan-1-amine (9)**

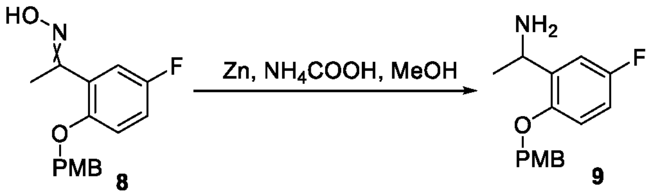


To a solution of 1-(5-fluoro-2-((4-methoxybenzyl)oxy)phenyl)ethan-1-one oxime (Compound **8,** 6.50 g, 22.47 mmol) in MeOH (150 mL) was added ammonium formate (14.7 g, 224.68 mmol) and Zn (14.69 g, 224.68 mmol). The mixture was stirred at 40 °C for 12 h, then filtered and the filtrate was concentrated. The residue was purified by chromatography column to give 1-(5-fluoro-2-((4-methoxybenzyl)oxy)phenyl)ethan-1-amine (2.8 g, 48.8%). **LCMS:** m/z (ESI), 276.0[M+H]$^+$. **$^1$H NMR:** (400 MHz, DMSO-d6) δ = 7.38 (br d, *J* = 8.6 Hz, 2H), 7.28 (dd, *J* = 3.1, 10.0 Hz, 1H), 7.06 - 7.00 (m, 1H), 6.99 - 6.93 (m, 3H), 5.05 - 4.96 (m, 2H), 4.29 - 4.21 (m, 1H), 3.76 (s, 3H), 1.21 - 1.17 (m, 3H).

**Step G: 3-(4-(((tert-butyldimethylsilyl)oxy)methyl)pyridin-2-yl)-N-(1-(5-fluoro-2-((4-methoxybenzyl)oxy)phenyl) ethyl)imidazo[1,2-*b*]pyridazin-6-amine (10)**

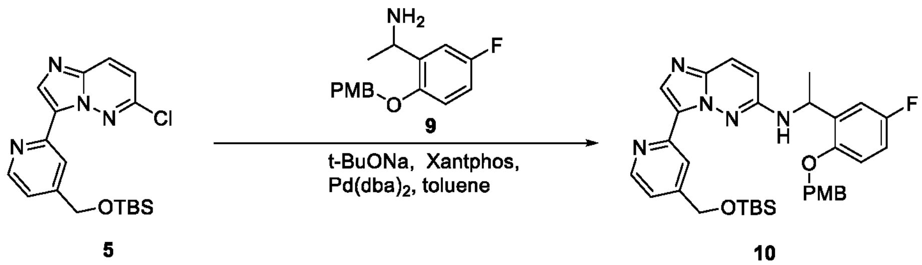


To a solution of 3-(4-(((tert-butyldimethylsilyl)oxy)methyl)pyridin-2-yl)-6-chloroimidazo[1,2 -***b***]pyridazine (Compound **5,** 100 mg, 0.267 mmol) in toluene (5 mL) was added 1-(5-fluoro-2-((4-methoxybenzyl)oxy)phenyl)ethan-1-amine (73.43 mg, 0.27 mmol), $Pd(dba)_2$ (15.34 mg, 0.027 mmol), t-BuONa (51.26 mg, 0.53 mmol) and Xantphos (15.43 mg, 0.027 mmol) in sequence. The mixture was stirred at 100 °C for 16 h. The mixture was concentrated and purified by chromatography column to give 3-(4-(((tert-butyldimethylsilyl)oxy)methyl)pyridine-2-yl)-N-(1-(5-fluoro-2-((4-methoxybenzyl)oxy)phenyl)ethyl)imidazo[1,2-***b***]pyridazin-6-amine (24 mg, 15%). **LCMS:** m/z (ESI), 614.5[M+H]$^+$.**$^1$H NMR:** (400 MHz, DMSO-*d*6) δ = 8.59 - 8.54 (m, 1H), 8.30 (s, 1H), 8.03 (s, 1H), 7.82 (d, *J* = 9.8 Hz, 1H), 7.30 - 7.28 (m, 2H), 7.28 - 7.26 (m, 2H), 7.25 - 7.24 (m, 1H), 7.20 (br d, *J* = 3.3 Hz, 2H), 6.89 - 6.80 (m, 1H), 6.68 (d, *J* = 8.6 Hz, 2H), 5.41 - 5.34 (m, 1H), 5.10 - 5.03 (m, 2H), 3.65 (s, 3H), 3.17 (s, 3H), 0.86 (s, 9H), 0.03 (d, *J* = 7.1 Hz, 6H).

**Step H: 3-(4-(hydroxymethyl)pyridin-2-yl)-N-(1-(5-fluoro-2-hydroxyphenyl)ethyl) imidazo[1,2-b]pyridazine-6-amine (11)**

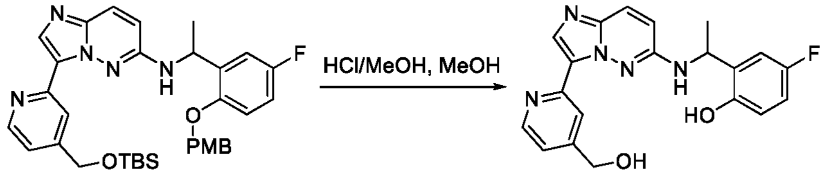


10 11

To a solution of 3-(4-(((tert-butyldimethylsilyl)oxy)methyl)pyridin-2-yl)-N-(1-(5-fluoro-2-((4-methoxybenzyl)oxy) phenyl)ethyl)imidazo[1,2-***b***]pyridazin-6-amine (Compound **10**, 24 mg, 0.039 mmol) in MeOH (3 mL) was added HCl/MeOH (4 M, 3 mL). The mixture was stirred at 25 °C for 2 h, then adjusted to pH = 7 with $NaHCO_3$ and filtered. The filtrate was concentrated and purified by chromatography column to give 3-(4-(hydroxymethyl)pyridin-2-yl)-N-(1-(5-fluoro -2-hydroxyphenyl)ethyl)imidazo[1,2-b]pyridazine-6-amine (12 mg, 81%).**LCMS:** m/z (ESI), 380[M+H]$^+$.

**Step I: (*E*)-5$^4$-fluoro-6-methyl-4-oxa-7-aza-1(3,6)-imidazo[1,2-*b*]pyridazine-2(2,4) -pyridina-5(1,2)-benzenacycloheptaphane (I-1)**

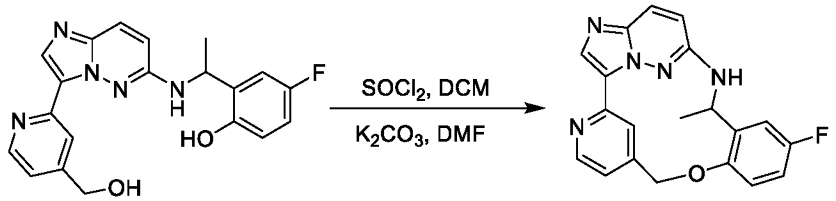


11 I-1

To a solution of 3-(4-(hydroxymethyl)pyridin-2-yl)-N-(1-(5-fluoro-2-hydroxyphenyl)ethyl) imidazo[1,2-***b***]pyridazine-6-amine (Compound **11,** 34.37 mg, 0.091mol) in DCM/DMF mixture solution (6 mL) was added $SOCl_2$ (215.56 mg, 1.81 mol). The mixture was stirred at 25 °C for 0.5 h. Then $K_2CO_3$ (500.84 mg, 3.62 mmol) was added to adjust pH > 7. The mixture was evaporated to remove DCM and stirred at 90 °C for another 1.5 h. The mixture was filtered and the filtrate was concentrated. The residue was purified by the Preparative Chromatography to give (***E***)-5$^4$-fluoro-6-methyl-4-oxa-7-aza-1(3,6)-imidazo[1,2-***b***]pyridazine-2(2,4)-pyridina-5(1,2)-benzenacycloheptaphane (3.8 mg, 11.3%). **LCMS:** m/z (ESI), 362[M+H]$^+$.**$^1$H NMR:** (400 MHz, DMSO-d6) δ = 9.69 (s, 1H), 8.51 (d, *J = 5.0* Hz, 1H), 8.00 (s, 1H), 7.98 - 7.90 (m, 1H), 7.87 (d, *J* = 9.8 Hz, 1H), 7.24 (d, *J* = 5.0 Hz, 1H), 7.10 - 7.03 (m, 2H), 6.89 (d, *J* = 9.8 Hz, 1H), 6.80 (br dd, *J* = 3.2, 7.9 Hz, 1H), 5.75 (d, *J* = 15.4 Hz, 1H), 5.47 (d, *J* = 16.0 Hz, 1H), 5.35 (br d, *J* = 6.0 Hz, 1H), 1.47 (d, *J* = 6.8 Hz, 3H).

**Example 2: (*E*)-5$^4$,5$^6$-difluoro-6-methyl-4-oxa-7-aza-1(3,6)-imidazo[1,2-*b*]pyridazine -2(2,4)-pyridina-5(1,2)-benzenacycloheptaphane (I-2)**

**Step A: 1-(3,5-difluoro-2-((4-methoxybenzyl)oxy)phenyl)ethan-1-one (12)**

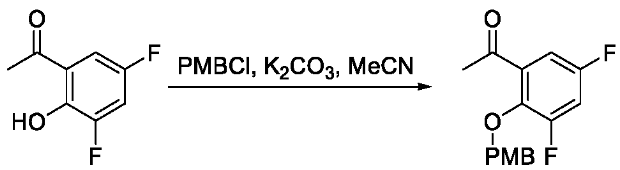


11 12

To solution of 1-(3,5-difluoro-2-hydroxy-phenyl)ethanone (10 g, 58.10 mmol) in DMF (200 mL) was added 4-methoxybenzylchloride (10.01 g, 63.91 mmol) and $K_2CO_3$ (24.09 g, 174.29 mmol). The reaction was stirred for 12 h at 60 °C, then diluted with water (300 mL), extracted with EA (200 mL) three times. The combined organic layer was washed with brine, dried over anhydrous $Na_2SO_4$, and concentrated. The residue was purified by the chromatography column to give 1-(3,5-difluoro-2-((4-methoxybenzyl)oxy)phenyl)ethan-1-one (14.5 g, 85.4%). **$^1$H NMR:** (400 MHz, CHLOROFORM-d) δ

= 7.33 (br d, *J* = 8.3 Hz, 2H), 7.15 (br d, *J* = 1.1 Hz, 1H), 7.05 (br s, 1H), 6.92 (br d, *J* = 8.4 Hz, 2H), 5.08 (s, 2H), 3.84 (s, 3H), 2.54 (s, 3H).

**Step B: 1-(3,5-difluoro-2-((4-methoxybenzyl)oxy)phenyl)ethan-1-one oxime (13)**

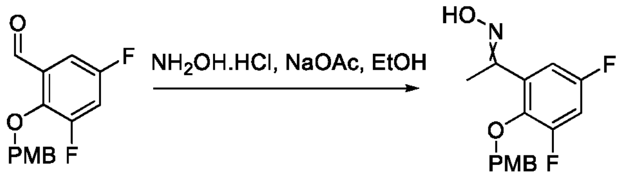


**12** **13**

To a solution of 1-(3,5-difluoro-2-((4-methoxybenzyl)oxy)phenyl)ethan-1-one (Compound **12,** 13.1 g, 44.82 mmol) in EtOH (180 mL) was added NaOAc (11.03 g, 134.46 mmol), $NH_2OH.HCl$ (3.74 g, 53.78 mmol) in sequence. The reaction mixture was stirred at 70 °C for 12 h and diluted with water (600 mL). The mixture was extracted with EA (200 mL) three times. The combined organic layer was washed with brine, dried over anhydrous $Na_2SO_4$, and concentrated. The residue was purified by the chromatography column to give 1-(3,5-difluoro-2-((4-methoxy benzyl)oxy)phenyl)ethan-1-one oxime (6.25 g, 45.4%). **$^1$H NMR:** (400 MHz, DMSO-d6) δ = 11.59 - 11.21 (m, 1H), 7.42 - 7.34 (m, 1H), 7.30 (br d, *J* = 8.0 Hz, 2H), 7.03 - 6.97 (m, 1H), 6.93 (br d, *J* = 8.1 Hz, 2H), 4.91 (s, 2H), 3.76 (s, 3H), 2.07 (s, 3H).

**Step C: 1-(3,5-difluoro-2-((4-methoxybenzyl)oxy)phenyl)ethan-1-amine(14)**

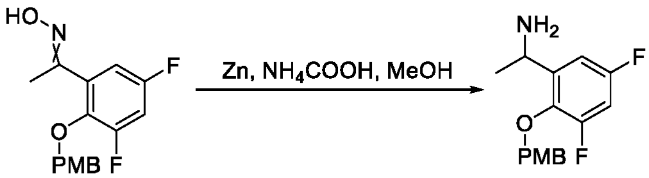


**13** **14**

To a solution of 1-(3,5-difluoro-2-((4-methoxybenzyl)oxy)phenyl)ethan-1-one oxime (Compund **13,** 6.20 g, 20.18 mmol) in MeOH (150 mL) was added ammonium formate (12.72 g, 201.76 mmol), Zn powder (13.19 g, 201.76 mmol) in sequence. The mixture was stirred at 40 °C for 12 h. The mixture was filtered and the filtrate was concentrated. The residue was purified by the chromatography column to give 1-(3,5-difluoro-2-((4-methoxybenzyl)oxy)phenyl)ethan-1-amine (2.6 g, 43.9%). **LCMS:** m/z (ESI), 294.2[M+H]$^+$. **$^1$H NMR:** (400 MHz, DMSO-d6) δ = 7.35 (d, *J* = 8.6 Hz, 2H), 7.21 - 7.10 (m, 2H), 6.95 (d, *J* = 8.6 Hz, 2H), 4.93 (s, 2H), 4.28 - 4.18 (m, 1H), 3.76 (s, 3H), 1.11 (d, *J* = 6.6 Hz, 3H).

**Step D: 3-(4-(((tert-butyldimethylsilyl)oxy)methyl)pyridin-2-yl)-N-(1-(3,5-difluoro-2-((4-methoxybenzyl)oxy)phenyl)ethyl)imidazo[1,2-b]pyridazin-6-amine (15)**

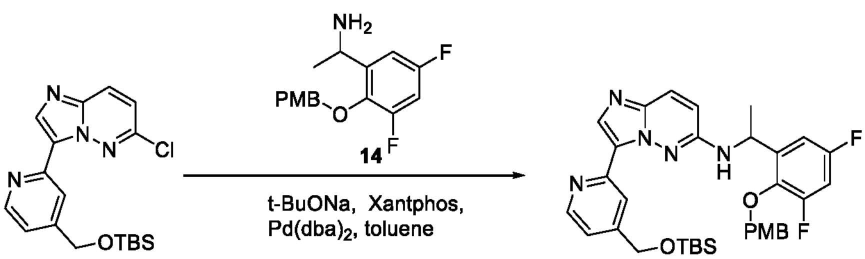


**5** **15**

To a solution of 3-(4-(((tert-butyldimethylsilyl)oxy)methyl)pyridin-2-yl)-6-chloroimidazo[1,2 -***b***]pyridazine (Compound **5,** 100 mg, 0.267 mmol) in toluene (5 mL) was added 1-(3,5-difluoro-2-((4-methoxybenzyl)oxy) phenyl)ethan-1-amine (Compound **14,** 78.23 mg, 0.27 mmol), Pd(dba)$_2$ (15.34 mg, 0.027 mmol), t-BuONa (51.26 mg, 0.53 mmol) and Xantphos (15.34 mg, 0.027 mmol) in sequence. The mixture was stirred at 100 °C for 16 h and concentrated. The residue was purified by the chromatography column to give 3-(4-(((tert-butyldimethylsilyl)oxy)methyl)pyridin-2-yl)-N-(1-(3,5-difluoro-2-((4-methoxybenzyl)oxy)phenyl)ethyl)imidazo[1,2-***b***]pyridazin-6-amine (44 mg, 26%). **LCMS:** m/z (ESI), 632.2 [M+H]$^+$. **$^1$H NMR:** (400 MHz, DMSO-d6) δ = 8.53 (d, *J* = 4.8 Hz, 1H), 8.11 (s, 1H), 7.98 (s, 1H), 7.79 (d, *J* = 9.6 Hz, 1H), 7.44 (br d, J = 7.0 Hz, 1H), 7.31 (br d, *J* = 8.4 Hz, 1H), 7.22 - 7.16 (m, 4H), 7.09 (br d, *J* = 9.3 Hz, 1H), 6.95 - 6.88 (m, 1H), 6.77 (d, *J* = 9.9 Hz, 1H), 5.29 (br d, *J* = 7.1 Hz, 1H), 4.97 - 4.84 (m, 2H), 4.70 - 4.53 (m, 2H), 3.59 (s, 3H), 1.40 (br d, *J* = 6.8 Hz, 3H), 0.84 (s, 9H), 0.00 (s, 6H).

**Step E: 3-(4-(hydroxymethyl)pyridin-2-yl)-N-(1-(3,5-difluoro-2-hydroxyphenyl)ethyl) imidazo[1,2-b]pyridazine-6-amine (16)**

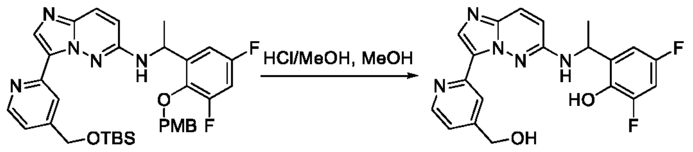


15 16

To a solution of 3-(4-(((tert-butyldimethylsilyl)oxy)methyl)pyridin-2-yl)-N-(1-(3,5-difluoro -2-((4-methoxybenzyl) oxy)phenyl)ethyl)imidazo[1,2-b]pyridazin-6-amine (Comound **15,** 44 mg, 0.069 mmol) in MeOH (3 mL) was added HCl/MeOH (4 M, 3 mL). The mixture was stirred at 25 °C for 1 h. The mixture was adjusted to pH = 7 with NaHCO$_3$, filtered and the filtrate was concentrated. The residue was purified by the chromatography column to give 3-(4-(hydroxymethyl) pyridin-2-yl)-N-(1-(3,5-difluoro-2-hydroxyphenyl)ethyl)imidazo[1,2-b]pyridazine-6-amine (36 mg). **LCMS:** m/z (ESI), 398[M+H]$^+$.

**Step F: (*E*)-5$^4$,5$^6$-difluoro-6-methyl-4-oxa-7-aza-1(3,6)-imidazo[1,2-*b*]pyridazine -2(2,4)-pyridina-5(1,2)-benzenacycloheptaphane (I-2)**

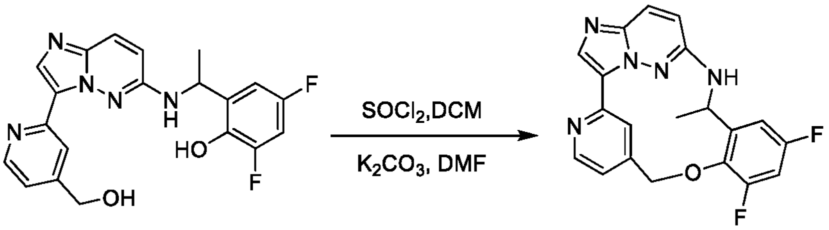


16 I-2

To a solution of 3-(4-(hydroxymethyl)pyridin-2-yl)-N-(1-(3,5-difluoro-2-hydroxyphenyl)ethyl) imidazo[1,2-***b***]pyridazine-6-amine (Compound **16,** 36 mg, 0.091 mol) in DCM/DMF mixture solution (6 mL) was added SOCl$_2$ (215.57 mg, 1.81 mol). Then K$_2$CO$_3$ (500.84 mg, 3.62 mmol) was added to adjust pH > 7 and the mixture was evaporated to remove DCM and stirred at 90 °C for another 1.5 h. The mixture was filtered and the filtrate was concentrated. The residue was purified by Preparative Chromatography to give (***E***)-5$^4$,5$^6$-difluoro-6-methyl-4-oxa-7-aza-1(3,6)-imidazo[1,2-***b***]pyridazine-2(2,4)-pyridina -5(1,2)-benzenacycloheptaphane (4.2 mg, 11.2%). **LCMS:** m/z (ESI), 380[M+H]$^+$. **$^1$H NMR:** (400 MHz, DMSO-d6) δ = 9.34 (s, 1H), 8.63 (d, *J* = 5.0 Hz, 2H), 8.54 (s, 1H), 8.11 (d, *J* = 9.9 Hz, 1H), 7.45 (d, *J* = 4.9 Hz, 1H), 7.35 (d, *J* = 9.9 Hz, 1H), 6.98 - 6.85 (m, 2H), 5.78 - 5.72 (m, 1H), 5.62 - 5.54 (m, 1H), 5.29 (br d, *J* = 6.7 Hz, 1H), 1.46 (d, *J* = 6.8 Hz, 3H).

**Example 3: (E)-6-methyl-4-oxa-7-aza-1(3,6)-imidazo[1,2-*b*]pyridazine-2(2,4)-pyridine-5(1,2)-benzenacycloheptaphane (I-3)**

**Step A: 1-(2-((4-methoxybenzyl)oxy)phenyl)ethan-1-one (17)**

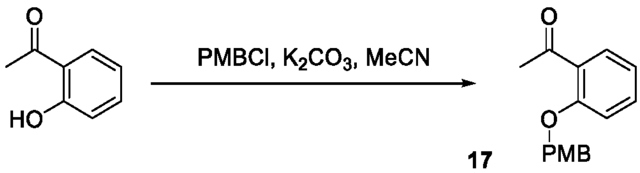


**17**

To solution of 1-(2-hydroxy-phenyl)ethanone (10 g, 73.45 mmol) in $CH_3CN$ (200 mL) was added 4-methoxybenzylchloride (13.80 g, 88.14 mmol), $K_2CO_3$ (30.45 g, 220.35 mmol). The reaction was stirred for 12 h at 60 °C and diluted with water (300 mL). The mixture was extracted with EA (200 mL) three times. The combined organic layer was washed with brine, dried over anhydrous $Na_2SO_4$, and concentrated. The residue was purified by the chromatography column to give 1-(2-((4-methoxybenzyl)oxy)phenyl)ethan-1-one (18 g, 95.6%). **$^1$H NMR:** (400 MHz, DMSO-d6) δ = 7.58 (dd, *J* = 1.8, 7.7 Hz, 1H), 7.55 - 7.50 (m, 1H), 7.44 (d, *J* = 8.6 Hz, 2H), 7.27 (d, *J* = 8.3 Hz, 1H), 7.04 - 7.00 (m, 1H), 6.99 - 6.94 (m, 2H), 5.15 (s, 2H), 3.76 (s, 3H), 2.48 (s, 3H).

**Step B: 1-(2-((4-methoxybenzyl)oxy)phenyl)ethan-1-one oxime (18)**

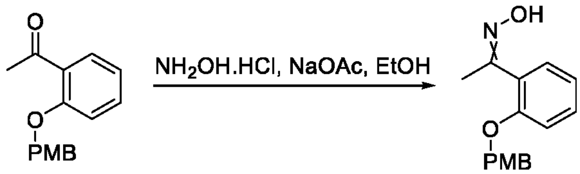


**17** **18**

To a solution of 1-(2-((4-methoxybenzyl)oxy)phenyl)ethan-1-one (Compound **17**, 18 g, 70.23 mmol) in EtOH (180 mL) was added NaOAc (17.28 g, 210.69 mmol), $NH_2OH.HCl$ (5.86 g, 84.28 mmol) in sequence. The reaction mixture was stirred at 70 °C for 2 h and diluted with water (600 mL). The mixture was extracted with EA (200 mL) three times. The combined organic layer was washed with brine, dried over anhydrous $Na_2SO_4$, and concentrated. The residue was purified by the chromatography column to give 1-(2-((4-methoxybenzyl)oxy)phenyl)ethan-1-one oxime (10 g, 50.4%). **$^1$H NMR:** (400 MHz, DMSO-d6) δ = 10.96 (s, 1H), 7.38 - 7.31 (m, 3H), 7.21 (dd, *J* = 1.6, 7.4 Hz, 1H), 7.14 (d, *J* = 8.3 Hz, 1H), 6.97 - 6.93 (m, 3H), 5.05 (s, 2H), 3.76 (s, 3H), 2.03 (s, 3H).

**Step C: 1-(2-((4-methoxybenzyl)oxy)phenyl)ethan-1-amine (19)**

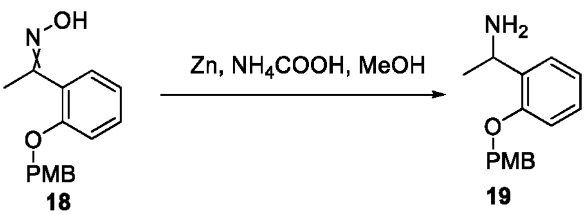


**18** **19**

To a solution of 1-(2-((4-methoxybenzyl)oxy)phenyl)ethan-1-one oxime (Compund **18,** 11.0 g, 40.54 mmol) in MeOH (150 mL) was added ammonium formate (25.57 g, 405.44 mmol) and Zn powder (26.67 g, 407.94 mmol) in sequence. The mixture was stirred at 40 °C for 12 h. The mixture was filtered and the filtrate was concentrated. The residue was purified by the chromatography column give 1-(2-((4-methoxybenzyl)oxy)phenyl)ethan-1-amine (7.8 g, 74%). **LCMS:** m/z (ESI), 258.2[M+H]$^+$. **$^1$H NMR:** (400 MHz, DMSO-d6) δ = 7.48 - 7.34 (m, 3H), 7.18 - 7.12 (m, 1H), 7.03 - 6.88 (m, 4H), 5.02 (d, *J* = 1.0 Hz, 2H), 4.27 (q, *J* = 6.6 Hz, 1H), 3.76 (s, 3H), 1.80 - 1.59 (m, 2H), 1.20 (d, *J* = 6.6 Hz, 3H).

**Step D: 3-(4-(((tert-butyldimethylsilyl)oxy)methyl)pyridin-2-yl)-N-(1-(2-((4-methoxybenzyl)oxy)phenyl)ethyl)imidazo[1,2-b]pyridazin-6-amine (20)**

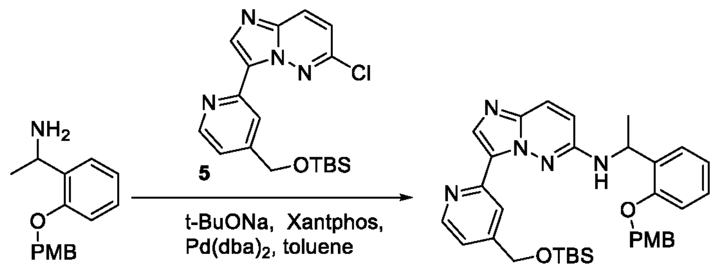


19

20

To a solution of 3-(4-(((tert-butyldimethylsilyl)oxy)methyl)pyridin-2-yl)-6-chloroimidazo[1,2 -***b***]pyridazine (Compound **19,** 1.9 g, 2.53 mmol) in toluene (50 mL) was added 1-(2-((4-methoxy benzyl)oxy)phenyl)ethan-1-amine (651.08 mg, 2.53 mmol), Pd(dba)$_2$ (215.89 mg, 0.38 mmol), t-BuONa (487.00 mg, 5.07 mmol) and Xantphos (219.91 mg, 0.38 mmol) in sequence. The mixture was stirred at 100 °C for 16 h, and concentrated. The residue was purified by the chromatography column to give 3-(4-(((tert-butyldimethylsilyl)oxy)methyl)pyridin-2-yl)-N-(1-(2-((4-methoxy benzyl)oxy) phenyl)ethyl)imidazo[1,2-b]pyridazin-6-amine (400 mg, 26.5%). **LCMS:** m/z (ESI), 596.0 $[M+H]^+$. **$^1$H NMR:** (400 MHz, DMSO-d6) δ = 8.57 (d, *J* = 4.9 Hz, 1 H), 8.33 (s, 1 H), 8.03 (s, 1 H), 7.79 (d, *J* = 9.6 Hz, 1 H), 7.42 (dd, *J* = 1.3, 7.4 Hz, 1 H), 7.32 (d, *J* = 7.3 Hz, 1 H), 7.27 (d, *J* = 8.6 Hz, 2 H), 7.24 - 7.20 (m, 2 H), 7.09 (d, *J* = 7.8 Hz, 1 H), 6.93 (t, *J* = 7.4 Hz, 1 H), 6.84 (d, *J* = 9.6 Hz, 1 H), 6.67 (d, *J* = 8.6 Hz, 2 H), 5.41 (t, *J* = 7.0 Hz, 1H), 5.13 - 5.04 (m, 2 H), 4.72 - 4.55 (m, 2 H), 3.65 (s, 3 H), 1.55 (d, *J* = 6.8 Hz, 3 H), 0.87 (s, 9 H), 0.04 (d, *J* = 7.8 Hz, 6 H).

**Step E: 3-(4-(hydroxymethyl)pyridin-2-yl)-N-(1-(2-hydroxyphenyl)ethyl)imidazo [1,2-b]pyridazine-6-amine (21)**

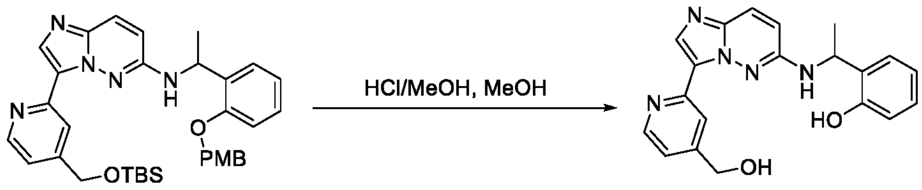


20

I-3

To a solution of 3-(4-(((tert-butyldimethylsilyl)oxy)methyl)pyridin-2-yl)-N-(1-(2-((4-methoxybenzyl)oxy)phenyl) ethyl)imidazo[1,2-***b***]pyridazin-6-amine (Comound **20,** 300 mg, 0.53 mmol) in MeOH (3 mL) was added HCl/MeOH (4 M, 3 mL). The mixture was stirred at 25 °C for 1 h. The mixture was adjusted to pH = 7 with $NaHCO_3$, filtered and the filtrate was concentrated. The residue was purified by the chromatography column to give 3-(4-(hydroxymethyl)pyridin-2-yl)-N -(1-(2-hydroxyphenyl)ethyl)imidazo[1,2-***b***]pyridazine-6-amine (70 mg, 34%). **LCMS:** m/z (ESI), 362$[M+H]^+$.

**Step F: (*E*)-6-methyl-4-oxa-7-aza-1(3,6)-imidazo[1,2-*b*]pyridazine-2(2,4)-pyridine -5(1,2)-benzenacycloheptaphane (I-3)**

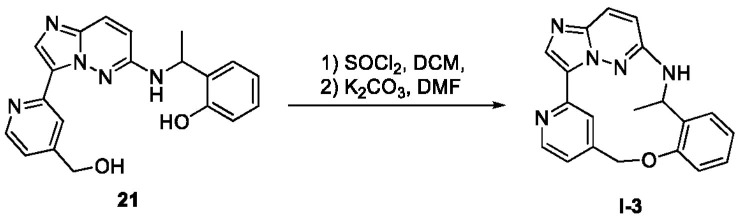


21

I-3

To a solution of 3-(4-(hydroxymethyl)pyridin-2-yl)-N-(1-(2-hydroxyphenyl)ethyl)imidazo [1,2-***b***]pyridazine-6-amine (Compound **21**, 25 mg, 0.066 mmol) in DCM /DMF mixture solution (6 mL) was added $SOCl_2$ (215.57 mg, 1.81 mmol). The mixture was stirred at 25 °C for 0.5 h, then $K_2CO_3$ (500.84 mg, 3.62 mmol) was added to adjust pH > 7 and the mixture was evaporated to remove DCM and stirred at 90 °C for another 1.5 h. The mixture was filtered. The filtrate was concentrated. The residue was purified by Preparative Chromatography to give (E)-6-methyl-4-oxa-7-aza-1(3,6)-imidazo [1,2-***b***]pyridazine-2(2,4)-pyridine-5(1,2)-benzenacycloheptaphane (19.9 mg, 39.2%). **LCMS:** m/z (ESI), 344 $[M+H]^+$. **$^1$H NMR:** (400 MHz, MeOD-d4) $\delta$ 9.90 (s, 1 H), 8.59 (d, *J* = 5.3 Hz, 1 H), 8.34 (s, 1 H), 7.99 (d, *J* = 9.9 Hz, 1 H), 7.51 (d, *J* = 5.3 Hz, 1 H), 7.39 - 7.26 (m, 2 H), 6.99 (d, *J* = 3.4 Hz, 2H), 6.79 (td, *J* = 4.1, 8.0 Hz, 1H), 5.82 (d, *J* = 15.9 Hz, 1 H), 5.60 - 5.48 (m, 2 H), 1.58 (d, *J* = 6.9 Hz, 3 H).

**Example 4: (*E*)-6-methyl-4-oxa-7-aza-1(3,6)-imidazo[1,2-*b*]pyridazina-2(2,4),5(2,3)-dipyridinacycloheptaphane (I-4)**

**Step A: 1-(2-((4-methoxybenzyl)oxy)pyridin-3-yl)ethan-1-one (22)**

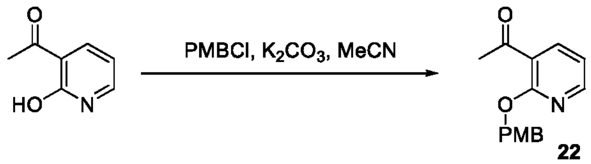


22

To solution of 1-(2-hydroxypyridin-3-yl)ethan-1-one (10 g, 72.99 mmol) in $CH_3CN$ (200 mL) was added 4-methoxybenzylchloride (13.80 g, 88.14 mmol), $K_2CO_3$ (30.45 g, 220.35 mmol). The reaction was stirred for 12 h at 60 °C. The mixture was diluted with water (300 mL) and extracted with EA (200 mL) three times. The combined organic layer was washed with brine, dried over anhydrous $Na_2SO_4$, and concentrated. The residue was purified by the chromatography column to give 1-(2-((4-methoxybenzyl)oxy)pyridin-3-yl)ethan-1-one (6 g, 32%). **LCMS:** m/z (ESI), 258.2$[M+H]^+$.

**Step B: 1-(2-((4-methoxybenzyl)oxy)pyridin-3-yl)ethan-1-one oxime (23)**

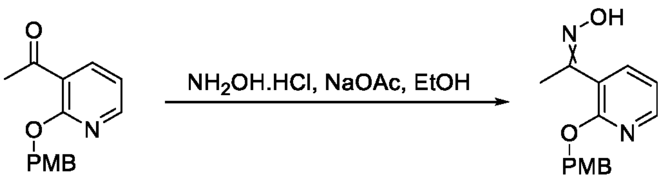


22

23

To a solution of 1-(2-((4-methoxybenzyl)oxy)pyridin-3-yl)ethan-1-one (Compound **22,** 6 g, 23.26 mmol) in EtOH (180 mL) was added NaOAc (5.76 g, 70.23 mmol), $NH_2OH.HCl$ (1.96 g, 28.08 mmol) in sequence. The reaction mixture was stirred at 70 °C for 2 h and diluted with water (200 mL). The mixture was extracted with EA (200 mL) three times. The combined organic layer was washed with brine, dried over anhydrous $Na_2SO_4$, and concentrated. The residue was purified by the chromatography column to give 1-(2-((4-methoxybenzyl)oxy)pyridin-3-yl)ethan-1-one oxime (3 g, 47.4%). **LCMS:** m/z (ESI), 273.2$[M+H]^+$.

**Step C: 1-(2-((4-methoxybenzyl)oxy)pyridin-3-yl)ethan-1-amine (24)**

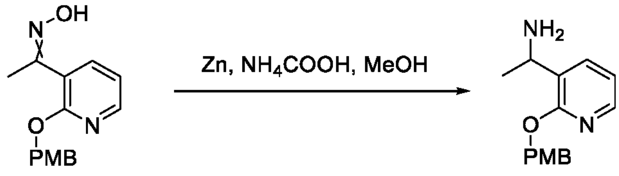


23 24

To a solution of 1-(2-((4-methoxybenzyl)oxy)pyridin-3-yl)ethan-1-one oxime (Compund **23**, 3.0 g, 11.03 mmol) and in MeOH (50 mL) was added ammonium formate (25.57 g, 405.44 mmol) and Zn powder (26.67 g, 407.94 mmol) in sequence. The mixture was stirred at 40 °C for 12 h. The mixture was filtered and the filtrate was concentrated. The residue was purified by the chromatography column to give **1-(2-((4-methoxybenzyl)oxy)pyridin-3-yl)ethan-1-amine** (2.1 g, 74%). **LCMS:** m/z (ESI), 259.2[M+H]$^+$.

**Step D: 3-(4-(((tert-butyldimethylsilyl)oxy)methyl)pyridin-2-yl)-N-(1-(2-((4-methoxy benzyl)oxy)pyridin-3-yl) ethyl)imidazo[1,2-*b*]pyridazin-6-amine (25)**

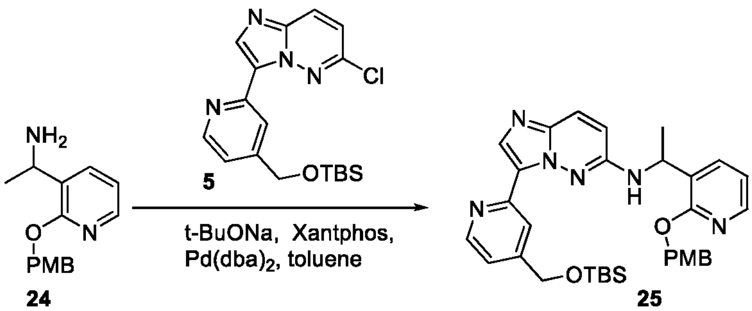


24 25

To a solution of 3-(4-(((tert-butyldimethylsilyl)oxy)methyl)pyridin-2-yl)-6-chloroimidazo[1,2 -b]pyridazine (Compound **5,** 1.9 g, 2.53 mmol) in toluene (50 mL) was added 1-(2-((4-methoxy benzyl)oxy)pyridin-3-yl)ethan-1-amine (Compound **24,** 651.08 mg, 2.53 mmol), $Pd(dba)_2$ (215.89 mg, 0.38 mmol), t-BuONa (487.00 mg, 5.07 mmol) and Xantphos (219.91 mg, 0.38 mmol) in sequence. The mixture was stirred at 100 °C for 16 h and concentrated. The residue was purified by the chromatography column to give **3-(4-(((tert-butyldimethylsilyl)oxy)methyl)pyridin-2-yl)-N-(1-(2-((4-methoxybenzyl)oxy)pyridin-3-yl)ethyl)imidazo[1,2-*b*]pyridazin-6-amine** (300 mg, 20%). **LCMS:** m/z (ESI), 597.0 [M+H]$^+$.

**Step E: 3-(4-(hydroxymethyl)pyridin-2-yl)-N-(1-(2-hydroxypyridin-3-yl)ethyl)imidazo [1,2-*b*]pyridazine-6-amine (26)**

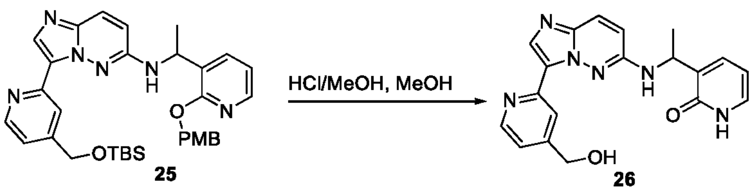


25 26

To a solution of 3-(4-(((tert-butyldimethylsilyl)oxy)methyl)pyridin-2-yl)-N-(1-(2-((4-methoxy benzyl)oxy)pyridin-3-yl)ethyl)imidazo[1,2-***b***]pyridazin-6-amine (Comound **25**, 300 mg, 0.52 mol) in MeOH (3 mL) was added HCl/MeOH (4 M, 3 mL). The mixture was stirred at 25 °C for 2 h. The mixture was adjusted to pH = 7 with $NaHCO_3$, filtered and the filtrate was concentrated. The residue was purified by chromatography column to give 3-(4-(hydroxymethyl)pyridin-2-yl)-N-(1 -(2-hydroxypyridin-3-yl)ethyl)imidazo[1,2-***b***]pyridazine-6-amine (50 mg, 24%). **LCMS:** m/z (ESI), 363[M+H]$^+$.

**Step F: (*E*)-6-methyl-4-oxa-7-aza-1(3,6)-imidazo[1,2-*b*]pyridazina-2(2,4),5(2,3)-dipyridinacycloheptaphane (I-4)**

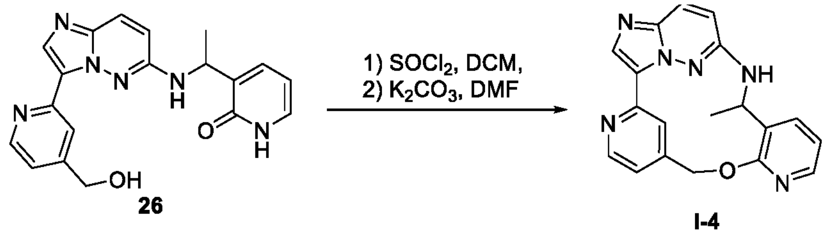


26

I-4

To a solution of 3-(4-(hydroxymethyl)pyridin-2-yl)-N-(1-(2-hydroxypyridin-3-yl)ethyl) imidazo[1,2-*b*]pyridazine-6-amine (Compound **26,** 50 mg, 0.132 mol) in DCM/DMF mixture solution (6 mL) was added $SOCl_2$ (215.57 mg, 1.81 mol). The mixture was stirred at 25 °C for 0.5 h. Then $K_2CO_3$ (500.84 mg, 3.62 mmol) was added to adjust pH > 7 and the mixture was evaporated to remove DCM and stirred at 90 °C for another 1.5 h. The mixture was filtered and the filtrate was concentrated. The residue was purified by Preparative Chromatography to give (***E***)-6-methyl-4-oxa-7-aza-1(3,6)-imidazo[1,2-***b***]pyridazina-2(2,4),5(2,3)-dipyridinacycloheptaphane (14.9 mg, 30.2%). **LCMS:** m/z (ESI), 345[M+H]$^+$.

**Example 5: (*E*)-5$^4$-fluoro-4-oxa-7-aza-1(3,6)-imidazo[1,2-*b*]pyridazina-2(2,4)-pyridina -5(1,2)-benzenacycloheptaphane (I-5)**

**Step A: 2-(aminomethyl)-4-fluoro-phenol (27)**

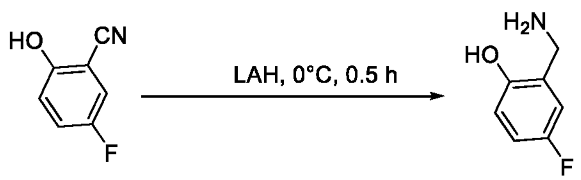


27

To solution of 5-fluoro-2-hydroxy-benzonitrile (2 g, 14.59 mmol) in THF (20 mL) was added LAH (1.11 g, 29.17 mmol). The reaction mixture was stirred for 1 h at 0 °C, then quenched by $Na_2SO_4.10H_2O$. Then the mixture was diluted with EtOAc (50 mL), filtered and the filtration was concentrated. The residue was purified by chromatography column to give 2-(aminomethyl)-4-fluoro-phenol (2.3 g). **$^1$H NMR:** (400 MHz, CHLOROFORM-*d*) δ = 6.74 - 6.48 (m, 2H), 6.29 - 6.11 (m, 1H), 3.57 - 3.46 (m, 2H).

**Step B: 4-fluoro-2-(((3-(4-(hydroxymethyl)-2-pyridyl]imidazo[1,2-b]pyridazin -6-yl)amino)methyl)phenol (28)**

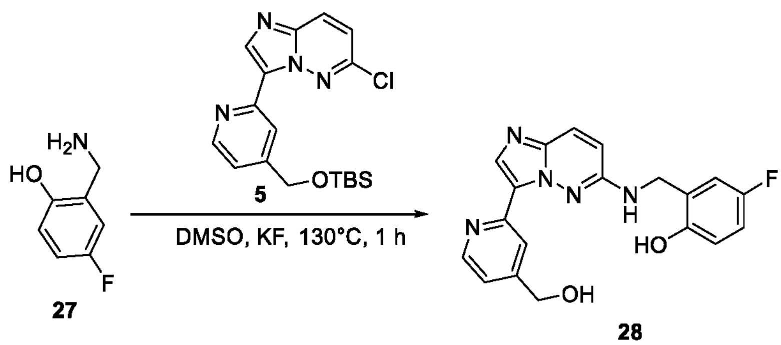


27

28

To a solution of 3-(4-(((tert-butyldimethylsilyl)oxy)methyl)pyridin-2-yl)-6-chloroimidazo[1,2 -***b***]pyridazine (Compound **27,** 100 mg, 0.267 mmol) in DMSO (3 mL) was added 2-(aminomethyl) -4-fluoro-phenol (188.22 mg, 1.33 mmol), t-BuONa (51.26 mg, 0.53 mmol) in sequence. The reaction was stirred for 1 h at 130 °C. The reaction mixture was filtered and the filtration was purified by Preparative Chromatography to give 4-fluoro-2-(((3-(4-(hydroxymethyl)-2-pyriyl]imidazo [1,2-***b***] pyridazin-6-yl)amino)methyl)phenol (48 mg, 49%). **LCMS:** m/z (ESI), 366.2 $[M+H]^+$. **$^1$H NMR** (400 MHz, DMSO-*d*6) δ = 8.59 - 8.49 (m, 2H), 8.19 (s, 1H), 8.07 (s, 1H), 7.84 (d, *J* = 9.8 Hz, 1H), 7.53 - 7.47 (m, 1H), 7.25 (d, *J* = 4.5 Hz, 1H), 7.12 - 7.06 (m, 1H), 6.92 - 6.85 (m, 3H), 4.54 - 4.44 (m, 4H).

**Step C: (*E*)-5$^4$-fluoro-4-oxa-7-aza-1(3,6)-imidazo[1,2-*b*]pyridazina-2(2,4)-pyridina -5(1,2)-benzenacycloheptaphane (I-5)**

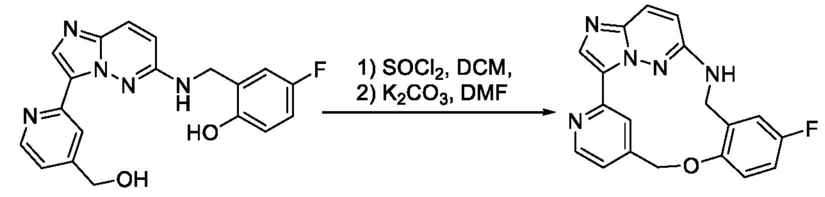


**28** **I-5**

To a solution of 4-fluoro-2-(((3-(4-(hydroxymethyl)-2-pyridyl)imidazo[1,2-b]pyridazin -6-yl)amino)methyl)phenol (Compound **28**, 47 mg, 0.122 mol) in DCM/DMF mixture solution (6 mL) was added $SOCl_2$ (215.57 mg, 1.81 mol). The reaction was stirred for 0.5 h at 25 °C. Then $K_2CO_3$ (500.84 mg, 3.62 mmol) was added to adjust pH > 7 and the mixture was evaporated to remove DCM and stirred at 90 °C for another 1.5 h. The reaction mixture was filtered and the filtration was concentrated and purified by Preparative Chromatography to give (***E***)-5$^4$-fluoro-4-oxa-7-aza-1(3,6)-imidazo[1,2-***b***]pyridazina-2(2,4) -pyridina-5(1,2)-benzenacycloheptaphane (5.2 mg, 11.6%). **LCMS:** m/z (ESI), 348$[M+H]^+$ . **$^1$H NMR** (400 MHz, DMSO-*d6*) δ = 9.69 - 9.62 (m, 1H), 8.72 - 8.65 (m, 1H), 8.61 (d, *J* = 5.3 Hz, 1H), 8.49 (s, 1H), 8.07 (d, *J* = 9.8 Hz, 1H), 7.43 (d, *J* = 4.3 Hz, 1H), 7.32 (d, *J* = 9.9 Hz, 1H), 7.14 (dd, *J* = 4.5, 9.4 Hz, 1H), 7.06 (dd, *J* = 3.1, 9.4 Hz, 1H), 6.87 - 6.80 (m, 1H), 5.79 (s, 1H), 5.51 (d, *J* = 15.8 Hz, 1H), 5.07 - 4.98 (m, 1H), 4.26 - 4.20 (m, 1H).

**Example 6: (*E*)-4-oxa-7-aza-1(3,6)-imidazo[1,2-*b*]pyridazina-2(2,4)-pyridina-5(1,2)-benzenacycloheptaphane (I-6)**

**Step A: 2-(((3-(4-(hydroxymethyl)pyridin-2-yl)imidazo[1,2-*b*]pyridazin-6-yl)amino) methyl)phenol (29)**

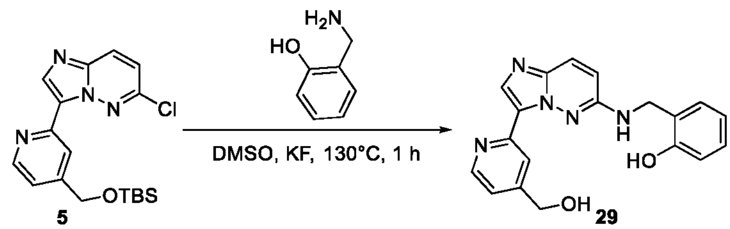


**5** **29**

To a solution of 3-(4-(((tert-butyldimethylsilyl)oxy)methyl)pyridin-2-yl)-6-chloroimidazo[1,2 -***b***]pyridazine (1 g, 2.13 mmol) in DMSO (16 mL) was added 2-(aminomethyl)- phenol (525.53 mg, 4.27 mmol), t-BuONa (51.26 mg, 0.53 mmol) in sequence. The reaction was stirred for 1 h at 130 °C. The reaction mixture was filtered. The filtration was purified by Preparative Chromatography to give 2-(((3-(4-(hydroxymethyl)pyridin-2-yl)imidazo[1,2-***b***]pyridazin-6-yl)amino) methyl)phenol (180 mg, 24%). **LCMS:** m/z (ESI), 348.2 $[M+H]^+$. **$^1$H NMR** (400 MHz, DMSO-d6) δ 9.73 - 9.53 (m, 1 H), 8.60 (s, 1 H), 8.54 (d, *J* = 5.0 Hz, 1 H), 8.06 (s, 1 H), 7.80 (d, *J* = 9.8 Hz, 1 H), 7.39 (t, *J* = 5.1 Hz, 1 H), 7.32 - 7.20 (m, 2 H), 7.09 (t, *J* = 7.4 Hz, 1 H), 6.93 - 6.85 (m, 2 H), 6.78 - 6.70 (m, 1 H), 5.39 (d, *J* = 6.9 Hz, 1H), 4.50 (s, 4 H).

**Step B**: **(*E*)-4-oxa-7-aza-1(3,6)-imidazo[1,2-*b*]pyridazina-2(2,4)-pyridina-5(1,2)-benzenacycloheptaphane (I-6)**

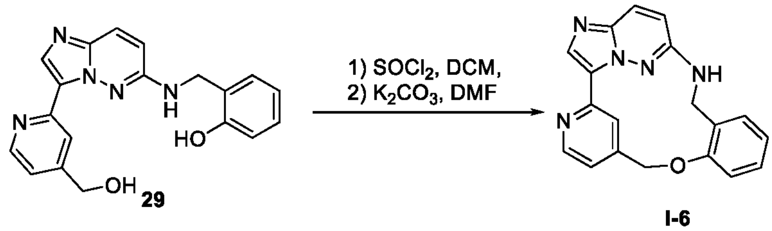


29

I-6

To a solution of 2-(((3-(4-(hydroxymethyl)pyridin-2-yl)imidazo[1,2-b]pyridazin-6-yl)amino) methyl)phenol (Compound **29,** 50 mg, 0.137 mol) in DCM/DMF mixture solution (6 mL) was added $SOCl_2$ (215.57 mg, 1.81 mol). The reaction was stirred for 0.5 h at 25 °C. Then $K_2CO_3$ (500.84 mg, 3.62 mmol) was added to adjust pH > 7 and the mixture was evaporated to remove DCM and stirred at 90 °C for another 1.5 h. The reaction mixture was filtered and the filtration was concentrated and purified by Preparative Chromatography to give (***E***)-4-oxa-7-aza-1(3,6)-imidazo[1,2-***b***]pyridazina-2(2,4)-pyridina-5(1,2)- benzenecycloheptaphane (6.8 mg, 13.6%). **LCMS:** m/z (ESI), 330[M+H]$^+$. **$^1$H NMR:** (400 MHz, DMSO-*d6*) δ 9.74 (s, 1 H), 8.92 - 8.74 (m, 1 H), 8.60 (d, *J* = 5.0 Hz, 1 H), 8.55 - 8.47 (m, 1 H), 8.06 (d, *J* = 9.8 Hz, 1 H), 7.45 (br d, *J* = 3.6 Hz, 1 H), 7.38 - 7.27 (m, 2 H), 7.10 (d, *J* = 8.4 Hz, 1 H), 7.04 - 6.94 (m, 1 H), 6.84 - 6.75 (m, 1 H), 5.85 (d, *J* = 15.9 Hz, 1 H), 5.49 (d, *J* = 15.9 Hz, 1 H), 5.05 (dd, *J* = 4.7, 15.3 Hz, 1 H), 4.21 (dd, *J* = 5.8, 15.4 Hz, 1 H).

**Example 7: (*E*)-5$^4$-fluoro-6-methyl-4-oxa-7-aza-1(3,6)-imidazo[1,2-*b*]pyridazine -2(3,5)-pyridina-5(1,2)-benzenacycloheptaphane (I-7)**

**Step A: (5-(6-chloroimidazo[1,2-*b*]pyridazin-3-yl)pyridin-3-yl)methanol (30)**

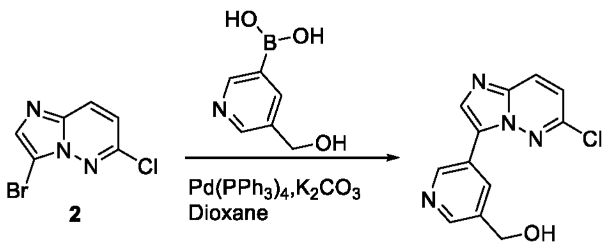


30

To 1,4-dioxane (50 mL) and water (5 mL) was added (5-(hydroxymethyl)pyridin-3-yl)boronic acid (500 mg, 3.27 mmol), 3-bromo-6-chloroimidazo[1,2-b]pyridazine (Compound **2,** 760 mg, 3.27 mmol), $Pd(PPh_3)_4$ (381 mg, 0.33 mmol), $K_2CO_3$ (11.28 g, 9.76 mmol) . The mixture was stirred at 100 °C for 16 h and diluted with water (1.5 L). The mixture was extracted with EA (500 mL) three times. The combined organic layer was washed with brine, dried over anhydrous $Na_2SO_4$, and concentrated. The residue was purified by the chromatography column to give (5-(6-chloroimidazo [1,2-b] pyridazin-3-yl)pyridin-3-yl)methanol (150 mg, 17%). **LCMS:** m/z (ESI), 261[M+H]$^+$.

**Step B: 3-(5-(((tert-butyldimethylsilyl)oxy)methyl)pyridin-3-yl)-6-chloroimidazo[1,2-*b*] pyridazine (31)**

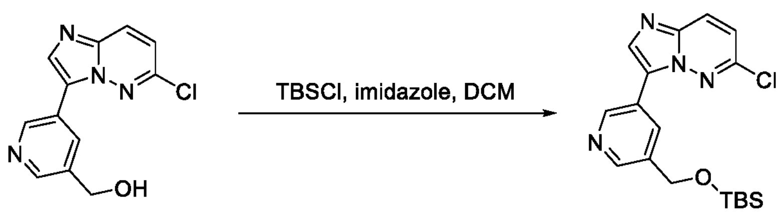


30

31

To a solution of (5-(6-chloroimidazo[1,2-***b***]pyridazin-3-yl)pyridin-3-yl)methanol (Compound **30,** 150 mg, 0.57 mmol) and imidazole (77.53 mg, 1.14 mmol) in DCM (10 mL) was added TBSCl (128.25 mg, 0.85 mmol) at 0°C. The

reaction mixture was stirred at room temperature for 16 h and quenched with water (50 mL). The mixture was extracted with EA (50 mL) three times. The combined organic layer was washed with brine, dried over anhydrous $Na_2SO_4$, and concentrated. The residue was purified by the chromatography column to give 3-(5-(((tert-butyldimethylsilyl)oxy)methyl) pyridin-3-yl)-6-chloroimidazo[1,2-b]pyridazine (70 mg, 32%). **LCMS:** m/z (ESI), 375[M+H]+.

**Step C: 3-(5-(((tert-butyldimethylsilyl)oxy)methyl)pyridin-3-yl)-*N*-(1-(5-fluoro-2-((4-methoxybenzyl)oxy)phenyl) ethyl)imidazo[1,2-*b*]pyridazin-6-amine (32)**

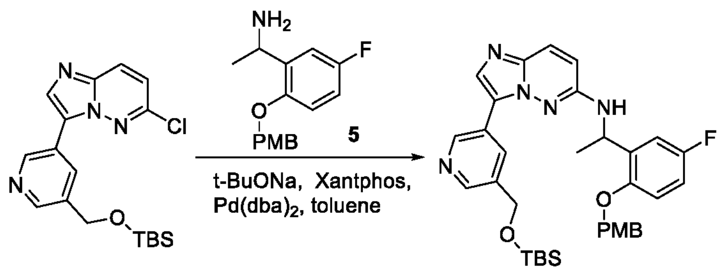


**31** **32**

To a solution of 3-(5-(((tert-butyldimethylsilyl)oxy)methyl)pyridin-3-yl)-6-chloroimidazo [1,2-b]pyridazine (Compound **31,** 70 mg, 0.187 mmol) in toluene (5 mL) was added 1-(5-fluoro -2-((4-methoxybenzyl)oxy)phenyl)ethan-1-amine (73.43 mg, 0.187 mmol), Pd(dba)2 (11.74 mg, 0.019 mmol), t-BuONa (35.93 mg, 0.37 mmol) and Xantphos (10.03 mg, 0.019 mmol) in sequence. The mixture was stirred at 100 °C for 16 h and concentrated. The residue was purified by the chromatography column to give 3-(5-(((tert-butyldimethylsilyl)oxy)methyl)pyridin-3-yl) -*N*-(1-(5-fluoro-2-((4-methoxybenzyl)oxy)phenyl)ethyl)imidazo[1,2-***b***]pyridazin-6-amine (12 mg, 10%). **LCMS:** m/z (ESI), 614.2[M+H]+.

**Step D: 3-(5-(hydroxymethyl)pyridin-3-yl)-*N*-(1-(5-fluoro-2-hydroxypehnyl)ethyl) imidazo[1,2-*b*]pyridazine-6-amine (33)**

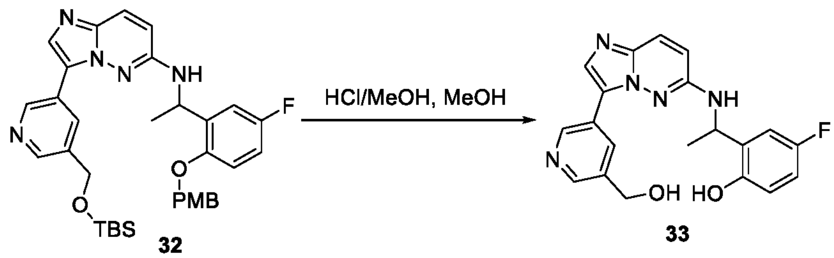


**32** **33**

To a solution of 3-(5-(((tert-butyldimethylsilyl)oxy)methyl)pyridin-3-yl)-N-(1-(5-fluoro -2-((4-methoxybenzyl)oxy) phenyl)ethyl)imidazo[1,2-b]pyridazin-6-amine (Comound **32,** 12 mg, 0.019 mmol) in MeOH (3 mL) was added HCl/MeOH (4 M, 3 mL). The mixture was stirred at 25 °C for 2 h. The mixture was adjusted to pH = 7 with $NaHCO_3$, filtered and the filtrate was concentrated. The residue was purified by chromatography column to give 3-(5-(hydroxymethyl) pyridin-3-yl)-*N*-(1-(5-fluoro-2-hydroxypehnyl)ethyl)imidazo[1,2-***b***]pyridazine-6-amine (5 mg). **LCMS:** m/z (ESI), 380[M+H]+.

**Step E: (*E*)-5$^4$-fluoro-6-methyl-4-oxa-7-aza-1(3,6)-imidazo[1,2-*b*]pyridazine -2(3,5)-pyridina-5(1,2)-benzenacycloheptaphane (I-7)**

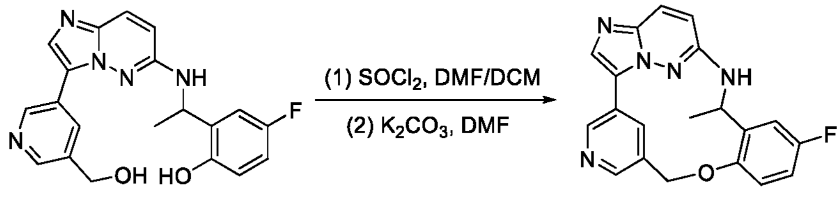


33 I-7

To a solution of 3-(5-(hydroxymethyl)pyridin-3-yl)-***N***-(1-(5-fluoro-2-hydroxypehnyl)ethyl) imidazo[1,2-***b***]pyridazine-6-amine (Compound **33,** 35.2 mg, 0.094 mmol) in DCM/DMF mixture solution (6 mL) was added $SOCl_2$ (215.57 mg, 1.81 mmol). The reaction was stirred for 0.5 h at 25 °C. Then $K_2CO_3$ (500.84 mg, 3.62 mmol) was added to adjust pH > 7 and the mixture was evaporated to remove DCM and stirred at 90 °C for another 1.5 h. The reaction mixture was filtered and the filtration was concentrated and purified by Preparative Chromatography to give (***E***)-$5^4$-fluoro-6-methyl-4-oxa-7-aza-1(3,6)-imidazo[1,2-***b***]pyridazine-2(3,5)-pyridina-5(1,2)-benzenacycloheptaphane (7.6 mg, 22.6%). **LCMS:** m/z (ESI), 362$[M+H]^+$. **$^1$H NMR:** (400 MHz, DMSO-*d*6) δ = 9.69 (s, 1H), 8.51 (d, *J* = 5.0 Hz, 1H), 8.00 (s, 1H), 7.98 - 7.90 (m, 1H), 7.87 (d, *J* = 9.8 Hz, 1H), 7.24 (d, *J* = 5.0 Hz, 1H), 7.10 - 7.03 (m, 2H), 6.89 (d, *J* = 9.8 Hz, 1H), 6.80 (br dd, *J* = 3.2, 7.9 Hz, 1H), 5.75 (d, *J* = 15.4 Hz, 1H), 5.47 (d, *J* = 16.0 Hz, 1H), 5.35 (br d, *J* = 6.0 Hz, 1H), 1.47 (d, *J* = 6.8 Hz, 3H).

**Example 8: (*E*)-6-oxa-3-aza-2(3,6)-imidazo[1,2-*b*]pyridazina-1(2,4)-pyridina-5(1,2)-benzenacyclooctaphane (I-8)**

**Step A: 2-bromo-4-hydroxyethylpyridine (34)**

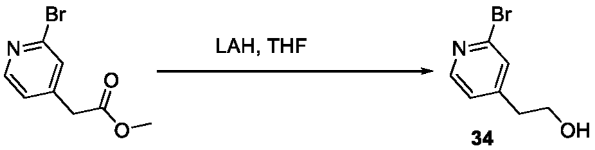


34

To a solution of ethyl 2-(2-bromo-4-pyridyl)acetate (1 g, 4.35 mmol) in THF (15 mL) slowly added LAH (142.03 mg, 6.52 mmol). The mixture was stirred at 25 °C for 1 h, then quenched with aqueous saturated $NH_4Cl$ solution (20 mL). The mixture was extracted by EtOAc (20 mL) two times. The combined organic layers were washed with brine, dried over anhydrous $Na_2SO_4$, and concentrated. The residue was purified by the chromatography column to give 2-bromo-4-hydroxyethylpyridine (870 mg). **LCMS:** m/z (ESI), 301$[M+H]^+$.

**Step B: 2-bromo-4-(2-((tert-butyldimethylsilyl)oxy)ethyl)pyridine (35)**

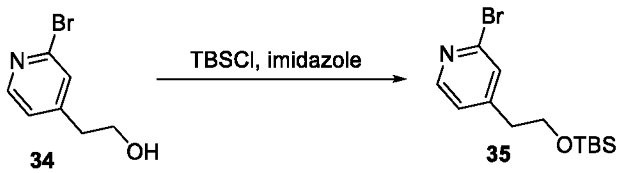


34 35

To a solution of 2-bromo-4-hydroxyethylpyridine (Compound **34,** 2.57 g, 12.72 mmol) in DCM (30 mL) was added TBSCl (3.83 g, 25.44 mmol) and imidazole (1.73 g, 25.44 mmol) at 0 °C in sequence. The mixture was stirred at 25 °C for 1 h and concentrated. The residue was purified by the chromatography column to give 2-bromo-4-(2-((tert-butyldimethylsilyl) oxy)ethyl)pyridine (4 g, 99%). **$^1$H NMR:** (400 MHz, CHLOROFORM -*d*) δ 8.24 - 8.27 (m, 1 H) 7.38 - 7.39 (m, 1 H) 7.10 - 7.13 (m, 1 H) 3.83 (t, *J* = 6.19 Hz, 2 H) 2.75 - 2.81 (m, 2 H) 0.86 (s, 9 H), 0.02 (s, 6 H).

**Step C: 4-(2-((tert-butyldimethylsilyl)oxy)ethyl)-2-(tributylstannyl)pyridine (36)**

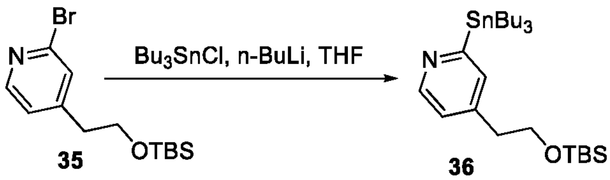


35 36

To a solution of 2-bromo-4-(2-((tert-butyldimethylsilyl)oxy)ethyl)pyridine (Compound **35,** 4 g, 12.65 mmol) in THF (10 mL) was added n-BuLi (2.5 M, 5.06 mL) at -78 °C under $N_2$ atmosphere. Then tributyl(chloro)stannane (6.17 g, 18.97 mmol) was added in and the reaction was stirred at -78 °C for 2 h. The reaction was quenched by aqueous saturated $NH_4Cl$ solution (50 mL). KF solution (50 mL) was added in and the reaction was stirred for another 1 h, then extracted by EtOAc (50 mL) two times. The combined organic layers were washed with brine, dried over anhydrous $Na_2SO_4$, and concentrated. The residue was purified by the chromatography column to give 4-(2-((tert-butyl dimethylsilyl)oxy)ethyl)-2-(tributylstannyl) pyridine (5 g, crude).

**Step D: 3-(4-(2-((tert-butyldimethylsilyl)oxy)ethyl)pyridin-2-yl)-6-chloroimidazo[1,2-*b*] pyridazine (37)**

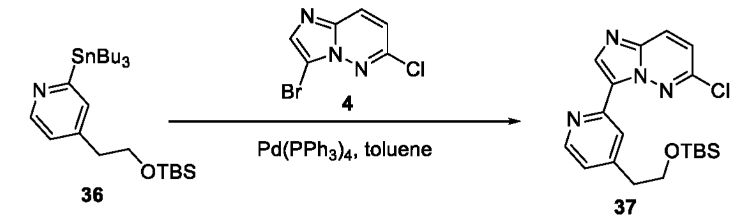


36 37

To a solution of 3-bromo-6-chloro-imidazo[1,2-***b***]pyridazine (Compound **36,** 2.21 g, 97.57 mmol) and Pd($PPh_3$)$_4$ (1.10 g, 949.75 umol) in toluene (10 mL) was added 4-(2-((tert-butyldimethy lsilyl)oxy)ethyl)-2-(tributylstannyl)pyridine (Compound **36,** 5 g, 9.50 mmol). The mixture was stirred at 80 °C for 12 h under $N_2$ atmosphere. The reaction mixture was filtered and concentrated. The residue was purified by the chromatography column to give 3-(4-(2-((tert-butyldimethylsilyl) oxy)ethyl)pyridin-2-yl)-6-chloroimidazo[1,2-***b***] pyridazine (270 mg, 7.31% yield). **$^1$H NMR:** (400 MHz, CHLOROFORM-*d*) δ ppm 8.59 - 8.62 (m, 1 H) 8.54 - 8.57 (m, 1 H) 8.39 - 8.42 (m, 1 H) 7.99 - 8.03 (m, 1 H) 7.12 - 7.18 (m, 2 H) 3.94 (t, *J* = 6.63 Hz, 2 H) 2.95 (t, *J* = 6.57 Hz, 2 H) 0.87 (br, 9 H), 0.00 - 0.02 (m, 6 H).

**Step E: 2-(((3-(4-(2-hydroxyethyl)pyridin-2-yl)imidazo[1,2-*b*]pyridazin-6-yl)amino) methyl)phenol (37-1)**

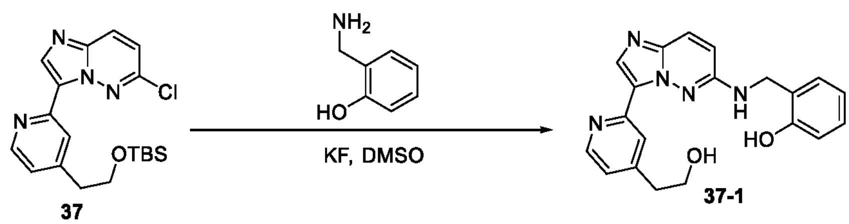


37 37-1

To a solution of 3-(4-(2-((tert-butyldimethylsilyl)oxy)ethyl)pyridin-2-yl)-6-chloroimidazo [1,2-***b***]pyridazine (Compound **37,** 250 mg, 0.64 mmol) in DMSO (5 mL) was added 2-(amino methyl)-phenol (158.31 mg, 1.29 mmol) and KF (298.74 mg, 5.14 mmol), and the reaction was stirred for 30 min at 130 °C under $N_2$ atmosphere. The reaction mixture was concentrated and purified by Preparative Chromatography to give **2-(((3-(4-(2-hydroxyethyl)pyridin-2-yl)imidazo[1,2-*b*]pyridazin-6-yl)amino) methyl)phenol** (100 mg, 43% yield). **LCMS:** m/z (ESI), 362.1[M+H]$^+$. **$^1$H NMR:** (400 MHz, METHANOL-d4) δ ppm 8.50 - 8.52 (m, 1 H) 8.43 (d, *J* = 5.00 Hz, 1 H) 8.06 (s, 1 H) 7.67 - 7.72 (m, 1 H) 7.26 (d, *J* = 7.50 Hz, 1 H) 7.16 - 7.21 (m, 1 H) 7.06 - 7.11 (m, 1 H) 6.87 (d, *J* = 9.76 Hz, 1 H) 6.83 (d, *J* = 8.00 Hz, 1 H) 6.77 (t, *J* = 7.50 Hz, 1 H) 4.63 (s, 2 H) 3.76 (t, *J* = 6.44 Hz, 2 H) 2.76 (t, *J* = 6.50 Hz, 2 H).

**Step F: (*E*)-6-oxa-3-aza-2(3,6)-imidazo[1,2-*b*]pyridazina-1(2,4)-pyridina-5(1,2)-benzenacyclooctaphane (I-8)**

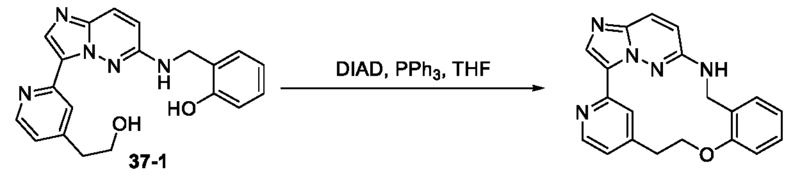


37-1

I-8

To a solution of 2-(((3-(4-(2-hydroxyethyl)pyridin-2-yl)imidazo[1,2-***b***]pyridazin-6-yl)amino) methyl)phenol (Compound **37-1,** 30 mg, 0.083 mmol) in THF (5 mL) was added $PPh_3$ (65.32 mg, 249.03 umol) and DIAD (50.36 mg, 249.03 umol). The mixture was stirred for 2 h at 60 °C under $N_2$ atomosphere. The reaction mixture was directly purified by Preparative Chromatography to give (***E***)-6-oxa-3-aza -2(3,6)-imidazo[1,2-***b***]pyridazina-1(2,4)-pyridina-5(1,2)-benzenacyclooctaphane (10 mg, 34% yield). **LCMS: m/z (ESI),** 344.0[M+H]$^+$. **$^1$H NMR:** (400 MHz, METHANOL-d4) $\delta$ ppm 9.42 (s, 1 H) 8.76 (br s, 1 H) 8.59 - 8.62 (m, 1 H) 8.46 - 8.50 (m, 1 H) 8.02 (d, *J* = 9.88 Hz, 1 H) 7.49 - 7.55 (m, 1 H) 7.40 - 7.44 (m, 1 H) 7.28 (d, *J* = 9.88 Hz, 1 H) 7.15 - 7.22 (m, 1 H) 6.91 - 7.00 (m, 2 H) 4.71 (br s, 2 H) 4.34 - 4.38 (m, 2 H) 3.27 (t, *J* = 4.88 Hz, 2 H).

**Example 9: (*E*)-4-methyl-6-oxa-3-aza-2(3,6)-imidazo[1,2-*b*]pyridazina-1(2,4)-pyridina-5(1,2)-benzenacyclooctaphane (I-9)**

**Step A: 3-(4-(2-((tert-butyldimethylsilyl)oxy)ethyl)pyridin-2-yl)-*N*-(1-(2-((4-methoxy benzyl)oxy)phenyl)ethyl) imidazo[1,2-*b*]pyridazin-6-amine (38)**

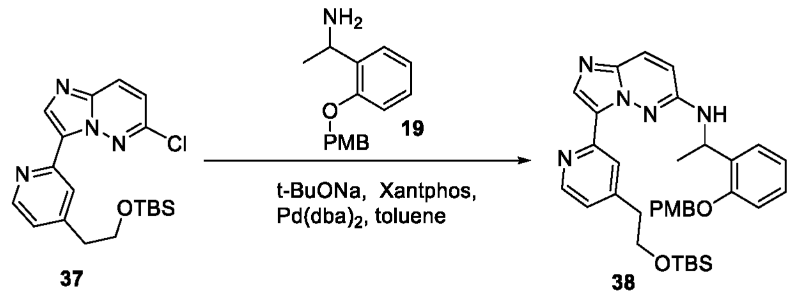


37

38

To a solution of 3-(4-(2-((tert-butyldimethylsilyl)oxy)ethyl)pyridin-2-yl)-6-chloroimidazo [1,2-***b***]pyridazine (Compound **37**, 500 mg, 1.28 mmol) and 1-(2-((4-methoxybenzyl)oxy)phenyl) ethan-1-amine (262.40 mg, 1.02 mmol) in 2-methyl-2-butanol (20 mL) was added t-BuONa (2M, 241.02 uL), RuPhos Pd G4 (13.44 mg, 0.016 mmol). The reaction was stirred for 2 h at 100 °C under $N_2$ atmosphere and diluted with water (20 mL), extracted with EA (20 mL) three times. The combined organic layer was washed with brine, dried over anhydrous $Na_2SO_4$, and concentrated. The residue was purified by the chromatography column to give 3-(4-(2-((tert-butyldimethylsilyl)oxy)ethyl) pyridin-2-yl)-***N***-(1-(2-((4-methoxybenzyl)oxy)phenyl)ethyl)imidazo[1,2-***b***]pyridazin-6-amine (130 mg, 83%). **LCMS:** m/z (ESI), 610.2[M+H]$^+$.

**Step B: 3-(4-(hydroxyethyl)pyridin-2-yl)-*N*-(1-(5-fluro2-hydroxyphenyl)ethyl)imidazo [1,2-*b*]pyridazine-6-amine (39)**

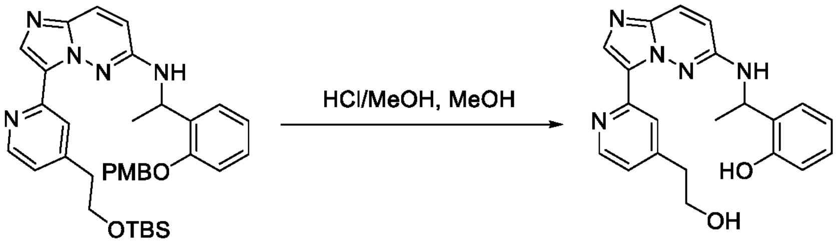


38

39

To a solution of 3-(4-(2-((tert-butyldimethylsilyl)oxy)ethyl)pyridin-2-yl)-***N***-(1-(2-((4-methoxy benzyl)oxy)phenyl) ethyl)imidazo[1,2-***b***]pyridazin-6-amine (Comound **38,** 120 mg, 0.196 mmol) in MeOH (10 mL) was added HCl/MeOH (4 M, 3 mL). The mixture was stirred at 25 °C for 2 h. The mixture was adjusted to pH = 7 with $NaHCO_3$, filtered and the filtrate was concentrated. The residue was purified by chromatography column to give 3-(4-(hydroxyethyl)pyridin-2-yl)-***N***-(1-(5-fluro2-hydroxyphenyl)ethyl)imidazo[1,2-***b***]pyridazine-6-amine (35 mg, 23%). **LCMS:** m/z (ESI), 376[M+H]$^+$. **$^1$H NMR:** (400 MHz, METHANOL-d4) δ = 8.43 - 8.39 (m, 2 H) 8.05 - 8.02 (m, 1 H) 7.69 - 7.65 (m, 1 H) 7.30 - 7.26 (m, 1 H) 7.21 - 7.18 (m, 1 H) 7.06 - 7.01 (m, 1 H) 6.89 (d, *J* = 9.6 Hz, 1 H) 6.82 - 6.74 (m, 2 H) 5.46 - 5.39 (m, 1 H) 3.92 - 3.76 (m, 2 H) 2.94 (t, *J* = 6.6 Hz, 2 H) 1.59 - 1.56 (m, 3 H).

**Step C: (*E*)-4-methyl-6-oxa-3-aza-2(3,6)-imidazo[1,2-*b*]pyridazina-1(2,4)-pyridina-5(1,2)-benzenacyclooctaphane (I-9)**

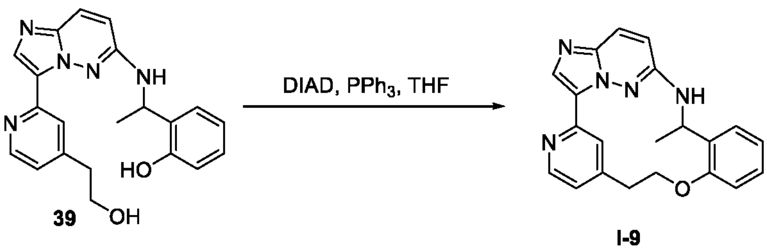


39

I-9

To a solution of 3-(4-(hydroxyethyl)pyridin-2-yl)-***N***-(1-(5-fluro2-hydroxyphenyl)ethyl) imidazo[1,2-***b***]pyridazine-6-amine (Compound **39,** 30 mg, 0.079 mol) in THF (5 mL) was added $PPh_3$ (65.32 mg, 0.25 mmol) and DIAD (50.36 mg, 0.25 mmol). The mixture was stirred at 60 °C for 2 h and directly purified by Preparative Chromatography to give (E)-4-methyl-6-oxa-3-aza-2(3,6)-imidazo [1,2-***b***]pyridazina-1(2,4)-pyridina-5(1,2)-benzenacyclooctaphane (6.74 mg, 18.4%). **LCMS: m/z (ESI),** 358.0[M+H]$^+$. **$^1$H NMR:** (400 MHz, DMSO-d6) δ = 9.32 - 9.28 (m, 1 H) 8.80 - 8.74 (m, 1 H) 8.64 - 8.60 (m, 1 H) 8.55 - 8.52 (m, 1 H) 8.07 - 8.02 (m, 1 H) 7.51 - 7.46 (m, 1 H) 7.46 - 7.42 (m, 1 H) 7.28 - 7.24 (m, 1 H) 7.20 - 7.14 (m, 1 H) 7.01 - 6.92 (m, 2 H) 5.76 - 5.66 (m, 1 H) 4.53 - 4.48 (m, 1 H) 4.22 (t, *J* = 9.2 Hz, 1 H) 3.30 (d, *J* = 4.4 Hz, 1 H) 3.21 (d, *J* = 4.8 Hz, 1 H) 1.45 - 1.41 (m, 3 H).

**Example 10: (*E*)-6-methyl-4-oxa-7-aza-1(3,6)-imidazo[1,2-*b*]pyridazina-2(4,6)-pyrimidina -5(1,2)-benzenacycloheptaphane**

**Step A: 6-(((tert-butyldimethylsilyl)oxy)methyl)-4-chloropyrimidine (40)**

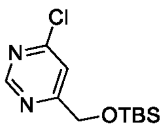

40

The title Compound **40** was prepared with reference to the method of Compound **2** by replacing Compound **1** with 6-chloro-4-hydroxymethylpyrimidine as the raw material. **LCMS:** m/z (ESI), 259[M+H]$^+$.

**Step B: 6-(((tert-butyldimethylsilyl)oxy)methyl)-4-(tributylstannyl)pyrimidine (41)**

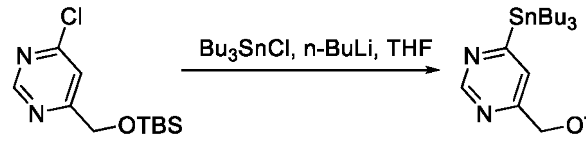


40 41

The title Compound **41** was prepared with reference to the method of Compound **3** by replacing Compound **2** with Compound **40** as the raw material. **LCMS:** m/z (ESI), 515.0[M+H]$^+$.

**Step C: 3-(6-(((tert-butyldimethylsilyl)oxy)methyl)pyrimidin-4-yl)-6-chloroimidazo [1,2-*b*]pyridazine (42)**

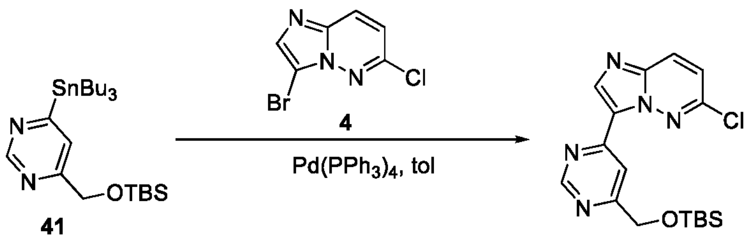


41 42

The title Compound **42** was prepared with reference to the method of Compound 5 by replacing Compound **3** with Compound **41** as the raw material. **LCMS:** m/z (ESI), 376.0[M+H]$^+$.

**Step D: 3-(6-(((tert-butyldimethylsilyl)oxy)methyl)pyrimidin-4-yl)-N-(1-(2-((4-methoxy benzyl)oxy)phenyl)ethyl) imidazo[1,2-*b*]pyridazin-6-amine (43)**

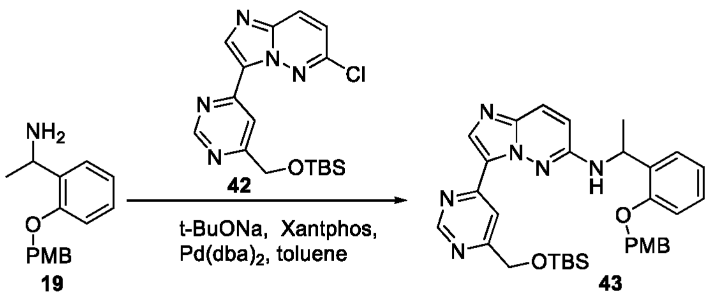


19 43

The title Compound **43** was prepared with reference to the method of Compound **20** by replacing Compound **5** with Compound **42** as the raw material. **LCMS:** m/z (ESI), 597.2[M+H]$^+$.

**Step E: 3-(6-(hydroxymethyl)pyrimidin-4-yl)-*N*-(1-(2-hydroxypehnyl)ethyl) imidazo[1,2-*b*]pyridazine-6-amine (44)**

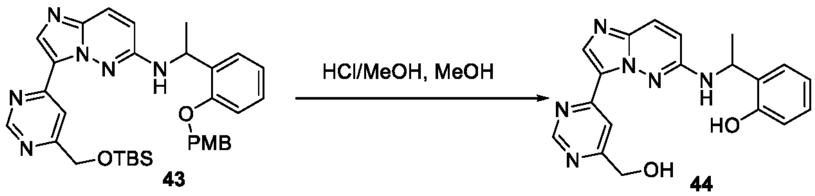


The title Compound **44** was prepared with reference to the method of Compound **11** by replacing Compound **10** with Compound **43** as the raw material. **LCMS:** m/z (ESI), 363[M+H]$^+$.

**Step F: (*E*)-6-methyl-4-oxa-7-aza-1(3,6)-imidazo[1,2-*b*]pyridazina-2(4,6)-pyrimidine -5(1,2)-benzenacycloheptaphane (I-10)**

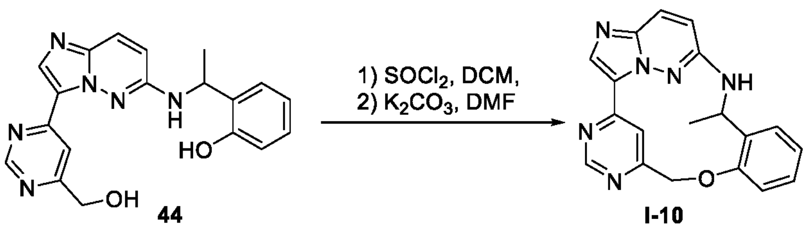


The title Compound **I-10** was prepared with reference to the method of Compound **I-1** by replacing Compound **11** with Compound **44** as the raw material. **LCMS:** m/z (ESI), 345[M+H]$^+$.

**Example 11: (*E*)-4-methyl-6-oxa-3-aza-2(3,6)-imidazo[1,2-*b*]pyridazina-1(2,4)-pyridina -5(1,2)-benzenacyclooctaphane (I-11)**

**Step A: 4-chloro-6-hydroxyethylpyrimidine (45)**

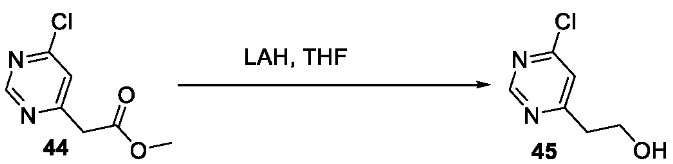


The title Compound **45** was prepared with reference to the method of Compound **34** by replacing Compound **33** with Compound **44** as the raw material. **LCMS:** m/z (ESI), 159.2[M+H]$^+$.

**Step B: 4-(2-((tert-butyldimethylsilyl)oxy)ethyl)-6-chloropyrimidine (46)**

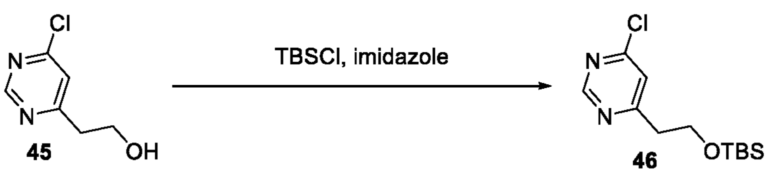


The title Compound **46** was prepared with reference to the method of Compound 35 by replacing Compound **34** with Compound **45** as the raw material. **LCMS:** m/z (ESI), 273.0[M+H]$^+$.

**Step C: 4-(2-((tert-butyldimethylsilyl)oxy)ethyl)-6-(tributylstannyl)pyrimidine (47)**

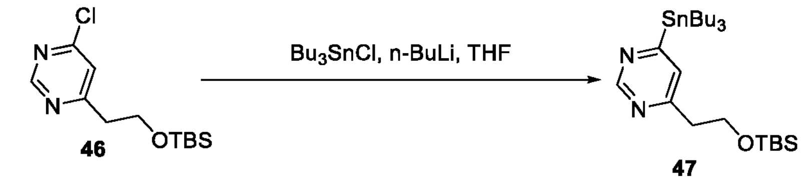


46 47

The title Compound **47** was prepared with reference to the method of Compound **36** by replacing Compound **35** with Compound **46** as the raw material. **LCMS:** m/z (ESI), 529.2[M+H]$^+$.

**Step D: 3-(6-(2-((tert-butyldimethylsilyl)oxy)ethyl)pyrimidin-4-yl)-6-chloroimidazo[1,2-*b*] pyridazine (48)**

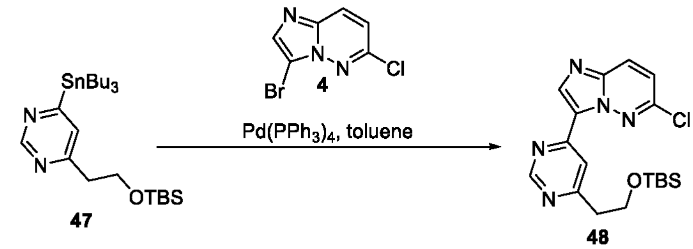


47 48

The title Compound **48** was prepared with reference to the method of Compound **37** by replacing Compound **36** with Compound **47** as the raw material. **LCMS:** m/z (ESI), 390.0[M+H]$^+$.

**Step E: 3-(6-(2-((tert-butyldimethylsilyl)oxy)ethyl)pyrimidin-4-yl)-*N*-(1-(2-((4-methoxy benzyl)oxy)phenyl)ethyl) imidazo[1,2-*b*]pyridazin-6-amine (48-1)**

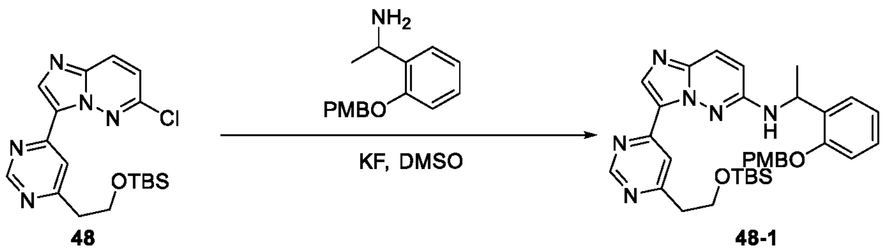


48 48-1

The title Compound **48-1** was prepared with reference to the method of Compound **38** by replacing Compound **37** with Compound **48** as the raw material. **LCMS:** m/z (ESI), 611.2[M+H]$^+$.

**Step F: 2-(((3-(6-(6-hydroxyethyl)pyrimidin-4-yl)imidazo[1,2-*b*]pyridazin-6-yl)amino) methyl)phenol (48-2)**

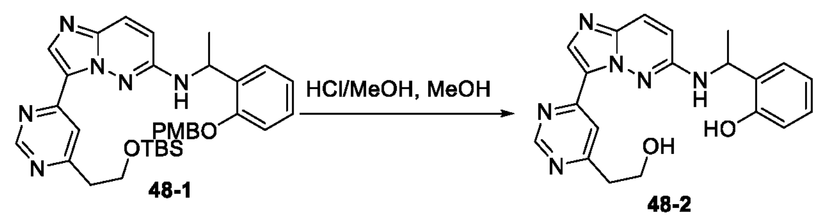


48-1 48-2

The title Compound **48-2** was prepared with reference to the method of Compound **39** by replacing Compound **38** with Compound **48-1** as the raw material. **LCMS:** m/z (ESI), 377.2[M+H]+.

**Step G: (*E*)-4-methyl-6-oxa-3-aza-2(3,6)-imidazo[1,2-*b*]pyridazina-1(4,6)- pyrimidina -5(1,2)-benzenacycloheptaphane (I-11)**

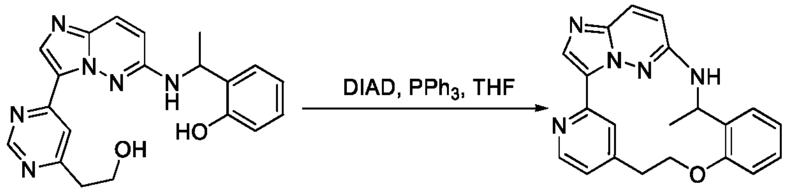


**48-2** **I-11**

The title Compound **I-11** was prepared with reference to the method of Compound **I-9** by replacing Compound 39 with Compound **48-2** as the raw material. **LCMS:** m/z (ESI), 359[M+H]+.

**Example 12: (*E*)-5⁵-fluoro-6-methyl-3-oxa-7-aza-1(3,6)-imidazo[1,2-*b*]pyridazina-2(2,4),5(2,3)-dipyridinacycloheptaphane (I-12)**

**Step** A: **2-bromo-4-((4-methoxybenzyl)oxy)pyridine (50)**

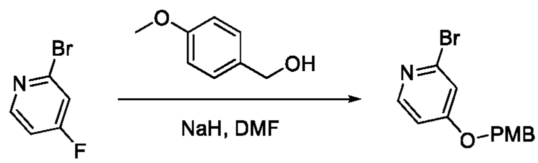


**49** **50**

To a solution of 2-bromo-4-fluoropyridine (Compound **49,** 10 g, 57.47 mmol) and 4-methoxy benzyl alcohol (9.52 g, 68.96 mmol) in DMF (100 mL) was added and NaH (3.45 g, 86.21 mmol) at 0 °C. The mixture was stirred at RT for 16 h, then quenched with water (1 L) and extracted with EA (500 mL) three times. The combined organic layer was washed with brine, dried over anhydrous $Na_2SO_4$, and concentrated. The residue was purified by the chromatography column to give 2-bromo-4-tert-butyldimethylsilyloxymethylpyridine (14.44 g, 85%). **LCMS:** m/z (ESI), 294[M+H]+.

**Step B: 6-chloro-3-(tributylstannyl)imidazo[1,2-*b*]pyridazine (51)**

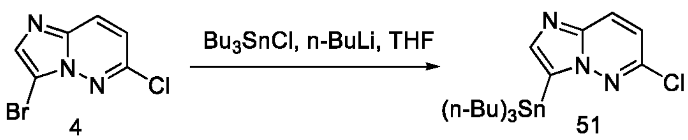


4 51

To a solution of 3- chloro -6-chloro-imidazo[1,2-b]pyridazine (Compound **4,** 10 g, 43.10 mmol) in THF (100 mL) was added n-BuLi (2.5 M, 17.24 mL) dropwise at -70 °C. The mixture was stirred for 30 min, and then tributyl(chloro) stannane (18.25 g, 43.10 mmol) was added in and the reaction was stirred at -70 °C for 2 h. The reaction was quenched by aqueous saturated $NH_4Cl$ solution (1.5 L) and extracted by EtOAc (500 mL) three times. The combined organic layers were washed with brine, dried over anhydrous $Na_2SO_4$, and concentrated. The residue was purified by the chromatography column to give 6-chloro-3-(tributylstannyl)imidazo[1,2-***b***]pyridazine (2.1 g, 11%). **LCMS:** m/z (ESI), 444.3[M+H]+.

**Step C: 6-chloro-3-(4-((4-methoxybenzyl)oxy)pyridin-2-yl)imidazo[1,2-*b*]pyridazine (52)**

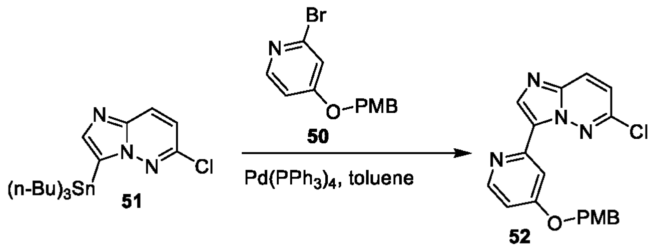


To a solution of 6-chloro-3-(tributylstannyl)imidazo[1,2-***b***]pyridazine (Compound **51**, 1 g, 2.25 mmol) in toluene (10 mL) was added 2-bromo-4-((4-methoxybenzyl)oxy)pyridine (Compound **50**, 661.3 mg, 2.25 mmol) and $Pd(PPh_3)_4$ (58.5 mg, 0.225 mmol) in sequence. The mixture was stirred at 100 °C for 16 h and diluted with water (1.5 L). The mixture was extracted by EtOAc (500 mL) three times. The combined organic layers were washed with brine, dried over anhydrous $Na_2SO_4$, concentrated. The residue was purified by the chromatography column to give 6-chloro-3-(4-((4-methoxybenzyl)oxy)pyridin-2-yl)imidazo[1,2-***b***]pyridazine (517 mg, 62%).

**Step D: methyl 3-bromo-5-fluoropyridine-2-carboxylate (54)**

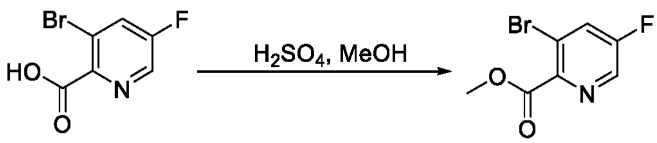


**53** **54**

To a solution of 3-bromo-5-fluoropyridine-2-carboxylic acid (Compound **53,** 10 g, 45.45 mmol) in MeOH (200 mL) was added concentrated sulfuric acid (2 mL). The mixture was stirred at 80 °C for 12 h and diluted with water (300 mL). The mixture was extracted by EtOAc (200 mL) three times. The combined organic layers were washed with brine, dried over anhydrous $Na_2SO_4$, and concentrated. The residue was purified by the chromatography column to give methyl 3-bromo-5-fluoropyridine -2-carboxylate (6.3 g, 59%). **LCMS:** m/z (ESI), 234.0[M+H]$^+$.

**Step E: methyl 3-(1-ethoxyvinyl)-5-fluoropyridine-2-carboxylate (55)**

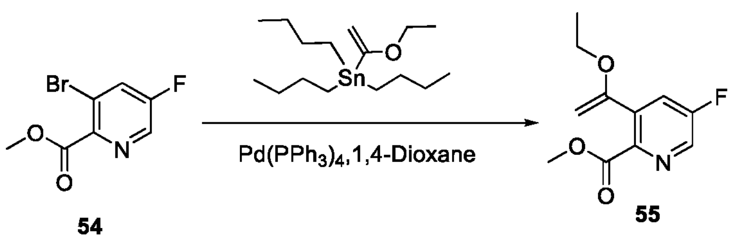


To a solution of methyl 3-bromo-5-fluoropyridine-2-carboxylate (Compound **54,** 3 g, 12.82 mmol) and in 1,4-dioxane (100 mL) was added $Pd(PPh_3)_4$ (147.84 mg, 0.128 mmol) and tributy (1-ethoxyvinyl)-λ4-stannane (6.94 g, 19.23 mmol). The mixture was stirred at 110 °C for 16 h and diluted with water (1.5 L). The mixture was extracted by EtOAc (500 mL) three times. The combined organic layers were washed with brine, dried over anhydrous $Na_2SO_4$, and concentrated. The residue was purified by the chromatography column to give methyl 3-(1-ethoxyvinyl)-5-fluoro pyridine-2-carboxylate (2.5 g, 86%). **LCMS:** m/z (ESI), 226.0[M+H]$^+$.

**Step F: (3-(1-ethoxyvinyl)-5-fluoropyridin-2-yl)methanol (56)**

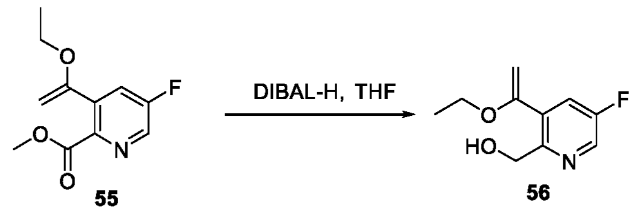


55

56

To a solution of methyl 3-(1-ethoxyvinyl)-5-fluoropyridine-2-carboxylate (Compound **55,** 1.1 g, 4.89 mmol) in THF (50 mL) was added DABAL-H (9.77 mL, 9.77 mmol). The mixture was stirred at 0 °C for 5 h. The reaction was quenched and diluted with water (600 mL) and extracted by EA (200 mL) three times. The combined organic layers were washed with brine, dried over anhydrous $Na_2SO_4$, and concentrated. The residue was purified by the chromatography column to give (3-(1-ethoxy vinyl)-5-fluoropyridin-2-yl)methanol (803 mg, 83%). **LCMS:** m/z (ESI), 198.0[M+H]+.

**Step G: (3-Acetyl-5-fluoropyridin-2-yl)methanol (57)**

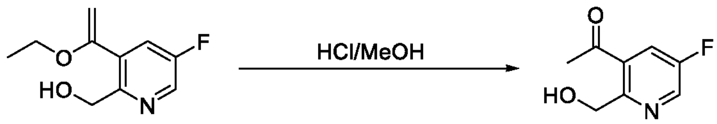


56

57

To a solution of (3-(1-ethoxyvinyl)-5-fluoropyridin-2-yl)methanol (Compound **56,** 803 mg, 4.07 mmol) in MeOH (50 mL) was added HCl/MeOH (1M, 10 mL). The mixture was stirred at 25 °C for 12 h and diluted with saturated $NaHCO_3$ solution (100 mL). The mixture was extracted by EA (200 mL) three times. The combined organic layers were washed with brine, dried over anhydrous $Na_2SO_4$, and concentrated. The residue was purified by the chromatography column to give (3-Acetyl-5-fluoropyridin-2-yl)methanol (650 mg, 94%). **LCMS:** m/z (ESI), 170.0[M+H]+.

**Step H: 1-(2-(((tert-butyldimethylsilyl)oxy)methyl)-5-fluoropyridin-3-yl)ethan-1-one (58)**

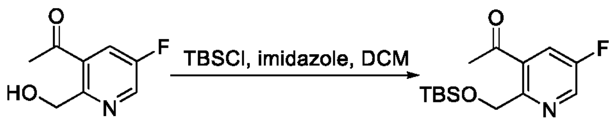


57

58

To a solution of (3-Acetyl-5-fluoropyridin-2-yl)methanol (Compound **57,** 650 mg, 3.84 mmol) in DCM (50 mL) was added imidazole (537.6 mg, 7.68 mmol), TBSCl (576 mg, 3.84 mmol) in sequence. The mixture was stirred at 25 °C for 12 h. Then the mixture was diluted with water (50 mL) and extracted by EA (50 mL) three times. The combined organic layers were washed with brine, dried over anhydrous $Na_2SO_4$, and concentrated. The residue was purified by the chromatography column to give 1-(2-(((tert-butyldimethylsilyl)oxy)methyl)-5-fluoropyridin-3-yl)ethan-1-one (343 mg, 32%). **LCMS:** 58 m/z (ESI), 284.0[M+H]+.

**Step I: 1-(2-(((tert-butyldimethylsilyl)oxy)methyl)-5-fluoropyridin-3-yl)ethan-1-one oxime(59)**

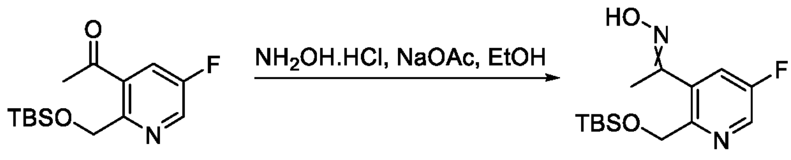


58 59

To a solution of 1-(2-(((tert-butyldimethylsilyl)oxy)methyl)-5-fluoropyridin-3-yl)ethan-1-one (Compound **58,** 343 mg, 1.21 mmol) in EtOH (20 mL) was added NaOAc (198 mg, 2.42 mmol) and $NH_2OH.HCl$ (168 mg, 2.42 mmol) in sequence. The reaction mixture was stirred at 70 °C for 2 h. The reaction was diluted with water (600 mL) and extracted with EA (200 mL) three times. The combined organic layer was washed with brine, dried over anhydrous $Na_2SO_4$, and concentrated. The residue was purified by the chromatography column to give 1-(2-(((tert-butyldimethylsilyl)oxy)methyl) -5-fluoropyridin-3-yl)ethan-1-one oxime (309 mg, 85.6%). **LCMS:** m/z (ESI), 299.0$[M+H]^+$.

**Step J: 1-(2-(((tert-butyldimethylsilyl)oxy)methyl)-5-fluoropyridin-3-yl)ethan-1-amine(60)**

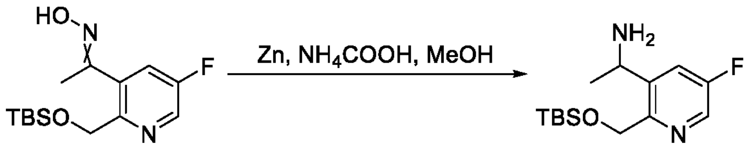


59 60

To a solution of 1-(2-(((tert-butyldimethylsilyl)oxy)methyl)-5-fluoropyridin-3-yl)ethan-1-one oxime (Compound **59,** 309 mg, 1.07 mmol) in MeOH (15 mL) was added ammonium formate (127 mg, 2.14 mmol) and Zn powder (139 mg, 2.14 mmol) in sequence. The mixture was stirred at 40 °C for 12 h. The mixture was filtered and the filtrate was concentrated. The residue was purified by the chromatography column to give 1-(2-(((tert-butyldimethylsilyl)oxy) methyl)-5-fluoropyridin-3-yl) ethan-1-amine (105 mg, 34.5%). **LCMS:** m/z (ESI), 285.0$[M+H]^+$.

**Step K: *N*-(1-(2-(((tert-butyldimethylsilyl)oxy)methyl)-5-fluoropyridin-3-yl)ethyl)-3-(4-((4-methoxybenzyl)oxy) pyridin-2-yl)imidazo[1,2-*b*]pyridazin-6-amine (61)**

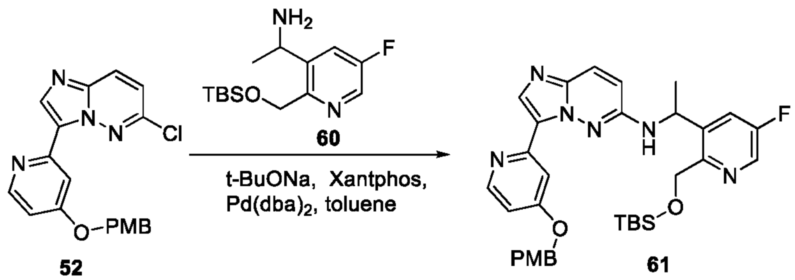


52 61

To a solution of 6-chloro-3-(4-((4-methoxybenzyl)oxy)pyridin-2-yl)imidazo[1,2-***b***]pyridazine (Compound **52,** 100 mg, 0.272 mmol) in Toluene (5 mL) was added 1-(2-(((tert-butyldimethyl silyl)oxy)methyl)-5-fluoro pyridin-3-yl)ethan-1-amine (93.11 mg, 0.328 mmol), $Pd(dba)_2$ (15.34 mg, 0.027 mmol), t-BuONa (51.26 mg, 0.53 mmol) and Xantphos (15.43 mg, 0.027 mmol) in sequence., the reaction was stirred for 16 h at 100 °C under $N_2$ atmosphere. The reaction mixture was concentrated and purified by the chromatography column to give ***N***-(1-(2-(((tert-butyldimethylsilyl) oxy)methyl)-5-fluoropyridin-3-yl)ethyl)-3-(4-((4-methoxybenzyl)oxy)pyridin-2-yl)imidazo[1,2-***b***]p yridazin-6-amine (42 mg, 25%). **LCMS:** m/z (ESI), 615.2$[M+H]^+$.

**Step L: 2-(6-((1-(5-fluoro-2-(hydroxymethyl)pyridin-3-yl)ethyl)amino)imidazo[1,2-*b*] pyridazin-3-yl)pyridin-4-ol (62)**

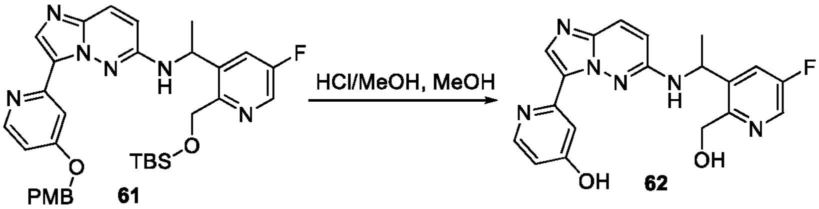


To a solution of N-(1-(2-(((tert-butyldimethylsilyl)oxy)methyl)-5-fluoropyridin-3-yl)ethyl)-3-(4-((4-methoxybenzyl)oxy)pyridin-2-yl)imidazo[1,2-***b***]pyridazin-6-amine (Compound **61,** 42 mg, 0.068 mmol) in MeOH (3 mL) was added HCl/MeOH (4 M, 3 mL). The mixture was stirred at 25 °C for 2 h. The mixture was adjusted to pH = 7 with $NaHCO_3$, filtered and the filtrate was concentrated and purified by chromatography column to give 2-(6-((1-(5-fluoro-2-(hydroxymethyl) pyridin-3-yl)ethyl)amino)imidazo[1,2-***b***]pyridazin-3-yl)pyridin-4-ol (22 mg, 85%). **LCMS:** m/z (ESI), 381.2[M+H]$^+$.

**Step M: (*E*)-5$^5$-fluoro-6-methyl-3-oxa-7-aza-1(3,6)-imidazo[1,2-*b*]pyridazina-2(2,4),5(2,3)-dipyridinacycloheptaphane (I-12)**

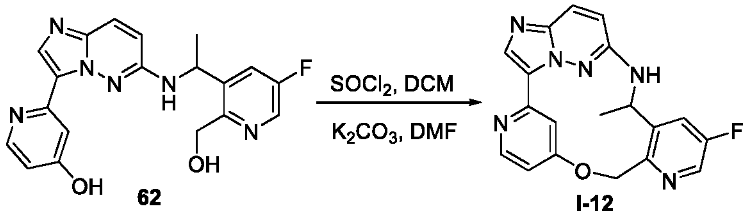


To a solution of 2-(6-((1-(5-fluoro-2-(hydroxymethyl)pyridin-3-yl)ethyl)amino)imidazo[1,2-***b***] pyridazin-3-yl)pyridin-4-ol (Compound **62,** 35.2 mg, 0.094 mol) in DCM/DMF mixture solution (6 mL) was added $SOCl_2$ (215.57 mg, 1.81 mol). The reaction was stirred for 0.5 h at 25 °C. Then $K_2CO_3$ (500.84 mg, 3.62 mmol) was added to adjust pH > 7 and the mixture was evaporated to remove DCM and stirred at 90 °C for another 1.5 h. The reaction mixture was filtered and the filtration was concentrated and purified by Preparative Chromatography to give (***E***)-5$^5$-fluoro-6-methyl-3-oxa-7-aza-1(3,6) -imidazo[1,2-***b***]pyridazina-2(2,4),5(2,3)-dipyridinacycloheptaphane (7.6 mg, 22.6%). **LCMS:** m/z (ESI), 363[M+H]$^+$.

**Example 13: (*E*)-5$^4$-fluoro-6-methyl-3-oxa-7-aza-1(3,6)-imidazo[1,2-*b*]pyridazina-2(2,4)-pyridina-5(1,2)-benzenacycloheptaphane (I-13)**

**Step A: methyl 2-bromo-4-fluorobenzoate (63)**

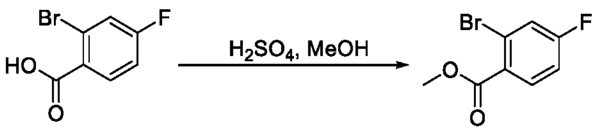


62 63

The title Compound **63** was prepared with reference to the method of Compound **54** by replacing Compound **53** with Compound **62** as the raw material. **LCMS:** m/z (ESI), 233.0[M+H]$^+$.

**Step B: methyl 2-(1-ethoxyvinyl)-4-fluorobenzoate (64)**

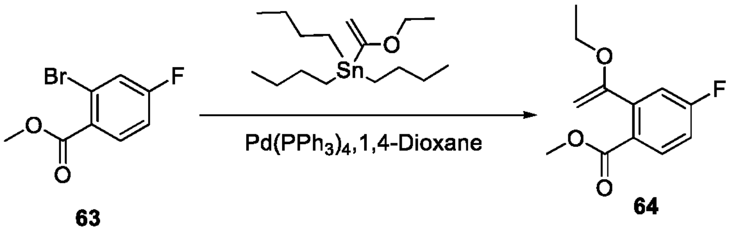


63 64

The title Compound **64** was prepared with reference to the method of Compound 55 by replacing Compound **54** with Compound **63** as the raw material. **LCMS:** m/z (ESI), 225.0[M+H]+.

**Step C: [2-(1-ethoxyvinyl)-4-fluorophenyl]methanol (65)**

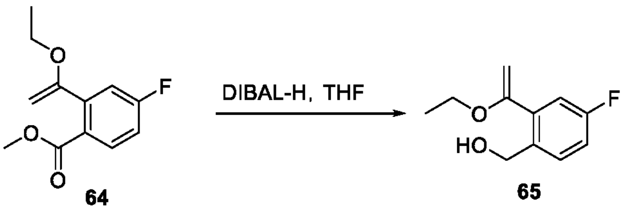


64 65

The title Compound **65** was prepared with reference to the method of Compound **56** by replacing Compound **55** with Compound **64** as the raw material. **LCMS:** m/z (ESI), 197.0[M+H]+.

**Step D: (2-Acetyl-4-fluoro-phenyl)methanol (66)**

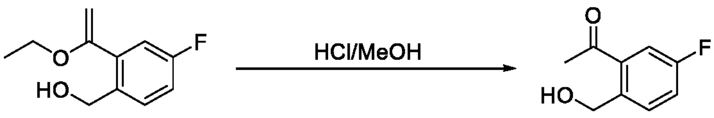


65 66

The title Compound **66** was prepared with reference to the method of Compound **57** by replacing Compound **56** with Compound **65** as the raw material. **LCMS:** m/z (ESI), 169.0[M+H]+.

**Step E: 1-(2-(((tert-butyldimethylsilyl)oxy)methyl)-5-fluorophenyl)ethan-1-one (67)**

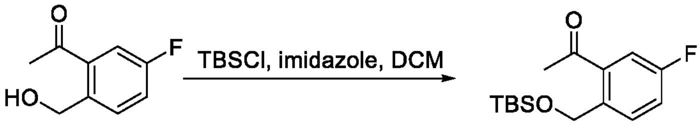


66 67

The title Compound **67** was prepared with reference to the method of Compound **58** by replacing Compound **57** with Compound **66** as the raw material. **LCMS:** m/z (ESI), 283.0[M+H]+.

**Step F: 1-(2-(((tert-butyldimethylsilyl)oxy)methyl)-5-fluorophenyl)ethan-1-one oxime(68)**

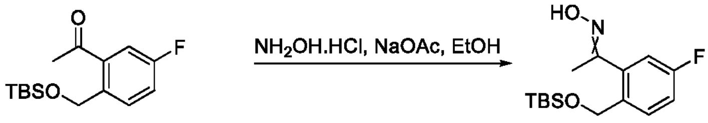


67 68

The title Compound **68** was prepared with reference to the method of Compound **59** by replacing Compound **58** with Compound **67** as the raw material. **LCMS:** m/z (ESI), 298.0[M+H]$^+$.

**Step G: 1-(2-(((tert-butyldimethylsilyl)oxy)methyl)-5-fluorophenyl)ethan-1-amine(69)**

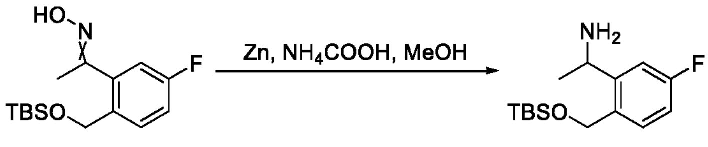


68 69

The title Compound **69** was prepared with reference to the method of Compound **60** by replacing Compound **59** with Compound **68** as the raw material. **LCMS:** m/z (ESI), 284.0[M+H]$^+$.

**Step H: *N*-(1-(2-(((tert-butyldimethylsilyl)oxy)methyl)-5-fluorophenyl)ethyl)-3-(4-((4-methoxybenzyl)oxy)pyridin-2-yl)imidazo[1,2-*b*]pyridazin-6-amine (70)**

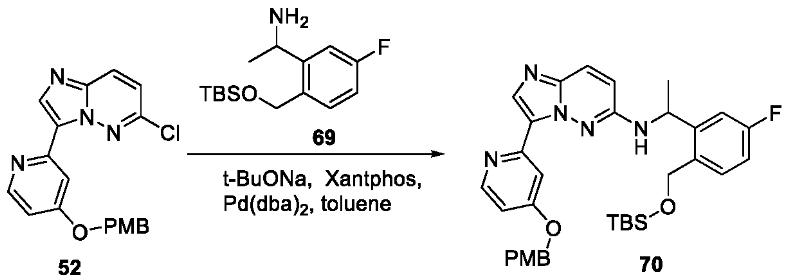


52 70

The title Compound **70** was prepared with reference to the method of Compound 61 by replacing Compound **60** with Compound **69** as the raw material. **LCMS:** m/z (ESI), 614.2[M+H]$^+$.

**Step I: 2-(6-((1-(5-fluoro-2-(hydroxymethyl)phenyl)ethyl)amino)imidazo[1,2-*b*] pyridazin-3-yl)pyridin-4-ol (70-1)**

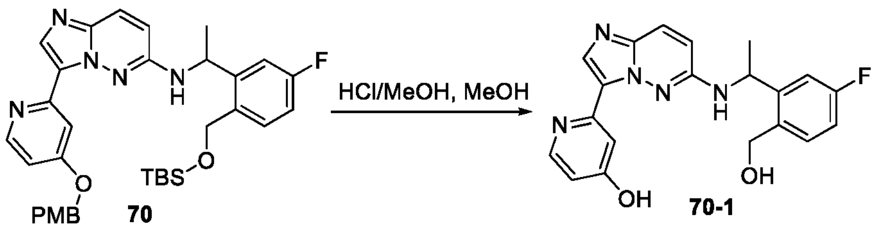


70 70-1

The title Compound **70-1** was prepared with reference to the method of Compound **62** by replacing Compound **61** with Compound **70** as the raw material. **LCMS:** m/z (ESI), 380.2[M+H]$^+$.

**Step J: (*E*)-54-fluoro-6-methyl-3-oxa-7-aza-1(3,6)-imidazo[1,2-*b*]pyridazina-2(2,4) -pyridina-5(1,2)-benzenacycloheptaphane (I-13)**

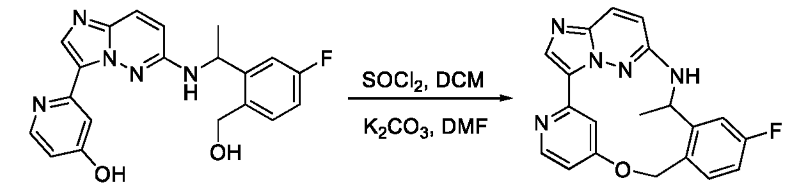


**70-1** **I-13**

The title Compound **I-13** was prepared with reference to the method of Compound **I-12** by replacing Compound **62** with Compound **70-1** as the raw material. **LCMS:** m/z (ESI), 362.2[M+H]$^+$.

**Example 14, 15: (*R*)-(*E*)-$5^4$-fluoro-6-methyl-4-oxa-7-aza-1(3,6)-imidazo[1,2-*b*]pyrid azina-2(2,4)-pyridina-5(1,2)-benzenacycloheptaphane (I-14), (*S*)-(*E*)-$5^4$-fluoro-6-methyl-4-oxa-7-aza-1(3,6)-imidazo[1,2-*b*]pyridazina-2(2,4)-pyridina-5(1,2)-benzenacycloheptaphane (I-15),**

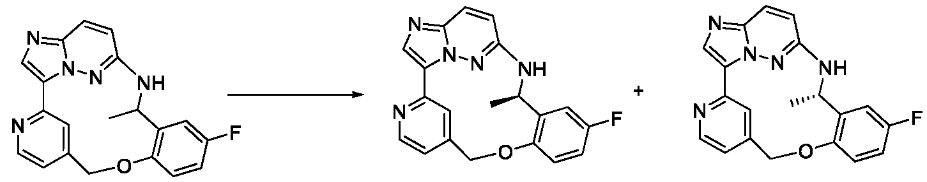

**I-1** **I-14** **I-15**

The title Compound **I-14 and I-15** were prepared by chiral preparative chromatography from Compound **I-1. LCMS:** m/z (ESI), 362.2[M+H]$^+$(I-14), **LCMS:** m/z (ESI), 362.2[M+H]$^+$(I-15).

**Example 16, 17: (*R*)-(*E*)-$5^4 5^6$-difluoro-6-methyl-4-oxa-7-aza-1(3,6)-imidazo[1,2-*b*] pyridazine-2(2,4)-pyridina-5(1,2)-benzenacycloheptaphane (I-16), (*S*)-(*E*)-$5^4$,$5^6$-difluoro-6-methyl-4-oxa-7-aza-1(3,6)-imidazo[1,2-*b*]pyridazine-2(2,4)-pyridina-5(1,2)-benzenacyclohe ptaphane (I-17)**

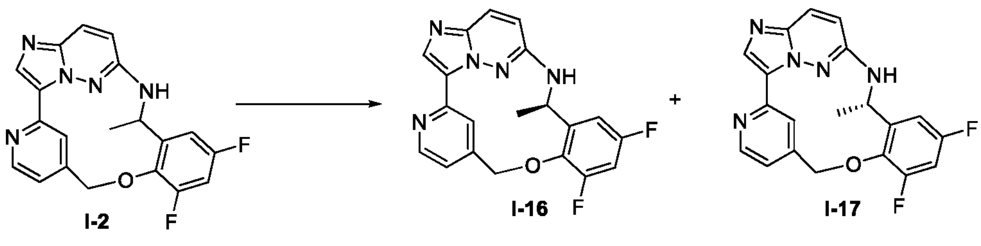

**I-2** **I-16** **I-17**

The title Compound **I-16 and I-17** were prepared by chiral preparative chromatography from Compound **I-2. LCMS:** m/z (ESI), 380.2[M+H]$^+$(I-16), **LCMS:** m/z (ESI), 380.0[M+H]$^+$(I-17).

**Example 18, 19: (*R*)-(*E*)-6-methyl-4-oxa-7-aza-1(3,6)-imidazo[1,2-*b*]pyridaz ine-2(2,4)-pyridina-5(1,2)-benzenacycloheptaphane (I-18), (*S*)-(*E*)-6-methyl-4-oxa-7-aza-1(3,6)-imidazo[1,2-*b*]pyridazine-2(2,4)-pyridina-5(1,2)-benzenacycloheptaphane (I-19)**

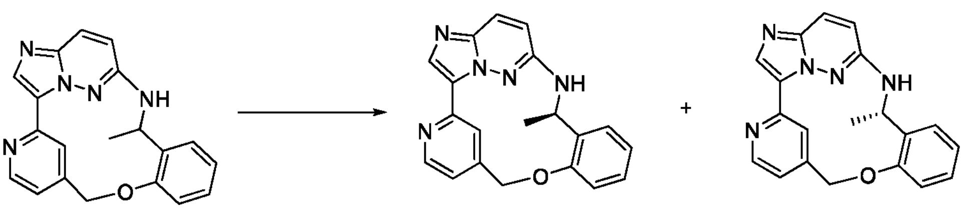

I-3 I-18 I-19

The title Compound **I-18** and **I-19** were prepared by chiral preparative chromatography from Compound **I-3. LCMS:** m/z (ESI), 344.2[M+H]$^+$(I-18), **LCMS:** m/z (ESI), 344.2[M+H]$^+$(I-19).

**Example 20, 21: (*R*)-(*E*)-6-methyl-4-oxa-7-aza-1(3,6)-imidazo[1,2-*b*]pyridazina-2(2,4), 5(2,3)-dipyridinacycloheptaphane (I-20), (6*S*,*E*)-6-methyl-4-oxa-7-aza-1(3,6)-imidazo[1,2-*b*] pyridazina-2(2,4),5(2,3)-dipyridinacycloheptaphane (I-21)**

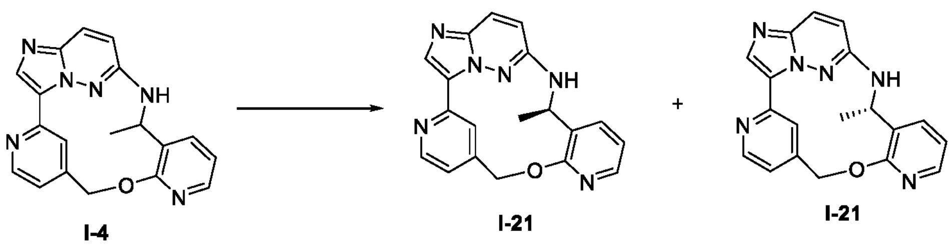

I-4 I-21 I-21

The title Compound **I-20 and I-21** were prepared by chiral preparative chromatography from Compound **I-4. LCMS:** m/z (ESI), 345.2[M+H]$^+$(I-20), **LCMS:** m/z (ESI), 345.2[M+H]$^+$(I-21).

**Example 22, 23: (*R*)-(*E*)-5$^4$-fluoro-6-methyl-4-oxa-7-aza-1(3,6)-imidazo[1,2-*b*]pyrida zina-2(3,5)-pyridina-5(1,2)-benzenacycloheptaphane (I-22), (*S*)-(*E*)-5$^4$-fluoro-6-methyl-4-oxa -7-aza-1(3,6)-imidazo[1,2-*b*]pyridazina-2(3,5)-pyridina-5(1,2)-benzenacycloheptaphane (I-23)**

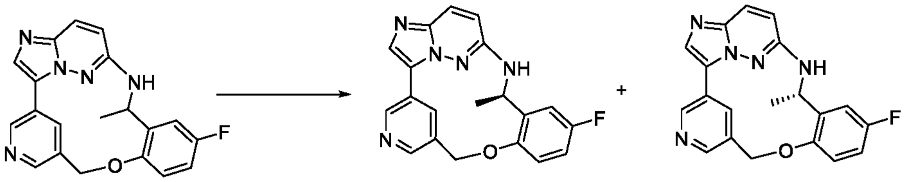

I-7 I-22 I-23

The title Compound **I-22 and I-23** were prepared by chiral preparative chromatography from Compound **I-7. LCMS:** m/z (ESI), 362.2[M+H]$^+$(I-22), **LCMS:** m/z (ESI), 362.2[M+H]$^+$(I-23).

**Example 24, 25: (*R*)-(*E*)-5$^5$-fluoro-6-methyl-3-oxa-7-aza-1(3,6)-imidazo[1,2-*b*]pyrida zina-2(2,4),5(2,3)-dipyridinacycloheptaphane (I-24), (*S*)-(*E*)-5$^5$-fluoro-6-methyl-3-oxa-7-aza-1(3,6)-imidazo[1,2-*b*]pyridazina-2(2,4),5(2,3)-dipyridinacycloheptaphane (I-25)**

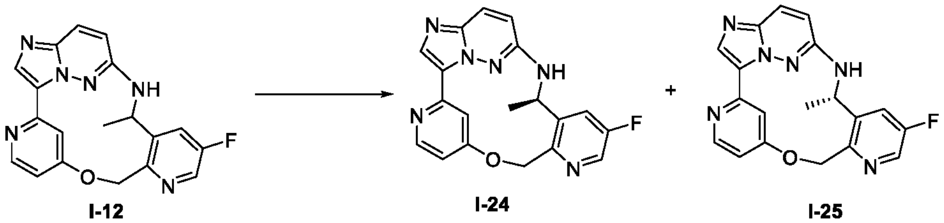

I-12 I-24 I-25

The title Compound **I-24** and **I-25** were prepared by chiral preparative chromatography from Compound **I-12. LCMS:** m/z (ESI), 363.2$[M+H]^+$(I-24), **LCMS:** m/z (ESI), 363.2$[M+H]^+$(I-25).

**Example 26, 27: (*R*)-(*E*)-6-methyl-4-oxa-7-aza-1(3,6)-imidazo[1,2-*b*]pyridazina-2(4,6)-pyrimidina-5(1,2)-benzenacycloheptaphane (I-26), (6*S*,*E*)-6-methyl-4-oxa-7-aza-1(3,6) -imidazo[1,2-*b*]pyridazina-2(4,6)-pyrimidina-5(1,2)-benzenacycloheptaphane (I-27)**

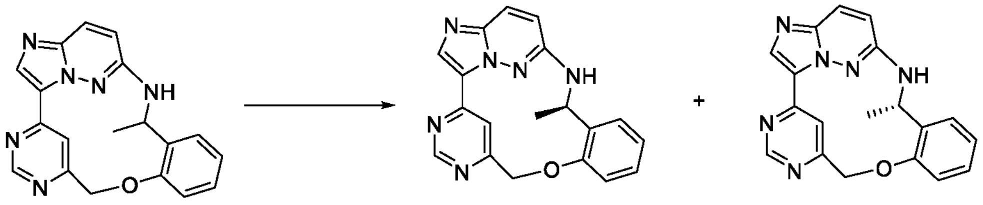

I-10 I-26 I-27

The title Compound **I-26 and I-27** were prepared by chiral preparative chromatography from Compound **I-10. LCMS:** m/z (ESI), 345.2$[M+H]^+$(I-26), **LCMS:** m/z (ESI), 345.2$[M+H]^+$(I-27).

**Example 28, 29: (4*R*,*E*)-4-methyl-6-oxa-3-aza-2(3,6)-imidazo[1,2-*b*]pyridazina-1(2,4)-pyridina-5(1,2)-benzenacyclooctaphane (I-28), (4*S*,*E*)-4-methyl-6-oxa-3-aza-2(3,6)-imidazo [1,2-*b*]pyridazina-1(2,4)-pyridina-5(1,2)-benzenacyclooctaphane (I-29)**

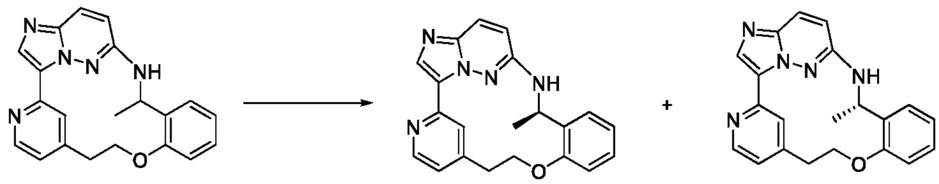

I-9 I-28 I-29

The title Compound **I-28 and I-29** were prepared by chiral preparative chromatography from Compound **I-9. LCMS:** m/z (ESI), 358.0$[M+H]^+$(I-28), **LCMS:** m/z (ESI), 358.0$[M+H]^+$(I-29).

**Example 30, 31: (*R*)-(*E*)-$5^1$-fluoro-6-methyl-3-oxa-7-aza-1(3,6)-imidazo[1,2-*b*]pyrida zina-2(2,4)-pyridina-5(1,2)-benzenacycloheptaphane (I-30), (*S*)-(*E*)-$5^4$-fluoro-6-methyl-3-oxa -7-aza-1(3,6)-imidazo[1,2-*b*]pyridazina-2(2,4)-pyridina-5(1,2)-benzenacycloheptaphane (I-31)**

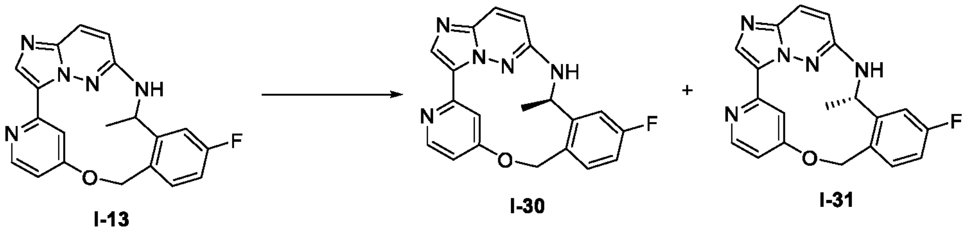

I-13 I-30 I-31

The title Compound **I-30 and I-31** were prepared by chiral preparative chromatography from Compound **I-13. LCMS:** m/z (ESI), 362.0[M+H]+(I-30), **LCMS:** m/z (ESI), 362.0[M+H]+(I-31).

**Example 32, 33: (*R*)-(*E*)-4-methyl-6-oxa-3-aza-2(3,6)-imidazo[1,2-*b*]pyridazina-1(4,6)-pyrimidina-5(1,2)-benzenacyclooctaphane (I-32), (*R*)-(*E*)-4-methyl-6-oxa -3-aza-2(3,6)-imidazo[1,2-*b*]pyridazina-1(4,6)-pyrimidina-5(1,2)-benzenacyclooctaphane (I-33)**

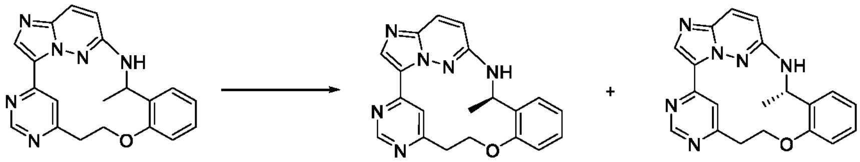

I-11 I-32 I-33

The title Compound **I-32 and I-33** were prepared by chiral preparative chromatography from Compound **I-11. LCMS:** m/z (ESI), 359.0[M+H]+(I-32), **LCMS:** m/z (ESI), 359.0[M+H]+(I-33).

**Example 34: (4*R*,7*R*,*E*)-5$^5$-4,7-dimethyl-6-oxa-3-aza-2(3,6)-imidazo[1,2-*b*]pyri dazina-1(2,4)-pyridina-5(1,2)-benzenacyclooctaphane (I-34)**

**Step A: (1R)-1-(5-fluoro-2-methoxyphenyl)ethan-1-amine (71)**

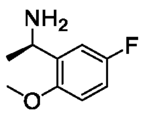

71

The title Compound **71** was prepared by the method in the reference of Journal of Medicinal Chemistry(1974),17(7), 708-15. **LCMS:** m/z (ESI), 170[M+H]+.

**Step B: (2*S*)-1-(2-bromopyridin-4-yl)propan-2-ol (73)**

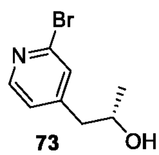

73

The title Compound **73** was prepared by the method in the reference of Tetrahedron Asymmetry,(2006),17(2),268-274. **LCMS:** m/z (ESI), 216.2[M+H]+.

**Step C: (*S*)-2-bromo-4-(2-((tert-butyldimethylsilyl)oxy)propyl)pyridine (74)**

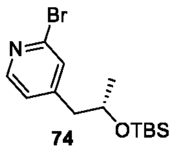

**74**

The title Compound **74** was prepared with reference to the method of Compound **35** by replacing Compound **34** with Compound **73** as the raw material. **LCMS:** m/z (ESI), 332.2[M+H]$^+$.

**Step D: (*S*)-4-(2-((tert-butyldimethylsilyl)oxy)propyl)-2-(tributylstannyl)pyridine (75)**

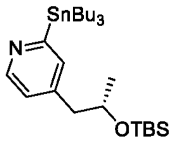

**75**

The title Compound **75** was prepared with reference to the method of Compound **36** by replacing Compound **35** with Compound **74** as the raw material. **LCMS:** m/z (ESI), 542.2[M+H]$^+$.

**Step E: (S)-3-(4-(2-((tert-butyldimethylsilyl)oxy)propyl)pyridin-2-yl)-6-chloroimidazo [1,2-*b*]pyridazine (76)**

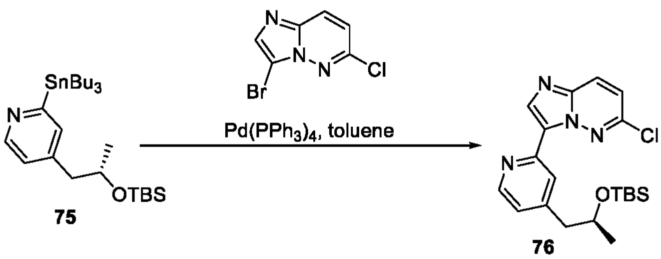


**75** **76**

The title Compound **76** was prepared with reference to the method of Compound **37** by replacing Compound **36** with Compound **75** as the raw material. **LCMS:** m/z (ESI), 403.2[M+H]$^+$.

**Step F: (*S*)-1-(2-(6-(((*R*)-1-(5-fluoro-2-methoxyphenyl)ethyl)amino)imidazo[1,2-*b*] pyridazin-3-yl)pyridin-4-yl) propan-2-ol (77)**

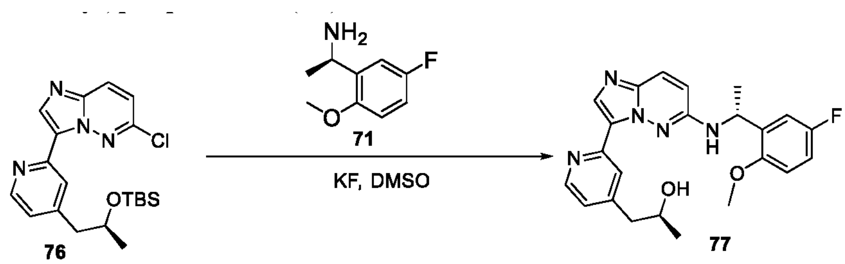


**76** **71** **77**

The title Compound **77** was prepared with reference to the method of Compound **38** by replacing Compound **37**

with Compound **76** and Compound **19** with Compound **71** as the raw material. **LCMS:** m/z (ESI), 422.1[M+H]+.

**Step G: (*S*)-1-(2-(6-(((*R*)-1-(2-hydroxy-5-fluoro-phenyl)ethyl)amino)imidazo[1,2-b] pyridazin-3-yl)pyridin-4-yl) propan-2-ol (78)**

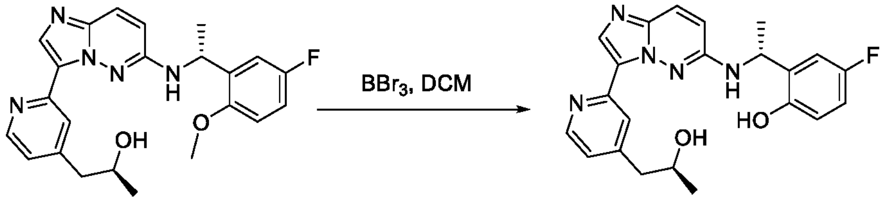


77 78

To a solution of (***S***)-1-(2-(6-(((***R***)-1-(5-fluoro-2-methoxyphenyl)ethyl)amino)imidazo[1,2-*b*] pyridazin-3-yl)pyridin-4-yl)propan-2-ol (Comound **77**, 74 mg, 0.183 mmol) in DCM (5 mL) was added $BBr_3$ (1 M, 0.5 mL). The mixture was stirred at 25 °C for 2 h. The mixture was adjusted to pH = 7 with $NaHCO_3$, filtered and the filtrate was concentrated. The residue was purified by chromatography column to give 4-fluoro-2-((***R***)-1-((3-(4-((S)-2-hydroxypropyl)pyridin-2-yl) imidazo[1,2-***b***]pyridazin-6-yl)amino)ethyl)phenol (25 mg, 35%). **LCMS:** m/z (ESI), 408.1[M+H]+.

**Step H: (4*R*,7*R*,*E*)-5$^5$-4,7-dimethyl-6-oxa-3-aza-2(3,6)-imidazo[1,2-*b*]pyri dazina-1(2,4)-pyridina-5(1,2)-benzenacyclooctaphane (I-34)**

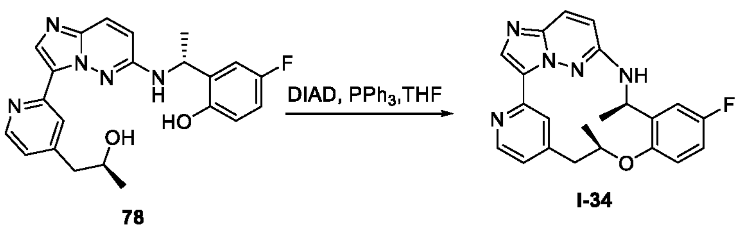


78 I-34

The title Compound **I-34** was prepared with reference to the method of Compound **I-9** by replacing Compound **39** with Compound **78** as the raw material. **LCMS:** m/z (ESI), 390[M+H]+.

**Example 35: (4*R*,7*R*,*E*)-5$^5$-4,7-dimethyl-6-oxa-3-aza-2(3,6)-imidazo[1,2-*b*] pyridazina-1(4,6)-pyrimidina-5(1,2)-benzenacyclooctaphane (I-35)**

**Step A: (*S*)-1-(6-chloropyrimidin-4-yl)propan-2-ol (79)**

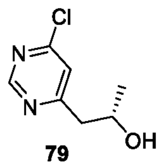

79

The title Compound **79** was prepared by the method in the reference of Tetrahedron Asymmetry,(2006),17(2),268-274. **LCMS:** m/z (ESI), 173.2[M+H]+.

**Step B: (*S*)-4-(2-((tert-butyldimethylsilyl)oxy)propyl)-6-chloropyrimidine (80)**

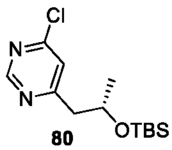

80

The title Compound **80** was prepared with reference to the method of Compound **74** by replacing Compound **73** with Compound **79** as the raw material. **LCMS:** m/z (ESI), 287.0[M+H]$^+$.

**Step C: (*S*)-4-(2-((tert-butyldimethylsilyl)oxy)propyl)-6-(tributylstannyl)pyrimidine (81)**

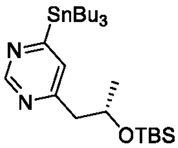

81

The title Compound **81** was prepared with reference to the method of Compound **75** by replacing Compound **74** with Compound **80** as the raw material. **LCMS:** m/z (ESI), 543.2[M+H]$^+$.

**Step D: (*S*)-3-(6-(2-((tert-butyldimethylsilyl)oxy)propyl)pyrimidin-4-yl)-6-chloroimidazo [1,2-*b*]pyridazine (82)**

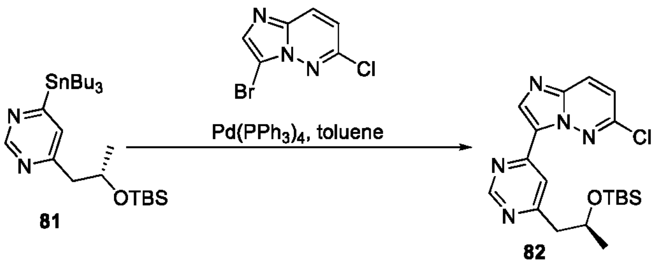


81

82

The title Compound **82** was prepared with reference to the method of Compound **76** by replacing Compound **75** with Compound **81** as the raw material. **LCMS:** m/z (ESI), 404.2[M+H]$^+$.

**Step E: (S)-1-(6-(6-(((R)-1-(5-fluoro-2-methoxyphenyl)ethyl)amino)imidazo[1,2-*b*] pyridazin-3-yl)pyrimidin-4-yl) propan-2-ol (82-1)**

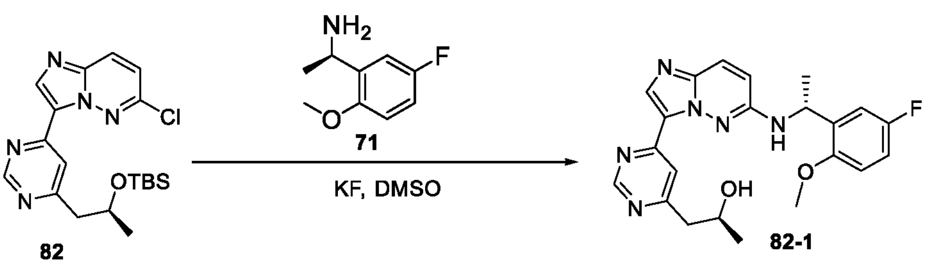


71

82

82-1

The title Compound **82-1** was prepared with reference to the method of Compound **77** by replacing Compound 76 with Compound **82** as the raw material. **LCMS:** m/z (ESI), 423.1[M+H]$^+$.

**Step F: (*S*)-1-(6-(6-(((R)-1-(2-hydroxy-5-fluoro-phenyl)ethyl)amino)imidazo[1,2-*b*] pyridazin-3-yl) pyrimidin-4-yl) propan-2-ol (82-2)**

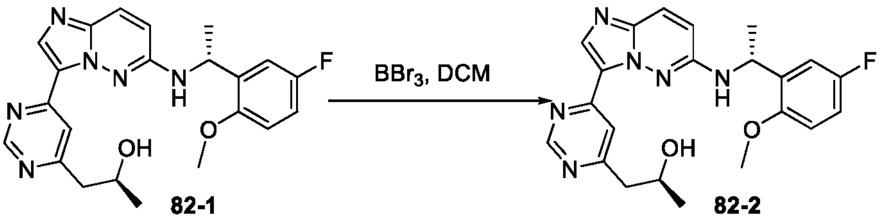

82-1
82-2

The title Compound **82-2** was prepared with reference to the method of Compound **78** by replacing Compound **77** with Compound **82-1** as the raw material. **LCMS:** m/z (ESI), 409. 1 [M+H]+.

**Step G: (4*R*,7*R*,*E*)-5⁵--4,7-dimethyl-6-oxa-3-aza-2(3,6)-imidazo[1,2-*b*] pyridazina-1(4,6)-pyrimidina-5(1,2)-benzenacyclooctaphane (I-35)**

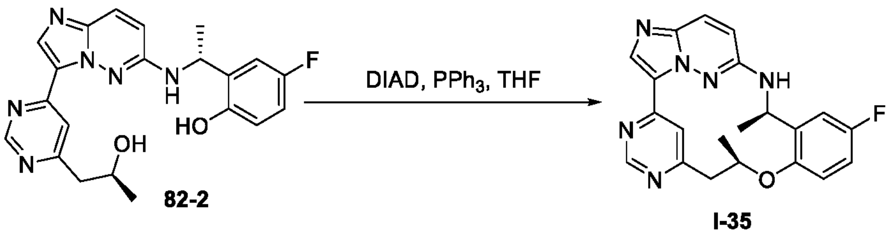

82-2
I-35

The title Compound **I-35** was prepared with reference to the method of Compound **I-9** by replacing Compound **39** with Compound **82-2** as the raw material. **LCMS:** m/z (ESI), 391[M+H]+.

**Example 36: (4*R*,7*R*,*E*)-5⁵--4,7-dimethyl-6-oxa-3-aza-2(3,6)-imidazo[1,2-*b*] pyridazina-1(2,4),5(3,2)-dipyridinacyclooctaphane (I-36)**

**Step A: (*S*)-1-(2-(6-(((R)-1-(5-fluoro-2-methoxypyridin-3-yl)ethyl)amino)imidazo [1,2-b]pyridazin-3-yl)pyridin-4-yl)propan-2-ol (76-1)**

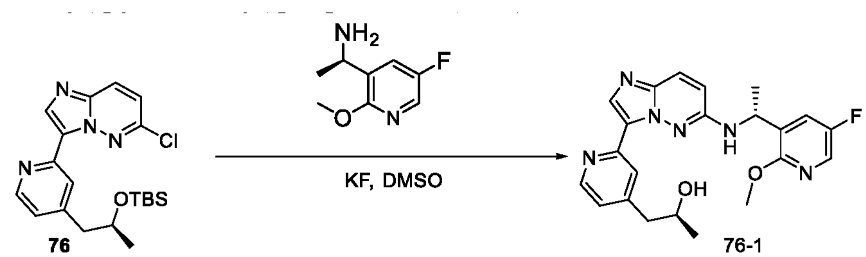

76
76-1

The title Compound **76-1** was prepared with reference to the method of Compound **77** by replacing Compound **71** with (1***R***)-1-(5-fluoro-2-methoxypyridin-3-yl)ethan-1-amine as the raw material. **LCMS:** m/z (ESI), 423.1[M+H]+.

**Step B: (*S*)-1-(2-(6-(((*R*)-1-(5-fluoro-2-methoxypyridin-3-yl)ethyl)amino)imidazo[1,2-*b*] pyridazin-3-yl)pyridin-4-yl)propan-2-ol (76-2)**

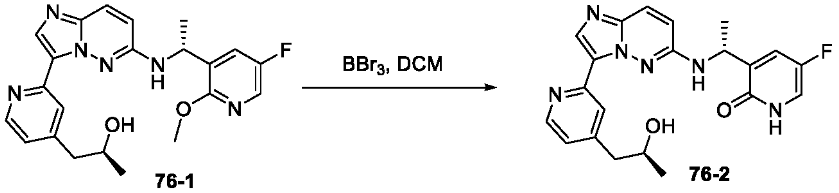


76-1 76-2

The title Compound **76-2** was prepared with reference to the method of Compound **78** by replacing Compound **77** with Compound **76-1** as the raw material. **LCMS:** m/z (ESI), 409. 1 [M+H]$^+$.

**Step C: (4*R*,7*R*,*E*)-5$^5$-4,7-dimethyl-6-oxa-3-aza-2(3,6)-imidazo[1,2-*b*] pyridazina-1(2,4),5(3,2)-dipyridinacyclooctaphane (I-36)**

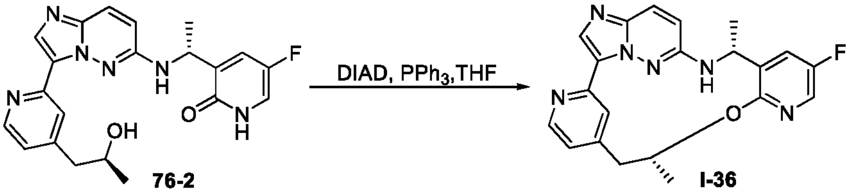


76-2 I-36

The title Compound **I-36** was prepared with reference to the method of Compound **I-9** by replacing Compound **39** with Compound **76-2** as the raw material. **LCMS:** m/z (ESI), 391[M+H]$^+$.

**Example 37: (3$^2$*R*,6*R*,*E*)-4$^5$-fluoro-6-methyl-5-oxa-2(3,6)-imidazo[1,2-*b*]pyridazina-1(2,4)-pyridina-3(1,2)-pyrrolidina-4(1,2)-benzenacycloheptaphane (I-37)**

**Step A: (*S*)-1-(2-(6-((*R*)-2-(5-fluoro-2-methoxyphenyl)pyrrolidin-1-yl)imidazo[1,2-b] pyridazin-3-yl)pyridin-4-yl) propan-2-ol (76-3)**

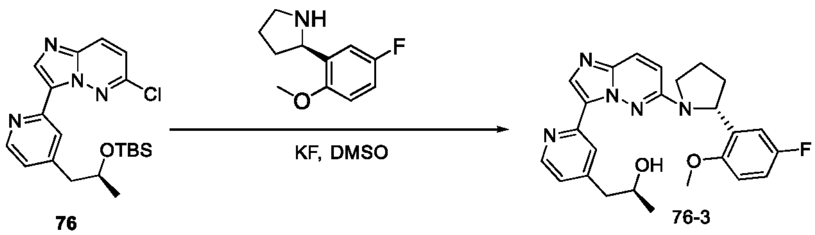


76 76-3

The title Compound **76-3** was prepared with reference to the method of Compound **77** by replacing Compound **71** with (***R***)-2-(5-fluoro-2-methoxyphenyl)pyrrolidine as the raw material. **LCMS:** m/z (ESI), 448.1[M+H]$^+$.

**Step B: 4-fluoro-2-((*R*)-1-(3-(4-((S)-2-hydroxypropyl)pyridin-2-yl)imidazo[1,2-*b*] pyridazin-6-yl)pyrrolidin-2-yl) phenol (76-4)**

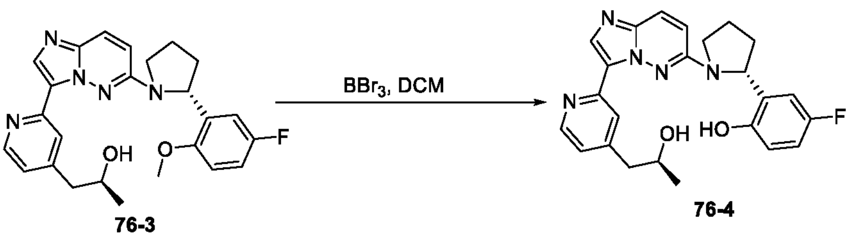


76-3 76-4

The title Compound **76-4** was prepared with reference to the method of Compound **78** by replacing Compound **77** with Compound **76-3** as the raw material. **LCMS:** m/z (ESI), 434.1[M+H]+.

**Step C: (3²*R*,6*R*,*E*)-4⁵-fluoro-6-methyl-5-oxa-2(3,6)-imidazo[1,2-*b*]pyridazina-1(2,4)-pyridina-3(1,2)-pyrrolidina-4(1,2)-benzenacycloheptaphane (I-37)**

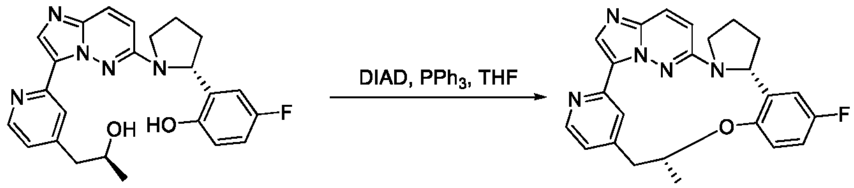


**76-4** **I-37**

The title Compound **I-37** was prepared with reference to the method of Compound **I-9** by replacing Compound **39** with Compound **76-4** as the raw material. **LCMS:** m/z (ESI), 416[M+H]+.

**Example 38: (3²*R*,6*R*,*E*)-4⁵-fluoro-6-methyl-5-oxa-2(3,6)-imidazo[1,2-*b*]pyridazina -1(2,4),4(3,2)-dipyridina-3(1,2)-pyrrolidinacycloheptaphane (I-38)**

**Step A: (*S*)-1-(2-(6-((*R*)-2-(5-fluoro-2-methoxypyridin-3-yl)pyrrolidin-1-yl)imidazo [1,2-b]pyridazin-3-yl)pyridin-4-yl)propan-2-ol (76-5)**

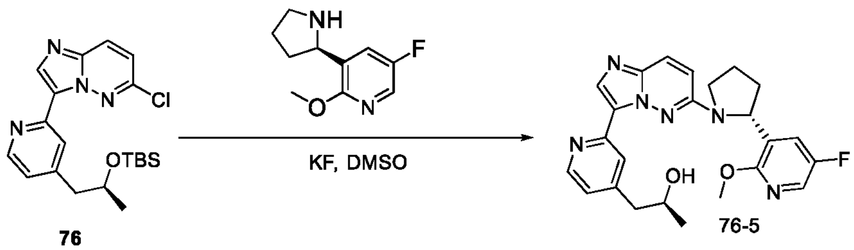


**76** 76-5

The title Compound **76-5** was prepared with reference to the method of Compound **77** by replacing Compound **71** with (***R***)-5-fluoro-2-methoxy-3-(pyrrolidin-2-yl)pyridine as the raw material. **LCMS:** m/z (ESI), 449.1 [M+H]+.

**Step B: 5-fluoro-3-((*R*)-1-(3-(4-((S*)*-2-hydroxypropyl)pyridin-2-yl)imidazo[1,2-*b*] pyridazin-6-yl)pyrrolidin-2-yl) pyridin-2-ol (76-6)**

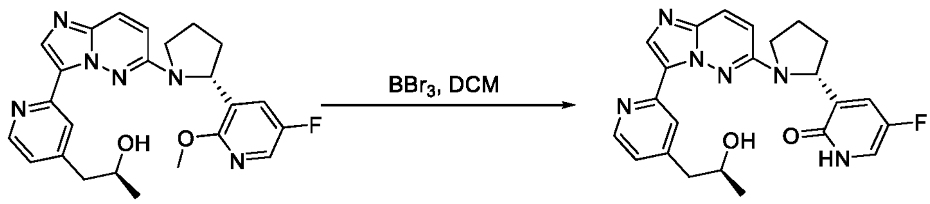


**76-5** **76-6**

The title Compound **76-6** was prepared with reference to the method of Compound **78** by replacing Compound **77** with Compound **76-5** as the raw material. **LCMS:** m/z (ESI), 435.1[M+H]+.

**Step C: ($3^2$*R*,6*R*,*E*)-$4^5$-fluoro-6-methyl-5-oxa-2(3,6)-imidazo[1,2-*b*]pyridazina-1(2,4), 4(3,2)-dipyridina-3(1,2)-pyrrolidinacycloheptaphane (I-38)**

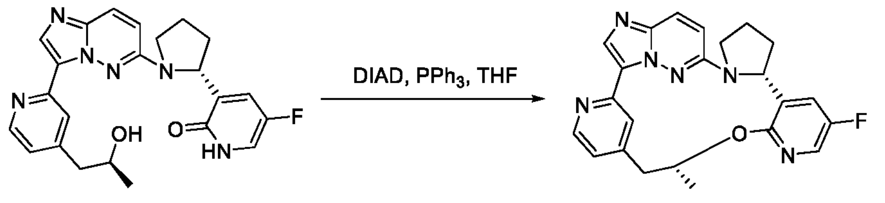


**76-6** **I-38**

The title Compound **I-38** was prepared with reference to the method of Compound **I-9** by replacing Compound **39** with Compound **76-6** as the raw material. **LCMS:** m/z (ESI), 417[M+H]$^+$.

**Example 39: ($3^2$*R*,$3^4$*S*,6*R*,*E*)-$3^4$,$4^5$-difluoro-6-methyl-5-oxa-2(3,6)-imidazo[1,2-*b*] pyridazina-1(2,4)-pyridina-3(1,2)-pyrrolidina-4(1,2)-benzenacycloheptaphane (I-39)**

**Step A: (*S*)-1-(2-(6-((2*R*,4*S*)-4-fluoro-2-(5-fluoro-2-methoxyphenyl)pyrrolidin-1-yl) imidazo[1,2-b]pyridazin-3-yl) pyridin-4-yl)propan-2-ol (76-7)**

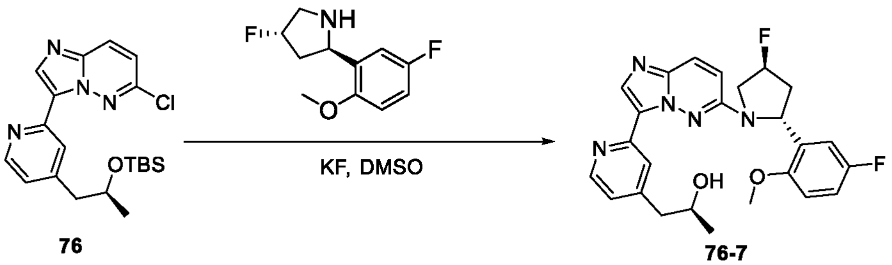


**76** **76-7**

The title Compound **76-7** was prepared with reference to the method of Compound **77** by replacing Compound **71** with (2***R***,4***S***)-4-fluoro-2-(5-fluoro-2-methoxyphenyl)pyrrolidine as the raw material. **LCMS:** m/z (ESI), 466.1 [M+H]$^+$.

**Step B: 4-fluoro-2-((2*R*,4*S*)-4-fluoro-1-(3-(4-((*S*)-2-hydroxypropyl)pyridin-2-yl)imidazo [1,2-*b*]pyridazin-6-yl)pyrrolidin-2-yl)phenol (76-8)**

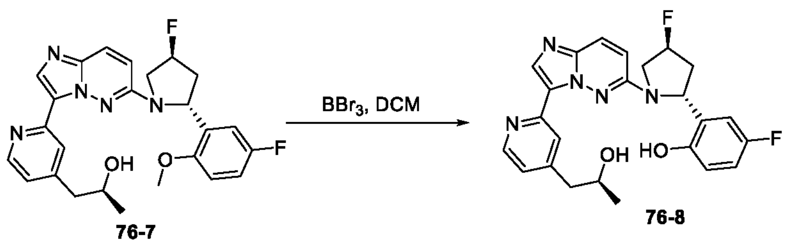


**76-7** **76-8**

The title Compound **76-8** was prepared with reference to the method of Compound **78** by replacing Compound **77** with Compound **76-7** as the raw material. **LCMS:** m/z (ESI), 452. 1 [M+H]$^+$.

**Step C: ($3^2$*R*,$3^4$*S*,6*R*,*E*)-$3^4$,$4^5$-difluoro-6-methyl-5-oxa-2(3,6)-imidazo[1,2-*b*]pyridazina -1(2,4)-pyridina-3(1,2)-pyrrolidina-4(1,2)-benzenacycloheptaphane(I-39)**

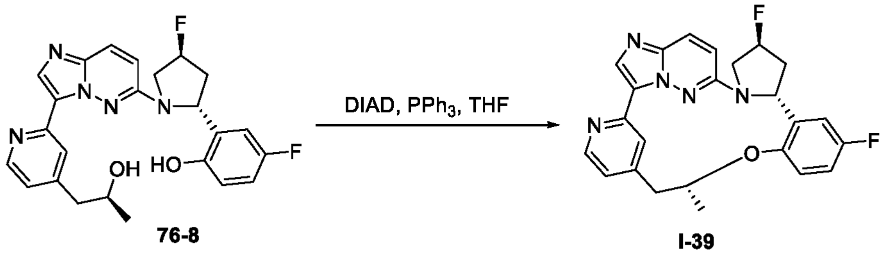


76-8

I-39

The title Compound **I-39** was prepared with reference to the method of Compound **I-9** by replacing Compound **39** with Compound **76-8** as the raw material. **LCMS:** m/z (ESI), 434[M+H]+.

**Example 40: *(*3$^2$*R*,3$^4$*S*,6*R*,*E*)-3$^4$,4$^5$-difluoro-6-methyl-5-oxa-2(3,6)-imidazo[1,2-*b*] pyridazina-1(2,4),4(3,2)-dipyridina-3(1,2)-pyrrolidinacycloheptaphane (I-40)**

**Step A: (*S*)-1-(2-(6-((2*R*,4*S*)-4-fluoro-2-(5-fluoro-2-methoxypyridin-3-yl)pyrrolidine -1-yl)imidazo[1,2-b]pyridazin-3-yl)pyridin-4-yl)propan-2-ol (76-9)**

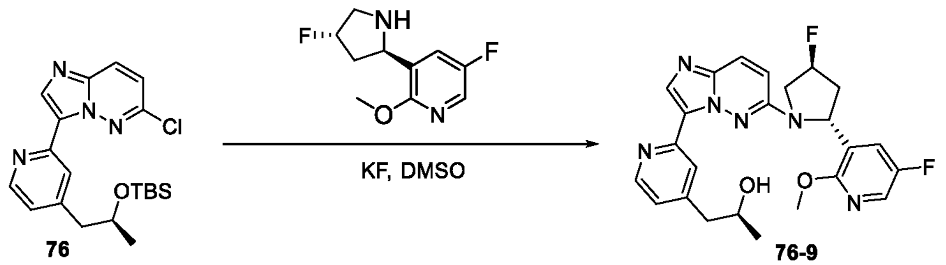


76

76-9

The title Compound **76-9** was prepared with reference to the method of Compound **77** by replacing Compound **71** with 5-fluoro-3-((2***R***,4***S***)-4-fluoropyrrolidin-2-yl)-2-methoxypyridine as the raw material. **LCMS:** m/z (ESI), 467.1[M+H]+.

**Step B: 5-fluoro-3-((2*R*,4*S*)-4-fluoro-1-(3-(4-((*S*)-2-hydroxypropyl)pyridin-2-yl)imidazo [1,2-b]pyridazin-6-yl)pyrrolidin-2-yl)pyridin-2-ol (76-10)**

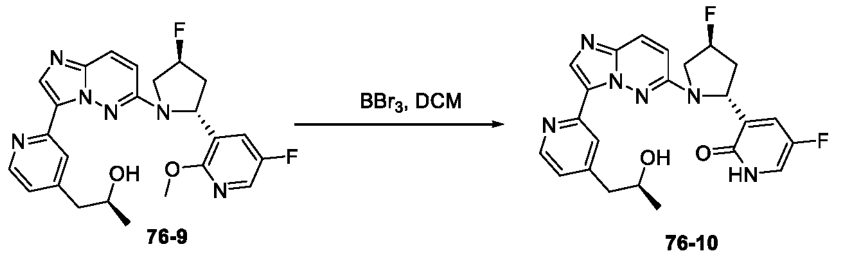


76-9

76-10

The title Compound **76-10** was prepared with reference to the method of Compound **78** by replacing Compound **77** with Compound **76-9** as the raw material. **LCMS:** m/z (ESI), 453.1[M+H]+.

**Step C: (3$^2$*R*,3$^4$*S*,6*R*,*E*)-3$^4$,4$^5$-difluoro-6-methyl-5-oxa-2(3,6)-imidazo[1,2-*b*]pyridazina -1(2,4),4(3,2)-dipyridina-3(1,2)-pyrrolidinacycloheptaphane (I-40)**

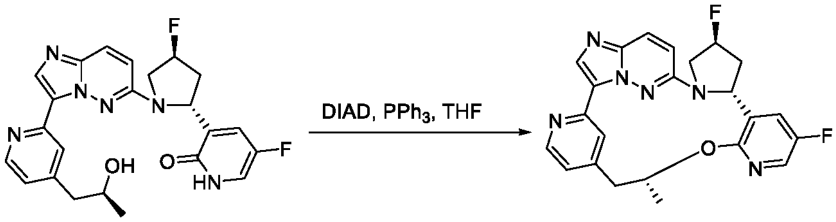


76-10 I-40

The title Compound **I-40** was prepared with reference to the method of Compound **I-9** by replacing Compound **39** with Compound **76-10** as the raw material. **LCMS:** m/z (ESI), 435[M+H]+.

**Example 41: (3$^3$*R*,6*R*,*E*)-5$^4$-fluoro-6-methyl-4-oxa-7-aza-1(3,6)-imidazo[1,2-*b*] pyridazina-2(2,4)-pyridina-3(1,3)-pyrrolidina-5(1,2)-benzenacycloheptaphane(I-41)**

**Step A: (*S*)-2-bromo-4-(3-((tert-butyldimethylsilyl)oxy)pyrrolidin-1-yl)pyridine (83-1)**

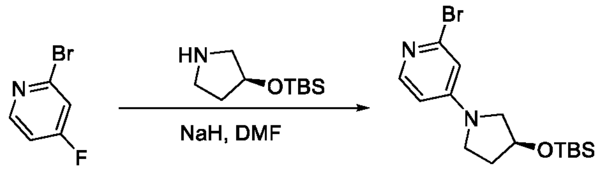


49 83-1

The title Compound **83-1** was prepared with reference to the method of Compound **50** by replacing 4-methoxy benzyl alcohol with (S)-3-((tert-butyldimethylsilyl)oxy)pyrrolidine as the raw material. **LCMS:** m/z (ESI), 359.1[M+H]+.

**Step B: (*S*)-3-(4-(3-((tert-butyldimethylsilyl)oxy)pyrrolidin-1-yl)pyridin-2-yl)-6-chloro imidazo[1,2-b]pyridazine (83)**

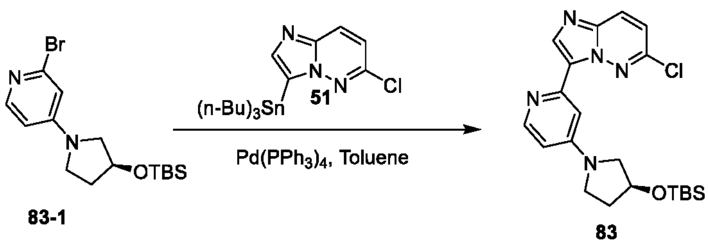


83-1 83

The title Compound **83** was prepared with reference to the method of Compound **52** by replacing Compound **50** with Compound **83-1** as the raw material. **LCMS:** m/z (ESI), 430.1[M+H]+.

**Step C: (*S*)-1-(2-(6-(((*R*)-1-(5-fluoro-2-methoxyphenyl)ethyl)amino)imidazo[1,2-*b*] pyridazin-3-yl)pyridin-4-yl) pyrrolidin-3-ol (83-2)**

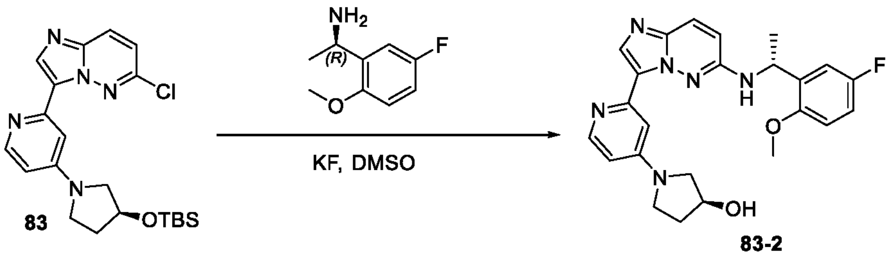


83

83-2

The title Compound **83-2** was prepared with reference to the method of Compound **77** by replacing Compound **76** with Compound **83** as the raw material. **LCMS:** m/z (ESI), 449.1 $[M+H]^+$.

**Step D: (*S*)-1-(2-(6-(((*R*)-1-(5-fluoro-2-hydroxyphenyl)ethyl)amino)imidazo[1,2-*b*] pyridazin-3-yl)pyridin-4-yl) pyrrolidin-3-ol (83-3)**

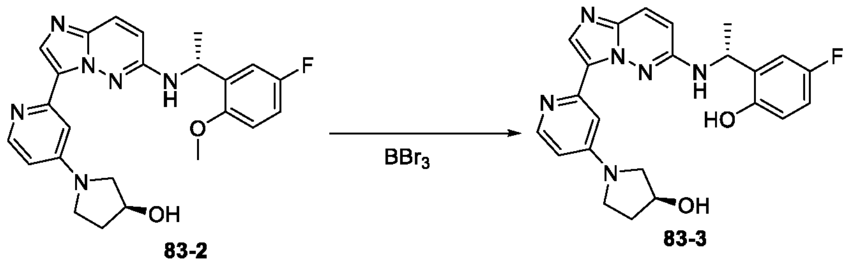


83-2

83-3

The title Compound **83-3** was prepared with reference to the method of Compound **78** by replacing Compound **77** with Compound **83-2** as the raw material. **LCMS:** m/z (ESI), 435.1 $[M+H]^+$.

**Step E: ($3^3$*R*,6*R*,*E*)-$5^4$-fluoro-6-methyl-4-oxa-7-aza-1(3,6)-imidazo[1,2-*b*]pyridazina-2(2,4)-pyridina-3(1,3)-pyrrolidina-5(1,2)-benzenacycloheptaphane(I-41)**

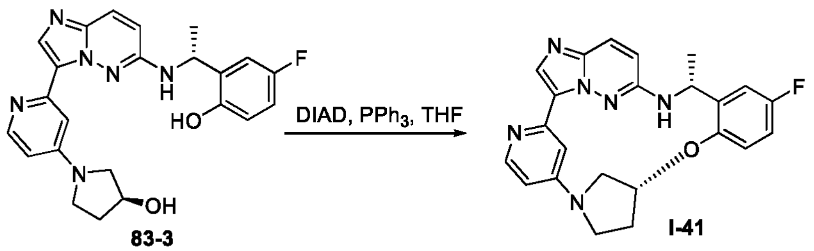


83-3

I-41

The title Compound **I-41** was prepared with reference to the method of Compound **I-9** by replacing Compound **39** with Compound **83-3** as the raw material. **LCMS:** m/z (ESI), 417$[M+H]^+$.

**Example 42: ($3^3$*S*,6*R*,*E*)-$5^4$-fluoro-6-methyl-4-oxa-7-aza-1(3,6)-imidazo[1,2-*b*] pyridazina-2(2,4)-pyridina-3(1,3)-pyrrolidina-5(1,2)-benzenacycloheptaphane (I-42)**

**Step A: (*R*)-2-bromo-4-(3-((tert-butyldimethylsilyl)oxy)pyrrolidin-1-yl)pyridine (84-1)**

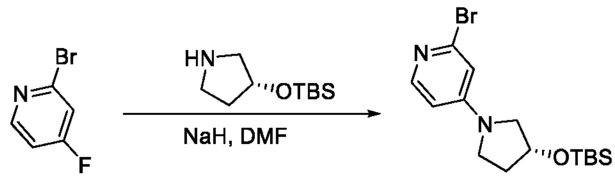


49 84-1

The title Compound **84-1** was prepared with reference to the method of Compound **50** by replacing 4-methoxy benzyl alcohol with (***R***)-3-((tert-butyldimethylsilyl)oxy)pyrrolidine as the raw material. **LCMS:** m/z (ESI), 359.1[M+H]+.

**Step B: (*R*)-3-(4-(3-((tert-butyldimethylsilyl)oxy)pyrrolidin-1-yl)pyridin-2-yl)-6-chloro imidazo[1,2-b]pyridazine (84)**

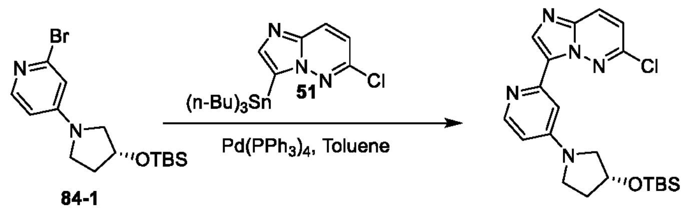


84-1 84

The title Compound **84** was prepared with reference to the method of Compound **52** by replacing Compound **50** with Compound **84-1** as the raw material. **LCMS:** m/z (ESI), 430.1[M+H]+.

**Step C: (*R*)-1-(2-(6-(((*R*)-1-(5-fluoro-2-methoxyphenyl)ethyl)amino)imidazo[1,2-*b*] pyridazin-3-yl)pyridin-4-yl) pyrrolidin-3-ol (84-2)**

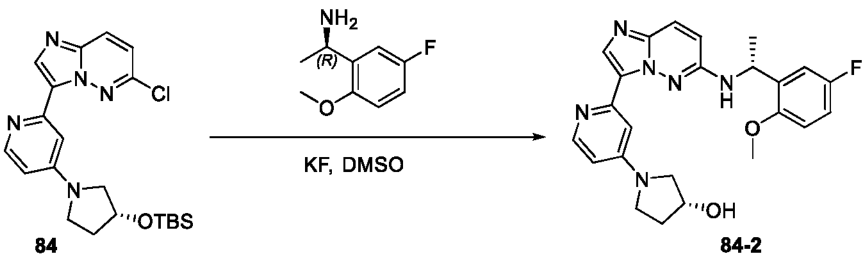


84 84-2

The title Compound **84-2** was prepared with reference to the method of Compound **77** by replacing Compound **76** with Compound **84** as the raw material. **LCMS:** m/z (ESI), 449.1 [M+H]+.

**Step D: (*R*)-1-(2-(6-(((*R*)-1-(5-fluoro-2-hydroxyphenyl)ethyl)amino)imidazo[1,2-*b*] pyridazin-3-yl)pyridin-4-yl) pyrrolidin-3-ol (84-3)**

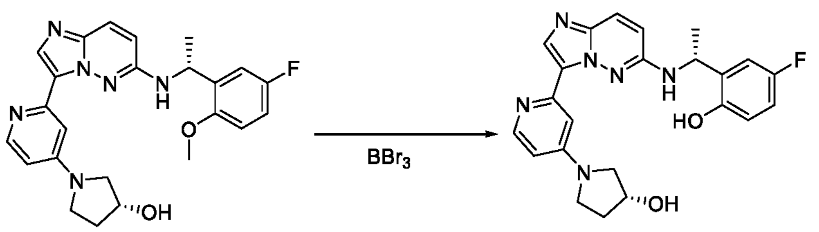


84-2 84-3

The title Compound **84-3** was prepared with reference to the method of Compound **78** by replacing Compound **77** with Compound **84-2** as the raw material. **LCMS:** m/z (ESI), 435.1 [M+H]$^+$.

**Step E: ($3^3$S,6R,E)-$5^4$-fluoro-6-methyl-4-oxa-7-aza-1(3,6)-imidazo[1,2-b]pyridazina-2(2,4)-pyridina-3(1,3)-pyrrolidina-5(1,2)-benzenacycloheptaphane(I-42)**

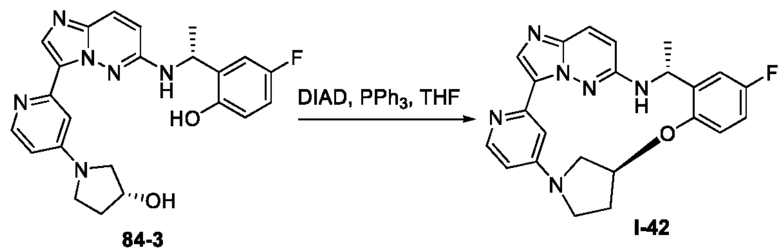


84-3 I-42

The title Compound **I-42** was prepared with reference to the method of Compound **I-9** by replacing Compound **39** with Compound **84-3** as the raw material. **LCMS:** m/z (ESI), 417[M+H]$^+$.

**Example 43: ($3^3$*R*,6*R*,*E*)-$5^4$-fluoro-6-methyl-4-oxa-7-aza-1(3,6)-imidazo[1,2-*b*] pyridazina-2(2,4)-pyridina-3(1,3)-piperidina-5(1,2)-benzenacycloheptaphane(I-43)**

**Step A: (S)-2-bromo-4-(3-((tert-butyldimethylsilyl)oxy)piperidin-1-yl)pyridine (87-1)**

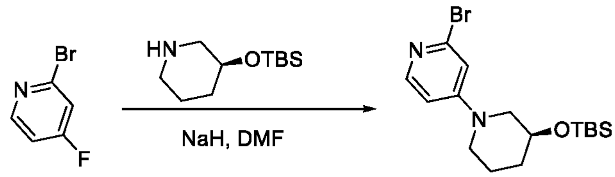


49 87-1

The title Compound **87-1** was prepared with reference to the method of Compound **50** by replacing 4-methoxy benzyl alcohol with (***S***)-3-((tert-butyldimethylsilyl)oxy)piperidine as the raw material. **LCMS:** m/z (ESI), 371.1[M+H]$^+$.

**Step B: (*S*)-3-(4-(3-((tert-butyldimethylsilyl)oxy)piperidin-1-yl)pyridin-2-yl)-6-chloro imidazo[1,2-*b*]pyridazine (87)**

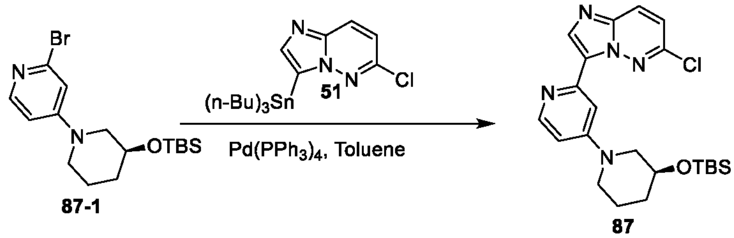


87-1

87

The title Compound **87** was prepared with reference to the method of Compound **52** by replacing Compound **50** with Compound **87-1** as the raw material. **LCMS:** m/z (ESI), 444. 1 [M+H]$^+$.

**Step C: (*S*)-1-(2-(6-(((*R*)-1-(5-fluoro-2-methoxyphenyl)ethyl)amino)imidazo[1,2-*b*] pyridazin-3-yl)pyridin-4-yl)piperidin-3-ol (87-2)**

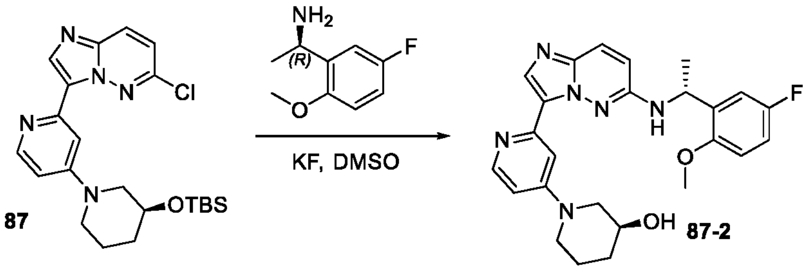


87

87-2

The title Compound **87-2** was prepared with reference to the method of Compound **77** by replacing Compound **76** with Compound **87** as the raw material. **LCMS:** m/z (ESI), 463.1[M+H]$^+$.

**Step D: (*S*)-1-(2-(6-(((*R*)-1-(5-fluoro-2-hydroxyphenyl)ethyl)amino)imidazo[1,2-*b*] pyridazin-3-yl)pyridin-4-yl)piperidin-3-ol (87-3)**

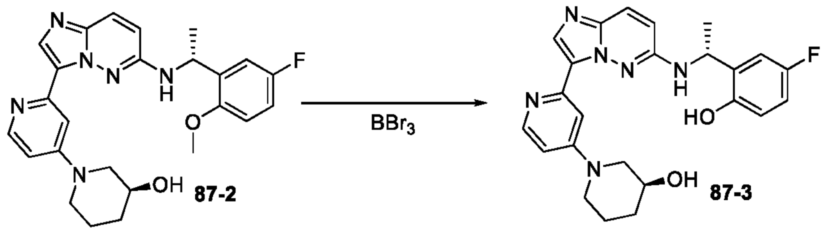


87-2

87-3

The title Compound **87-3** was prepared with reference to the method of Compound **78** by replacing Compound **77** with Compound **87-2** as the raw material. **LCMS:** m/z (ESI), 449.0[M+H]$^+$.

**Step E: (3$^3$*R*,6*R*,*E*)-5$^4$-fluoro-6-methyl-4-oxa-7-aza-1(3,6)-imidazo[1,2-*b*]pyridazina-2(2,4)-pyridina-3(1,3)-piperidina-5(1,2)-benzenacycloheptaphane(I-43)**

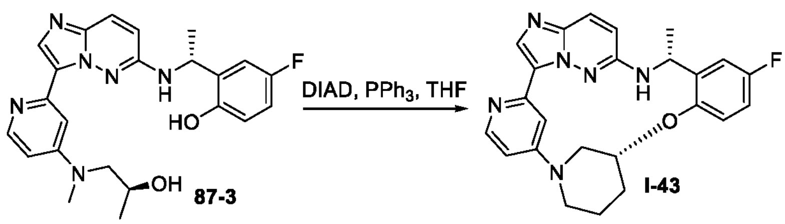


87-3

I-43

The title Compound **I-43** was prepared with reference to the method of Compound **I-9** by replacing Compound **39** with Compound **87-3** as the raw material. **LCMS:** m/z (ESI), 431 $[M+H]^+$.

**Example 44: ($3^3$*S*,6*R*,*E*)-$5^4$-fluoro-6-methyl-4-oxa-7-aza-1(3,6)-imidazo[1,2-*b*] pyridazina-2(2,4)-pyridina-3(1,3)-piperidina-5(1,2)-benzenacycloheptaphane(I-44)**

**Step A: (*R*)-2-bromo-4-(3-((tert-butyldimethylsilyl)oxy)piperidin-1-yl)pyridine (88-1)**

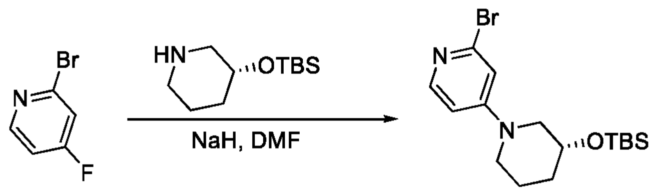


**49** **88-1**

The title Compound **88-1** was prepared with reference to the method of Compound **50** by replacing 4-methoxy benzyl alcohol with (***R***)-3-((tert-butyldimethylsilyl)oxy)piperidine as the raw material. **LCMS:** m/z (ESI), 371.1$[M+H]^+$.

**Step B: (R)-3-(4-(3-((tert-butyldimethylsilyl)oxy)piperidin-1-yl)pyridin-2-yl)-6-chloro imidazo[1,2-*b*]pyridazine (88)**

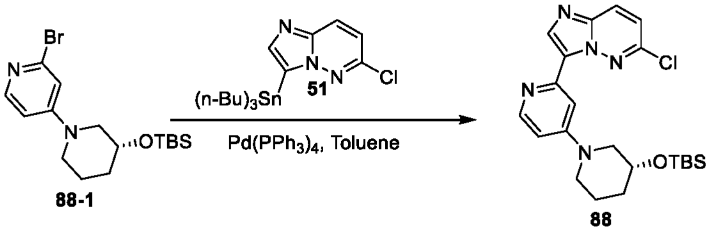


**88-1** **88**

The title Compound **88** was prepared with reference to the method of Compound **52** by replacing Compound **50** with Compound **88-1** as the raw material. **LCMS:** m/z (ESI), 444. 1 $[M+H]^+$.

**Step C: (*R*)-1-(2-(6-(((*R*)-1-(5-fluoro-2-methoxyphenyl)ethyl)amino)imidazo[1,2-*b*] pyridazin-3-yl)pyridin-4-yl)piperidin-3-ol (88-2)**

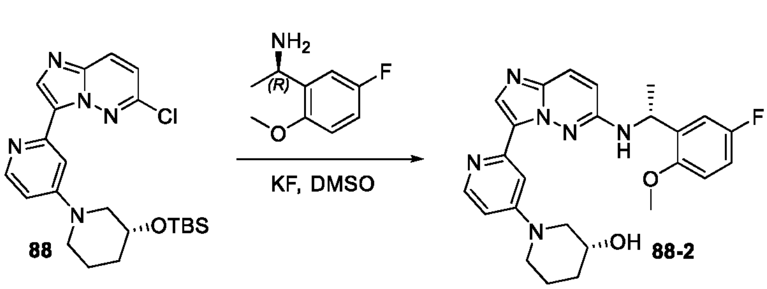


**88** **88-2**

The title Compound **88-2** was prepared with reference to the method of Compound **77** by replacing Compound **76** with Compound **88** as the raw material. **LCMS:** m/z (ESI), 463.1$[M+H]^+$.

**Step D: (*R*)-1-(2-(6-(((*R*)-1-(5-fluoro-2-hydroxyphenyl)ethyl)amino)imidazo[1,2-*b*] pyridazin-3-yl)pyridin-4-yl)piperidin-3-ol (88-3)**

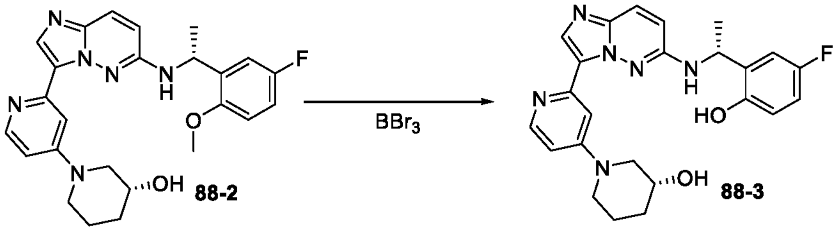


The title Compound **88-3** was prepared with reference to the method of Compound **78** by replacing Compound **77** with Compound **88-2** as the raw material. **LCMS:** m/z (ESI), 449. 1 [M+H]+.

**Step E: ($3^3$*S*,6*R*,*E*)-$5^4$-fluoro-6-methyl-4-oxa-7-aza-1(3,6)-imidazo[1,2-*b*]pyridazina-2(2,4)-pyridina-3(1,3)-piperidina-5(1,2)-benzenacycloheptaphane(I-44)**

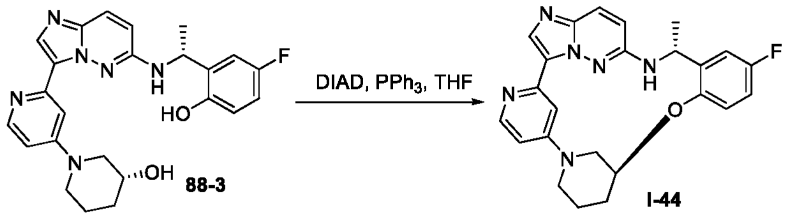


The title Compound **I-44** was prepared with reference to the method of Compound **I-9** by replacing Compound **39** with Compound **88-3** as the raw material. **LCMS:** m/z (ESI), 431[M+H]+.

**Example 45: (8a*R*,11a*R*,16b*R*,*E*)-15-fluoro-8a,10,11,11a,16b,17,18,19-octahydro-9*H*-1,21-etheno-4,8-(metheno) benzo[*b*]cyclopenta[*o*]imidazo[1,5-*h*]pyrrolo[2,1-*d*][1]oxa[5,7,8,11] tetraazacyclohexadecine(I-45)**

**Step A: 2-bromo-4-((1*R*,2*S*)-2-((tert-butyldimethylsilyl)oxy)cyclopentyl)pyridine(103-1)**

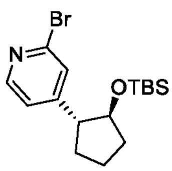

**103-1**

The title Compound **103-1** was prepared by the method in the reference of Tetrahedron Asymmetry, 2004, 15, (3), 481 - 488. **LCMS:** m/z (ESI), 358.1[M+H]+.

**Step B: 3-(4-((1*R*,2*S*)-2-((tert-butyldimethylsilyl)oxy)cyclopentyl)pyridin-2-yl)-6-chloro imidazo[1,2-*b*]pyridazine (103)**

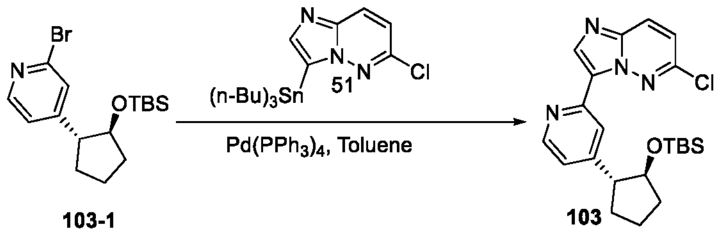


103-1 103

The title Compound **103** was prepared with reference to the method of Compound **52** by replacing Compound **50** with Compound **103-1** as the raw material. **LCMS:** m/z (ESI), 429. 1 [M+H]+.

**Step C: (1*S*,2*R*)-2-(2-(6-((*R*)-2-(5-fluoro-2-methoxyphenyl)pyrrolidin-1-yl)imidazo [1,2-*b*]pyridazin-3-yl)pyridin-4-yl)cyclopentan-1-ol (103-2)**

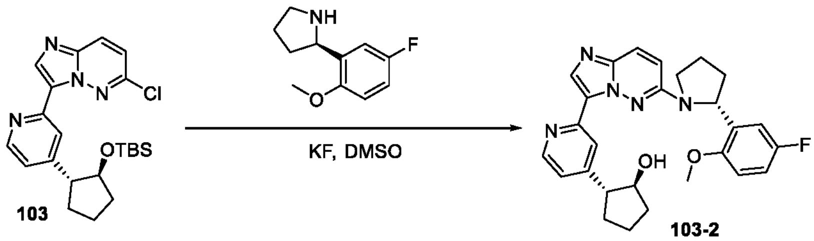


103 103-2

The title Compound **103-2** was prepared with reference to the method of Compound **77** by replacing Compound **76** with Compound **103** as the raw material. **LCMS:** m/z (ESI), 474. 1 [M+H]+.

**Step D: (1*S*,2*R*)-2-(2-(6-((*R*)-2-(5-fluoro-2-hydroxyphenyl)pyrrolidin-1-yl)imidazo [1,2-*b*]pyridazin-3-yl)pyridin-4-yl)cyclopentan-1-ol (103-3)**

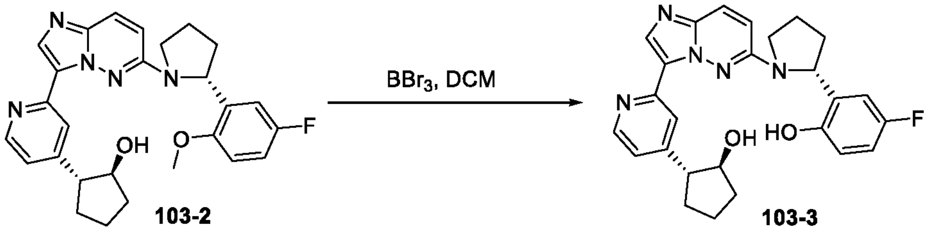


103-2 103-3

The title Compound **103-3** was prepared with reference to the method of Compound **78** by replacing Compound **77** with Compound **103-2** as the raw material. **LCMS:** m/z (ESI), 460.1[M+H]+.

**Step E: (8a*R*,11a*R*,16b*R*,*E*)-15-fluoro-8a,10,11,11a,16b,17,18,19-octahydro-9*H*-1,21-etheno-4,8-(metheno)benzo [b]cyclopenta[*o*]imidazo[1,5-*h*]pyrrolo[2,1-*d*][1]oxa[5,7,8,11]tetra azacyclohexadecine(I-45)**

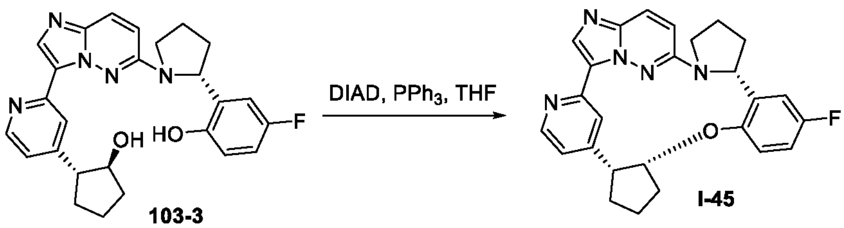


103-3 I-45

The title Compound **I-45** was prepared with reference to the method of Compound **I-9** by replacing Compound **39** with Compound **103-3** as the raw material. **LCMS:** m/z (ESI), 442[M+H]+.

**Example 46: (8a*S*,11a*R*,16b*R*,*E*)-15-fluoro-8a,10,11,11a,16b,17,18,19-octahydro-9*H*-1,21-etheno-4,8-(metheno) benzo[*b*]cyclopenta[*o*]imidazo[1,5-*h*]pyrrolo[2,1-*d*][1]oxa[5,7,8,11] tetraazacyclohexadecine(I-46)**

**Step A: 2-bromo-4-((1*S*,2*S*)-2-((tert-butyldimethylsilyl)oxy)cyclopentyl)pyridine(104-1)**

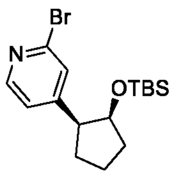

**104-1**

The title Compound **104-1** was prepared by the method in the reference of Tetrahedron Asymmetry, 2004, 15, (3), 481 - 488. **LCMS:** m/z (ESI), 358.1[M+H]+.

**Step B: 3-(4-((1*S*,2*S*)-2-((tert-butyldimethylsilyl)oxy)cyclopentyl)pyridin-2-yl)-6-chloro imidazo[1,2-b]pyridazine (104)**

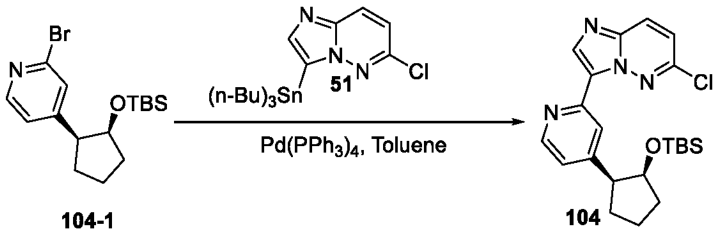


**104-1** **104**

The title Compound **104** was prepared with reference to the method of Compound **52** by replacing Compound **50** with Compound **104-1** as the raw material. **LCMS:** m/z (ESI), 429. 1 [M+H]+.

**Step C: (1*S*,2*S*)-2-(2-(6-((*R*)-2-(5-fluoro-2-methoxyphenyl)pyrrolidin-1-yl)imidazo [1,2-b]pyridazin-3-yl)pyridin-4-yl)cyclopentan-1-ol (104-2)**

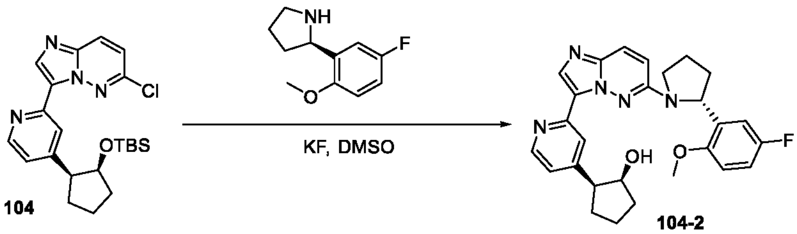


**104** **104-2**

The title Compound **104-2** was prepared with reference to the method of Compound **77** by replacing Compound **76** with Compound **104** as the raw material. **LCMS:** m/z (ESI), 474. 1 [M+H]+.

**Step D: (1*S*,2*S*)-2-(2-(6-((*R*)-2-(5-fluoro-2-hydroxyphenyl)pyrrolidin-1-yl)imidazo [1,2-*b*]pyridazin-3-yl)pyridin-4-yl)cyclopentan-1-ol (104-3)**

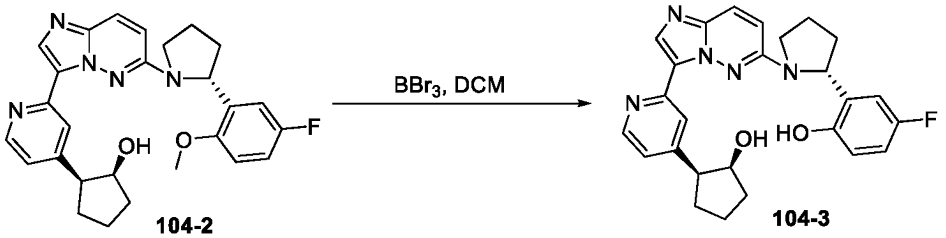


104-2 104-3

The title Compound **104-3** was prepared with reference to the method of Compound **78** by replacing Compound **77** with Compound **104-2** as the raw material. **LCMS:** m/z (ESI), 460.1[M+H]$^+$.

**Step E: (8a*S*,11a*R*,16b*R*,*E*)-15-fluoro-8a,10,11,11a,16b,17,18,19-octahydro-9*H*-1,21-etheno-4,8-(metheno)benzo[*b*]cyclopenta[*o*]imidazo[1,5-*h*]pyrrolo[2,1-*d*][1]oxa[5,7,8,11]tetra azacyclohexadecine(I-46)**

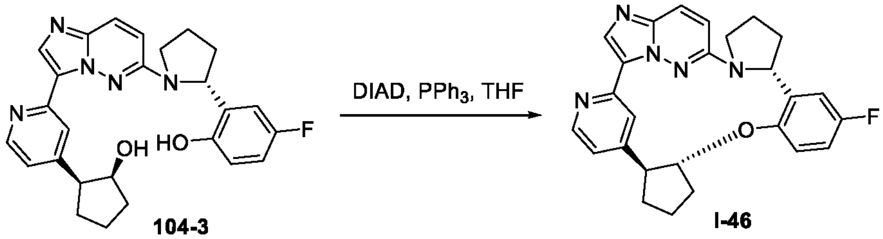


104-3 I-46

The title Compound **I-46** was prepared with reference to the method of Compound **I-9** by replacing Compound **39** with Compound **104-3** as the raw material. **LCMS:** m/z (ESI), 442[M+H]$^+$.

**Example 47: (*E*)-4'-fluorospiro[cyclopropane-1,6'-4-oxa-7-aza-1(3,6)-imidazo[1,2-*b*] pyridazina-2(2,4)-pyridina-5(1,2)-benzenacycloheptaphane] (I-47)**

**Step A: 1-(5-fluoro-2-((4-methoxybenzyl)oxy)phenyl)cyclopropan-1-amine(111)**

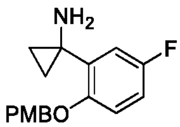

111

The title Compound **111** was prepared by the method in the reference of CN112110938. **LCMS:** m/z (ESI),182.0 [M+H]$^+$.

**Step B: 3-(4-(((tert-butyldimethylsilyl)oxy)methyl)pyridin-2-yl)-N-(1-(5-fluoro-2-((4-methoxybenzyl)oxy)phenyl) cyclopropyl)imidazo[1,2-*b*]pyridazin-6-amine (112)**

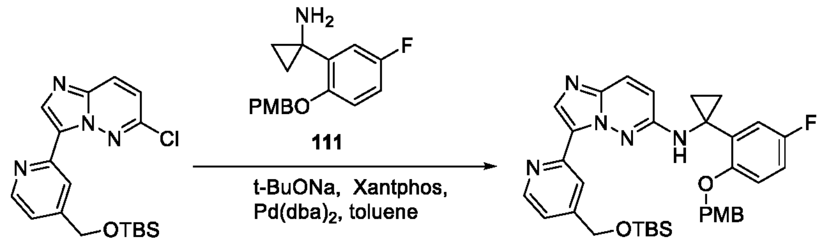


5 112

The title Compound **112** was prepared with reference to the method of Compound **10** by replacing Compound **9** with Compound **111** as the raw material. **LCMS:** m/z (ESI), 626.2[M+H]$^+$.

**Step C: 4-fluoro-2-(1-((3-(4-(hydroxymethyl)pyridin-2-yl)imidazo[1,2-*b*]pyridazin -6-yl)amino)cyclopropyl)phenol (112-1)**

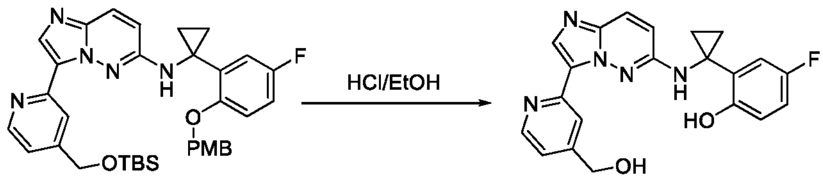


112 112-1

The title Compound **112-1** was prepared with reference to the method of Compound **11** by replacing Compound **10** with Compound **112** as the raw material. **LCMS:** m/z (ESI), 392[M+H]$^+$.

**Step D: (*E*)-4'-fluorospiro[cyclopropane-1,6'-4-oxa-7-aza-1(3,6)-imidazo[1,2-*b*] pyridazina-2(2,4)-pyridina-5(1,2)-benzenacycloheptaphane] (I-47)**

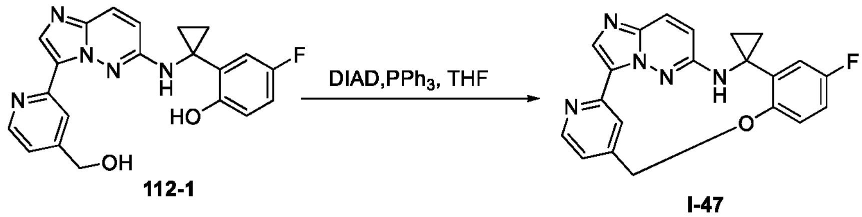


112-1 I-47

The title Compound **I-47** was prepared with reference to the method of Compound **I-9** by replacing Compound 39 with Compound **112-1** as the raw material. **LCMS:** m/z (ESI), 374[M+H]$^+$.

**Example 48: (*E*)-5$^4$-fluoro-6,6-dimethyl-4-oxa-7-aza-1(3,6)-imidazo[1,2-*b*]pyridazina -2(2,4)-pyridina-5(1,2)-benzenacycloheptaphane(I-48)**

**Step A: 2-(5-fluoro-2-((4-methoxybenzyl)oxy)phenyl)propan-2-amine(113)**

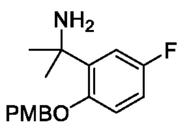

113

The title Compound **113** was prepared by the method in the reference of US2016221948. **LCMS:** m/z (ESI),184.0 [M+H]+.

**Step B: 3-(4-(((tert-butyldimethylsilyl)oxy)methyl)pyridin-2-yl)-*N*-(2-(5-fluoro-2-((4-methoxybenzyl)oxy)phenyl) propan-2-yl)imidazo[1,2-*b*]pyridazin-6-amine (114)**

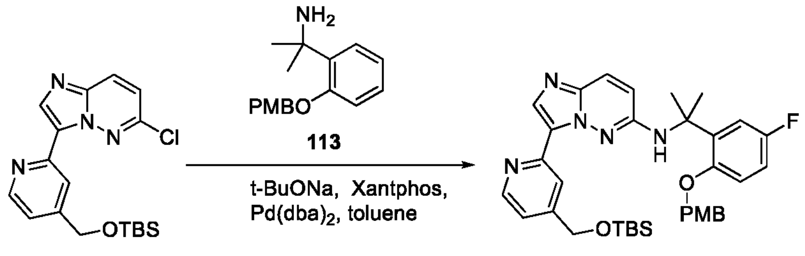


5 114

The title Compound **114** was prepared with reference to the method of Compound **10** by replacing Compound **9** with Compound **113** as the raw material. **LCMS:** m/z (ESI), 628.2[M+H]+.

**Step C: 4-fluoro-2-(2-((3-(4-(hydroxymethyl)pyridin-2-yl)imidazo[1,2-*b*]pyridazin-6-yl) amino)propan-2-yl)phenol (114-1)**

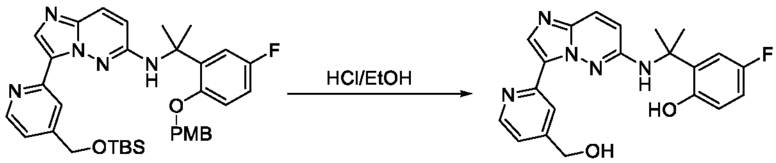


114 114-1

The title Compound **114-1** was prepared with reference to the method of Compound **11** by replacing Compound **10** with Compound **114** as the raw material. **LCMS:** m/z (ESI), 394[M+H]+.

**Step D: (*E*)-5⁴-fluoro-6,6-dimethyl-4-oxa-7-aza-1(3,6)-imidazo[1,2-*b*]pyridazina-2(2,4) -pyridina-5(1,2)-benzenacycloheptaphane(I-48)**

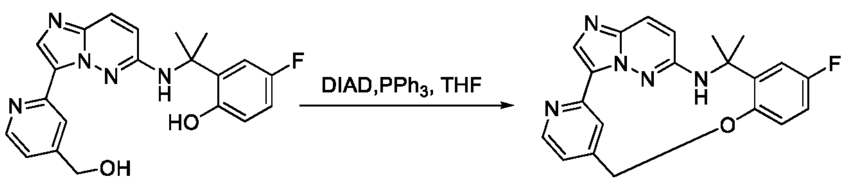


114-1 I-48

The title Compound **I-48** was prepared with reference to the method of Compound **I-9** by replacing Compound **39** with Compound **114-1** as the raw material.**LCMS:** m/z (ESI), 376[M+H]+.

**Example 49: (14b*R*,*E*)-13-fluoro-14b,15,16,17-tetrahydro-9*H*-1,19-etheno-4,8-(metheno)benzo[*b*]imidazo[1,5-*h*] pyrrolo[2,1-*d*][1]oxa[5,7,8,11]tetraazacyclopentadecine(I-4 9)**

**Step A: (*R*)-3-(4-(((tert-butyldimethylsilyl)oxy)methyl)pyridin-2-yl)-6-(2-(5-fluoro-2-methoxyphenyl)pyrrolidin-1-yl)imidazo[1,2-*b*]pyridazine (115)**

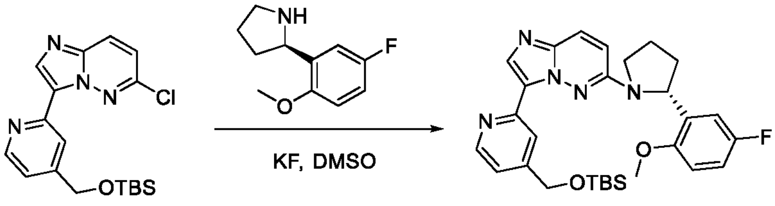


5

115

The title Compound **115** was prepared with reference to the method of Compound **10** by replacing Compound **9** with (***R***)-2-(5-fluoro-2-methoxyphenyl)pyrrolidine as the raw material. **LCMS:** m/z (ESI), 420.1 $[M+H]^+$.

**Step B: (*R*)-4-fluoro-2-(1-(3-(4-(hydroxymethyl)pyridin-2-yl)imidazo[1,2-*b*]pyridazin -6-yl)pyrrolidin-2-yl)phenol (115-1)**

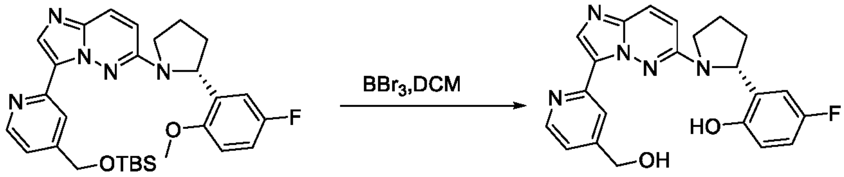


115

115-1

The title Compound **115-1** was prepared with reference to the method of Compound **11** by replacing Compound **10** with Compound **115** as the raw material. **LCMS:** m/z (ESI), 406.1$[M+H]^+$.

**Step C: (14b*R*,*E)*-13-fluoro-14b,15,16,17-tetrahydro-9*H*-1,19-etheno-4,8-(metheno) benzo[b]imidazo[1,5-*h*]pyrrolo[2,1-*d*][1]oxa[5,7,8,11]tetraazacyclopentadecine(I-49)**

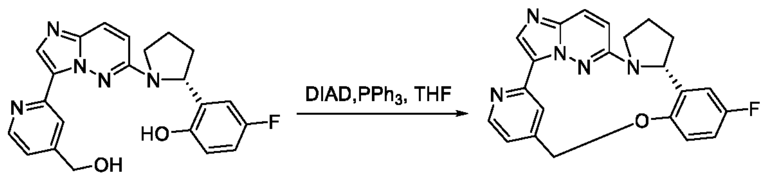


115-1

I-49

The title Compound **I-49** was prepared with reference to the method of Compound **I-9** by replacing Compound **39** with Compound **115-1** as the raw material. **LCMS:** m/z (ESI), 388$[M+H]^+$.

**Example 50: (14b*R*,16*S*,*E*)-13,16-difluoro-14b,15,16,17-tetrahydro-9*H*-1,19-etheno-4,8-(metheno)benzo[*b*]imidazo[1,5-*h*]pyrrolo[2,1-*d*][1]oxa[5,7,8,11]tetraazacyclopentadecine(I-5 0)**

**Step A: 3-(4-(((tert-butyldimethylsilyl)oxy)methyl)pyridin-2-yl)-6-((2*R*,4*S*)-4-fluoro-2-(5-fluoro-2-methoxyphenyl)pyrrolidin-1-yl)imidazo[1,2-*b*]pyridazine (116)**

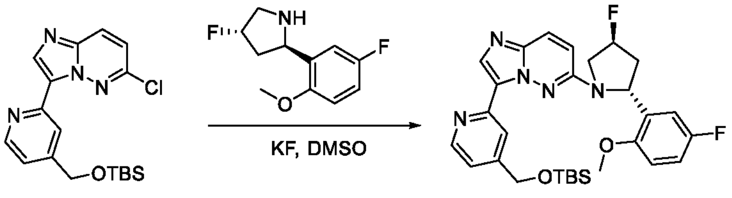


5 116

The title Compound **115** was prepared with reference to the method of Compound **10** by replacing Compound **9** with (2***R***,4***S***)-4-fluoro-2-(5-fluoro-2-methoxyphenyl)pyrrolidine as the raw material. **LCMS:** m/z (ESI), 438.1[M+H]+.

**Step B: 4-fluoro-2-((2*R*,4*S*)-4-fluoro-1-(3-(4-(hydroxymethyl)pyridin-2-yl)imidazo [1,2-*b*]pyridazin-6-yl)pyrrolidin-2-yl)phenol (116-1)**

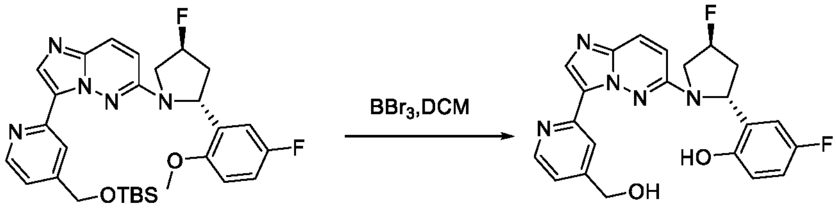


116 116-1

The title Compound **116-1** was prepared with reference to the method of Compound **11** by replacing Compound **10** with Compound **116** as the raw material. **LCMS:** m/z (ESI), 424.1[M+H]+.

**Step C: (14b*R*,16*S*,*E*)-13,16-difluoro-14b,15,16,17-tetrahydro-9*H*-1,19-etheno-4,8-(metheno)benzo[*b*]imidazo [1,5-*h*]pyrrolo[2,1-*d*][1]oxa[5,7,8,11]tetraazacyclopentadecine(I-5 0)**

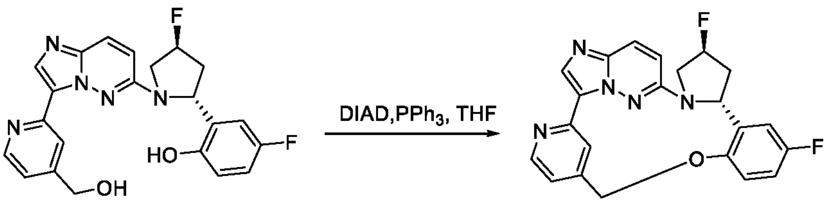


116-1 I-50

The title Compound **I-50** was prepared with reference to the method of Compound **I-9** by replacing Compound **39** with Compound **116-1** as the raw material. **LCMS:** m/z (ESI), 406[M+H]+.

**Example 51: (14b*R*,*E*)-13-fluoro-14b,15,16,17-tetrahydro-9*H*-1,19-etheno-4,8-(metheno)imidazo[1,5-*h*]pyrido [2,3-*b*]pyrrolo[2,1-*d*][1]oxa[5,7,8,11]tetraazacyclopentadecine (I-51)**

**Step A: (*R*)-(2-(6-(2-(5-fluoro-2-methoxypyridin-3-yl)pyrrolidin-1-yl)imidazo[1,2-*b*] pyridazin-3-yl)pyridin-4-yl) methanol (116-2)**

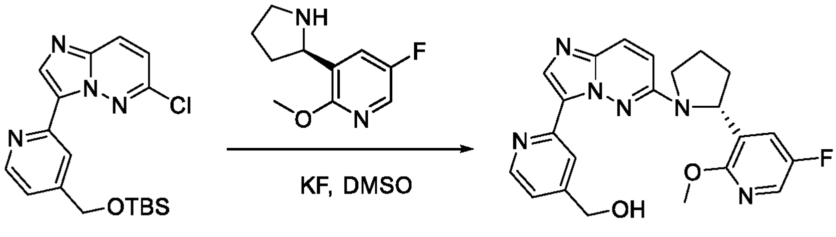


5

116-2

The title Compound **116-2** was prepared with reference to the method of Compound **10** by replacing Compound **9** with (***R***)-5-fluoro-2-methoxy-3-(pyrrolidin-2-yl)pyridine as the raw material. **LCMS:** m/z (ESI), 421.1[M+H]$^+$.

**Step B: (*R*)-5-fluoro-3-(1-(3-(4-(hydroxymethyl)pyridin-2-yl)imidazo[1,2-*b*]pyridazin -6-yl)pyrrolidin-2-yl)pyridin-2(1*H*)-one (116-3)**

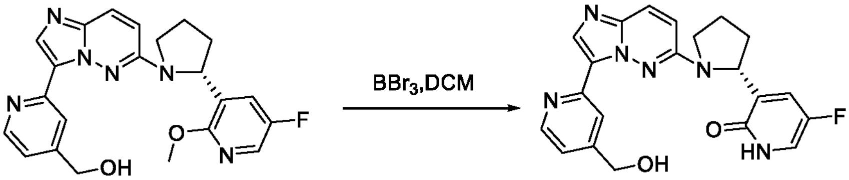


116-2

116-3

The title Compound **116-3** was prepared with reference to the method of Compound **11** by replacing Compound **10** with Compound **116-2** as the raw material. **LCMS:** m/z (ESI), 407.1[M+H]$^+$.

**Step C: (14b*R*,*E*)-13-fluoro-14b,15,16,17-tetrahydro-9*H*-1,19-etheno-4,8-(metheno) imidazo[1,5-*h*]pyrido[2,3-*b*] pyrrolo[2,1-*d*][1]oxa[5,7,8,11]tetraazacyclopentadecine(I-51)**

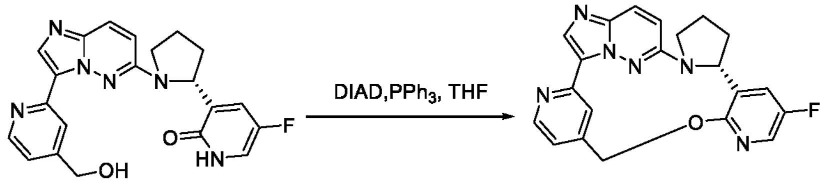


116-3

I-51

The title Compound **I-51** was prepared with reference to the method of Compound **I-9** by replacing Compound **39** with Compound **116-3** as the raw material. **LCMS:** m/z (ESI), 389[M+H]$^+$.

**Example 52: (14b*R*,16*S*,*E*)-13,16-difluoro-14b,15,16,17-tetrahydro-9*H*-1,19-etheno-4,8-(metheno)imidazo[1,5-*h*] pyrido[2,3-*b*]pyrrolo[2,1-*d*][1]oxa[5,7,8,11]tetraazacyclopentadecine (I-52)**

**Step A: (2-(6-((2*R*,4*S*)-4-fluoro-2-(5-fluoro-2-methoxypyridin-3-yl)pyrrolidin-1-yl) imidazo[1,2-*b*]pyridazin-3-yl) pyridin-4-yl)methanol (116-4)**

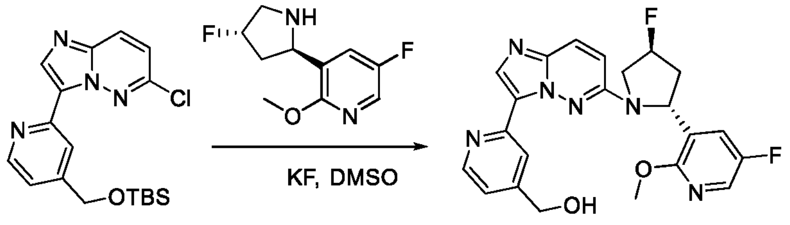


5 116-4

The title Compound **116-4** was prepared with reference to the method of Compound **10** by replacing Compound **9** with 5-fluoro-3-((2***R***,4***S***)-4-fluoropyrrolidin-2-yl)-2-methoxypyridine as the raw material. **LCMS:** m/z (ESI), 439.1[M+H]$^+$.

**Step B: 5-fluoro-3-((2*R*,4*S*)-4-fluoro-1-(3-(4-(hydroxymethyl)pyridin-2-yl)imidazo [1,2-*b*]pyridazin-6-yl)pyrrolidin-2-yl)pyridin-2(1*H*)-one (116-5)**

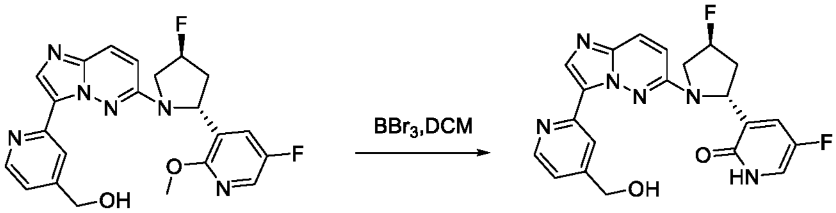


116-4 116-5

The title Compound **116-5** was prepared with reference to the method of Compound **11** by replacing Compound **10** with Compound **116-4** as the raw material. **LCMS:** m/z (ESI), 425.1[M+H]$^+$.

**Step C: (14b*R*,16*S*,*E*)-13,16-difluoro-14b,15,16,17-tetrahydro-9*H*-1,19-etheno-4,8-(metheno)imidazo[1,5-*h*]pyrido[2,3-*b*]pyrrolo[2,1-*d*][1]oxa[5,7,8,11]tetraazacyclopentadecine (I-52)**

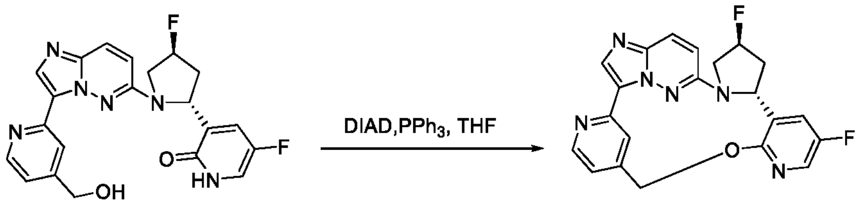


116-5 I-52

The title Compound **I-52** was prepared with reference to the method of Compound **I-9** by replacing Compound **39** with Compound **116-5** as the raw material. **LCMS:** m/z (ESI), 407[M+H]$^+$.

**Example 53: (3$^2$*R*,*E*)-4$^5$-fluoro-5-oxa-2(3,6)-imidazo[1,2-*b*]pyridazina-1(2,4)-pyridina-3(1,2)-pyrrolidina-4(1,2)-benzenacycloheptaphane(I-53)**

**Step A: (*R*)-2-(2-(6-(2-(5-fluoro-2-methoxyphenyl)pyrrolidin-1-yl)imidazo[1,2-*b*]pyri dazin-3-yl)pyridin-4-yl) ethan-1-ol (117)**

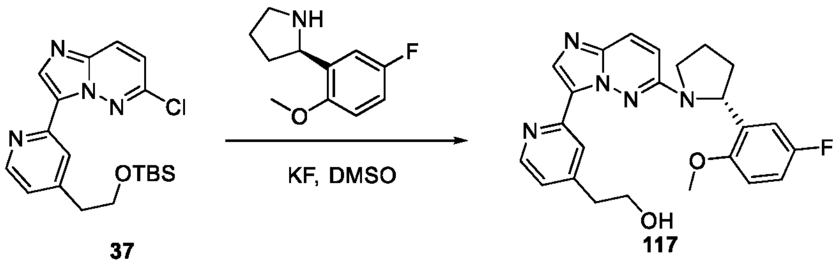


37 117

The title Compound **117** was prepared with reference to the method of Compound **38** by replacing Compound **37** with (R)-2-(5-fluoro-2-methoxyphenyl)pyrrolidine as the raw material. **LCMS:** m/z (ESI), 434.1 $[M+H]^+$.

**Step *B*: (*R*)-4-fluoro-2-(1-(3-(4-(2-hydroxyethyl)pyridin-2-yl)imidazo[1,2-*b*]pyridazin -6-yl)pyrrolidin-2-yl)phenol (117-1)**

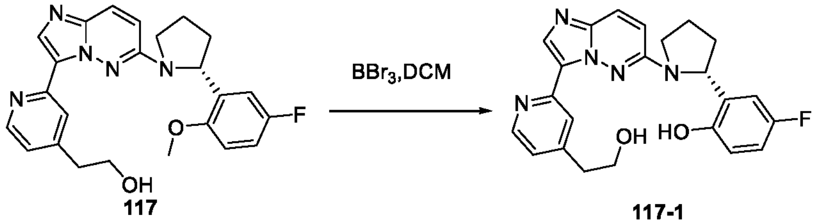


117 117-1

The title Compound **117-1** was prepared with reference to the method of Compound **11** by replacing Compound **10** with Compound **117** as the raw material. **LCMS:** m/z (ESI), 420.1$[M+H]^+$.

**Step C: ($3^2$*R*,*E*)-$4^5$-fluoro-5-oxa-2(3,6)-imidazo[1,2-*b*]pyridazina-1(2,4)-pyridina-3(1,2)-pyrrolidina-4(1,2)-benzenacycloheptaphane(I-53)**

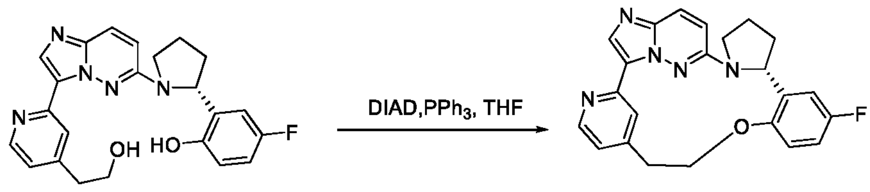


117-1 I-53

The title Compound **I-53** was prepared with reference to the method of Compound **I-9** by replacing Compound **39** with Compound **117-1** as the raw material. **LCMS:** m/z (ESI), 402$[M+H]^+$.

**Example 54: ($3^2$*R*,$3^4$*S*,*E*)-$3^4$,$4^5$-difluoro-5-oxa-2(3,6)-imidazo[1,2-*b*]pyridazina-1(2,4)-pyridina-3(1,2)-pyrrolidina-4(1,2)-benzenacycloheptaphane(I-54)**

**Step A: 2-(2-(6-((2*R*,4*S*)-4-fluoro-2-(5-fluoro-2-methoxyphenyl)pyrrolidin-1-yl) imidazo[1,2-b]pyridazin-3-yl)pyridin-4-yl)ethan-1-ol (118)**

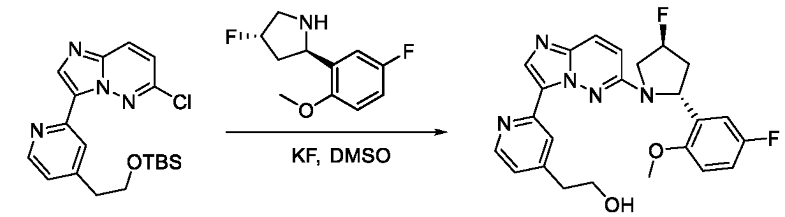


37

118

The title Compound **118** was prepared with reference to the method of Compound **38** by replacing Compound **37** with (2***R***,4***S***)-4-fluoro-2-(5-fluoro-2-methoxyphenyl)pyrrolidine as the raw material. **LCMS:** m/z (ESI), 452.1 [M+H]$^+$.

**Step B: 4-fluoro-2-((2*R*,4*S*)-4-fluoro-1-(3-(4-(2-hydroxyethyl)pyridin-2-yl)imidazo [1,2-*b*]pyridazin-6-yl)pyrrolidin-2-yl)phenol (118-1)**

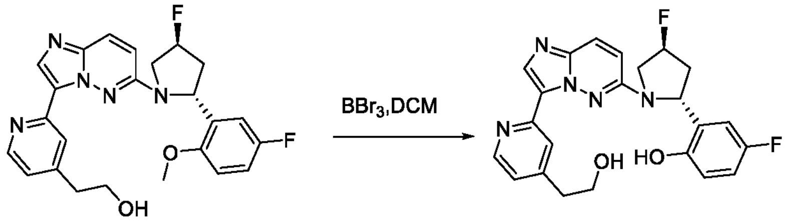


118

118-1

The title Compound **118-1** was prepared with reference to the method of Compound **11** by replacing Compound **10** with Compound **118** as the raw material. **LCMS:** m/z (ESI), 438.1[M+H]$^+$.

**Step C: (3$^2$*R*,3$^4$*S*,*E*)-3$^4$,4$^5$-difluoro-5-oxa-2(3,6)-imidazo[1,2-*b*]pyridazina-1(2,4)-pyridina-3(1,2)-pyrrolidina-4(1,2)-benzenacycloheptaphane(I-54)**

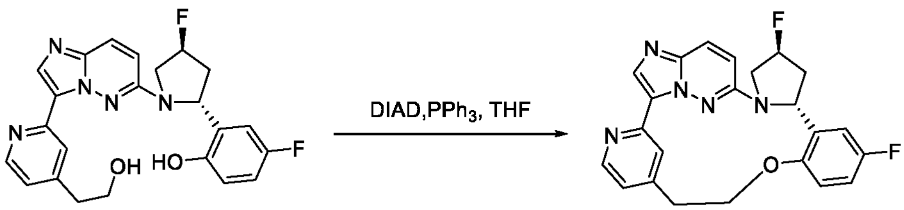


118-1

I-54

The title Compound **I-54** was prepared with reference to the method of Compound **I-9** by replacing Compound **39** with Compound **118-1** as the raw material. **LCMS:** m/z (ESI), 420[M+H]$^+$.

**Example 55: (3$^2$*R*,*E*)-4$^5$-fluoro-5-oxa-2(3,6)-imidazo[1,2-*b*]pyridazina-1(2,4),4(3,2)-dipyridina-3(1,2)-pyrrolidinacycloheptaphane (I-55)**

**Step A: (*R*)-2-(2-(6-(2-(5-fluoro-2-methoxypyridin-3-yl)pyrrolidin-1-yl)imidazo[1,2-*b*] pyridazin-3-yl)pyridin-4-yl) ethan-1-ol (118-2)**

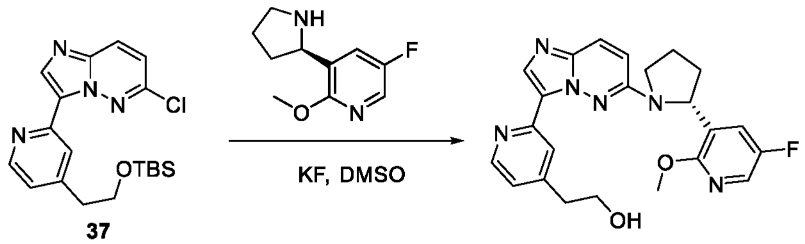


37

118-2

The title Compound **118-2** was prepared with reference to the method of Compound **38** by replacing Compound **19** with (***R***)-5-fluoro-2-methoxy-3-(pyrrolidin-2-yl)pyridine as the raw material. **LCMS:** m/z (ESI), 435.1[M+H]+.

**Step B: (*R*)-5-fluoro-3-(1-(3-(4-(2-hydroxyethyl)pyridin-2-yl)imidazo[1,2-*b*]pyridazin -6-yl)pyrrolidin-2-yl)pyridin-2(1*H*)-one (118-3)**

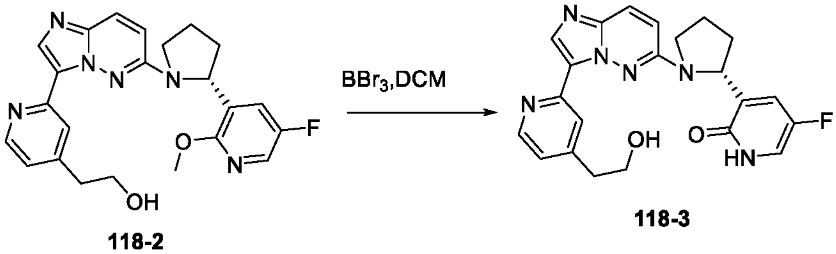


118-2

118-3

The title Compound **118-3** was prepared with reference to the method of Compound **11** by replacing Compound **10** with Compound **118-2** as the raw material. **LCMS:** m/z (ESI), 421.1[M+H]+.

**Step C: (3²*R*,*E*)-4⁵-fluoro-5-oxa-2(3,6)-imidazo[1,2-*b*]pyridazina-1(2,4),4(3,2)-dipyridina-3(1,2)-pyrrolidinacycloheptaphane(I-55)**

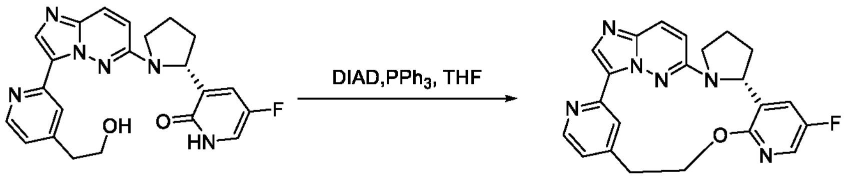


118-3

I-55

The title Compound **I-55** was prepared with reference to the method of Compound **I-9** by replacing Compound **39** with Compound **118-3** as the raw material. **LCMS:** m/z (ESI), 403 [M+H]+.

**Example 56: (3²*R*,3⁴*S*,*E*)-3⁴,4⁵-difluoro-5-oxa-2(3,6)-imidazo[1,2-*b*]pyridazina-1(2,4), 4(3,2)-dipyridina-3(1,2)-pyrrolidinacycloheptaphane(I-56)**

**Step A: 2-(2-(6-((2*R*,4*S*)-4-fluoro-2-(5-fluoro-2-methoxypyridin-3-yl)pyrrolidin-1-yl)imidazo[1,2-*b*]pyridazin-3-yl) pyridin-4-yl)ethan-1-ol (118-4)**

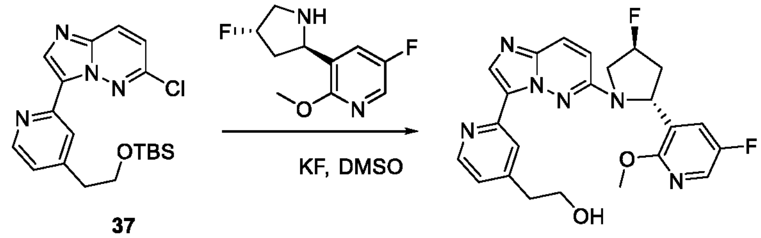


37

118-4

The title Compound **117** was prepared with reference to the method of Compound **9** by replacing Compound **10** with 5-fluoro-3-((2R,4S)-4-fluoropyrrolidin-2-yl)-2-methoxypyridine as the raw material. **LCMS:** m/z (ESI), 453.1[M+H]$^+$.

**Step B: 5-fluoro-3-((2*R*,4*S*)-4-fluoro-1-(3-(4-(2-hydroxyethyl)pyridin-2-yl)imidazo [1,2-*b*]pyridazin-6-yl)pyrrolidin-2-yl)pyridin-2(1*H*)-one (118-5)**

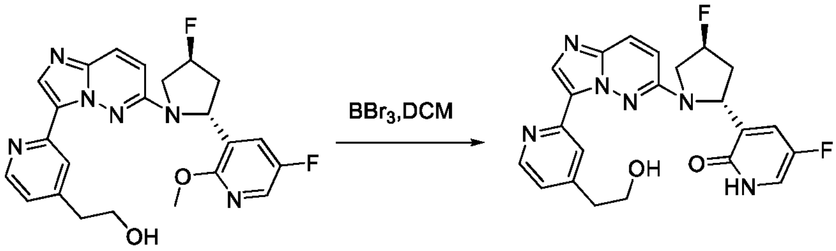


118-4

118-5

The title Compound **118-5** was prepared with reference to the method of Compound **11** by replacing Compound **10** with Compound **118-4** as the raw material. **LCMS:** m/z (ESI), 439. 1 [M+H]$^+$.

**Step C: (3$^2$*R*,3$^4$*S*,*E*)-3$^4$,4$^5$-difluoro-5-oxa-2(3,6)-imidazo[1,2-*b*]pyridazina-1(2,4),4(3,2)-dipyridina-3(1,2)-pyrrolidinacycloheptaphane(I-56)**

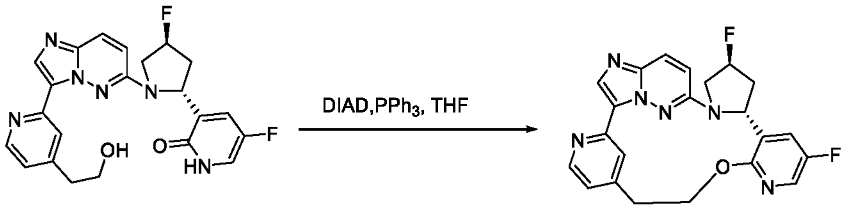


118-5

I-56

The title Compound **I-56** was prepared with reference to the method of Compound **I-9** by replacing Compound **39** with Compound **118-5** as the raw material. **LCMS:** m/z (ESI), 421 [M+H]$^+$.

**Example 57: (14b*R*,*E*)-13-fluoro-14b,15,16,17-tetrahydro-9*H*-1,19-etheno-4,8-(metheno)benzo[*n*]imidazo[5,1-*h*] pyrrolo[1,2-*l*][1]oxa[4,6,9,10,12]pentaazacyclopentadecine( I-57)**

**Step A: (*R*)-3-(6-(((tert-butyldimethylsilyl)oxy)methyl)pyrimidin-4-yl)-6-(2-(5-fluoro-2-methoxyphenyl)pyrrolidin-1-yl)imidazo[1,2-*b*]pyridazine (117A)**

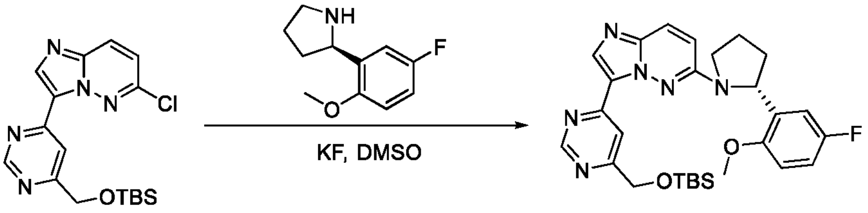


42

117A

The title Compound **117A** was prepared with reference to the method of Compound **43** by replacing Compound **19** with **(*R*)-2-(5-fluoro-2-methoxyphenyl)pyrrolidine** as the raw material. LCMS: m/z (ESI), 535.1[M+H]$^+$.

**Step B: (*R*)-4-fluoro-2-(1-(3-(6-(hydroxymethyl)pyrimidin-4-yl)imidazo[1,2-*b*] pyridazin-6-yl)pyrrolidin-2-yl)phenol (117A-1)**

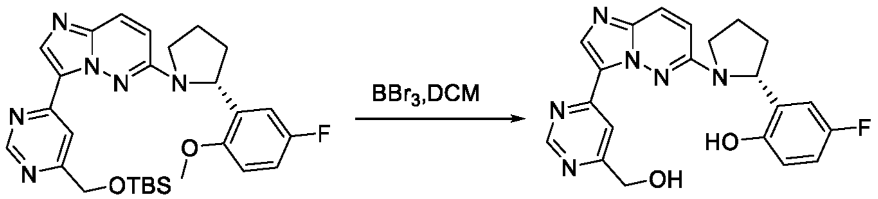


117A

117A-1

The title Compound **117A-1** was prepared with reference to the method of Compound **11** by replacing Compound **10** with Compound **117A** as the raw material. **LCMS:** m/z (ESI), 407.1[M+H]$^+$.

**Step C: (14b*R*,*E*)-13-fluoro-14b,15,16,17-tetrahydro-9*H*-1,19-etheno-4,8-(metheno) benzo[*n*]imidazo[5,1-*h*]pyrrolo[1,2-*l*][1]oxa[4,6,9,10,12]pentaazacyclopentadecine(I-57)**

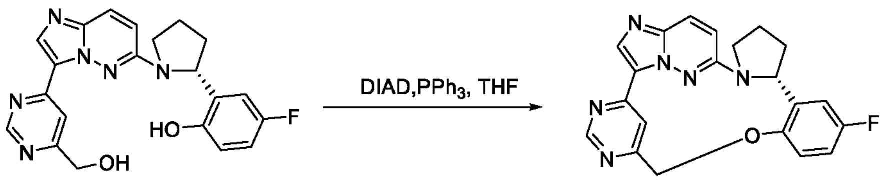


117A-1

I-57

The title Compound **I-57** was prepared with reference to the method of Compound **I-9** by replacing Compound **39** with Compound **117A-1** as the raw material. **LCMS:** m/z (ESI), 389[M+H]$^+$.

**Example 58: (14b*R*,16*S*,*E*)-13,16-difluoro-14b,15,16,17-tetrahydro-9*H*-1,19-etheno-4,8-(metheno)benzo[*n*]imidazo[5,1-*h*]pyrrolo[1,2-*l*][1]oxa[4,6,9,10,12]pentaazacyclopentadecine( I-58)**

**Step A: 3-(6-(((tert-butyldimethylsilyl)oxy)methyl)pyrimidin-4-yl)-6-((2*R*,4*S*)-4-fluoro -2-(5-fluoro-2-methoxyphenyl)pyrrolidin-1-yl)imidazo[1,2-*b*]pyridazine (118A)**

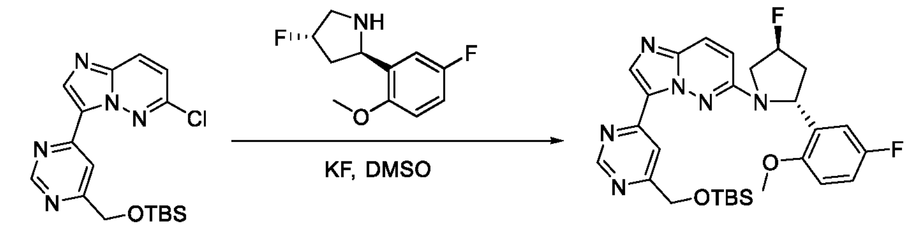


42

118A

The title Compound **118A** was prepared with reference to the method of Compound **43** by replacing Compound **19** with (2***R***,4***S***)-4-fluoro-2-(5-fluoro-2-methoxyphenyl)pyrrolidine as the raw material. LCMS: m/z (ESI), 553.1[M+H]$^+$.

**Step B: 4-fluoro-2-((2*R*,4*S*)-4-fluoro-1-(3-(6-(hydroxymethyl)pyrimidin-4-yl)imidazo [1,2-*b*]pyridazin-6-yl)pyrrolidin-2-yl)phenol (118A-1)**

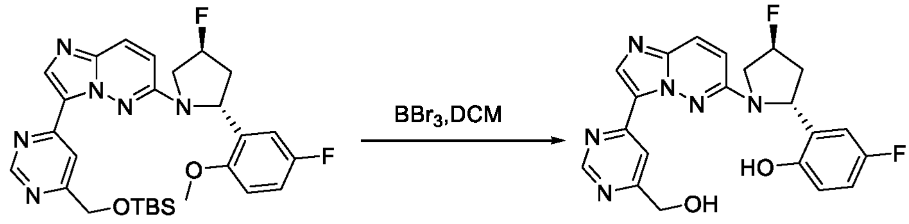


118A

118A-1

The title Compound **118A-1** was prepared with reference to the method of Compound **11** by replacing Compound **10** with Compound **118A** as the raw material. **LCMS:** m/z (ESI), 425.1[M+H]$^+$.

**Step C: (14b*R*,16*S*,*E*)-13,16-difluoro-14b,15,16,17-tetrahydro-9*H*-1,19-etheno-4,8-(metheno)benzo[*n*]imidazo [5,1-*h*]pyrrolo[1,2-*l*][1]oxa[4,6,9,10,12]pentaazacyclopentadecine( I-58)**

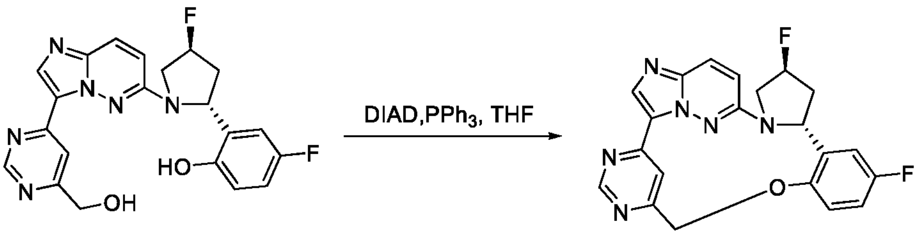


118A-1

I-58

The title Compound **I-58** was prepared with reference to the method of Compound **I-9** by replacing Compound **39** with Compound **118A-1** as the raw material. **LCMS:** m/z (ESI), 407[M+H]$^+$.

**Example 59: (14b*R*,*E*)-13-fluoro-14b,15,16,17-tetrahydro-9*H*-1,19-etheno-4,8-(metheno)imidazo[5,1-*h*]pyrido [3,2-*n*]pyrrolo[1,2-*l*][1]oxa[4,6,9,10,12]pentaazacyclopentade cine(I-59)**

**Step A: (*R*)-(6-(6-(2-(5-fluoro-2-methoxypyridin-3-yl)pyrrolidin-1-yl)imidazo[1,2-*b*] pyridazin-3-yl)pyrimidin-4-yl) methanol (118A-2)**

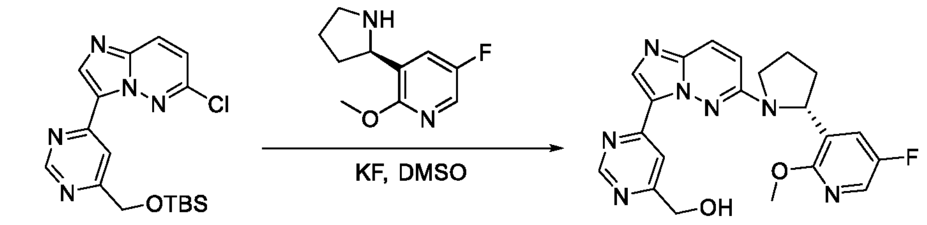


42

118A-2

The title Compound **118A-2** was prepared with reference to the method of Compound **43** by replacing Compound **19** with (***R***)-5-fluoro-2-methoxy-3-(pyrrolidin-2-yl)pyridine as the raw material. **LCMS:** m/z (ESI), 422.1[M+H]+.

**Step B: (R)-5-fluoro-3-(1-(3-(6-(hydroxymethyl)pyrimidin-4-yl)imidazo[1,2-b] pyridazin-6-yl)pyrrolidin-2-yl)pyridin-2(1*H*)-one (118A-3)**

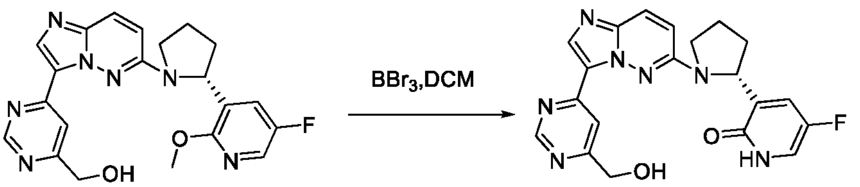


118A-2

118A-3

The title Compound **118A-3** was prepared with reference to the method of Compound **11** by replacing Compound **10** with Compound **118A-2** as the raw material. **LCMS:** m/z (ESI), 408.1[M+H]+.

**Step C: (14b*R*,*E)*-13-fluoro-14b,15,16,17-tetrahydro-9*H*-1,19-etheno-4,8-(metheno) imidazo[5,1-*h*]pyrido[3,2-*n*] pyrrolo[1,2-*l*][1]oxa[4,6,9,10,12]pentaazacyclopentadecine(I-59)**

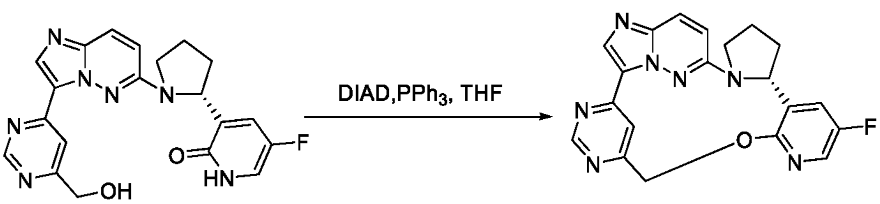


118A-3

I-59

The title Compound **I-59** was prepared with reference to the method of Compound **I-9** by replacing Compound **39** with Compound **118A-3** as the raw material. **LCMS:** m/z (ESI), 390[M+H]+.

**Example 60: (14b*R*,16*S*,*E*)-13,16-difluoro-14b,15,16,17-tetrahydro-9*H*-1,19-etheno-4,8-(metheno)imidazo[5,1-*h*] pyrido[3,2-*n*]pyrrolo[1,2-*l*][1]oxa[4,6,9,10,12]pentaazacyclopentade cine(I-60)**

**Step A: (6-(6-((2*R*,4*S*)-4-fluoro-2-(5-fluoro-2-methoxypyridin-3-yl)pyrrolidin-1-yl) imidazo[1,2-*b*]pyridazin-3-yl) pyrimidin-4-yl)methanol (118A-4)**

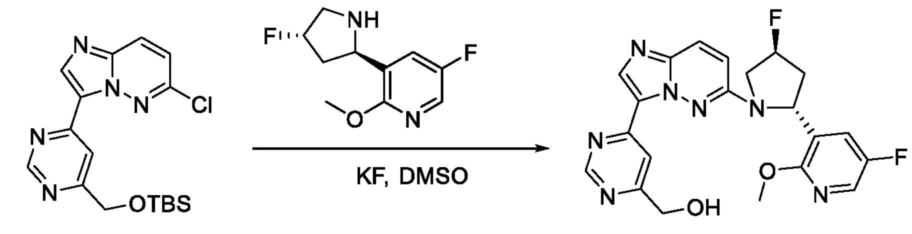


42

118A-4

The title Compound **118A-4** was prepared with reference to the method of Compound **43** by replacing Compound **19** with 5-fluoro-3-((2*R*,4*S*)-4-fluoropyrrolidin-2-yl)-2-methoxypyridine as the raw material. **LCMS:** m/z (ESI), 440.1 $[M+H]^+$.

**Step B: 5-fluoro-3-((2*R*,4*S*)-4-fluoro-1-(3-(6-(hydroxymethyl)pyrimidin-4-yl)imidazo [1,2-*b*]pyridazin-6-yl)pyrrolidin-2-yl)pyridin-2(1H)-one (118A-5)**

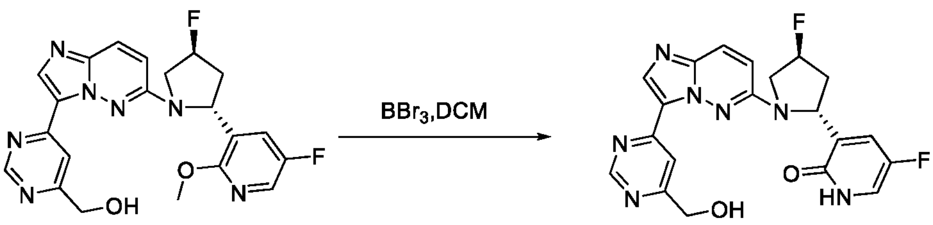


118A-4

118A-5

The title Compound **118A-5** was prepared with reference to the method of Compound **11** by replacing Compound **10** with Compound **118A-4** as the raw material. **LCMS:** m/z (ESI), 426.1$[M+H]^+$.

**Step C: (14b*R*,16*S*,*E*)-13,16-difluoro-14b,15,16,17-tetrahydro-9*H*-1,19-etheno-4,8-(metheno)imidazo[5,1-*h*]pyrido[3,2-*n*]pyrrolo[1,2-1][1]oxa[4,6,9,10,12]pentaazacyclopentade cine(I-60)**

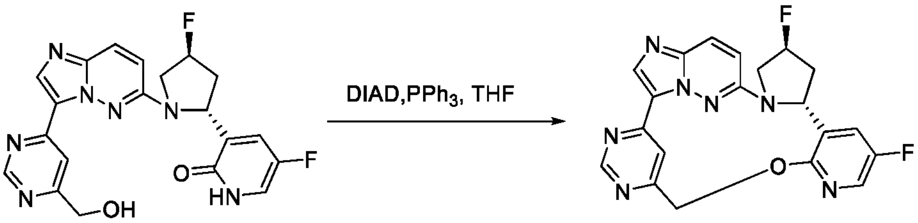


118A-5

I-60

The title Compound **I-60** was prepared with reference to the method of Compound **I-9** by replacing Compound **39** with Compound **118A-5** as the raw material. **LCMS:** m/z (ESI), 408$[M+H]^+$.

**Example 61: (14b*R*,*E*)-13-fluoro-14b,15,16,17-tetrahydro-9*H*-4,8-(azeno)-1,19-etheno benzo[*b*]imidazo[1,5-*h*] pyrrolo[2,1-*d*][1]oxa[5,7,8,11]tetraazacyclopentadecine(I-61)**

**Step A: 4-(((tert-butyldimethylsilyl)oxy)methyl)-2-chloropyrimidine (119)**

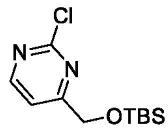

119

The title Compound **119** was prepared by the method in the reference of WO2016196244. **LCMS:** m/z (ESI),259.0[M+H]$^+$.

**Step B: 4-(((tert-butyldimethylsilyl)oxy)methyl)-2-(tributylstannyl)pyrimidine(120)**

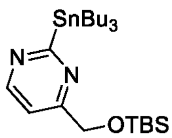

120

The title Compound **120** was prepared with reference to the method of Compound **3** by replacing Compound **2** with Compound **119** as the raw material. **LCMS:** m/z (ESI),515.0[M+H]$^+$.

Step C: 3-(4-(((tert-butyldimethylsilyl)oxy)methyl)pyrimidin-2-yl)-6-chloroimidazo [1,2-b]pyridazine(121)

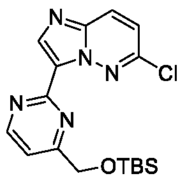

121

The title Compound **121** was prepared with reference to the method of Compound **5** by replacing Compound **3** with Compound **120** as the raw material. **LCMS:** m/z (ESI),376.0[M+H]$^+$.

**Step D: (*R*)-3-(4-(((tert-butyldimethylsilyl)oxy)methyl)pyrimidin-2-yl)-6-(2-(5-fluoro -2-methoxyphenyl)pyrrolidin-1-yl)imidazo[1,2-*b*]pyridazine (122)**

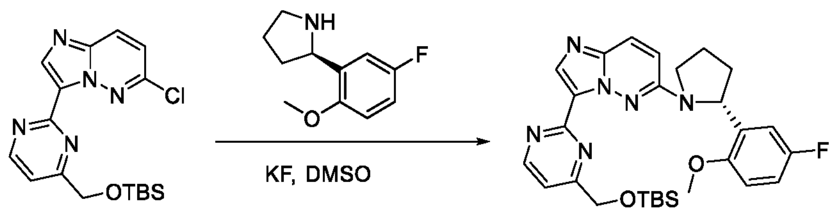


121 122

The title Compound **122** was prepared with reference to the method of Compound **43** by replacing Compound **19** with (***R***)-2-(5-fluoro-2-methoxyphenyl)pyrrolidine and Compound **42** with Compound **121** as the raw material. **LCMS:** m/z (ESI), 535.1[M+H]$^+$.

**Step E: (*R*)-4-fluoro-2-(1-(3-(4-(hydroxymethyl)pyrimidin-2-yl)imidazo[1,2-*b*] pyridazin-6-yl)pyrrolidin-2-yl)phenol(122-1)**

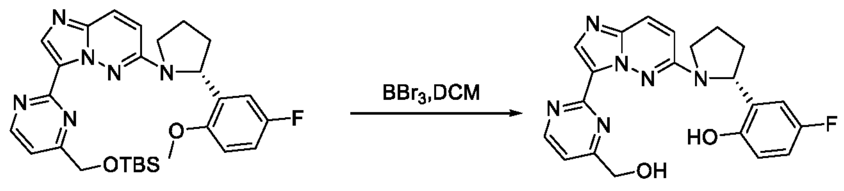


**122** **122-1**

The title Compound **122-1** was prepared with reference to the method of Compound **11** by replacing Compound **10** with Compound **122** as the raw material. **LCMS:** m/z (ESI), 407.1[M+H]$^+$.

**Step F: (14b*R*,*E*)-13-fluoro-14b,15,16,17-tetrahydro-9*H*-4,8-(azeno)-1,19-etheno benzo[*b*]imidazo[1,5-*h*]pyrrolo [2,1-*d*][1]oxa[5,7,8,11]tetraazacyclopentadecine(I-61)**

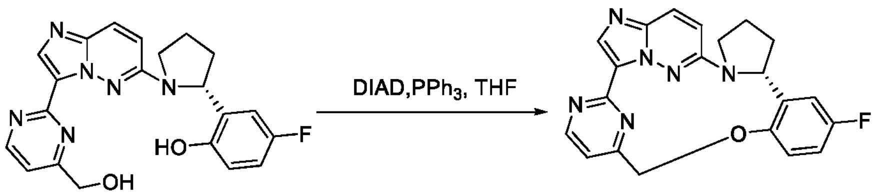


**122-1** **I-61**

The title Compound **I-61** was prepared with reference to the method of Compound **I-11** by replacing Compound **48-2** with Compound **122-1** as the raw **material.LCMS:** m/z (ESI), 389[M+H]$^+$.

**Example 62: (14b*R*,16*S*,*E*)-13,16-difluoro-14b,15,16,17-tetrahydro-9*H*-4,8-(azeno)-1,19-ethenobenzo[*b*]imidazo [1,5-*h*]pyrrolo[2,1-*d*][1]oxa[5,7,8,11]tetraazacyclopentadecine(I-62)**

**Step A: 3-(4-(((tert-butyldimethylsilyl)oxy)methyl)pyrimidin-2-yl)-6-((2*R*,4*S*)-4-fluoro -2-(5-fluoro-2-methoxyphenyl)pyrrolidin-1-yl)imidazo[1,2-*b*]pyridazine (123)**

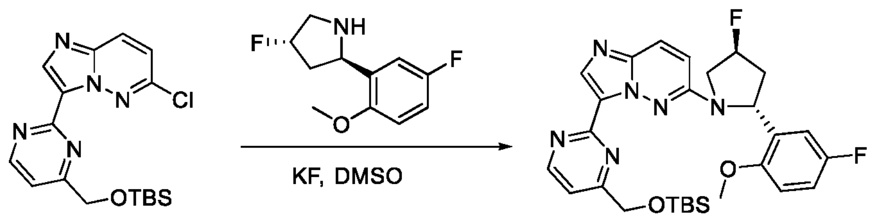


**121** **123**

The title Compound **122** was prepared with reference to the method of Compound **43** by replacing Compound **19** with (2***R***,4***S***)-4-fluoro-2-(5-fluoro-2-methoxyphenyl)pyrrolidine and Compound **42** with Compound **121** as the raw material. **LCMS:** m/z (ESI), 553.1[M+H]$^+$.

**Step B: 4-fluoro-2-((2*R*,4*S*)-4-fluoro-1-(3-(4-(hydroxymethyl)pyrimidin-2-yl)imidazo [1,2-*b*]pyridazin-6-yl)pyrrolidin-2-yl)phenol (123-1)**

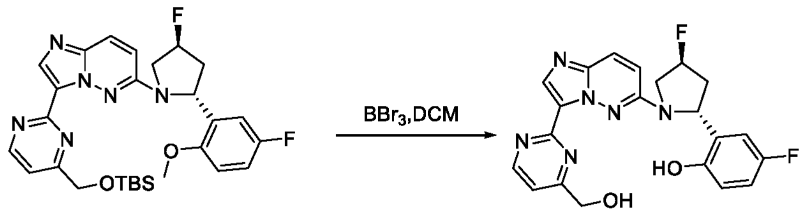


123

123-1

The title Compound **123-1** was prepared with reference to the method of Compound **11** by replacing Compound **10** with Compound **123** as the raw material. **LCMS:** m/z (ESI), 425.1[M+H]$^+$.

**Step C: (14b*R*,16*S*,*E*)-13,16-difluoro-14b,15,16,17-tetrahydro-9*H*-4,8-(azeno)-1,19-ethenobenzo[*b*]imidazo[1,5-*h*]pyrrolo[2,1-*d*][1]oxa[5,7,8,11]tetraazacyclopentadecine(I-62)**

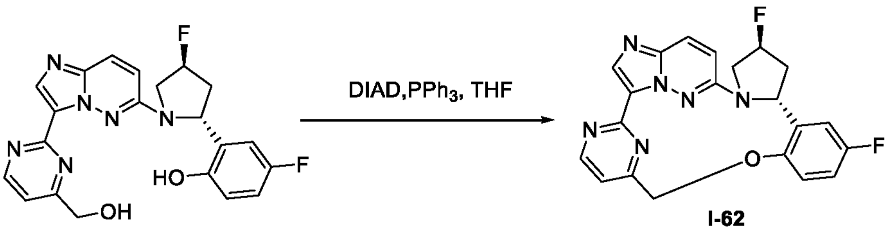


123-1

I-62

The title Compound **I-62** was prepared with reference to the method of Compound **I-9** by replacing Compound **39** with Compound **123-1** as the raw material. **LCMS:** m/z (ESI), 407[M+H]$^+$.

**Example 63: (14b*R*,*E*)-13-fluoro-14b,15,16,17-tetrahydro-9*H*-4,8-(azeno)-1,19-etheno imidazo[1,5-*h*]pyrido[2,3-*b*]pyrrolo[2,1-*d*][1]oxa[5,7,8,11]tetraazacyclopentadecine(I-63)**

**Step A: (*R*)-(2-(6-(2-(5-fluoro-2-methoxypyridin-3-yl)pyrrolidin-1-yl)imidazo[1,2-*b*] pyridazin-3-yl)pyrimidin-4-yl) methanol (123-2)**

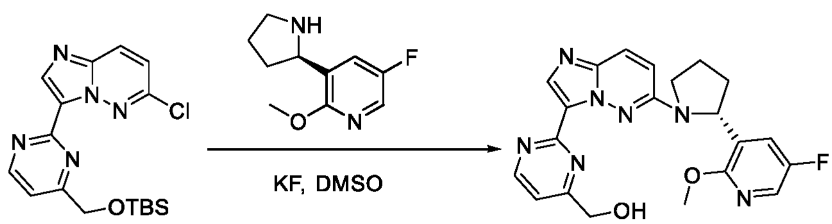


121

123-2

The title Compound **123-2** was prepared with reference to the method of Compound **43** by replacing Compound **19** with (***R***)-5-fluoro-2-methoxy-3-(pyrrolidin-2-yl)pyridine and Compound **42** with Compound **121** as the raw material. **LCMS:** m/z (ESI), 422.1[M+H]$^+$.

**Step B: (*R*)-5-fluoro-3-(1-(3-(4-(hydroxymethyl)pyrimidin-2-yl)imidazo[1,2-*b*] pyridazin-6-yl)pyrrolidin-2-yl)pyridin-2(1*H*)-one (123-3)**

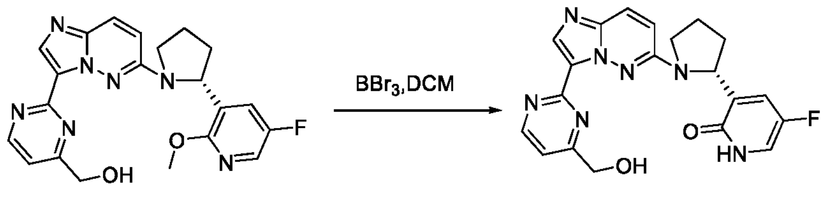


123-2 123-3

The title Compound **123-3** was prepared with reference to the method of Compound **11** by replacing Compound **10** with Compound **123-2** as the raw material. **LCMS:** m/z (ESI), 408.1[M+H]$^+$.

**Step C: (14b*R*,*E*)-13-fluoro-14b,15,16,17-tetrahydro-9*H*-4,8-(azeno)-1,19-etheno imidazo[1,5-*h*]pyrido[2,3-*b*]pyrrolo[2,1-*d*][1]oxa[5,7,8,11]tetraazacyclopentadecine(I-63)**

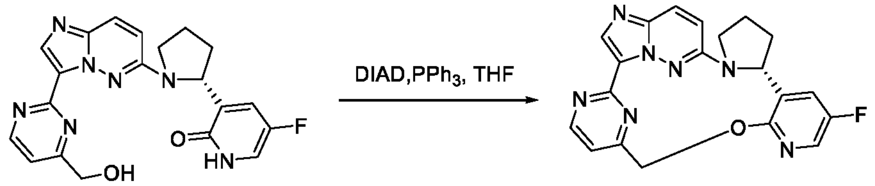


I-63

123-3

The title Compound **I-63** was prepared with reference to the method of Compound **I-9** by replacing Compound **39** with Compound **123-3** as the raw material. **LCMS:** m/z (ESI), 390[M+H]$^+$.

**Example 64: (14b*R*,16*S*,*E*)-13,16-difluoro-14b,15,16,17-tetrahydro-9*H*-4,8-(azeno)-1,19-ethenoimidazo[1,5-*h*]pyrido[2,3-*b*]pyrrolo[2,1-*d*][1]oxa[5,7,8,11]tetraazacyclopentadeci ne(I-64)**

**Step A: (2-(6-((2*R*,4*S*)-4-fluoro-2-(5-fluoro-2-methoxypyridin-3-yl)pyrrolidin-1-yl) imidazo[1,2-*b*]pyridazin-3-yl) pyrimidin-4-yl)methanol (123-4)**

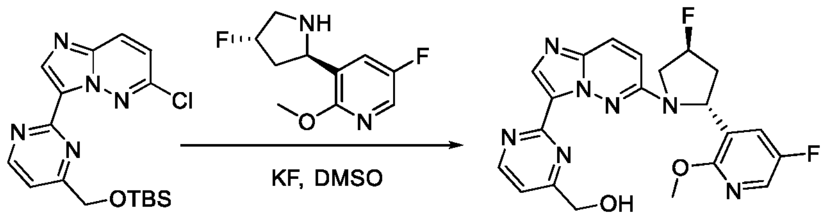


121

123-4

The title Compound **123-4** was prepared with reference to the method of Compound **43** by replacing Compound **19** with 5-fluoro-3-((2***R***,4***S***)-4-fluoropyrrolidin-2-yl)-2-methoxypyridine and Compound **42** with Compound **121** as the raw material. **LCMS:** m/z (ESI), 440.1 [M+H]$^+$.

**Step B: 5-fluoro-3-((2R,4S)-4-fluoro-1-(3-(4-(hydroxymethyl)pyrimidin-2-yl)imidazo [1,2-b]pyridazin-6-yl)pyrrolidin-2-yl)pyridin-2(1H)-one (123-5)**

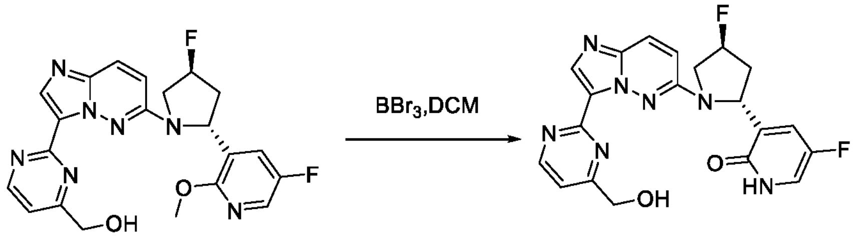


123-4 123-5

The title Compound **123-5** was prepared with reference to the method of Compound **11** by replacing Compound **10** with Compound **123-4** as the raw material. **LCMS:** m/z (ESI), 426.1[M+H]$^+$.

**Step C: (14b*R*,16*S*,*E*)-13,16-difluoro-14b,15,16,17-tetrahydro-9*H*-4,8-(azeno)-1,19-ethenoimidazo[1,5-*h*]pyrido[2,3-*b*]pyrrolo[2,1-*d*][1]oxa[5,7,8,11]tetraazacyclopentadeci ne(I-64)**

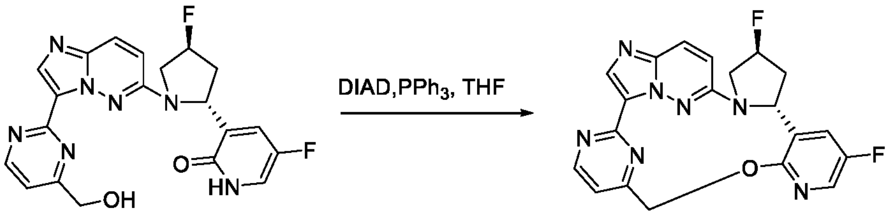


123-5 I-64

The title Compound **I-64** was prepared with reference to the method of Compound **I-9** by replacing Compound **39** with Compound **123-5** as the raw material. **LCMS:** m/z (ESI), 408[M+H]$^+$.

**Example 65: (14b*R*,*E*)-13-fluoro-14b,15,16,17-tetrahydro-9*H*-1,19-etheno-4,8-(metheno)benzo[n]imidazo[5,1-*h*]pyrrolo[1,2-*l*][1]oxa[5,6,9,10,12]pentaazacyclopentadecine( I-65)**

**Step A: (6-chloropyridazin-4-yl)methanol(124)**

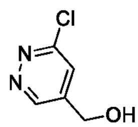

**124**

The title Compound **124** was prepared by the method in the reference of **Bioorganic and Medicinal Chemistry Letters, 2019,** vol. 29, # 23. **LCMS:** m/z (ESI),145.0[M+H]$^+$.

**Step B: 5-(((tert-butyldimethylsilyl)oxy)methyl)-3-chloropyridazine(125)**

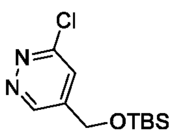

**125**

The title Compound **125** was prepared with reference to the method of Compound **2** by replacing Compound **1** with Compound **124** as the raw material. **LCMS:** m/z (ESI),259.0[M+H]+.

**Step C: 5-(((tert-butyldimethylsilyl)oxy)methyl)-3-(tributylstannyl)pyridazine(126)**

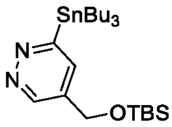

**126**

The title Compound **126** was prepared with reference to the method of Compound 3 by replacing Compound **2** with Compound **125** as the raw material. **LCMS:** m/z (ESI),515.0[M+H]+.

**Step D: 3-(5-(((tert-butyldimethylsilyl)oxy)methyl)pyridazin-3-yl)-6-chloroimidazo [1,2-b]pyridazine(127)**

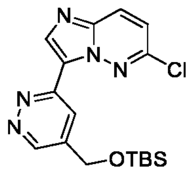

**127**

**Step E: (*R*)-3-(5-(((tert-butyldimethylsilyl)oxy)methyl)pyridazin-3-yl)-6-(2-(5-fluoro-2-methoxyphenyl)pyrrolidin-1-yl)imidazo[1,2-b]pyridazine (128)**

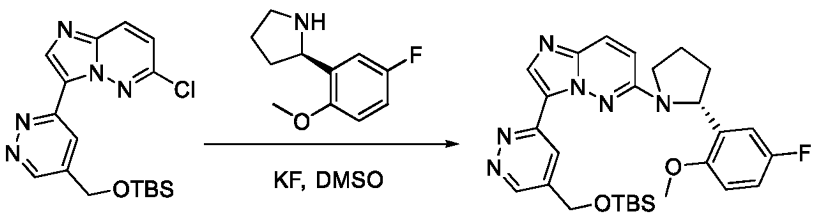


**127** **128**

The title Compound **128** was prepared with reference to the method of Compound **43** by replacing Compound **19** with (R)-2-(5-fluoro-2-methoxyphenyl)pyrrolidine and Compound **42** with Compound **127** as the raw material. **LCMS:** m/z (ESI), 535.1[M+H]+.

**Step F: (*R*)-4-fluoro-2-(1-(3-(5-(hydroxymethyl)pyridazin-3-yl)imidazo[1,2-b]pyridazin -6-yl)pyrrolidin-2-yl)phenol (128-1)**

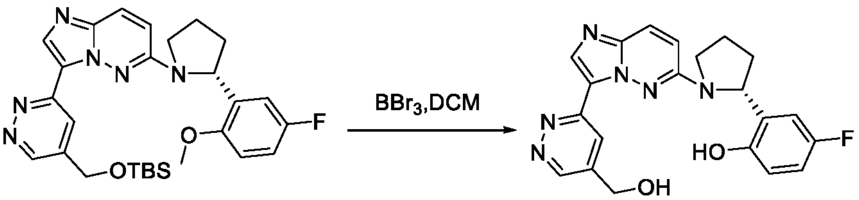

128

128-1

The title Compound **128-1** was prepared with reference to the method of Compound **11** by replacing Compound **10** with Compound **128** as the raw material. **LCMS:** m/z (ESI), 407.1[M+H]+.

**Step F: (14b*R*,*E)*-13-fluoro-14b,15,16,17-tetrahydro-9*H*-1,19-etheno-4,8-(metheno) benzo[*n*]imidazo[5,1-*h*]pyrrolo[1,2-*l*][1]oxa[5,6,9,10,12]pentaazacyclopentadecine(I-65)**

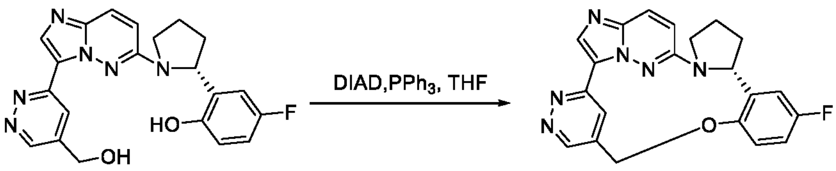

128-1

I-65

The title Compound **I-65** was prepared with reference to the method of Compound **I-9** by replacing Compound **39** with Compound **128-1** as the raw material. **LCMS:** m/z (ESI), 389[M+H]+

**Example 66: (14b*R*,16*S*,*E*)-13,16-difluoro-14b,15,16,17-tetrahydro-9*H*-1,19-etheno-4,8-(metheno)benzo[*n*]imidazo[5,1-*h*]pyrrolo[1,2-*l*][1]oxa[5,6,9,10,12]pentaazacyclopentadec ine(I-66)**

**Step A: 3-(5-(((tert-butyldimethylsilyl)oxy)methyl)pyridazin-3-yl)-6-((2*R*,4*S*)-4-fluoro -2-(5-fluoro-2-methoxyphenyl)pyrrolidin-1-yl)imidazo[1,2-b]pyridazine (129)**

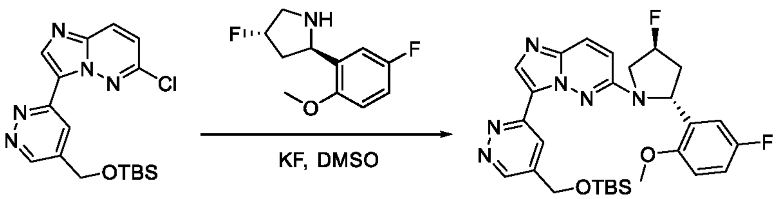

127

129

The title Compound **129** was prepared with reference to the method of Compound **43** by replacing Compound **19** with (2***R***,4***S***)-4-fluoro-2-(5-fluoro-2-methoxyphenyl)pyrrolidine and Compound **42** with Compound **127** as the raw material. **LCMS:** m/z (ESI), 553.1[M+H]+.

**Step B: 4-fluoro-2-((2*R*,4*S*)-4-fluoro-1-(3-(5-(hydroxymethyl)pyridazin-3-yl)imidazo [1,2-b]pyridazin-6-yl)pyrrolidin-2-yl)phenol (129-1)**

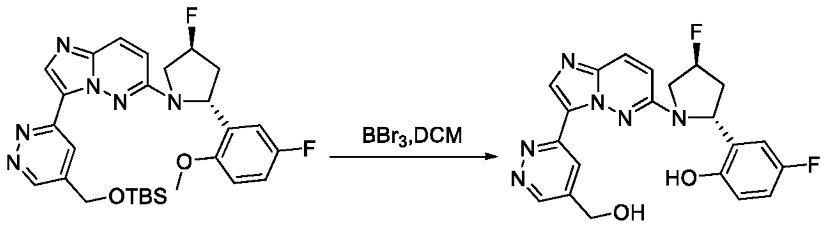


129

129-1

The title Compound **129-1** was prepared with reference to the method of Compound **11** by replacing Compound **10** with Compound **129** as the raw material. **LCMS:** m/z (ESI), 425.1[M+H]$^+$.

**Step C: (14b*R*,16*S*,*E*)-13,16-difluoro-14b,15,16,17-tetrahydro-9*H*-1,19-etheno-4,8-(metheno)benzo[*n*]imidazo [5,1-*h*]pyrrolo[1,2-*l*][1]oxa[5,6,9,10,12]pentaazacyclopentadec ine(I-66)**

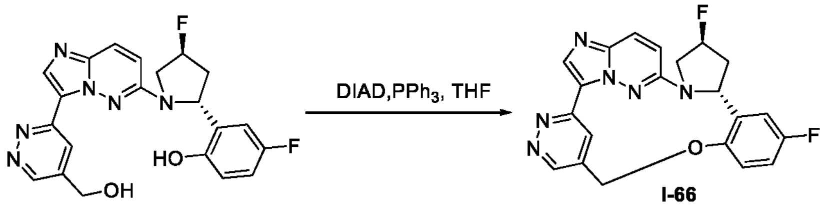


129-1

I-66

The title Compound **I-66** was prepared with reference to the method of Compound **I-9** by replacing Compound **39** with Compound **129-1** as the raw material. **LCMS:** m/z (ESI), 407[M+H]$^+$.

**Example 67: (14b*R*,*E*)-13-fluoro-14b,15,16,17-tetrahydro-9*H*-1,19-etheno-4,8-(metheno)imidazo[5,1-*h*]pyrido [3,2-*n*]pyrrolo[1,2-*l*][1]oxa[5,6,9,10,12]pentaazacyclopentade cine(I-67)**

**Step A: (*R*)-(6-(6-(2-(5-fluoro-2-methoxypyridin-3-yl)pyrrolidin-1-yl)imidazo[1,2-*b*] pyridazin-3-yl)pyridazin-4-yl) methanol (129-2)**

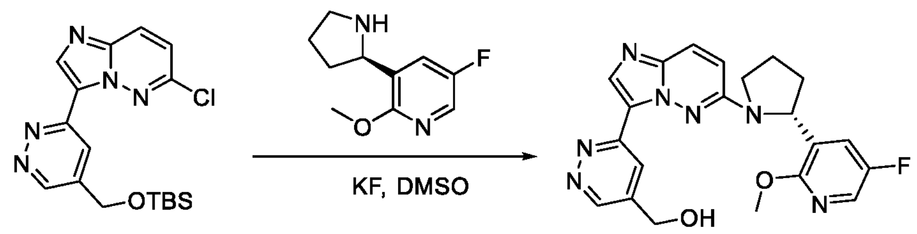


127

129-2

The title Compound **129-2** was prepared with reference to the method of Compound **43** by replacing Compound **19** with (***R***)-5-fluoro-2-methoxy-3-(pyrrolidin-2-yl)pyridine and Compound **42** with Compound **127** as the raw material. **LCMS:** m/z (ESI), 422.1[M+H]$^+$.

**Step B: (*R)*-5-fluoro-3-(1-(3-(5-(hydroxymethyl)pyridazin-3-yl)imidazo[1,2-*b*] pyridazin-6-yl)pyrrolidin-2-yl)pyridin-2(1*H*)-one (129-3)**

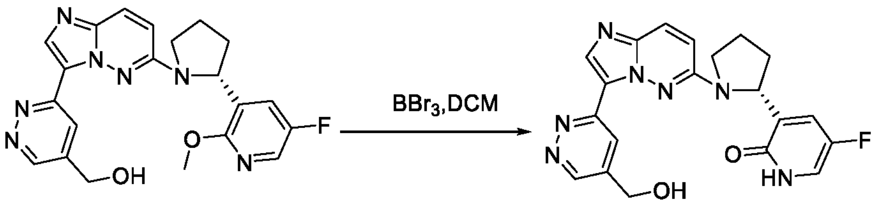


129-2

129-3

The title Compound **129-3** was prepared with reference to the method of Compound **11** by replacing Compound **10** with Compound **129-2** as the raw material. **LCMS:** m/z (ESI), 408.1[M+H]$^+$.

**Step C: (14b*R*,*E*)-13-fluoro-14b,15,16,17-tetrahydro-9*H*-1,19-etheno-4,8-(metheno) imidazo[5,1-*h*]pyrido[3,2-*n*] pyrrolo[1,2-*l*][1]oxa[5,6,9,10,12]pentaazacyclopentadecine(I-67)**

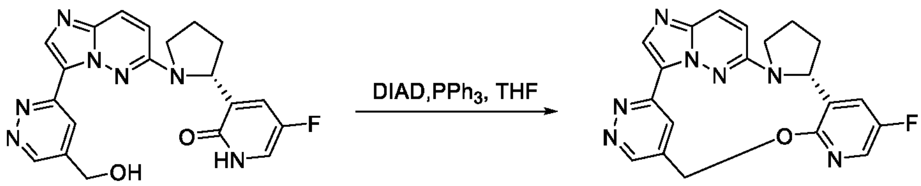


129-3

I-67

The title Compound **I-67** was prepared with reference to the method of Compound **I-9** by replacing Compound **39** with Compound **129-3** as the raw material. **LCMS:** m/z (ESI), 390[M+H]$^+$.

**Example 68: (14b*R*,16*S*,*E*)-13,16-difluoro-14b,15,16,17-tetrahydro-9*H*-1,19-etheno -4,8-(metheno)imidazo[5,1-*h*] pyrido[3,2-*n*]pyrrolo[1,2-*l*][1]oxa[5,6,9,10,12]pentaazacyclopen tadecine(I-68)**

**Step A: (6-(6-((2*R*,4*S*)-4-fluoro-2-(5-fluoro-2-methoxypyridin-3-yl)pyrrolidin-1-yl) imidazo[1,2-b]pyridazin-3-yl) pyridazin-4-yl)methanol (129-4)**

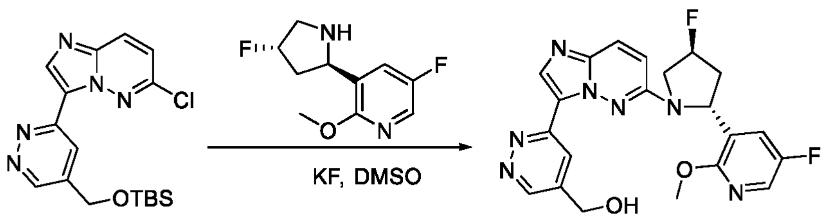


127

129-4

The title Compound **129-4** was prepared with reference to the method of Compound **43** by replacing Compound **19** with 5-fluoro-3-((2***R***,4***S***)-4-fluoropyrrolidin-2-yl)-2-methoxypyridine and Compound **42** with Compound **127** as the raw material. **LCMS:** m/z (ESI), 440.1 [M+H]$^+$.

**Step B: 5-fluoro-3-((2*R*,4*S*)-4-fluoro-1-(3-(5-(hydroxymethyl)pyridazin-3-yl)imidazo [1,2-*b*]pyridazin-6-yl)pyrrolidin-2-yl)pyridin-2(1*H*)-one (129-5)**

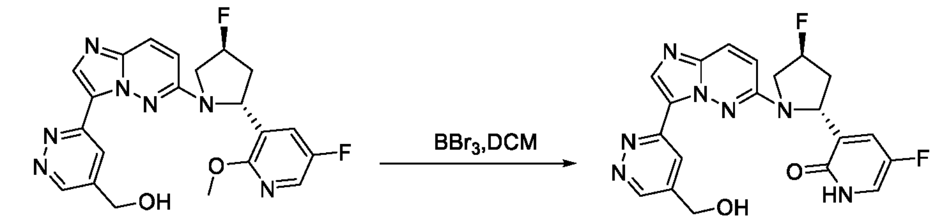


129-4 129-5

The title Compound **129-5** was prepared with reference to the method of Compound **11** by replacing Compound **10** with Compound **129-4** as the raw material. **LCMS:** m/z (ESI), 426.1[M+H]$^+$.

**Step C: (14b*R*,16*S*,*E*)-13,16-difluoro-14b,15,16,17-tetrahydro-9*H*-1,19-etheno-4,8-(metheno)imidazo[5,1-*h*]pyrido[3,2-*n*]pyrrolo[1,2-*l*][1]oxa[5,6,9,10,12]pentaazacyclopentade cine(I-68)**

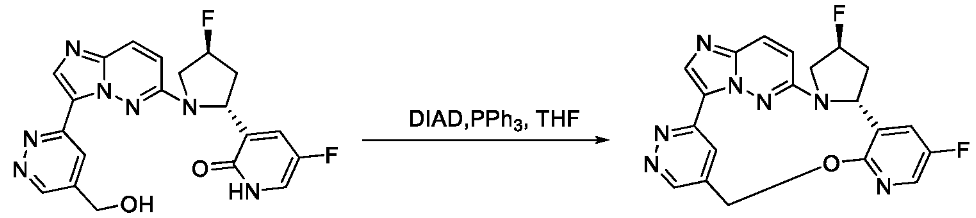


129-5 I-68

The title Compound **I-68** was prepared with reference to the method of Compound **I-9** by replacing Compound **39** with Compound **129-5** as the raw material. **LCMS:** m/z (ESI), 408[M+H]$^+$

**Example 69: (3$^2$*R*,*E*)-4$^5$-fluoro-5-oxa-2(3,6)-imidazo[1,2-*b*]pyridazina-1(4,6)-pyrimi dina-3(1,2)-pyrrolidina-4(1,2)-benzenacycloheptaphane(I-69)**

**Step A: (*R*)-2-(6-(6-(2-(5-fluoro-2-methoxyphenyl)pyrrolidin-1-yl)imidazo[1,2-*b*] pyridazin-3-yl)pyrimidin-4-yl) ethan-1-ol (130)**

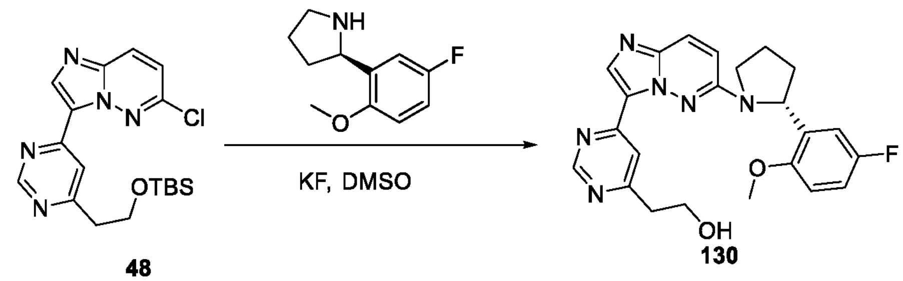


48 130

The title Compound **130** was prepared with reference to the method of Compound **48-1** by replacing Compound **19** with (***R***)-2-(5-fluoro-2-methoxyphenyl)pyrrolidine as the raw material. **LCMS:** m/z (ESI), 435.1[M+H]$^+$.

**Step B: (*R*)-4-fluoro-2-(1-(3-(6-(2-hydroxyethyl)pyrimidin-4-yl)imidazo[1,2-*b*] pyridazin-6-yl)pyrrolidin-2-yl)phenol (130-1)**

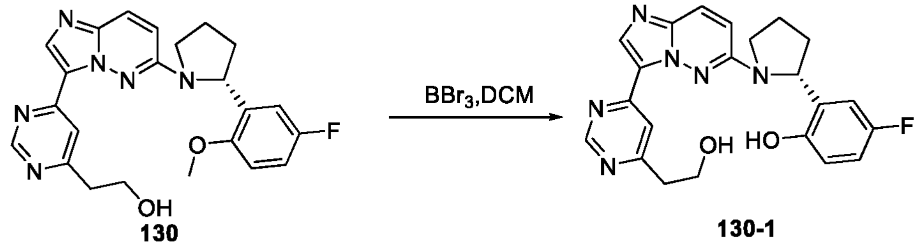


130 130-1

The title Compound **130-1** was prepared with reference to the method of Compound **11** by replacing Compound **10** with Compound **130** as the raw material. **LCMS:** m/z (ESI), 421.1[M+H]$^+$.

**Step C: ($3^2$*R*,*E*)-$4^5$-fluoro-5-oxa-2(3,6)-imidazo[1,2-*b*]pyridazina-1(4,6)-pyrimidina -3(1,2)-pyrrolidina-4(1,2)-benzenacycloheptaphane(I-69)**

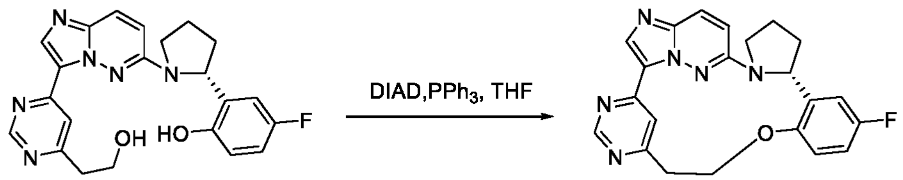


130-1 I-69

The title Compound **I-69** was prepared with reference to the method of Compound **I-9** by replacing Compound **39** with Compound **130-1** as the raw material. **LCMS:** m/z (ESI), 403 [M+H]$^+$.

**Example 70: ($3^2$*R*,$3^4$*S*,*E*)-$3^4$,$4^5$-difluoro-5-oxa-2(3,6)-imidazo[1,2-*b*]pyridazina-1(4,6)-pyrimidina-3(1,2)-pyrrolidina-4(1,2)-benzenacycloheptaphane(I-70)**

**Step A: 2-(6-(6-((2R,4S)-4-fluoro-2-(5-fluoro-2-methoxyphenyl)pyrrolidin-1-yl)imidazo [1,2-b]pyridazin-3-yl)pyrimidin-4-yl)ethan-1-ol (131)**

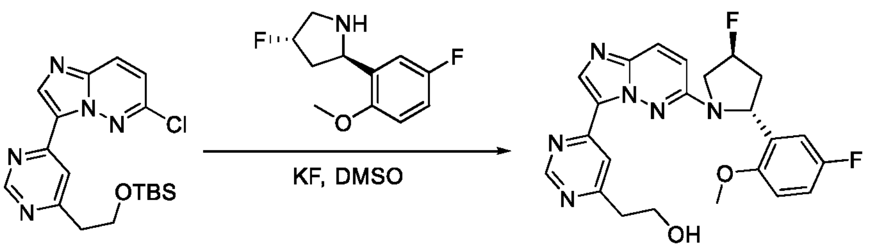


48 131

The title Compound **131** was prepared with reference to the method of Compound **48-1** by replacing Compound **19** with (2*R*,4*S*)-4-fluoro-2-(5-fluoro-2-methoxyphenyl)pyrrolidine as the raw material. **LCMS:** m/z (ESI), 453.1[M+H]$^+$.

**Step B: 4-fluoro-2-((2*R*,4*S*)-4-fluoro-1-(3-(6-(2-hydroxyethyl)pyrimidin-4-yl)imidazo [1,2-b]pyridazin-6-yl)pyrrolidin-2-yl)phenol (131-1)**

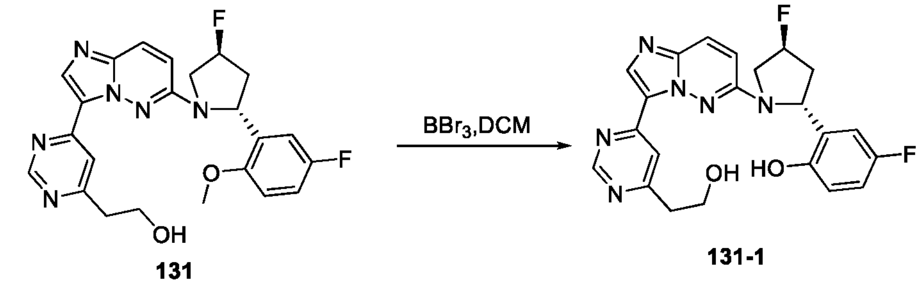


131

131-1

The title Compound **131-1** was prepared with reference to the method of Compound **11** by replacing Compound **10** with Compound **131** as the raw material. **LCMS:** m/z (ESI), 439.1[M+H]$^+$.

**Step C: (3$^2$*R*,3$^4$*S*,*E*)-3$^4$,4$^5$-difluoro-5-oxa-2(3,6)-imidazo[1,2-*b*]pyridazina-1(4,6)-pyrimidina-3(1,2)-pyrrolidina-4(1,2)-benzenacycloheptaphane(I-70)**

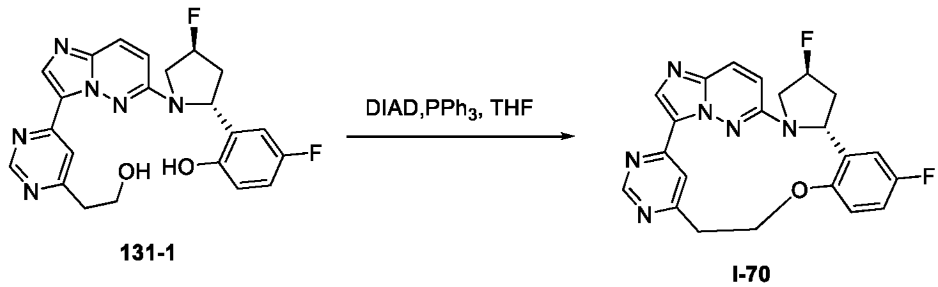


131-1

I-70

The title Compound **I-70** was prepared with reference to the method of Compound **I-9** by replacing Compound **39** with Compound **131-1** as the raw material. **LCMS:** m/z (ESI), 421 [M+H]$^+$.

**Example 71: (3$^2$*R*,*E*)-4$^5$-fluoro-5-oxa-2(3,6)-imidazo[1,2-*b*]pyridazina-1(4,6)-pyrimidina-4(3,2)-pyridina-3(1,2)-pyrrolidinacycloheptaphane(I-71)**

**Step A: (*R*)-2-(6-(6-(2-(5-fluoro-2-methoxypyridin-3-yl)pyrrolidin-1-yl)imidazo[1,2-b] pyridazin-3-yl)pyrimidin-4-yl)ethan-1-ol (131-2)**

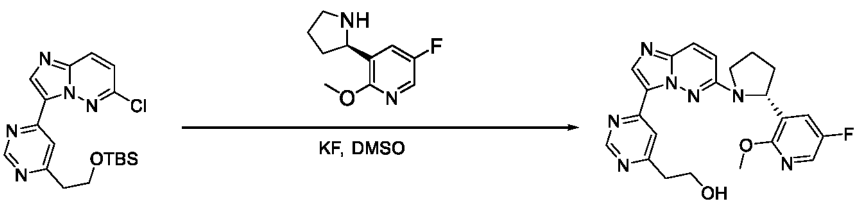


48

131-2

The title Compound **131-2** was prepared with reference to the method of Compound **48-1** by replacing Compound **19** with (R)-5-fluoro-2-methoxy-3-(pyrrolidin-2-yl)pyridine as the raw **material.LCMS:** m/z (ESI), 436.1 [M+H]$^+$.

**Step B: (*R*)-5-fluoro-3-(1-(3-(6-(2-hydroxyethyl)pyrimidin-4-yl)imidazo[1,2-*b*] pyridazin-6-yl)pyrrolidin-2-yl)pyridin-2(1*H*)-one (131-3)**

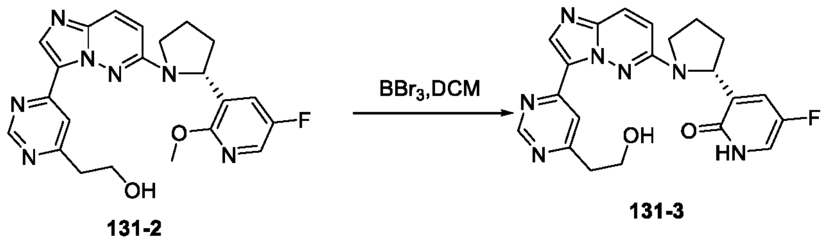


131-2 131-3

The title Compound **131-3** was prepared with reference to the method of Compound **11** by replacing Compound **10** with Compound **131-2** as the raw material. **LCMS:** m/z (ESI), 422.1[M+H]+.

**Step C: (3$^2$*R*,*E*)-4$^5$-fluoro-5-oxa-2(3,6)-imidazo[1,2-*b*]pyridazina-1(4,6)-pyrimidina -4(3,2)-pyridina-3(1,2)-pyrrolidinacycloheptaphane(I-71)**

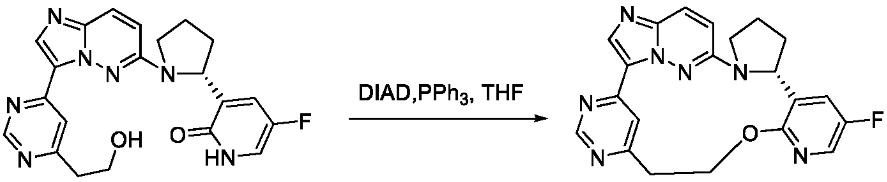


131-3 I-71

The title Compound **I-71** was prepared with reference to the method of Compound **I-9** by replacing Compound **39** with Compound **131-3** as the raw material. **LCMS:** m/z (ESI), 404[M+H]+.

**Example 72: (3$^2$*R*,3$^4$*S*,*E*)-3$^4$,4$^5$-difluoro-5-oxa-2(3,6)-imidazo[1,2-*b*]pyridazina-1(4,6)-pyrimidina-4(3,2)-pyridina-3(1,2)-pyrrolidinacycloheptaphane(I-72)**

**Step A: 2-(6-(6-((2*R*,4*S*)-4-fluoro-2-(5-fluoro-2-methoxypyridin-3-yl)pyrrolidin-1-yl) imidazo[1,2-b]pyridazin-3-yl)pyrimidin-4-yl)ethan-1-ol (131-4)**

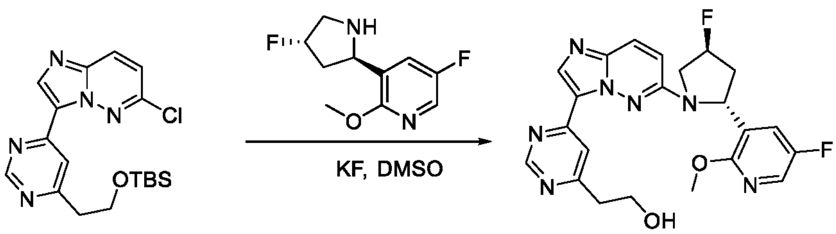


48 131-4

The title Compound **131-4** was prepared with reference to the method of Compound **48-1** by replacing Compound **19** with 5-fluoro-3-((2*R*,4*S*)-4-fluoropyrrolidin-2-yl)-2-methoxypyridine as the raw material. **LCMS:** m/z (ESI), 454.1 [M+H]+.

**Step B: 5-fluoro-3-((2*R*,4*S*)-4-fluoro-1-(3-(6-(2-hydroxyethyl)pyrimidin-4-yl)imidazo [1,2-b]pyridazin-6-yl)pyrrolidin-2-yl)pyridin-2(1H)-one (131-5)**

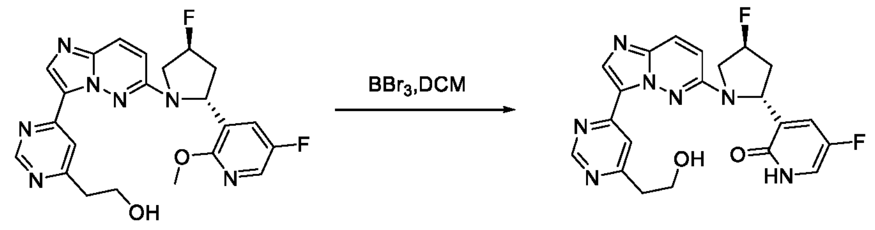


131-4 131-5

The title Compound **131-5** was prepared with reference to the method of Compound **11** by replacing Compound **10** with Compound **131-4** as the raw material. **LCMS:** m/z (ESI), 440. 1 [M+H]+.

**Step C: ($3^2$*R*,$3^4$*S*,*E*)-$3^4$,$4^5$-difluoro-5-oxa-2(3,6)-imidazo[1,2-*b*]pyridazina-1(4,6)-pyrimidina-4(3,2)-pyridina-3(1,2)-pyrrolidinacycloheptaphane(I-72)**

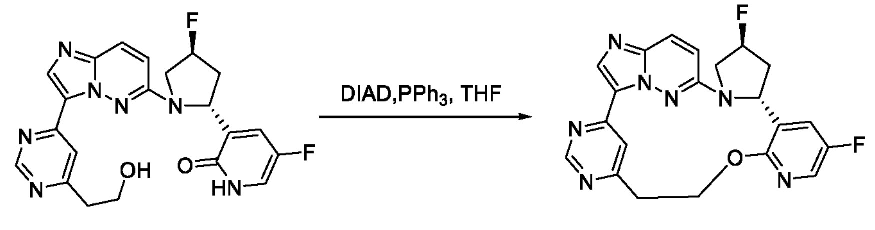


131-5 I-72

The title Compound **I-72** was prepared with reference to the method of Compound **I-9** by replacing Compound **39** with Compound **131-5** as the raw material. **LCMS:** m/z (ESI), 422[M+H]+.

**Example 73: (4*R*,*E*)-$5^5$-fluoro-4-methyl-6-oxa-3-aza-2(3,6)-imidazo[1,2-*b*]pyridazina -1(4,6)-pyrimidina-5(3,2)-pyridinacyclooctaphane(I-73)**

**Step A: (*R*)-2-(6-(6-((1-(5-fluoro-2-methoxypyridin-3-yl)ethyl)amino)imidazo[1,2-*b*] pyridazin-3-yl)pyrimidin-4-yl)ethan-1-ol (48-2)**

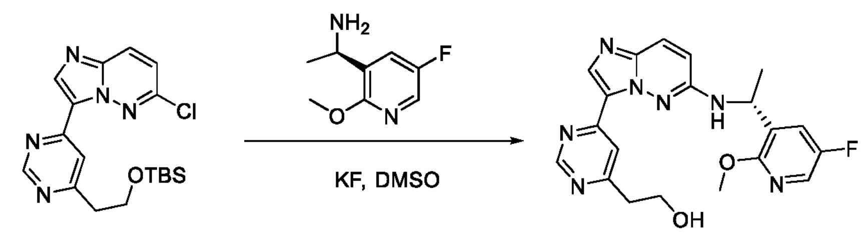


48 48-2

The title Compound **48-2** was prepared with reference to the method of Compound **48-1** by replacing Compound **19** with (1***R***)-1-(5-fluoro-2-methoxypyridin-3-yl)ethan-1-amine as the raw material. **LCMS:** m/z (ESI), 410.1 [M+H]+.

**Step B: (*R*)-5-fluoro-3-(1-((3-(6-(2-hydroxyethyl)pyrimidin-4-yl)imidazo[1,2-*b*] pyridazin-6-yl)amino)ethyl)pyridin-2(1*H*)-one (48-3)**

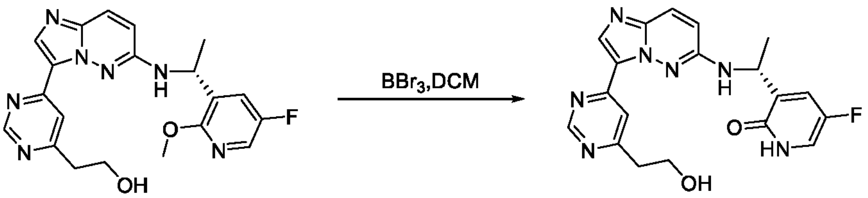


48-2 48-3

The title Compound **48-3** was prepared with reference to the method of Compound **11** by replacing Compound **10** with Compound **48-2** as the raw material. **LCMS:** m/z (ESI), 396.1[M+H]$^+$.

**Step C: (4*R*,*E*)-5$^5$-fluoro-4-methyl-6-oxa-3-aza-2(3,6)-imidazo[1,2-*b*]pyridazina -1(4,6)-pyrimidina-5(3,2)-pyridinacyclooctaphane(I-73)**

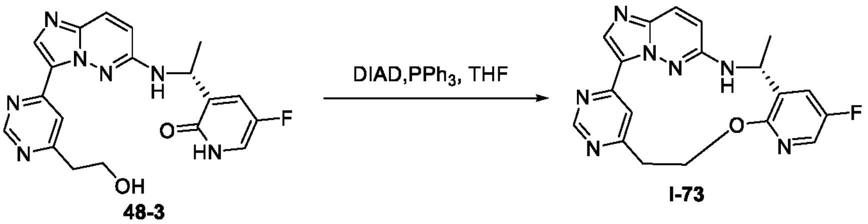


48-3 I-73

The title Compound **I-73** was prepared with reference to the method of Compound **I-9** by replacing Compound **39** with Compound **48-3** as the raw material. **LCMS:** m/z (ESI), 378[M+H]$^+$.

**Example 74: (4*R*,*E*)-5$^5$-fluoro-4-methyl-6-oxa-3-aza-2(3,6)-imidazo[1,2-*b*]pyridazina -1(4,6)-pyrimidina-5(1,2)-benzenacyclooctaphane(I-74)**

**Step A: (*R*)-2-(6-(6-((1-(5-fluoro-2-hydroxyphenyl)ethyl)amino)imidazo[1,2-*b*] pyridazin-3-yl)pyrimidin-4-yl) ethan-1-ol (48-4)**

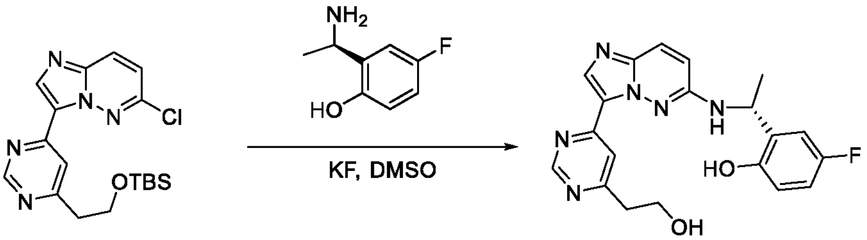


48 48-4

The title Compound **48-4** was prepared with reference to the method of Compound **48-1** by replacing Compound **19** with (1***R***)-1-(5-fluoro-2-hydroxyphenyl)ethan-1-amine as the raw material. **LCMS:** m/z (ESI), 395.1[M+H]$^+$.

**Step B: (4*R*,*E*)-5$^5$-fluoro-4-methyl-6-oxa-3-aza-2(3,6)-imidazo[1,2-*b*]pyridazina-1(4,6)-pyrimidina-5(1,2)-benzenacyclooctaphane(I-74)**

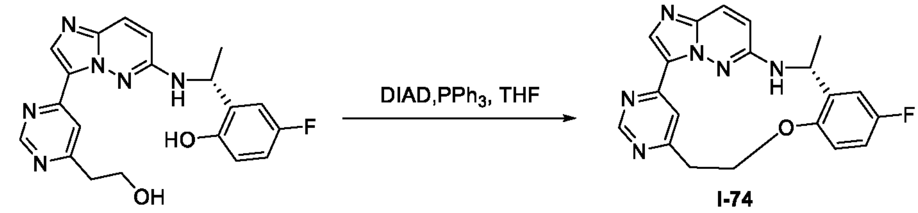


48-4 I-74

The title Compound **I-74** was prepared with reference to the method of Compound **I-9** by replacing Compound **39** with Compound **48-4** as the raw material. **LCMS:** m/z (ESI), 377[M+H]$^+$.

**Example 75: (4*R*,*E*)-5$^5$-fluoro-4-methyl-6-oxa-3-aza-2(3,6)-imidazo[1,2-*b*]pyridazina-1(2,4),5(3,2)-dipyridinacyclooctaphane(I-75)**

**Step A: (*R*)-2-(2-(6-((1-(5-fluoro-2-methoxypyridin-3-yl)ethyl)amino)imidazo[1,2-*b*] pyridazin-3-yl)pyridin-4-yl) ethan-1-ol (37-2)**

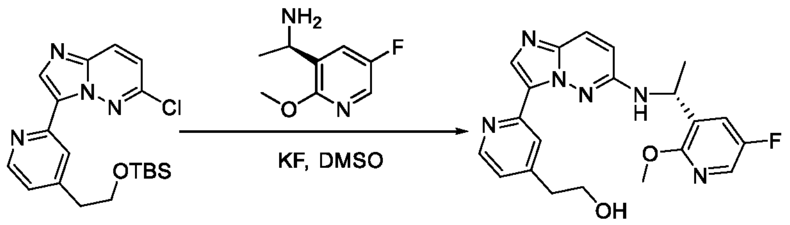


37 37-2

The title Compound **37-2** was prepared with reference to the method of Compound **38** by replacing Compound **19** with (1***R***)-1-(5-fluoro-2-methoxypyridin-3-yl)ethan-1-amine as the raw material. **LCMS:** m/z (ESI), 394.1[M+H]$^+$.

**Step B: (*R*)-5-fluoro-3-(1-((3-(4-(2-hydroxyethyl)pyridin-2-yl)imidazo[1,2-*b*]pyridazin -6-yl)amino)ethyl)pyridin-2(1*H*)-one (37-3)**

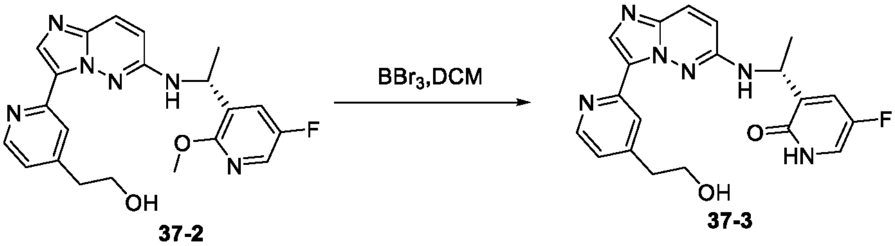


37-2 37-3

The title Compound **37-3** was prepared with reference to the method of Compound **11** by replacing Compound **10** with Compound **37-2** as the raw material. **LCMS:** m/z (ESI), 380.1[M+H]$^+$.

**Step C: (4*R*,*E*)-5$^5$-fluoro-4-methyl-6-oxa-3-aza-2(3,6)-imidazo[1,2-*b*]pyridazina-1(2,4),5(3,2)-dipyridinacyclooctaphane(I-75)**

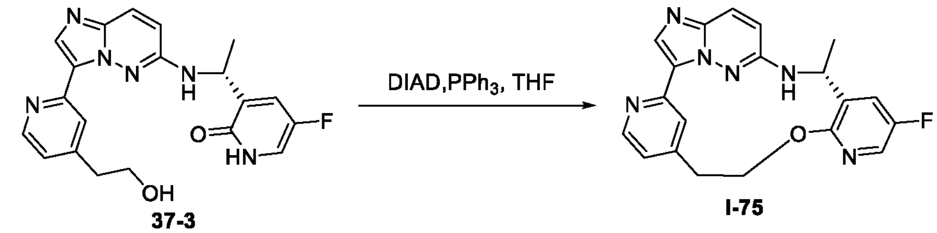


37-3 I-75

The title Compound **I-75** was prepared with reference to the method of Compound **I-9** by replacing Compound **39** with Compound **37-3** as the raw material. **LCMS:** m/z (ESI), 362[M+H]+.

**Example 76: (4*R*,*E*)-5⁵-fluoro-4-methyl-6-oxa-3-aza-2(3,6)-imidazo[1,2-*b*]pyridazina-1(2,4)-pyridina-5(1,2)-benzenacyclooctaphane(I-76)**

**Step A: (*R*)-4-fluoro-2-(1-((3-(4-(2-hydroxyethyl)pyridin-2-yl)imidazo[1,2-*b*] pyridazin-6-yl)amino)ethyl)phenol (37-4)**

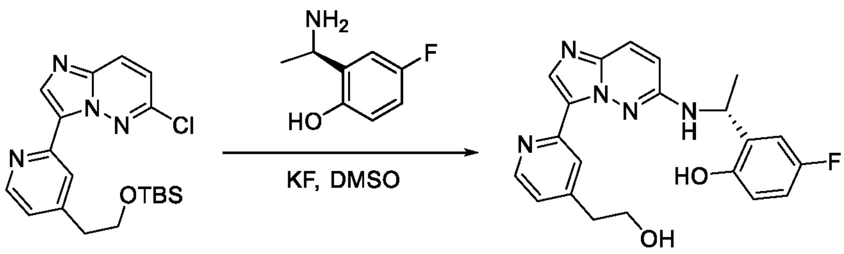


37 37-4

The title Compound **37-4** was prepared with reference to the method of Compound **38** by replacing Compound **19** with (1***R***)-1-(5-fluoro-2-hydroxyphenyl)ethan-1-amine as the raw material. **LCMS:** m/z (ESI), 394.1[M+H]+.

**Step B: (4*R*,*E*)-5⁵-fluoro-4-methyl-6-oxa-3-aza-2(3,6)-imidazo[1,2-*b*]pyridazina-1(2,4)-pyridina-5(1,2)-benzenacyclooctaphane(I-76)**

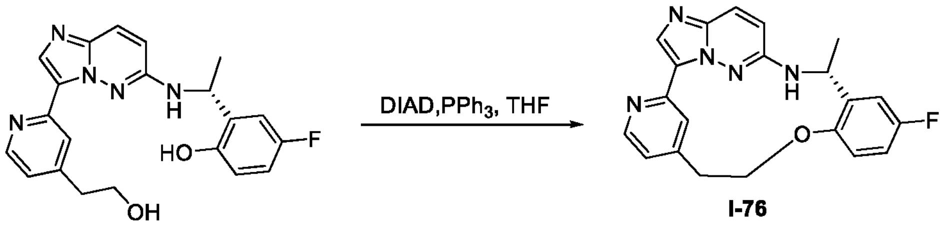


37-4 I-76

The title Compound **I-76** was prepared with reference to the method of Compound **I-9** by replacing Compound **39** with Compound **37-4** as the raw material. **LCMS:** m/z (ESI), 377[M+H]+

**Example 77: (6*R*,*E*)-5⁵-fluoro-6-methyl-4-oxa-7-aza-1(3,6)-imidazo[1,2-*b*]pyridazina-2(4,6)-pyrimidina-5(2,3)-pyridinacycloheptaphane(I-77)**

**Step A: (*R*)-(6-(6-((1-(5-fluoro-2-methoxypyridin-3-yl)ethyl)amino)imidazo[1,2-*b*]pyri dazin-3-yl)pyrimidin-4-yl) methanol (42-2)**

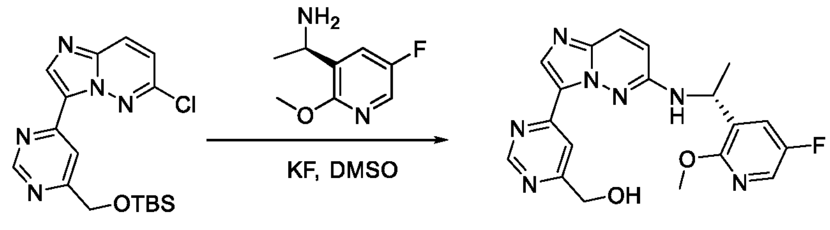


42 42-2

The title Compound **42-2** was prepared with reference to the method of Compound **43** by replacing Compound **19** with (1***R***)-1-(5-fluoro-2-methoxypyridin-3-yl)ethan-1-amine as the raw material. **LCMS:** m/z (ESI), 396.1[M+H]+.

**Step B: (*R*)-5-fluoro-3-(1-((3-(6-(hydroxymethyl)pyrimidin-4-yl)imidazo[1,2-*b*] pyridazin-6-yl)amino)ethyl)pyridin-2(1H)-one (42-3)**

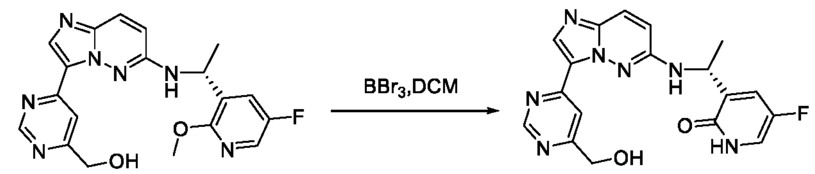


42-2 42-3

The title Compound **42-3** was prepared with reference to the method of Compound **11** by replacing Compound **10** with Compound **42-2** as the raw material. **LCMS:** m/z (ESI), 382.1[M+H]+.

**Step C: (6*R*,*E*)-$5^5$-fluoro-6-methyl-4-oxa-7-aza-1(3,6)-imidazo[1,2-*b*]pyridazina-2(4,6)-pyrimidina-5(2,3)-pyridinacycloheptaphane(I-77)**

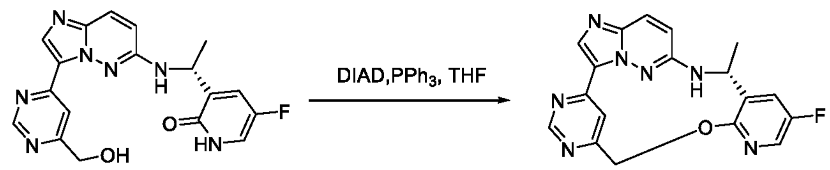


42-3 I-77

The title Compound **I-77** was prepared with reference to the method of Compound **I-9** by replacing Compound **39** with Compound **42-3** as the raw material. **LCMS:** m/z (ESI), 364[M+H]+.

**Example 78: (6*R*,*E*)-$5^4$-fluoro-6-methyl-4-oxa-7-aza-1(3,6)-imidazo[1,2-*b*]pyridazina-2(4,6)-pyrimidina-5(1,2)-benzenacycloheptaphane(I-78)**

**Step A: (*R*)-4-fluoro-2-(1-((3-(6-(hydroxymethyl)pyrimidin-4-yl)imidazo[1,2-*b*] pyridazin-6-yl)amino)ethyl)phenol (42-4)**

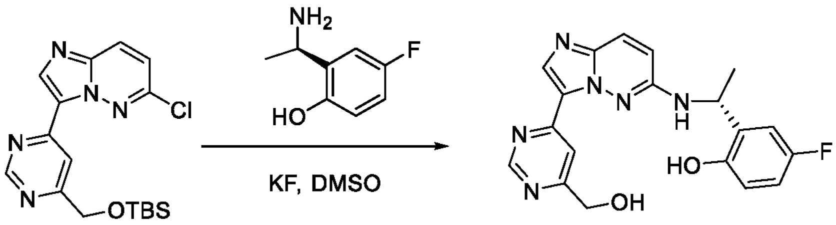


42 42-4

The title Compound **42-4** was prepared with reference to the method of Compound **43** by replacing Compound **19** with (***R***)-1-(5-fluoro-2-hydroxyphenyl)ethan-1-amine as the raw material. **LCMS:** m/z (ESI), 381.1[M+H]+.

**Step B: (6*R*,*E*)-5⁴-fluoro-6-methyl-4-oxa-7-aza-1(3,6)-imidazo[1,2-*b*]pyridazina-2(4,6)-pyrimidina-5(1,2)-benzenacycloheptaphane(I-78)**

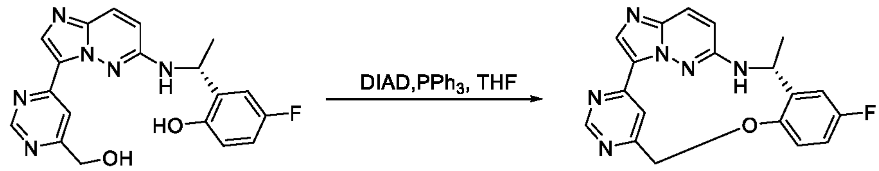


42-4 I-78

The title Compound **I-78** was prepared with reference to the method of Compound **I-9** by replacing Compound **39** with Compound **42-4** as the raw material. **LCMS:** m/z (ESI), 363[M+H]+.

**Example 79: (6*R*,*E*)-5⁵-fluoro-6-methyl-4-oxa-7-aza-1(3,6)-imidazo[1,2-*b*]pyridazina-2(2,4)-pyrimidina-5(2,3)-pyridinacycloheptaphane(I-79)**

**Step A: (*R*)-(2-(6-((1-(5-fluoro-2-methoxypyridin-3-yl)ethyl)amino)imidazo[1,2-*b*] pyridazin-3-yl)pyrimidin-4-yl) methanol (121-2)**

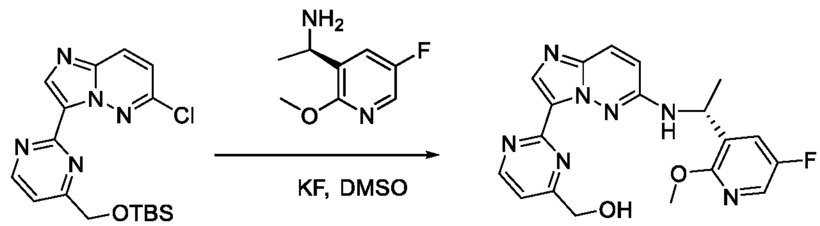


121 121-2

The title Compound **121-2** was prepared with reference to the method of Compound **43** by replacing Compound **19** with (***R***)-1-(5-fluoro-2-methoxypyridin-3-yl)ethan-1-amine and Compound **42** with Compound **121** as the raw material. **LCMS:** m/z (ESI), 396.1[M+H]+.

**Step B: (*R*)-5-fluoro-3-(1-((3-(4-(hydroxymethyl)pyrimidin-2-yl)imidazo[1,2-*b*] pyridazin-6-yl)amino)ethyl)pyridin-2(1H)-one** (121-3)

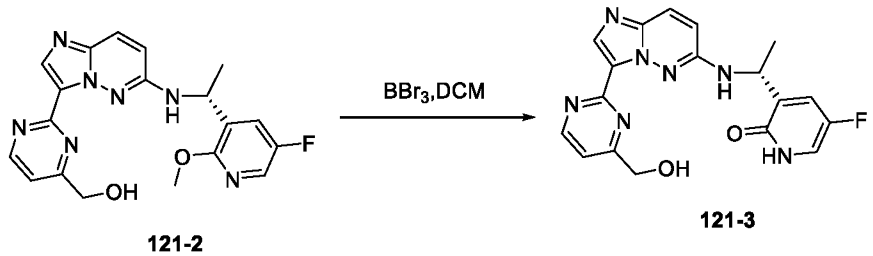


121-2 121-3

The title Compound **121-3** was prepared with reference to the method of Compound **11** by replacing Compound **10** with Compound **121-2** as the raw material. **LCMS:** m/z (ESI), 382.1[M+H]$^+$.

**Step C: (6*R*,*E*)-5$^5$-fluoro-6-methyl-4-oxa-7-aza-1(3,6)-imidazo[1,2-*b*]pyridazina-2(2,4)-pyrimidina-5(2,3)-pyridinacycloheptaphane(I-79)**

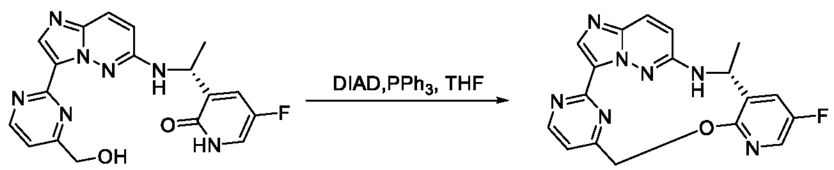


121-3 I-79

The title Compound **I-79** was prepared with reference to the method of Compound **I-9** by replacing Compound **39** with Compound **121-3** as the raw material. **LCMS:** m/z (ESI), 364[M+H]$^+$.

**Example 80: (6*R*,*E*)-5$^4$-fluoro-6-methyl-4-oxa-7-aza-1(3,6)-imidazo[1,2-*b*]pyridazina-2(2,4)-pyrimidina-5(1,2)-benzenacycloheptaphane(I-80)**

**Step A: (*R*)-(2-(6-((1-(5-fluoro-2-hydroxyphenyl)ethyl)amino)imidazo[1,2-*b*] pyridazin-3-yl)pyrimidin-4-yl)ethyl) methanol (121-4)**

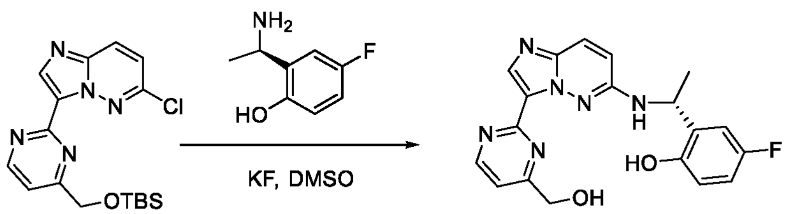


121 121-4

The title Compound **121-4** was prepared with reference to the method of Compound **43** by replacing Compound **19** with (***R***)-1-(5-fluoro-2-hydroxyphenyl)ethan-1-amine and Compound **42** with Compound **121** as the raw material. **LCMS:** m/z (ESI), 381.1[M+H]$^+$.

**Step B: (6*R*,*E*)-5$^4$-fluoro-6-methyl-4-oxa-7-aza-1(3,6)-imidazo[1,2-*b*]pyridazina-2(2,4)-pyrimidina-5(1,2)-benzenacycloheptaphane(I-80)**

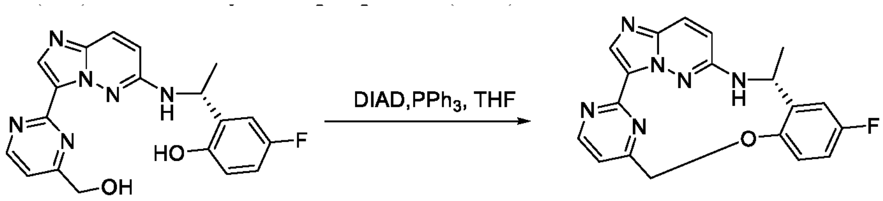


121-4 I-80

The title Compound **I-80** was prepared with reference to the method of Compound **I-9** by replacing Compound **39** with Compound **121-4** as the raw material. **LCMS:** m/z (ESI), 363[M+H]$^+$.

**Example 81: (6*R*,*E*)-5$^5$-fluoro-6-methyl-4-oxa-7-aza-1(3,6)-imidazo[1,2-*b*]pyridazina-2(3,5)-pyridazina-5(2,3)-pyridinacycloheptaphane(I-81)**

**Step A: (*R*)-(6-(6-((1-(5-fluoro-2-methoxypyridin-3-yl)ethyl)amino)imidazo[1,2-*b*] pyridazin-3-yl)pyridazin-4-yl) methanol (127-2)**

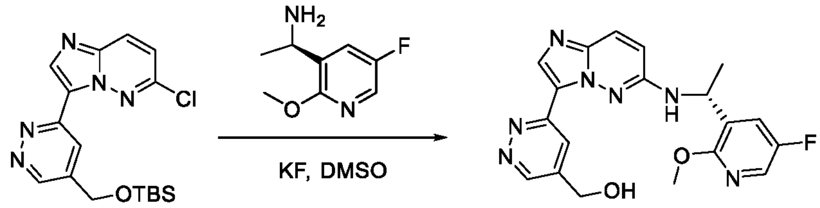


127 127-2

The title Compound **127-2** was prepared with reference to the method of Compound **43** by replacing Compound **19** with (***R***)-1-(5-fluoro-2-methoxypyridin-3-yl)ethan-1-amine and Compound **42** with Compound **127** as the raw material. **LCMS:** m/z (ESI),396.1[M+H]$^+$.

**Step B: (*R*)-5-fluoro-3-(1-((3-(5-(hydroxymethyl)pyridazin-3-yl)imidazo[1,2-*b*] pyridazin-6-yl)amino)ethyl)pyridin-2(1H)-one (127-3)**

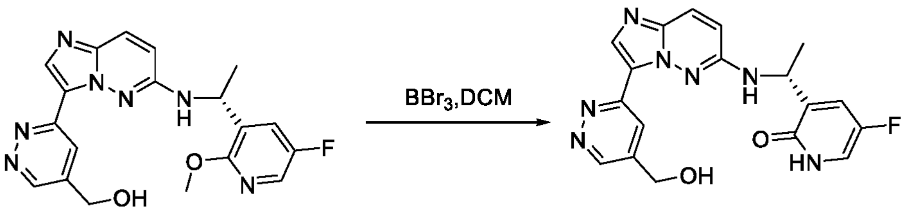


127-2 127-3

The title Compound **127-3** was prepared with reference to the method of Compound **11** by replacing Compound **10** with Compound **127-2** as the raw material. **LCMS:** m/z (ESI), 382.1 [M+H]$^+$.

**Step C: (6*R*,*E*)-5$^5$-fluoro-6-methyl-4-oxa-7-aza-1(3,6)-imidazo[1,2-*b*]pyridazina-2(3,5)-pyridazina-5(2,3)-pyridinacycloheptaphane(I-81)**

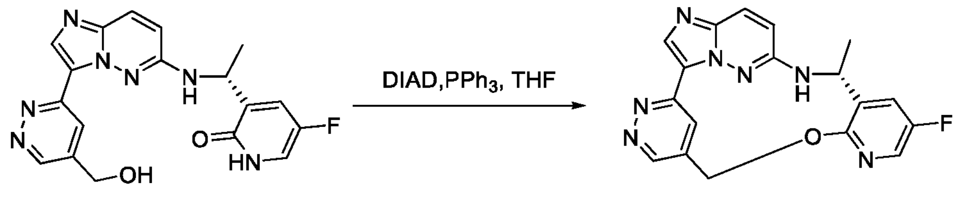


127-3 I-81

The title Compound **I-81** was prepared with reference to the method of Compound **I-9** by replacing Compound **39** with Compound **127-3** as the raw material. **LCMS:** m/z (ESI), 364[M+H]$^+$.

**Example 82: (6*R*,*E*)-54-fluoro-6-methyl-4-oxa-7-aza-1(3,6)-imidazo[1,2-*b*]pyridazina-2(3,5)-pyridazina-5(1,2)-benzenacycloheptaphane(I-82)**

**Step A: (*R*)-(6-(6-((1-(5-fluorohydroxyphenyl)ethyl)amino)imidazo[1,2-*b*] pyridazin-3-yl)pyridazin-4-yl)methanol (127-4)**

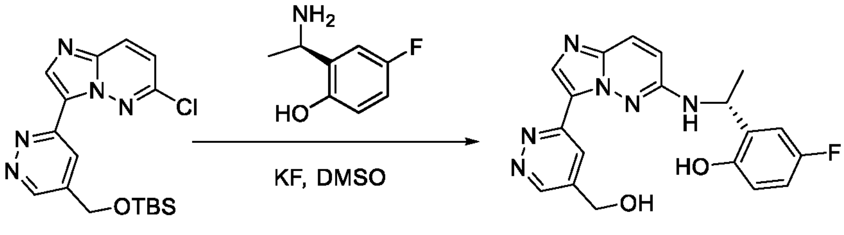


127 127-4

The title Compound **127-4** was prepared with reference to the method of Compound **43** by replacing Compound **19** with (R)-1-(5-fluoro-2-hydroxyphenyl)ethan-1-amine and Compound **42** with Compound **127** as the raw material. **LCMS:** m/z (ESI), 381.1[M+H]$^+$.

**Step B: (6*R*,*E*)-54-fluoro-6-methyl-4-oxa-7-aza-1(3,6)-imidazo[1,2-*b*]pyridazina-2(3,5)-pyridazina-5(1,2)-benzenacycloheptaphane(I-82)**

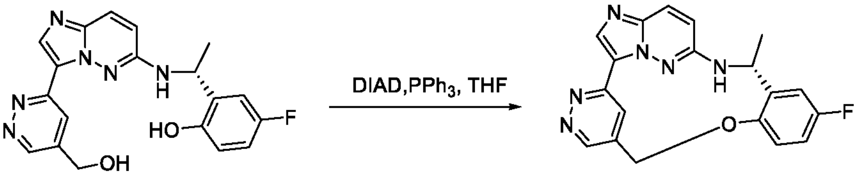


127-4 I-82

The title Compound **I-82** was prepared with reference to the method of Compound **I-9** by replacing Compound **39** with Compound **127-4** as the raw material. **LCMS:** m/z (ESI), 363[M+H]$^+$.

**Example 83: (4*R*,*E*)-5$^5$-fluoro-4-methyl-6-oxa-3-aza-2(3,6)-imidazo[1,2-*b*]pyridazina -1(2,4)-pyrimidina-5(3,2)-pyridinacyclooctaphane(I-83)**

**Step A: (*R*)-2-(2-(6-((1-(5-fluoro-2-methoxypyridin-3-yl)ethyl)amino)imidazo[1,2-*b*] pyridazin-3-yl)pyrimidin-4-yl)ethan-1-ol (137-2)**

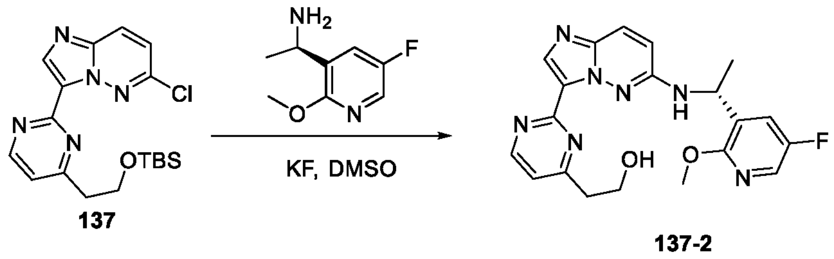


137

137-2

The title Compound **137-2** was prepared with reference to the method of Compound **43** by replacing Compound **19** with (***R***)-1-(5-fluoro-2-methoxypyridin-3-yl)ethan-1-amine and Compound **42** with Compound **137** as the raw material. **LCMS:** m/z (ESI),410.1[M+H]+.

**Step B: (*R*)-5-fluoro-3-(1-((3-(4-(2-hydroxyethyl)pyrimidin-2-yl)imidazo[1,2-*b*] pyridazin-6-yl)amino)ethyl)pyridin-2(1H)-one (137-3)**

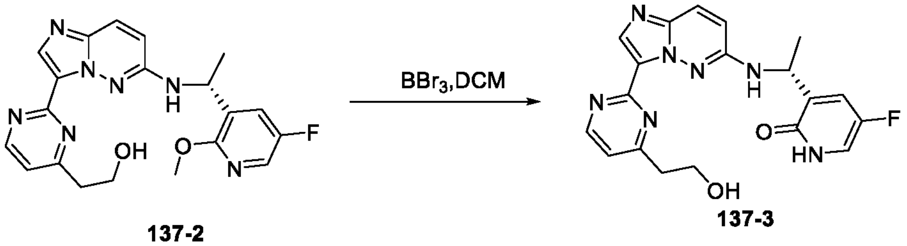


137-2

137-3

The title Compound **137-3** was prepared with reference to the method of Compound **11** by replacing Compound **10** with Compound **137-2** as the raw material. **LCMS:** m/z (ESI), 396.1[M+H]+.

**Step C: (4*R*,*E*)-5[5]-fluoro-4-methyl-6-oxa-3-aza-2(3,6)-imidazo[1,2-*b*]pyridazina-1(2,4) -pyrimidina-5(3,2)-pyridinacyclooctaphane(I-83)**

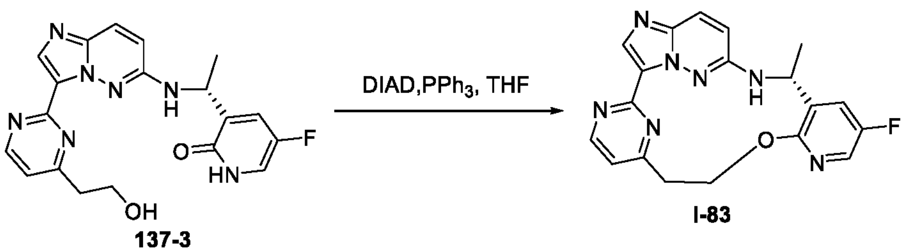


137-3

I-83

The title Compound **I-83** was prepared with reference to the method of Compound **I-9** by replacing Compound **39** with Compound **137-3** as the raw material. **LCMS:** m/z (ESI), 378[M+H]+.

**Example 84: (4*R*,*E*)-5[5]-fluoro-4-methyl-6-oxa-3-aza-2(3,6)-imidazo[1,2-*b*]pyridazina -1(2,4)-pyrimidina-5(1,2)-benzenacyclooctaphane(I-74)**

**Step A: Example 129: (*R*)-2-(2-(6-((1-(5-fluoro-2-hydroxyphenyl)ethyl)amino)imidazo[1,2-*b*] pyridazin-3-yl)pyrimidin-4-yl)ethan-1-ol (137-4)**

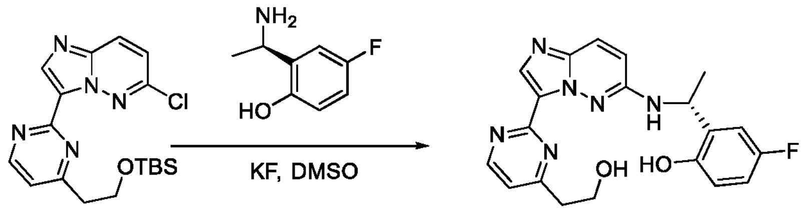


137

137-4

The title Compound **137-4** was prepared with reference to the method of Compound **43** by replacing Compound **19** with (***R***)-1-(5-fluoro-2-hydroxyphenyl)ethan-1-amine and Compound **137** with Compound **42** as the raw material. **LCMS:** m/z (ESI), 395.1[M+H]+.

**Step B: (4*R*,*E*)-5$^5$-fluoro-4-methyl-6-oxa-3-aza-2(3,6)-imidazo[1,2-*b*]pyridazina-1(2,4)-pyrimidina-5(1,2)-benzenacyclooctaphane(I-84)**

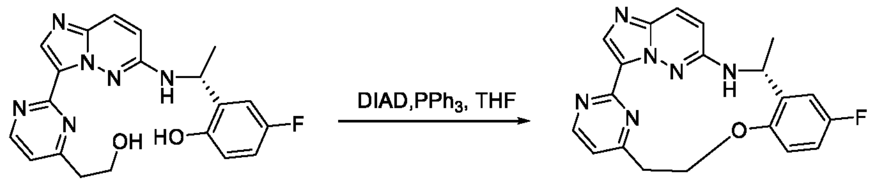


I-84

137-4

The title Compound **I-84** was prepared with reference to the method of Compound **I-9** by replacing Compound **39** with Compound **137-4** as the raw material. **LCMS:** m/z (ESI), 377[M+H]+.

**Example 85: (3$^2$*R*,*E*)-4$^5$-fluoro-5-oxa-2(3,6)-imidazo[1,2-*b*]pyridazina-1(2,4)-pyrimidina-3(1,2)-pyrrolidina-4(1,2)-benzenacycloheptaphane(I-85)**

**Step A: (*R*)-3-(4-(2-((tert-butyldimethylsilyl)oxy)ethyl)pyrimidin-2-yl)-6-(2-(5-fluoro-2-methoxyphenyl)pyrrolidin-1-yl)imidazo[1,2-*b*]pyridazine (138)**

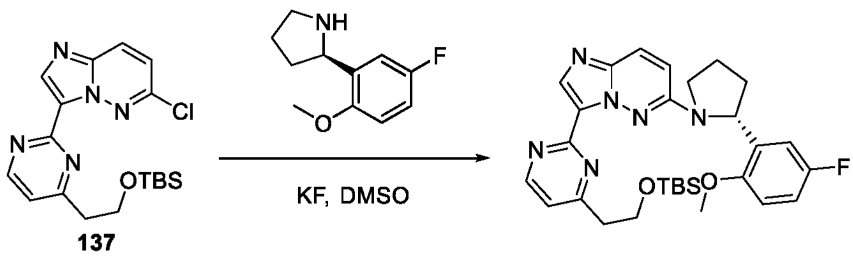


137

138

The title Compound **138** was prepared with reference to the method of Compound **48-1** by replacing Compound **19** with (***R***)-2-(5-fluoro-2-methoxyphenyl)pyrrolidine and Compound **48** with Compound **137** as the raw material. **LCMS:** m/z (ESI), 435.1[M+H]+.

**Step B: (*R*)-4-fluoro-2-(1-(3-(4-(2-hydroxyethyl)pyrimidin-2-yl)imidazo[1,2-*b*] pyridazin-6-yl)pyrrolidin-2-yl)phenol (138-1)**

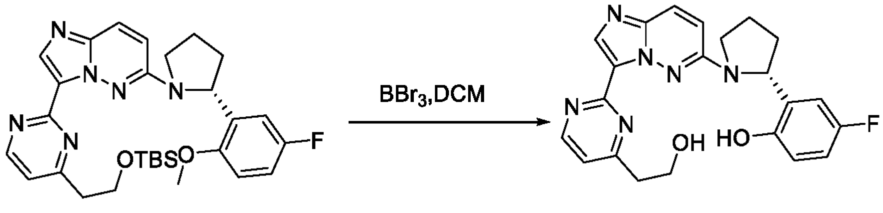


138 138-1

The title Compound **138-3** was prepared with reference to the method of Compound **11** by replacing Compound **10** with Compound **138-2** as the raw material. **LCMS:** m/z (ESI), 421.1[M+H]$^+$.

**Step C: ($3^2$*R*,*E*)-$4^5$-fluoro-5-oxa-2(3,6)-imidazo[1,2-*b*]pyridazina-1(2,4)-pyrimidina -3(1,2)-pyrrolidina-4(1,2)-benzenacycloheptaphane(I-85)**

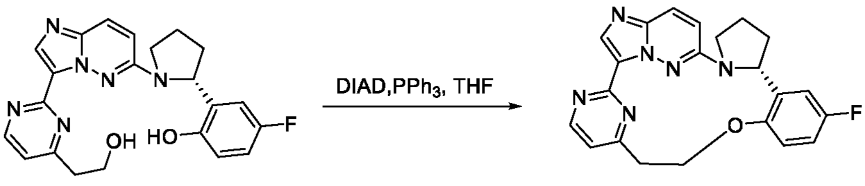


138-1 I-85

The title Compound **I-85** was prepared with reference to the method of Compound **I-9** by replacing Compound **39** with Compound **138-1** as the raw material. **LCMS:** m/z (ESI), 403 [M+H]$^+$.

**Example 86: ($3^2$*R*,$3^4$*S*,*E*)-$3^4$,$4^5$-difluoro-5-oxa-2(3,6)-imidazo[1,2-*b*]pyridazina-1(2,4)-pyrimidina-3(1,2)-pyrrolidina-4(1,2)-benzenacycloheptaphane(I-86)**

**Step A: 3-(4-(2-((tert-butyldimethylsilyl)oxy)ethyl)pyrimidin-2-yl)-6-((2*R*,4*S*)-4-fluoro -2-(5-fluoro-2-methoxyphenyl)pyrrolidin-1-yl)imidazo[1,2-*b*]pyridazine (139)**

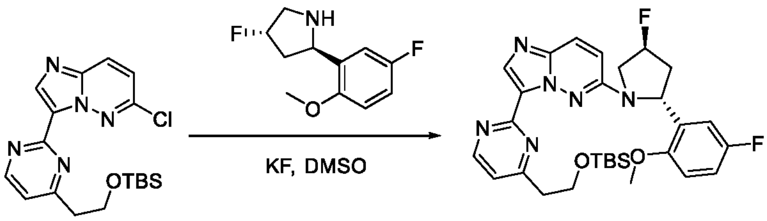


137 139

The title Compound **139** was prepared with reference to the method of Compound **48-1** by replacing Compound **19** with (2***R***,4***S***)-4-fluoro-2-(5-fluoro-2-methoxyphenyl)pyrrolidine and Compound **48** with Compound **137** as the raw material. **LCMS:** m/z (ESI), 453.1[M+H]$^+$.

**Step B: 4-fluoro-2-((2*R*,4*S*)-4-fluoro-1-(3-(4-(2-hydroxyethyl)pyrimidin-2-yl)imidazo [1,2-*b*]pyridazin-6-yl)pyrrolidin-2-yl)phenol (139-1)**

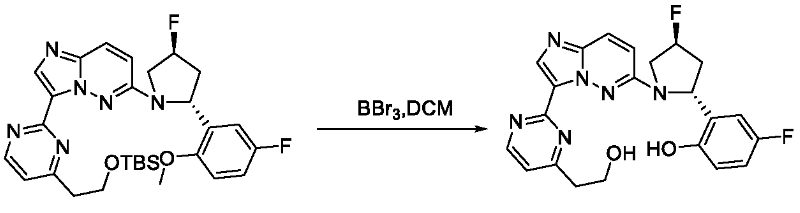


139 139-1

The title Compound **139-1** was prepared with reference to the method of Compound **11** by replacing Compound **10** with Compound **139** as the raw material. **LCMS:** m/z (ESI), 439. 1 [M+H]$^+$.

**Step C: (3$^2$*R*,3$^4$*S*,*E*)-3$^4$,4$^5$-difluoro-5-oxa-2(3,6)-imidazo[1,2-*b*]pyridazina-1(2,4)-pyrimidina-3(1,2)-pyrrolidina-4(1,2)-benzenacycloheptaphane(I-86)**

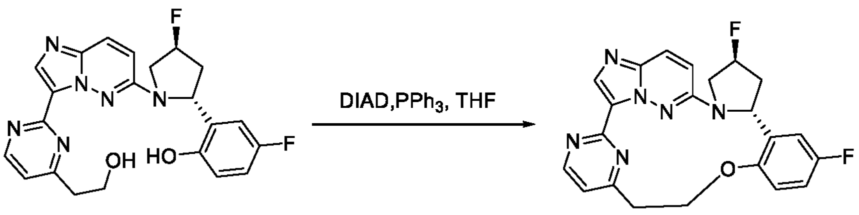


139-1 I-86

The title Compound **I-86** was prepared with reference to the method of Compound **I-9** by replacing Compound **39** with Compound **139-1** as the raw material. **LCMS:** m/z (ESI), 421 [M+H]$^+$.

**Example 87: (3$^2$*R*,*E*)-4$^5$-fluoro-5-oxa-2(3,6)-imidazo[1,2-*b*]pyridazina-1(2,4)-pyrimidina-4(3,2)-pyridina-3(1,2)-pyrrolidinacycloheptaphane(I-87)**

**Step A: (*R*)-2-(2-(6-(2-(5-fluoro-2-methoxypyridin-3-yl)pyrrolidin-1-yl)imidazo [1,2-b]pyridazin-3-yl)pyrimidin-4-yl)ethan-1-ol (139-2)**

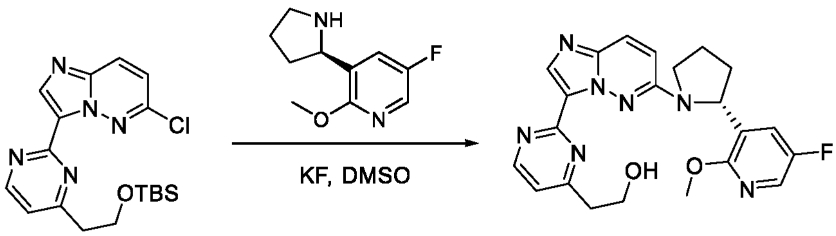


137 139-2

The title Compound **139-2** was prepared with reference to the method of Compound **48-1** by replacing Compound **19** with (***R***)-5-fluoro-2-methoxy-3-(pyrrolidin-2-yl)pyridine and Compound **48** with Compound **137** as the raw material. **LCMS:** m/z (ESI), 436.1[M+H]$^+$.

**Step B: (*R*)-5-fluoro-3-(1-(3-(4-(2-hydroxyethyl)pyrimidin-2-yl)imidazo[1,2-*b*] pyridazin-6-yl)pyrrolidin-2-yl)pyridin-2(1H)-one (139-3)**

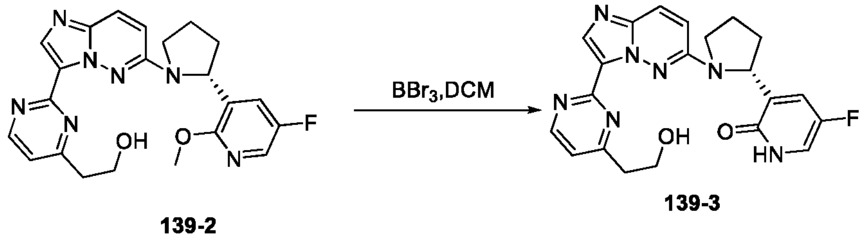


139-2

139-3

The title Compound **139-3** was prepared with reference to the method of Compound **11** by replacing Compound **10** with Compound **139-2** as the raw material. **LCMS:** m/z (ESI), 422. 1 $[M+H]^+$.

**Step C: ($3^2$*R*,*E*)-$4^5$-fluoro-5-oxa-2(3,6)-imidazo[1,2-*b*]pyridazina-1(2,4)-pyrimidina -4(3,2)-pyridina-3(1,2)-pyrrolidinacycloheptaphane(I-87)**

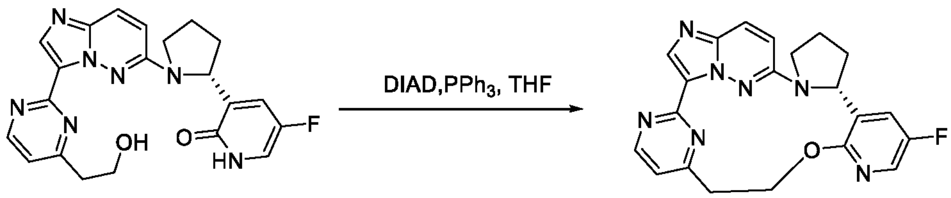


139-3

I-87

The title Compound **I-87** was prepared with reference to the method of Compound **I-9** by replacing Compound **39** with Compound **139-3** as the raw material. **LCMS:** m/z (ESI), 404$[M+H]^+$.

**Example 88: ($3^2$*R*,$3^4$*S*,*E*)-$3^4$,$4^5$-difluoro-5-oxa-2(3,6)-imidazo[1,2-*b*]pyridazina-1(2,4)-pyrimidina-4(3,2)-pyridina-3(1,2)-pyrrolidinacycloheptaphane(I-88)**

**Step A: 2-(2-(6-((2*R*,4*S*)-4-fluoro-2-(5-fluoro-2-methoxypyridin-3-yl)pyrrolidin-1-yl) imidazo[1,2-*b*]pyridazin-3-yl)pyrimidin-4-yl)ethan-1-ol (139-4)**

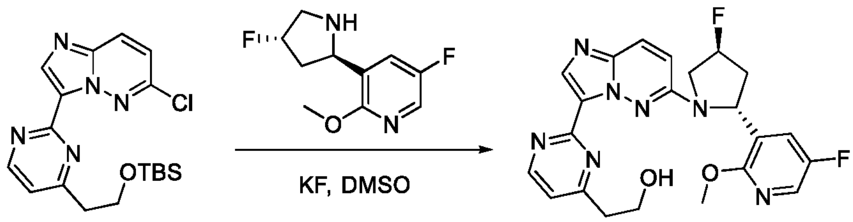


137

139-4

The title Compound **139-4** was prepared with reference to the method of Compound **48-1** by replacing Compound **19** with 5-fluoro-3-((2*R*,4*S*)-4-fluoropyrrolidin-2-yl)-2-methoxypyridine and Compound **48** with Compound **137** as the raw material. **LCMS:** m/z (ESI), 454.1 $[M+H]^+$.

**Step B: 5-fluoro-3-((2*R*,4*S*)-4-fluoro-1-(3-(4-(2-hydroxyethyl)pyrimidin-2-yl)imidazo [1,2-*b*]pyridazin-6-yl)pyrrolidin-2-yl)pyridin-2(1H)-one (139-5)**

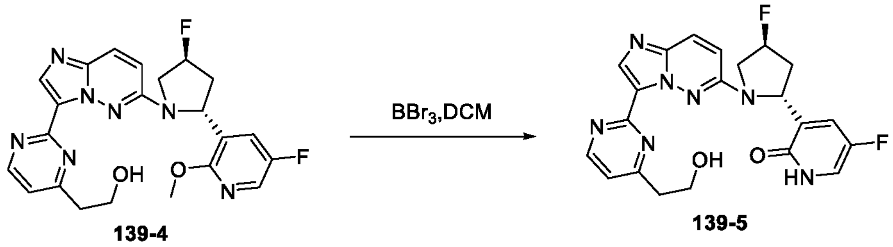


The title Compound **139-5** was prepared with reference to the method of Compound **11** by replacing Compound **10** with Compound **139-4** as the raw material. **LCMS:** m/z (ESI), 440.1[M+H]$^+$.

**Step C: ($3^2$*R*,$3^4$*S*,*E*)-$3^4$,$4^5$-difluoro-5-oxa-2(3,6)-imidazo[1,2-*b*]pyridazina-1(2,4)-pyrimidina-4(3,2)-pyridina-3(1,2)-pyrrolidinacycloheptaphane(I-88)**

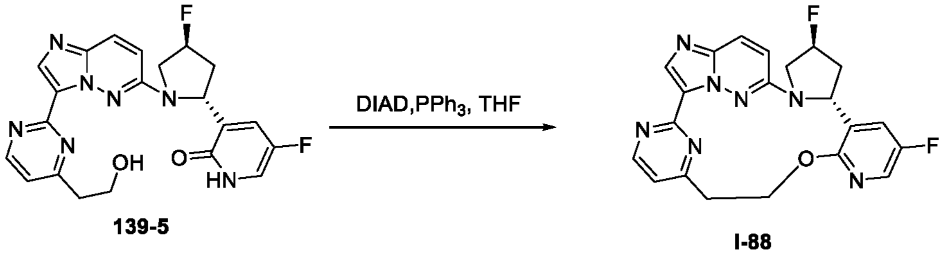


The title Compound **I-88** was prepared with reference to the method of Compound **I-9** by replacing Compound **39** with Compound **139-5** as the raw material. **LCMS:** m/z (ESI), 422[M+H]$^+$.

**Example 89: (4*R*,*E*)-$5^5$-fluoro-4-methyl-6-oxa-3-aza-2(3,6)-imidazo[1,2-*b*]pyridazina-1(3,5)-pyridazina-5(3,2)-pyridinacyclooctaphane(I-89)**

**Step A: (*R*)-2-(6-(6-((1-(5-fluoro-2-methoxypyridin-3-yl)ethyl)amino)imidazo[1,2-*b*] pyridazin-3-yl)pyridazin-4-yl) ethan-1-ol (143-2)**

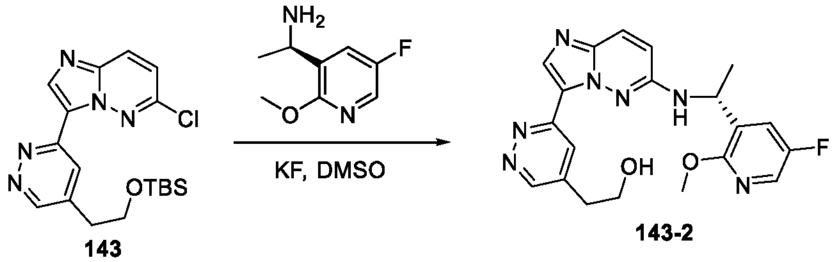


The title Compound **143-2** was prepared with reference to the method of Compound **48-1** by replacing Compound **19** with (***R***)-1-(5-fluoro-2-methoxypyridin-3-yl)ethan-1-amine and Compound **48** with Compound **143** as the raw material. **LCMS:** m/z (ESI),410.1[M+H]$^+$.

**Step B: (*R*)-5-fluoro-3-(1-((3-(5-(2-hydroxyethyl)pyridazin-3-yl)imidazo[1,2-*b*] pyridazin-6-yl)amino)ethyl)pyridin-2(1*H*)-one (143-3)**

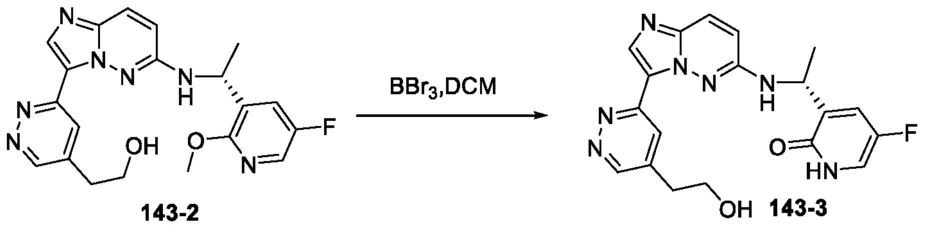


143-2 143-3

The title Compound **143-3** was prepared with reference to the method of Compound **11** by replacing Compound **10** with Compound **143-2** as the raw material. **LCMS:** m/z (ESI), 396.1[M+H]+.

**Step C: (4*R*,*E*)-5⁵-fluoro-4-methyl-6-oxa-3-aza-2(3,6)-imidazo[1,2-*b*]pyridazina-1(3,5)-pyridazina-5(3,2)-pyridinacyclooctaphane(I-89)**

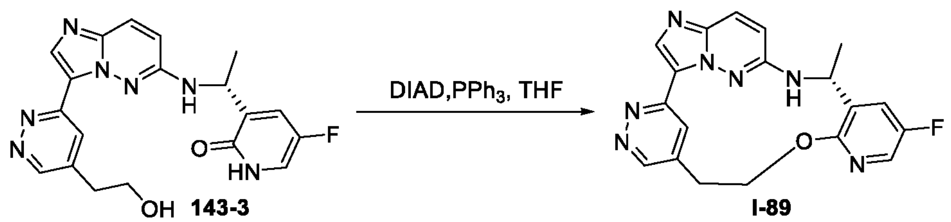


143-3 I-89

The title Compound **I-89** was prepared with reference to the method of Compound **I-9** by replacing Compound **39** with Compound **143-3** as the raw material. **LCMS:** m/z (ESI), 378[M+H]+.

**Example 90: (4*R*,*E*)-5⁵-fluoro-4-methyl-6-oxa-3-aza-2(3,6)-imidazo[1,2-*b*]pyridazina -1(3,5)-pyridazina-5(1,2)-benzenacyclooctaphane(I-90)**

**Step A: (*R*)-2-(6-(6-((1-(5-fluoro-2-hydroxyphenyl)ethyl)amino)imidazo[1,2-*b*] pyridazin-3-yl)pyridazin-4-yl) ethan-1-ol (143-4)**

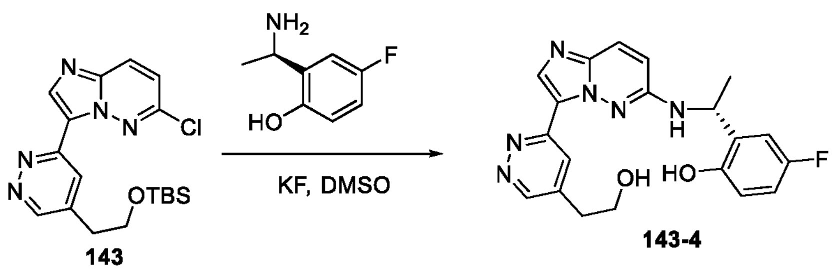


143 143-4

The title Compound **143-4** was prepared with reference to the method of Compound **48-1** by replacing Compound **19** with (***R***)-1-(5-fluoro-2-hydroxyphenyl)ethan-1-amine and Compound **48** with Compound **143** as the raw material. **LCMS:** m/z (ESI),395.1[M+H]+.

**Step B: (4*R*,*E*)-5⁵-fluoro-4-methyl-6-oxa-3-aza-2(3,6)-imidazo[1,2-*b*]pyridazina-1(3,5)-pyridazina-5(1,2)-benzenacyclooctaphane(I-90)**

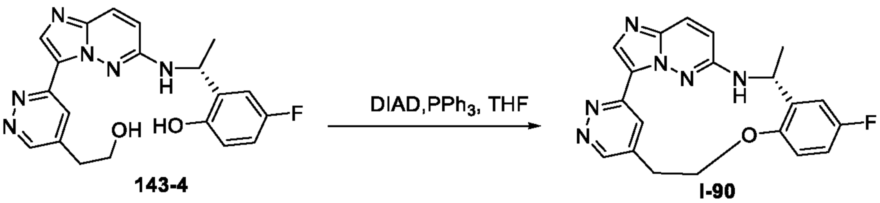


143-4 I-90

The title Compound **I-90** was prepared with reference to the method of Compound **I-9** by replacing Compound **39** with Compound **143-4** as the raw material. **LCMS:** m/z (ESI), 377[M+H]$^+$.

**Example 91: (3$^2$*R*,*E*)-4$^5$-fluoro-5-oxa-2(3,6)-imidazo[1,2-*b*]pyridazina-1(3,5)-pyridazina-3(1,2)-pyrrolidina-4(1,2)-benzenacycloheptaphane(I-91)**

**Step A: (R)-3-(5-(2-((tert-butyldimethylsilyl)oxy)ethyl)pyridazin-3-yl)-6-(2-(5-fluoro -2-methoxyphenyl)pyrrolidin-1-yl)imidazo[1,2-b]pyridazine (144)**

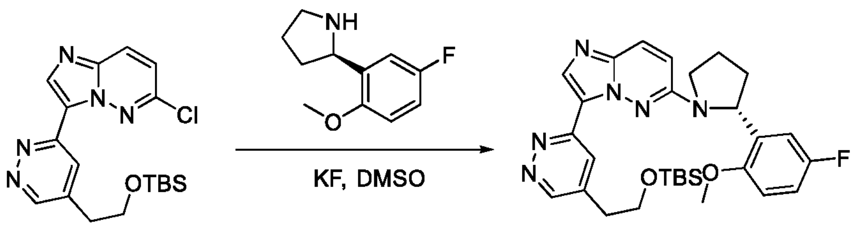


143 144

The title Compound **144** was prepared with reference to the method of Compound **48-1** by replacing Compound **19** with (***R***)-2-(5-fluoro-2-methoxyphenyl)pyrrolidine and Compound **48** with Compound **143** as the raw material. LCMS: m/z (ESI), 435.1[M+H]$^+$.

**Step B: (*R*)-4-fluoro-2-(l-(3-(5-(2-hydroxyethyl)pyridazin-3-yl)imidazo [1,2-*b*] pyridazin-6-yl)pyrrolidin-2-yl)phenol (144-1)**

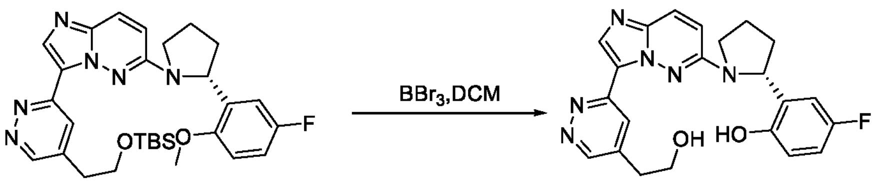


144 144-1

The title Compound **144-1** was prepared with reference to the method of Compound **11** by replacing Compound **10** with Compound **144** as the raw material. **LCMS:** m/z (ESI), 421.1 [M+H]$^+$.

**Step C: (3$^2$*R*,*E*)-4$^5$-fluoro-5-oxa-2(3,6)-imidazo[1,2-*b*]pyridazina-1(3,5)-pyridazina -3(1,2)-pyrrolidina-4(1,2)-benzenacycloheptaphane(I-91)**

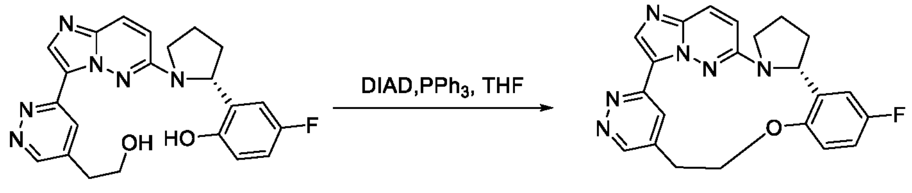


144-1 I-91

The title Compound **I-91** was prepared with reference to the method of Compound **I-9** by replacing Compound **39** with Compound **144-1** as the raw material. **LCMS:** m/z (ESI), 403 [M+H]$^+$.

**Example 92: ($3^2$*R*,$3^4$*S*,*E*)-$3^4$,$4^5$-difluoro-5-oxa-2(3,6)-imidazo[1,2-*b*]pyridazina-1(3,5)-pyridazina-3(1,2)-pyrrolidina-4(1,2)-benzenacycloheptaphane(I-92)**

**Step A: 3-(5-(2-((tert-butyldimethylsilyl)oxy)ethyl)pyridazin-3-yl)-6-((2*R*,4*S*)-4-fluoro -2-(5-fluoro-2-methoxyphenyl)pyrrolidin-1-yl)imidazo[1,2-b]pyridazine (145)**

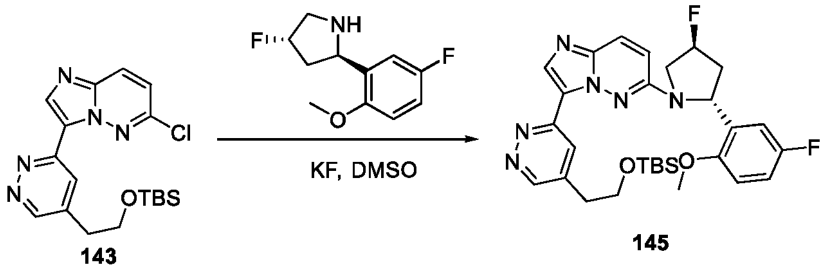


143 145

The title Compound **145** was prepared with reference to the method of Compound **48-1** by replacing Compound **19** with (2***R***,4***S***)-4-fluoro-2-(5-fluoro-2-methoxyphenyl)pyrrolidine and Compound **48** with Compound **143** as the raw material. **LCMS:** m/z (ESI), 439.1[M+H]$^+$.

**Step B: 4-fluoro-2-((2*R*,4*S*)-4-fluoro-1-(3-(5-(2-hydroxyethyl)pyridazin-3-yl)imidazo [1,2-*b*]pyridazin-6-yl)pyrrolidin-2-yl)phenol (145-1)**

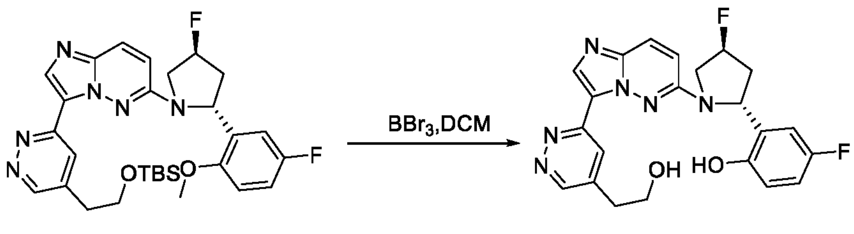


145 145-1

The title Compound **145-1** was prepared with reference to the method of Compound **11** by replacing Compound **10** with Compound **145** as the raw material.**LCMS:** m/z (ESI), 439.1[M+H]$^+$.

**Step C: ($3^2$*R*,$3^4$*S*,*E*)-$3^4$,$4^5$-difluoro-5-oxa-2(3,6)-imidazo[1,2-*b*]pyridazina-1(3,5)-pyridazina-3(1,2)-pyrrolidina-4(1,2)-benzenacycloheptaphane(I-92)**

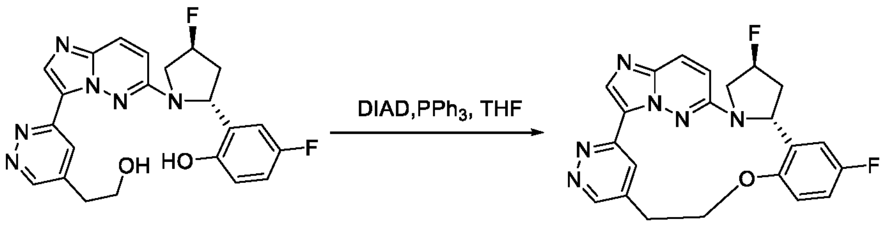


145-1 I-92

The title Compound **I-92** was prepared with reference to the method of Compound **I-9** by replacing Compound **39** with Compound **145-1** as the raw material. **LCMS:** m/z (ESI), 421 [M+H]+.

**Example 93: (3²*R*,*E*)-4⁵-fluoro-5-oxa-2(3,6)-imidazo[1,2-*b*]pyridazina-1(3,5)-pyridazina-4(3,2)-pyridina-3(1,2)-pyrrolidinacycloheptaphane(I-93)**

**Step A: (*R*)-2-(6-(6-(2-(5-fluoro-2-methoxypyridin-3-yl)pyrrolidin-1-yl)imidazo [1,2-*b*]pyridazin-3-yl)pyridazin-4-yl)ethan-1-ol (145-2)**

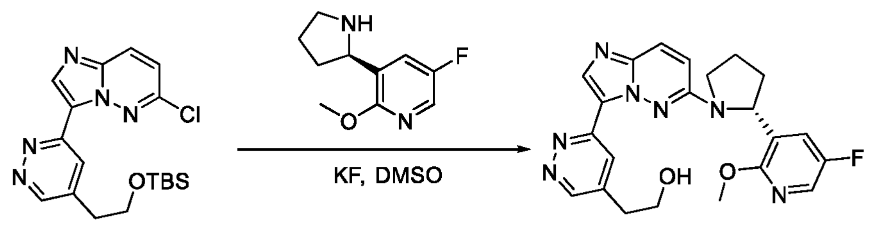


143 145-2

The title Compound **145-2** was prepared with reference to the method of Compound **48-1** by replacing Compound **19** with (***R***)-5-fluoro-2-methoxy-3-(pyrrolidin-2-yl)pyridine and Compound **48** with Compound **143** as the raw material. **LCMS:** m/z (ESI), 436.1[M+H]+.

**Step B: (*R*)-5-fluoro-3-(1-(3-(5-(2-hydroxyethyl)pyridazin-3-yl)imidazo [1,2-*b*] pyridazin-6-yl)pyrrolidin-2-yl)pyridin-2(1*H*)-one (145-3)**

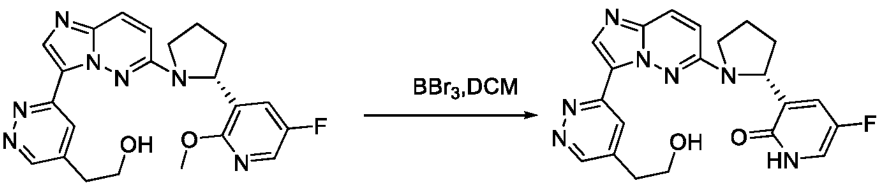


145-2 145-3

The title Compound **145-3** was prepared with reference to the method of Compound **11** by replacing Compound **10** with Compound **145-2** as the raw material. **LCMS:** m/z (ESI), 422.1[M+H]+.

**Step C: (3²*R*,*E*)-4⁵-fluoro-5-oxa-2(3,6)-imidazo[1,2-*b*]pyridazina-1(3,5)-pyridazina -4(3,2)-pyridina-3(1,2)-pyrrolidinacycloheptaphane(I-93)**

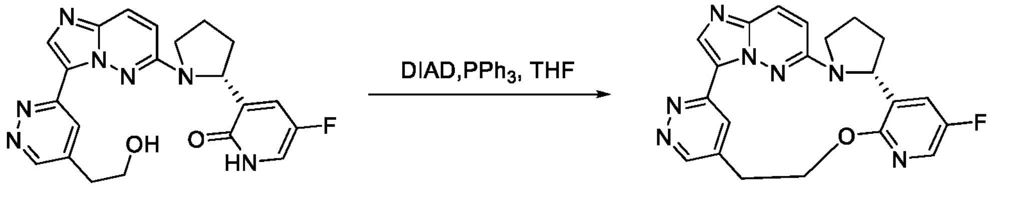


145-3 I-93

The title Compound **I-93** was prepared with reference to the method of Compound **I-9** by replacing Compound **39** with Compound **145-3** as the raw material. **LCMS:** m/z (ESI), 404[M+H]$^+$.

**Example 94: (3$^2$*R*,3$^4$*S*,*E*)-3$^4$,4$^5$-difluoro-5-oxa-2(3,6)-imidazo[1,2-*b*]pyridazina-1(3,5) -pyridazina-4(3,2)-pyridina-3(1,2)-pyrrolidinacycloheptaphane(I-94)**

**Step A: 2-(6-(6-((2*R*,4*S*)-4-fluoro-2-(5-fluoro-2-methoxypyridin-3-yl)pyrrolidin-1-yl) imidazo[1,2-*b*]pyridazin-3-yl)pyridazin-4-yl)ethan-1-ol (145-4)**

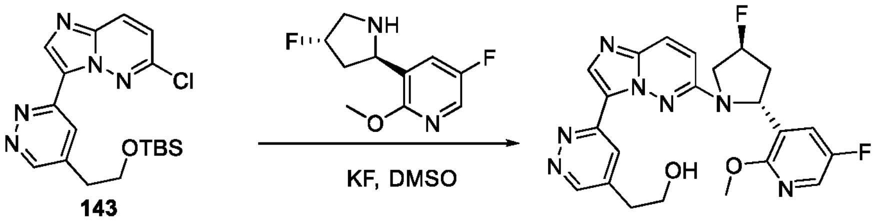


143

145-4

The title Compound **145-4** was prepared with reference to the method of Compound **48-1** by replacing Compound **19** with 5-fluoro-3-((2*R*,4*S*)-4-fluoropyrrolidin-2-yl)-2-methoxypyridine and Compound **48** with Compound **143** as the raw material. **LCMS:** m/z (ESI), 454.1 [M+H]$^+$.

**Step B: 5-fluoro-3-((2*R*,4*S*)-4-fluoro-1-(3-(5-(2-hydroxyethyl)pyridazin-3-yl)imidazo [1,2-*b*]pyridazin-6-yl)pyrrolidin-2-yl)pyridin-2(1H)-one (145-5)**

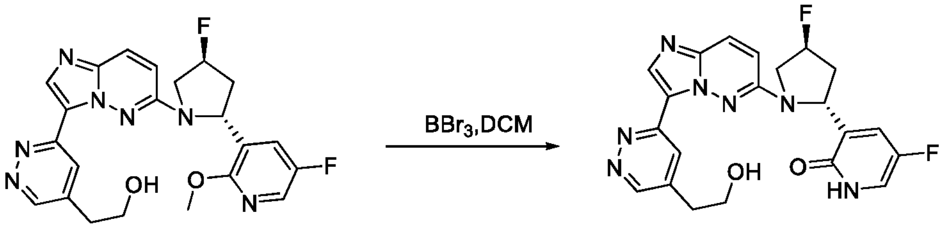


145-4 145-5

The title Compound **145-5** was prepared with reference to the method of Compound **11** by replacing Compound **10** with Compound **145-4** as the raw material.**LCMS:** m/z (ESI), 440. 1 [M+H]$^+$.

**Step C: (3$^2$*R*,3 $^4$*S*,*E*)-3$^4$4$^5$-difluoro-5-oxa-2(3,6)-imidazo[1,2-*b*]pyridazina-1(3,5) -pyridazina-4(3,2)-pyridina-3(1,2)-pyrrolidinacycloheptaphane(I-94)**

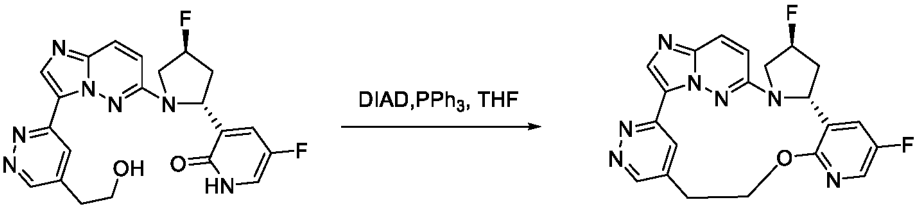


145-5 I-94

The title Compound **I-94** was prepared with reference to the method of Compound **I-9** by replacing Compound **39** with Compound **145-4** as the raw material. **LCMS:** m/z (ESI), 422[M+H]$^+$.

**Example 95: (3$^2$*R*,6*S*,*E*)-4$^5$-fluoro-6-methyl-5-oxa-2(3,6)-imidazo[1,2-*b*]pyridazina-1(2,4)-pyrimidina-4(3,2)-pyridina-3(1,2)-pyrrolidinacycloheptaphane(I-95)**

**Step A: (2R)-1-(2-chloropyrimidin-4-yl)propan-2-ol (147)**

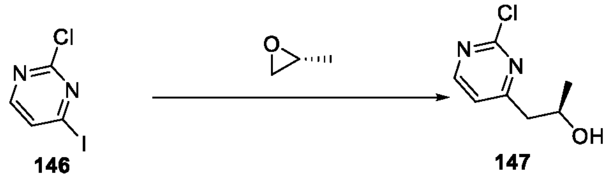

146 147

The title Compound **147** was prepared with reference to the method of Compound **79** by replacing Compound **78** with 2-iodo-4-chloropyrimidine as the raw material. **LCMS:** m/z (ESI),173.0[M+H]$^+$.

**Step B: (*R*)-4-(2-((tert-butyldimethylsilyl)oxy)propyl)-2-chloropyrimidine(148)**

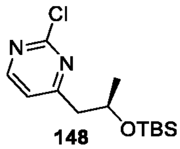

148

The title Compound **148** was prepared with reference to the method of Compound **2** by replacing Compound **1** with Compound **147** as the raw material. **LCMS:** m/z (ESI),287.0[M+H]$^+$.

**Step C: (*R*)-4-(2-((tert-butyldimethylsilyl)oxy)propyl)-2-(tributylstannyl)pyrimidine(149)**

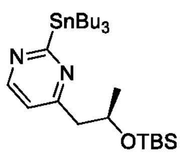

149

The title Compound **149** was prepared with reference to the method of Compound **3** by replacing Compound **2** with Compound **148** as the raw material. **LCMS:** m/z (ESI),543.0[M+H]$^+$.

**Step D: (*R*)-3-(4-(2-((tert-butyldimethylsilyl)oxy)propyl)pyrimidin-2-yl)-6-chloro imidazo[1,2-*b*]pyridazine(150)**

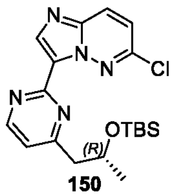

150

The title Compound **150** was prepared with reference to the method of Compound **5** by replacing Compound **3** with Compound **149** as the raw material. **LCMS:** m/z (ESI), 404.0 [M+H]+.

**Step E: (R)-1-(2-(6-((R)-2-(5-fluoro-2-methoxypyridin-3-yl)pyrrolidin-1-yl)imidazo [1,2-b]pyridazin-3-yl)pyrimidin-4-yl)propan-2-ol (151)**

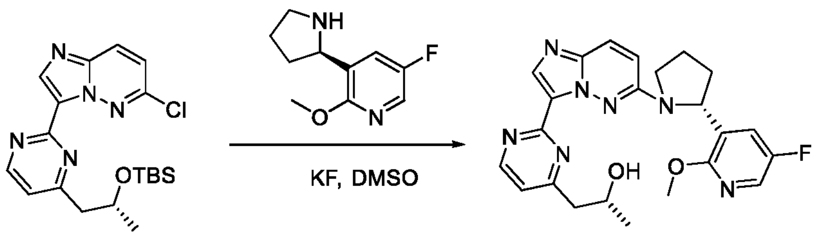


150

151

The title Compound **151** was prepared with reference to the method of Compound **48-1** by replacing Compound **19** with (***R***)-5-fluoro-2-methoxy-3-(pyrrolidin-2-yl)pyridine and Compound **48** with Compound **150** as the raw material. **LCMS:** m/z (ESI),436.1[M+H]+.

**Step F: 5-fluoro-3-((R)-1-(3-(4-((R)-2-hydroxypropyl)pyrimidin-2-yl)imidazo[1,2-b] pyridazin-6-yl)pyrrolidin-2-yl) pyridin-2(1*H*)-one (152)**

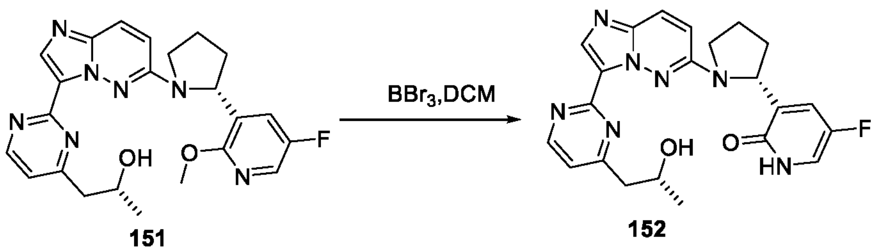


151

152

The title Compound **152** was prepared with reference to the method of Compound **11** by replacing Compound **10** with Compound **151** as the raw material. **LCMS:** m/z (ESI), 436.1[M+H]+.

**Step G: ($3^2$*R*,6*S*,*E*)-$4^5$-fluoro-6-methyl-5-oxa-2(3,6)-imidazo[1,2-*b*]pyridazina-1(2,4)-pyrimidina-4(3,2)-pyridina-3(1,2)-pyrrolidinacycloheptaphane(I-95)**

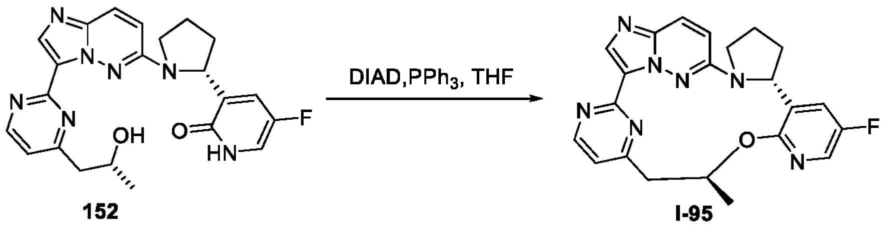


152 I-95

The title Compound **I-95** was prepared with reference to the method of Compound **I-9** by replacing Compound **39** with Compound **152** as the raw material. **LCMS:** m/z (ESI), 418[M+H]$^+$.

**Example 96: (3$^2$*R*,3$^1$*S*,6*S*,*E*)-3*,4$^5$-difluoro-6-methyl-5-oxa-2(3,6)-imidazo[1,2-*b*] pyridazina-1(2,4)-pyrimidina-4(3,2)-pyridina-3(1,2)-pyrrolidinacycloheptaphane(I-96)**

**Step A: (*R*)-1-(2-(6-((2*R*,4*S*)-4-fluoro-2-(5-fluoro-2-methoxypyridin-3-yl)pyrrolidine -1-yl)imidazo[1,2-b]pyridazin-3-yl)pyrimidin-4-yl)propan-2-ol (153)**

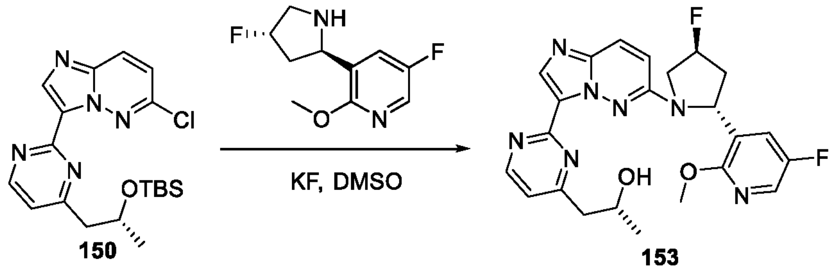


150 153

The title Compound **153** was prepared with reference to the method of Compound **48-1** by replacing Compound **19** with 5-fluoro-3-((2***R***,4***S***)-4-fluoropyrrolidin-2-yl)-2-methoxypyridine and Compound **48** with Compound **150** as the raw material. **LCMS:** m/z (ESI), 454.1 [M+H]$^+$.

**Step B: 5-fluoro-3-((2R,4S)-4-fluoro-1-(3-(4-((R)-2-hydroxypropyl)pyrimidin-2-yl) imidazo[1,2-*b*]pyridazin-6-yl) pyrrolidin-2-yl)pyridin-2(1*H*)-one (154)**

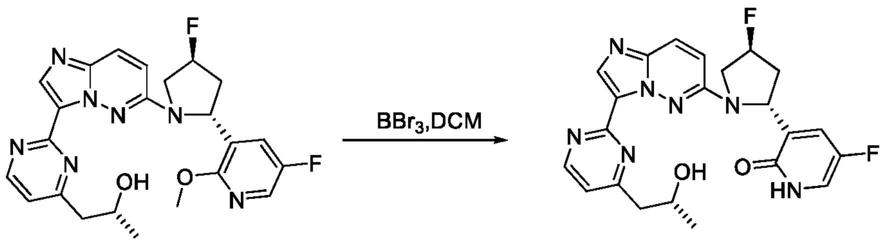


153 154

The title Compound **154** was prepared with reference to the method of Compound **11** by replacing Compound **10** with Compound **153** as the raw material. **LCMS:** m/z (ESI), 454.1 [M+H]$^+$.

**Step C: (3$^2$*R*,3$^4$*S*,6*S*,*E*)-3$^4$,4$^5$-difluoro-6-methyl-5-oxa-2(3,6)-imidazo[1,2-*b*]pyridazina -1(2,4)-pyrimidina-4(3,2)-pyridina-3(1,2)-pyrrolidinacycloheptaphane(I-96)**

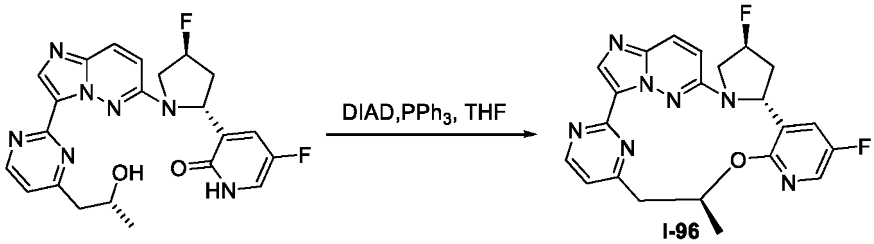


154

I-96

The title Compound **I-96** was prepared with reference to the method of Compound **I-9** by replacing Compound **39** with Compound **154** as the raw material. **LCMS:** m/z (ESI), 436[M+H]+.

**Example 97: (3$^2$*R*,6*R*,*E*)-4$^5$-fluoro-6-methyl-5-oxa-2(3,6)-imidazo[1,2-*b*]pyridazina-1(4,6)-pyrimidina-3(1,2)-pyrrolidina-4(1,2)-benzenacycloheptaphane(I-97)**

**Step A: (*R*)-3-(6-((*S*)-2-hydroxypropyl)pyrimidin-4-yl)-6-(5-fluoro-2-methoxyl phenyl)pyrrolidin-1-yl)imidazo [1,2-b]pyridazine (155)**

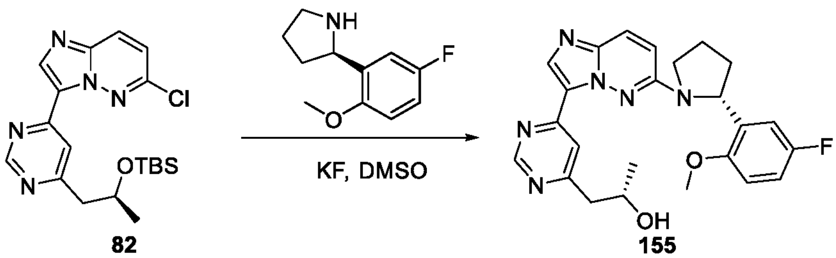


82

155

The title Compound **155** was prepared with reference to the method of Compound **82-1** by replacing Compound **71** with (***R***)-2-(5-fluoro-2-methoxyphenyl)pyrrolidine as the raw material. **LCMS:** m/z (ESI), 449.1[M+H]+.

**Step B: 4-fluoro-2-((*R*)-1-(3-(6-((*S*)-2-hydroxypropyl)pyrimidin-4-yl)imidazo [1,2-*b*]pyridazin-6-yl)pyrrolidin-2-yl) phenol (156)**

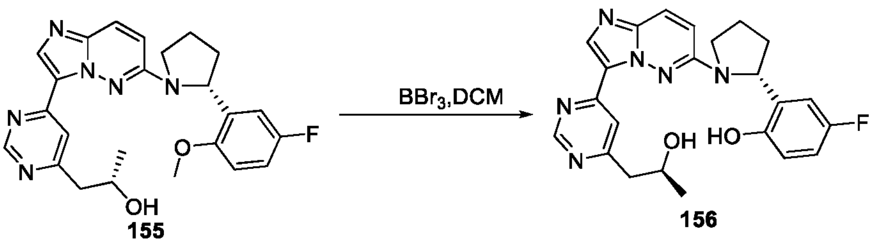


155

156

The title Compound **156** was prepared with reference to the method of Compound **78** by replacing Compound 77 with Compound **155** as the raw material. **LCMS:** m/z (ESI), 434.1 [M+H]+.

**Step C: (3$^2$*R*,6*R*,*E*)-4$^5$-fluoro-6-methyl-5-oxa-2(3,6)-imidazo[1,2-*b*]pyridazina-1(4,6)-pyrimidina-3(1,2)-pyrrolidina-4(1,2)-benzenacycloheptaphane(I-97)**

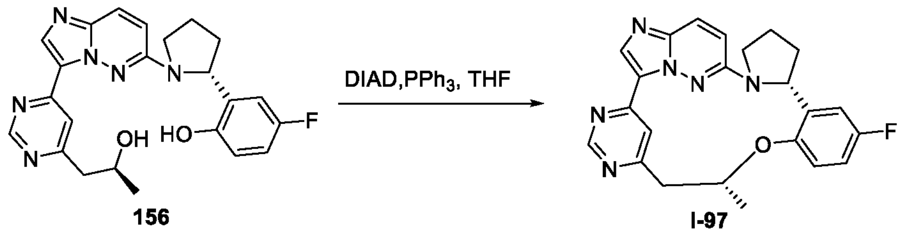


156

I-97

The title Compound **I-97** was prepared with reference to the method of Compound **I-9** by replacing Compound **39** with Compound **156** as the raw material. **LCMS:** m/z (ESI), 417[M+H]+.

**Example 98: ($3^2$*R*,$3^4$*S*,6*R*,*E*)-$3^4$,$4^5$-difluoro-6-methyl-5-oxa-2(3,6)-imidazo[1,2-*b*] pyridazina-1(4,6)-pyrimidina-3(1,2)-pyrrolidina-4(1,2)-benzenacycloheptaphane (I-98)**

**Step A: (*R*)-3-(6-((*S*)-2-hydroxypropyl)pyrimidin-4-yl)-6-(2-(5-fluoro-2-methoxyl phenyl)-(*S*)-4-fluoropyrrolidin-1-yl)imidazo[1,2-b]pyridazine (157)**

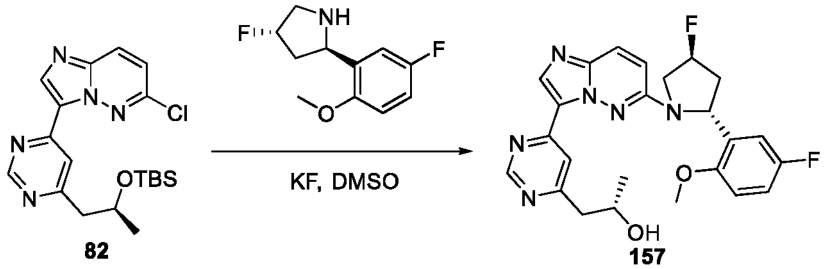


82

157

The title Compound **157** was prepared with reference to the method of Compound **82-1** by replacing Compound **71** with (2***R***,4***S***)-4-fluoro-2-(5-fluoro-2-methoxyphenyl)pyrrolidine as the raw material. **LCMS:** m/z (ESI), 467.1 [M+H]+.

**Step B: 4-fluoro-2-((2*R*,4*S*)-4-fluoro-1-(3-(6-((*S*)-2-hydroxypropyl)pyrimidin-4-yl) imidazo[1,2-*b*]pyridazin-6-yl) pyrrolidin-2-yl)phenol (158)**

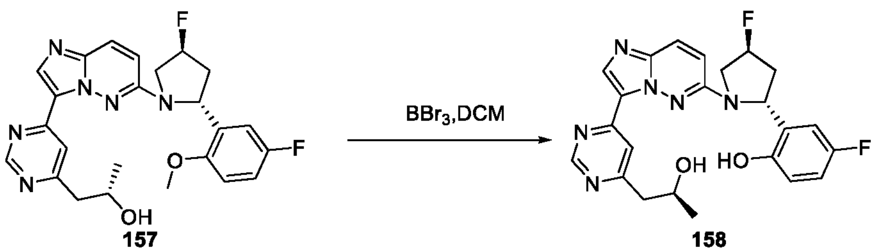


157

158

The title Compound 158 was prepared with reference to the method of Compound 78 by replacing Compound 77 with Compound 157 as the raw material. LCMS: m/z (ESI), 453.1[M+H]+.

Step C: **($3^2$*R*,$3^4$*S*,6*R*,*E*)-$3^4$,$4^5$-difluoro-6-methyl-5-oxa-2(3,6)-imidazo[1,2-*b*]pyridazina -1(4,6)-pyrimidina-3(1,2)-pyrrolidina-4(1,2)-benzenacycloheptaphane(I-98)**

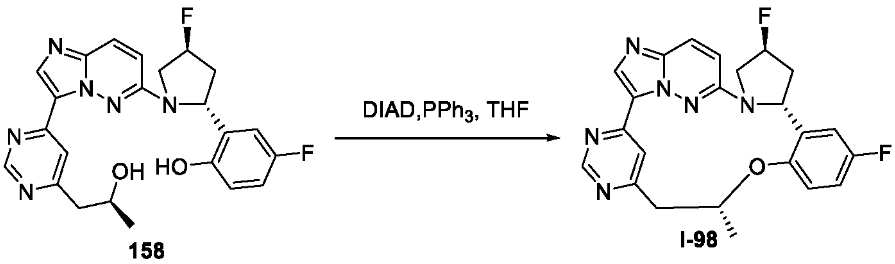


158

I-98

The title Compound **I-98** was prepared with reference to the method of Compound **I-9** by replacing Compound 39 with Compound 158 as the raw material. **LCMS:** m/z (ESI), 435[M+H]+.

Example 99: **Biological Evaluation**

Assay 1: **inhibitory activity of compounds against TRKA and TRKA(F589L) Kinases**

Experimental Method: Compound Preparation: The compounds from Examples 1-98 were separately pre-dissolved in 100% DMSO to prepare 10 mM stock solutions, and then the solutions were stored at -20 °C.

The chemical structure of the compound Loxo-195 is as follows. The compound Loxo-195 is commercially available and can be prepared with reference to WO2010/48314.

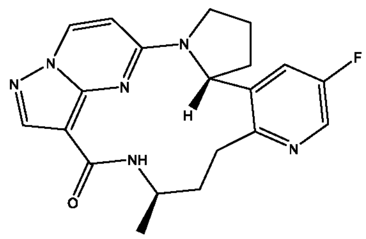

Loxo-195

The chemical structure of the compound TPX-0005 is as follows. The compound TPX-0005 is commercially available and can be prepared with reference to WO2017004342.

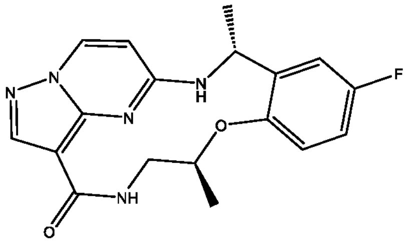

TPX-0005

Kinase Assay Process: 1× Kinase buffer was prepeared. Prepareation of the compound solutions with gradient concentrations: The test concentrations of the compounds from Examples 1-96 and Compound Loxo-195 were set at 1000 nM, and diluted in 100% DMSO solution to a final concentration of 100-fold in a 384-well plate. The compounds were serially diluted into 10 concentrations (1000, 333.3, 111.1, 37.0, 12.3, 4.1, 1.4, 0.16, 0.15, and 0.05 nM) using a Precision 3-fold dilution method. By using the Echo550 liquid handler, 250nL of the 100-fold final concentration compound was transferred to an OptiPlate-384F plate. a 2.5× final concentration kinase solution was prepared using 1× Kinase buffer. 10μL of the 2. 5× final concentration kinase solution was added to the compound wells and the positive control wells, and 10μL of 1× Kinase buffer was added to the negative control wells. The plate was centrifuged at 1000 rpm for 30 seconds, vortexed for mixing, and then incubated at room temperature for 10 minutes. A mixture of 5/3× final concentration of ATP and Kinase Substrate 22 was prepared using 1× Kinase buffer. 15μL of the mixture of the 5/3× final concentration ATP and the substrate was added to initiate the reaction. the 384-well plate was centrifuged at 1000 rpm for 30 seconds, vortexed for mixing, and incubated at room temperature for the required time. 30μL of the termination solution was added to stop the kinase reaction, the plate was centrifuged at 1000 rpm for 30 seconds, and vortexed for mixing. The conversion rate was measured using the Caliper EZ Reader.

Data Analysis: the inhibition rate was calculated as follows:

%Inhibition=(Conversion%_max-conversion%_sample)÷(Conversion%_max-conversion%_ min)x 100

Conversion%_sample is the conversion rate reading for the sample; Conversion %_min is the average value of the negative control wells, representing the conversion rate for wells without enzyme activity; Conversion %_max is the average value of the positive control wells, representing the conversion rate for wells without compound inhibition. A dose-response curve was fitted using the log concentration of the compound as the X-axis and the percentage inhibition as the Y-axis. The log(inhibitor) vs. response -variable slope of the GraphPad Prism 5 software was used to fit the dose-response curve, and the IC50 value for each compound was obtained. The calculation formula is:

$$Y = Bottom+(Top-Bottom)/(1+10^{((LogIC_{50}-X) * HillSlope)}).$$

wherein X represents the concentration of the compound to be tested; Y represents the inhibition rate at the concentration X; Top refers to the maximum response; Bottom refers to the baseline response; HillSlope refers to the slope of the curve (i.e., the gradient).

The inhibitory activity IC50 values of the compound against kinase are shown in Tables 1 and 2 below.

Table 1: the inhibitory activity $IC_{50}$ value(nM) of the compounds against $TRKA^{WT}$ Kinase in vitro

| Example | $TRKA^{WT}$ | Example | $TRKA^{WT}$ | Example | $TRKA^{WT}$ | Example | $TRKA^{WT}$ |
|---|---|---|---|---|---|---|---|
| I-1 | 2.57 | I-26 | <3 | I-50 | <3 | I-74 | <3 |
| I-2 | 2.59 | I-27 | 3.7 | I-51 | <3 | I-75 | <3 |
| I-3 | 5.9 | I-28 | <3 | I-52 | <3 | I-76 | <3 |
| I-4 | <3 | I-29 | 4.9 | I-53 | <3 | I-77 | <3 |
| I-5 | 1.36 | I-30 | <3 | I-54 | <3 | I-78 | <3 |
| I-6 | 2.76 | I-31 | 5.7 | I-55 | <3 | I-79 | <3 |
| I-7 | <3 | I-32 | <3 | I-56 | <3 | I-80 | <3 |
| I-8 | 2.46 | I-33 | 5.9 | I-57 | <3 | I-81 | <3 |
| I-9 | <3 | I-34 | <3 | I-58 | <3 | I-82 | <3 |
| I-10 | <3 | I-35 | <3 | I-59 | <3 | I-83 | <3 |
| I-11 | <3 | I-36 | <3 | I-60 | <3 | I-84 | <3 |
| I-12 | <3 | I-37 | <3 | I-61 | <3 | I-85 | <3 |
| I-13 | <3 | I-38 | <3 | I-62 | <3 | I-86 | <3 |
| I-14 | <3 | I-39 | <3 | I-63 | <3 | I-87 | <3 |
| I-15 | 4.8 | I-40 | <3 | I-64 | <3 | I-88 | <3 |
| I-16 | <3 | I-41 | <3 | I-65 | <3 | I-89 | <3 |
| I-17 | 5.2 | I-42 | <3 | I-66 | <3 | I-91 | <3 |
| I-18 | <3 | I-43 | <3 | I-67 | <3 | I-92 | <3 |
| I-19 | 5.7 | I-44 | <3 | I-68 | <3 | I-93 | <3 |
| I-20 | <3 | I-45 | <3 | I-69 | <3 | I-94 | <3 |
| I-21 | 6.1 | I-46 | <3 | I-70 | <3 | I-95 | <3 |
| I-22 | <3 | I-47 | <3 | I-71 | <3 | I-96 | <3 |
| I-23 | 4.8 | I-48 | <3 | I-72 | <3 | I-97 | <3 |
| I-24 | <3 | I-49 | <3 | I-73 | <3 | I-98 | <3 |
| I-25 | 5.1 | | | | | LOXO-195 | 3.5 |

The above results show that most of the compounds of the invention have better or equivalent activity against $TRKA^{WT}$ kinases compared to LOXO-195 in vitro.

Table 2: the inhibitory activity $IC_{50}$ value(nM) of the compounds of the invention against $TRKA^{F589L}$ Kinase in vitro

| Example | $TRKA^{F589L}$ | Example | $TRKA^{F589L}$ | Example | $TRKA^{F589L}$ | Example | $TRKA^{F589L}$ |
|---|---|---|---|---|---|---|---|
| I-1 | 5.4 | I-26 | <5 | I-50 | <5 | I-74 | <5 |
| I-2 | 6.2 | I-27 | ---- | I-51 | <5 | I-75 | <5 |
| I-3 | 7.1 | I-28 | <5 | I-52 | <5 | I-76 | <5 |
| I-4 | 5.8 | I-29 | ---- | I-53 | <5 | I-77 | <5 |
| I-5 | <5 | I-30 | <5 | I-54 | <5 | I-78 | <5 |
| I-6 | 6.1 | I-31 | ---- | I-55 | <5 | I-79 | <5 |
| I-7 | <5 | I-32 | <5 | I-56 | <5 | I-80 | <5 |
| I-8 | <5 | I-33 | ---- | I-57 | <5 | I-81 | <5 |
| I-9 | <5 | I-34 | <5 | I-58 | <5 | I-82 | <5 |
| I-10 | <5 | I-35 | <5 | I-59 | <5 | I-83 | <5 |
| I-11 | <5 | I-36 | <5 | I-60 | <5 | I-84 | <5 |
| I-12 | <5 | I-37 | <5 | I-61 | <5 | I-85 | <5 |
| I-13 | <5 | I-38 | <5 | I-62 | <5 | I-86 | <5 |
| I-14 | <5 | I-39 | <5 | I-63 | <5 | I-87 | <5 |
| I-15 | ---- | I-40 | <5 | I-64 | <5 | I-88 | <5 |
| I-16 | <5 | I-41 | <5 | I-65 | <5 | I-89 | <5 |
| I-17 | ---- | I-42 | <5 | I-66 | <5 | I-91 | <5 |
| I-18 | <5 | I-43 | <5 | I-67 | <5 | I-92 | <5 |
| I-19 | ---- | I-44 | <5 | I-68 | <5 | I-93 | <5 |
| I-20 | <5 | I-45 | <5 | I-69 | <5 | I-94 | <5 |
| I-21 | ---- | I-46 | <5 | I-70 | <5 | I-95 | <5 |
| I-22 | <5 | I-47 | <5 | I-71 | <5 | I-96 | <5 |
| I-23 | ---- | I-48 | <5 | I-72 | <5 | I-97 | <5 |
| I-24 | <5 | I-49 | <5 | I-73 | <5 | I-98 | <5 |
| I-25 | ---- | | | | | LOXO-195 | 6.5 |
| ---- indicates not tested or not detected. | | | | | | | |

The above results show that most of the compounds of the invention have better activity against $TRKA^{F589L}$ kinases compared to LOXO-195 in vitro.

**Assay 2: Inhibitory Effect of Compounds on the Growth of Ba/F3-TPM3-NTRK1-G667C Cells.**

**Reagents:**

| **Reagents** | **Catalog #** |
|---|---|
| RPMI 1640 Medium | L210KJ |
| Fetal Bovine Serum | FSP500 |
| PBS | B310KJ |
| Penicillin Streptomycin, Liquid (P/S) | SV30010 |

(continued)

| Reagents | Catalog # |
|---|---|
| Cell Titer-Glo (CTG) Reagent Kit | G7573 |

**Cell Line:**

| Cell line | Culture conditions |
|---|---|
| BaF3-NTRK1 WT | RPMI 1640 + 10% FBS + 1% P/S |
| BaF3-NTRK1 G595R | RPMI 1640 + 10% FBS + 1% P/S |
| BaF3-NTRK1 G667C | RPMI 1640 + 10% FBS + 1% P/S |

Solution Preparation: In a biological safety cabinet, the compounds of Example 1-96 and compound Loxo-101 were dissolved respectively in 100% DMSO to prepare 10 mM stock solutions, and the solutions were stored at -20 °C. Then the solutions were subjected to a gradient dilution with DMSO: a clean, sterile V-bottom 96-well microplate was placed in the biological safety cabinet. First, 4 μL of DMSO was added to well B2 and 10 μL of DMSO was added to wells B3-B11 respectively; Next, 6 μL of the 10 mM solution was added to well B2. The microplate was pipetted up and down 10 times for mixing. This results in a final concentration of 6 mM in well B2; 5 μL of the solution from well B2 was transferred to well B3. Mixing was performed by pipetting up and down 10 times. The solution was diluted in order until well B10. only DMSO was added to well B11 (no compound).

6X Compound Solution Preparation (6-fold dilution, X represents dilution factor): (Initial final concentration: 10 μM), a clean, sterile V-bottom 96-well microplate was placed in the biological safety cabinet. 99 μL of cell culture medium was added to each well from B2 to G11. By using a 12-channel pipettor, 1 μL of the compound solution prepared in the previous step was added into the corresponding wells. The solutions were pipetted up and down 10 times to mix the solutions. The highest concentration of compound in this step was 60 μM.

Treatment of Cells: the above-prepared cell culture plate was taken out of the incubator. 20 μL of compound solut ion from each well was added to the corresponding cell culture wells. The lowest pipetting speed was used and touching the bottom of the plate was avoided. The culture plate was gently shaked to mix the compound solution, then the plate was returned to the incubator for further culture for 72 hours.

The highest treatment concentration of the compound was 10 μM. After 72 hours of treatment, the Cell Titer-Glo (CTG) assay was performed. First, the cell culture plate was removed from the $CO_2$ incubator and allowed to stand at room temperature for 30 minutes to ensure uniform temperature of the plate. The pre-prepared Cell Titer-Glo reagent (following the manufacturer's instructions) was taken and allowed to stand at room temperature to thaw. 60 μL of Cell Titer-Glo reagent was added to each well containing cells. The cells were incubated on a horizontal shaker in a dark place at 225 rpm for 1 hour at room temperature. Then, a Tecan Spark microplate reader was used to read the plate using chemiluminescence according to the manufacturer's instructions. IC50 Test Results of some compounds on different cell lines are shown in Tables

Table 3: The inhibitory activity IC50 value (nM) of the compounds against NTRK G667C on Ba/F3 engineered cell line in vitro

| Exa mpl e | Ba/F3-TPM3-N TRK1-G667C | Exa mpl e | Ba/F3-TPM3-N TRK1-G667C | Exa mpl e | Ba/F3-TPM3-N TRK1-G667C | Exa mpl e | Ba/F3-TPM3-N TRK1-G667C |
|---|---|---|---|---|---|---|---|
| I-1 | 91.21 | I-26 | <100 | I-50 | 0.3 | I-74 | <100 |
| I-2 | 32.52 | I-27 | ---- | I-51 | <100 | I-75 | <100 |
| I-3 | 526 | I-28 | <100 | I-52 | 0.88 | I-76 | <100 |
| I-4 | <100 | I-29 | ---- | I-53 | <100 | I-77 | <100 |
| I-5 | 94 | I-30 | <100 | I-54 | 2.1 | I-78 | <100 |
| I-6 | 526 | I-31 | ---- | I-55 | <100 | I-79 | <100 |
| I-7 | 220 | I-32 | <100 | I-56 | 9.4 | I-80 | <100 |

(continued)

| Exa mpl e | Ba/F3-TPM3-N TRK1-G667C | Exa mpl e | Ba/F3-TPM3-N TRK1-G667C | Exa mpl e | Ba/F3-TPM3-N TRK1-G667C | Exa mpl e | Ba/F3-TPM3-N TRK1-G667C |
|---|---|---|---|---|---|---|---|
| I-8 | 562 | I-33 | ---- | I-57 | <100 | I-81 | <100 |
| I-9 | 308 | I-34 | <100 | I-58 | 12.7 | I-82 | <100 |
| I-10 | 378 | I-35 | <100 | I-59 | <100 | I-83 | <100 |
| I-11 | 419 | I-36 | <100 | I-60 | 22.5 | I-84 | <100 |
| I-12 | <100 | I-37 | <100 | I-61 | <100 | I-85 | <100 |
| I-13 | <100 | I-38 | <100 | I-62 | 13.6 | I-86 | 13.9 |
| I-14 | <100 | I-39 | 0.3 | I-63 | <100 | I-87 | <100 |
| I-15 | ---- | I-40 | 0.1 | I-64 | 8.5 | I-88 | 10.1 |
| I-16 | <100 | I-41 | <100 | I-65 | <100 | I-89 | <100 |
| I-17 | ---- | I-42 | <100 | I-66 | 1.3 | I-91 | <100 |
| I-18 | <100 | I-43 | <100 | I-67 | <100 | I-92 | 4.3 |
| I-19 | ---- | I-44 | <100 | I-68 | 2.8 | I-93 | <100 |
| I-20 | <100 | I-45 | <100 | I-69 | <100 | I-94 | 2.5 |
| I-21 | ---- | I-46 | <100 | I-70 | 4.6 | I-95 | <100 |
| I-22 | <100 | I-47 | <100 | I-71 | <100 | I-96 | 3.7 |
| I-23 | ---- | I-48 | <100 | I-72 | 7.4 | I-97 | <100 |
| I-24 | <100 | I-49 | <100 | I-73 | <100 | I-98 | 16.3 |
| I-25 | ---- | | | TPX-0005 | 50 | LOX 0-195 | 169 |
| ---- indicates not tested or undetectable | | | | | | | |

The above results show that most of the compounds in this application exhibit stronger inhibition in the Ba/F3-TPM3-NTRK1-G667C cell line compared to compound Loxo-195. The inhibitory ability of compounds I-2, I-39, I-40, I-50, I-52, I-54, I-56, I-58, I-60, I-62, I-64, I-66, I-68, I-70, I-72, I-86, I-88, I-92, I-94, I-96, and I-98 in the in vitro inhibition experiment of Ba/F3-TPM3-NTRK1-G667C cells was stronger than that of compound TPX-0005.

Table 4: The inhibitory activity IC50 value (nM) of the compounds of the present invention against NTRK G595R on Ba/F3 engineered cell line in vitro

| Exa mpl e | Ba/F3-TPM3-NTR K1-G667C | Exa mpl e | Ba/F3-TPM3-NTR K1-G667C | Exa mpl e | Ba/F3-TPM3-N TRK1-G667C | Example | Ba/F3-TPM3-NTR K1-G667C |
|---|---|---|---|---|---|---|---|
| I-1 | 72.21 | I-10 | 378 | I-58 | 2.1 | I-86 | 2.3 |
| I-2 | 244.58 | I-11 | 419 | I-60 | 2.5 | I-88 | 1.7 |
| I-3 | 527 | I-39 | 0.1 | I-62 | 0.7 | I-92 | 0.6 |
| I-5 | 91.55 | I-40 | 0.1 | I-64 | 0.9 | I-94 | 1 |
| I-6 | 527 | I-50 | 0.2 | I-66 | 0.4 | I-96 | 1.4 |
| I-7 | 198 | I-52 | 0.3 | I-68 | 1.6 | I-98 | 1.5 |
| I-8 | 562 | I-54 | 0.4 | I-70 | 1.1 | TPX-00 05 | 19.5 |
| I-9 | 297 | I-56 | 0.9 | I-72 | 1.5 | LOXO-195 | 10 |

The experimental results indicate that compounds I-39, I-40, I-50, I-52, I-54, I-56, I-58, I-60, I-62, I-64, I-66, I-68,I-70, I-72, I-86, I-88, I-92, I-94, I-96, and I-98 in this application have stronger inhibitory abilities than compounds Loxo-195 and TPX-0005 in the in vitro inhibition experiment of Ba/F3-TPM3-NTRK1-G595R cells.

Table 5: The inhibitory activity IC50 value (nM) of the compounds against NTRK WT on Ba/F3 engineered cell line in vitro

| Exa mpl e | Ba/F3-TPM3-N TRK1-G667C | Exa mpl e | Ba/F3-TPM3-N TRK1-G667C | Exa mpl e | Ba/F3-TPM3-N TRK1-G667C | Exa mpl e | Ba/F3-TPM3-N TRK1-G667C |
|---|---|---|---|---|---|---|---|
| I-1 | 3.66 | I-10 | 4.6 | I-58 | 0.14 | I-86 | 0.25 |
| I-2 | 24.80 | I-11 | 2.5 | I-60 | 0.13 | I-88 | 0.12 |
| I-3 | 17 | I-39 | 0.01 | I-62 | 0.21 | I-92 | 0.18 |
| I-5 | 7.94 | I-40 | 0.01 | I-64 | 0.30 | I-94 | 0.09 |
| I-6 | 18 | I-50 | 0.1 | I-66 | 0.05 | I-96 | 0.11 |
| I-7 | 2.8 | I-52 | 0.02 | I-68 | 0.06 | I-98 | 0.22 |
| I-8 | 3.4 | I-54 | 0.18 | I-70 | 0.12 | TPX-0005 | 1.51 |
| I-9 | 11 | I-56 | 0.2 | I-72 | 0.07 | LOX O-195 | 3.07 |

The experimental results indicate that the inhibitory ability of compounds I-39, I-40, I-50, I-52, I-54, I-56, I-58, I-60, I-62, I-64, I-66, I-68,I-70, I-72, I-86, I-88, I-92, I-94, I-96, and I-98 in the in vitro inhibition experiment of Ba/F3-TPM3-NTRK1-WT cells is stronger than that of compounds Loxo-195 and TPX-0005.

In vivo efficacy study

**Aaasy 3: Evaluation of the efficacy of compounds against Ba/F3-TPM3-NTRK1-WT subcutaneous xenograft tumor model of NOD-SCID mice in vivo**

Cell culture: Ba/F3-TPM3-NTRK1-WT cells were cultured in RPMI 1640 medium containing 10% FBS. Ba/F3-TPM3-NTRK1-WTcells in logarithmic growth phase were collected, the required total number of cells was calculated, and the cells were resuspended in a suitable volume of PBS mixture (PBS: Matrigel=1:1 (v/v)) with a cell suspension concentration of 5x106/0.1mL for subcutaneous inoculation on the right side of NOD-SCID mice.

Method: On Day 0, all animals were subcutaneously inoculated with 0.1 mL (5 × 106 Ba/F3-TPM3-NTRK1-WT cells) of cell suspension (PBS: Matrigel=1:1, V/V) on the right back. After the tumor growed to an average volume of 178.3 mm3, the mice were grouped andadministrated. The average tumor volume of the enrolled animals is 178.3 $mm^3$. The mice were divided into two groups, Group-1 (Vehicle) and Group-2 (10 mg/kg, I-52). Each group consisted of 6 animals. All groups were administered orally by gavage with a frequency of BID, for a total of 29 days.

Tumor volume inhibition rate $TGI_{TV}$ (%): TGI%=(1- △T/△C) × 100%, wherein ΔC is the tumor volume $C_t$-$C_0$ of the control group, $C_0$ is the average tumor volume of the control group at the time of grouping, $C_1$ is the average tumor volume of the control group after treatment, ΔT is the tumor volume $T_t$-$T_0$ of the treatment group, $T_0$ is the average tumor volume of the control group at grouping, and $T_t$ is the average tumor volume of the control group after treatment.

Pharmacological activity was evaluated based on tumor suppression rate ($TGI_{TV}$), and tolerance was evaluated based on changes in animal weight and mortality.

**Table 6: Pharmacodynamic Analysis of Tumor Volume on Day 29 in Ba/F3-TMP3-NTRK1-WT Subcutaneous Xenograft Model**

| ***Group*** | ***Day 29*** | | | |
|---|---|---|---|---|
| | **Tumor Volume($mm^3$) (average)** | **Relative Tumor Volume (%) (average)** | **$TGI_{Tv}$** | **Tumor Regression Rate** |
| Group-1: vehicle | 1643.0 | 900.3 | - | - |
| Group-2: I-52; 10 mg/kg; | 0.0 | - | 112% | 100% |

**Table 7: Original Records of Average Tumor Volume During the Experiment (Unit: $mm^3$)**

| Groups | 4 | 6 | 8 | 10 | 12 | 14 | 15 |
|---|---|---|---|---|---|---|---|
| Group-1 | 181.4 | 218.5 | 261.7 | 324.9 | 476.8 | 662.2 | 763.9 |
| Group-2 | 182.5 | 192.7 | 176.5 | 146.3 | 113.1 | 74.4 | 67.9 |
| **Groups** | **17** | **19** | **21** | **23** | **25** | **27** | **29** |
| Group-1 | 1061.3 | 1310.1 | 1643.0 | 2211.7 | 2455.6 | 2663.3 | 2522.9 |
| Group-2 | 60.1 | 32.8 | 0.0 | 0.0 | 0.0 | 0.0 | 0.0 |

Compared with the tumor volume of the vehicle group (Group 1), Group 2 shows a significant difference in average tumor volume. At the end of the experiment, tumors were collected and weighed. In Group 2, tumors disappeared completely in all mice, achieving a tumor regression rate of 100%.

The results indicate that the test compound I-52 exhibits significant pharmacodynamic activity in inhibiting the proliferation of Ba/F3-TPM3-NTRK1-WT subcutaneous xenografts in mice. At a dose of 10 mg/kg, the tumor regression rate reaches 100%. The compound is well tolerated, with no significant changes in body weight and no notable clinical abnormalities is observed.

**Aaasy 4: Evaluation of the efficacy of compounds against Ba/F3-TPM3-NTRK1-G667C subcutaneous xenograft tumor model of NOD-SCID mice in vivo**

Cell Culture: Ba/F3-TPM3-NTRK1-G667C cells were cultured in RPMI 1640 medium containing 10% FBS. Log-phase cells were collected, and the total number of cells required was calculated. Cells were resuspended in an appropriate volume of PBS mixture solution (PBS:Matrigel = 1:1, v/v) at a concentration of $5 \times 10^6$ cells/0.1 mL for subcutaneous inoculation into the right flank of NOD-SCID mice.

Method: at Day 0, all animals were subcutaneously inoculated with 0.1 mL ($5 \times 10^6$ cells) of Ba/F3-TPM3-NTRK1-G667C cell suspension (PBS: Matrigel = 1:1, v/v) on the right flank. When the tumors reached an average volume of 197.2 $mm^3$, dosing and grouping began. The enrolled animals had an average tumor volume of 197.2 $mm^3$. The animals were divided into two groups: Group 1: Vehicle control; Group 2: 10 mg/kg of compound I-52. Each group contained six mice. All groups were administered orally via gavage twice daily (BID) for 29 days.

Tumor Volume Inhibition Rate $TGI_{TV}$(%): TGI% = (1-△T/△C) × 100%; Where: ΔC is the tumor volume $C_t$-$C_0$ of the control group, $C_0$ is the average tumor volume of the control group at the time of grouping, $C_t$ is the average tumor volume of the control group after treatment, ΔT is the tumor volume $T_t$-$T_0$ of the treatment group, $T_0$ is the average tumor volume of the control group at grouping, and Tt is the average tumor volume of the control group after treatment.

Pharmacological activity was evacuated based on tumor suppression rate ($TGI_{TV}$), and tolerance wa assessed based on changes in animal weight and mortality.

**Table 8: Pharmacodynamic Analysis of Tumor Volume on Day 29 in Ba/F3-TMP3-NTRK1-G667C Subcutaneous Xenograft Model**

| ***Group*** | ***Day 29*** | | |
|---|---|---|---|
| | **Tumor Volume($mm^3$) (average)** | **Relative Tumor Volume (%) (average)** | **$TGI_{Tv}$** |
| Group-1: vehicle | 2953.8 | 1522.3 | - |
| Group-2: I-52; 10 mg/kg; | 636.5 | 308.6 | 84% |

**Table 9: Original Records of Average Body Weight During the Experiment (Unit: g)**

| Groups | 2 | 7 | 9 | 11 | 13 | 15 | 17 | 19 | 21 | 23 | 25 | 27 | 29 |
|---|---|---|---|---|---|---|---|---|---|---|---|---|---|
| Group-1 | 19.2 | 19.8 | 19.7 | 20.2 | 20.7 | 21.4 | 22.3 | 22.3 | 22.7 | 26.4 | 27.3 | 27.4 | 24.4 |
| Group-2 | 19.1 | 20.9 | 21.0 | 21.8 | 22.0 | 22.8 | 23.0 | 23.7 | 23.3 | 24.3 | 24.7 | 25.3 | 24.6 |

Compared with the tumor volume of the vehicle group (Group 1), Group 2 showed a significant difference in average tumor volume. At the end of the experiment, tumors were collected and weighed. The average tumor weights were consistent with the average tumor volume analysis.

The results indicate that the test compound I-52 exhibits significant pharmacodynamic activity in inhibiting the

proliferation of Ba/F3-TPM3-NTRK1-G667C subcutaneous xenografts in mice. At a dose of 10 mg/kg, the tumor growth inhibition rate reached 84%. The compound was well tolerated, without significant changes in body weight and notable clinical abnormalities observed.

The above description is only a specific embodiment of the present invention, but the scope of protection of the present invention is not limited thereto, and any person skilled in the art who is familiar with the technical field of the present invention can readily think of changes and modifications within the technical scope of the present invention as disclosed herein shall be covered within the scope of protection of the present invention. Therefore, the scope of protection of the present invention shall be in accordance with the scope of protection of the claims.

## Claims

1. A macrocyclic imidazo [1,2-b] pyridazine compound represented by formula I, or an isotopically labeled compound, an optical isomer, a geometric isomer, a tautomer, an isomers mixture, a pharmaceutically acceptable salt, a prodrug, or a metabolite thereof,

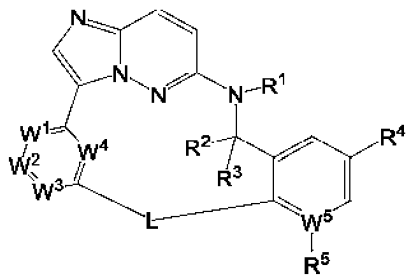

Formula I

wherein,

$W^1$, $W^2$, $W_3$, $W^4$ and $W^5$ are each independently selected from carbon or nitrogen, and at least one of $W^1$, $W^2$, $W_3$, $W^4$ and $W^5$ is nitrogen;

$R_1$, $R^2$ and $R^3$ are each independently selected from hydrogen, deuterium, halogen, cyano, hydroxyl, amino, substituted or unsubstituted saturated or unsaturated $C_1$-$C_6$ alkyl, substituted or unsubstituted saturated or unsaturated $C_3$-$C_6$ cycloalkyl, substituted or unsubstituted saturated or unsaturated $C_1$-$C_6$ alkoxy, substituted or unsubstituted saturated or unsaturated $C_3$-$C_6$ cycloalkoxy, or $R^1$ and $R^2$ together with the N and C atoms to which they are connected

form a substituted or unsubstituted 4- to 10- membered heterocycloalkyl containing 1 to 3 heteroatoms selected from N, O and S, or a substituted or unsubstituted 5- to 14- membered heteroaryl containing 1 to 3 heteroatoms selected from N, O and S, or $R^2$ and $R^3$ together with the C atom to which they are connected form a substituted or unsubstituted saturated or unsaturated $C_3$-$C_6$ cycloalkyl; wherein the "substituted" refers to selectively having 1 to 4

substituents selected from deuterium, hydroxyl, halogen, cyano, sulfonyl, amino, $C_1$-$C_4$ alkyl, $C_3$-$C_6$ cycloalkyl, $C_1$-$C_4$ alkoxy and $C_3$-$C_6$ cycloalkoxy;

$R_4$ is selected from hydrogen, deuterium, halogen, cyano, hydroxyl, amino, saturated or unsaturated $C_1$-$C_6$ alkyl, saturated or unsaturated $C_3$-$C_6$ cycloalkyl, saturated or unsaturated $C_1$-$C_6$ alkoxy, and saturated or unsaturated $C_3$-$C_6$ cycloalkoxy;

$R^5$ is selected from hydrogen, deuterium, halogen, cyano, hydroxyl, amino, saturated or unsaturated $C_1$-$C_6$ alkyl, saturated or unsaturated $C_3$-$C_6$ cycloalkyl, substituted or unsaturated $C_1$-$C_6$ alkoxy, and saturated or unsaturated $C_3$-$C_6$ cycloalkoxy, or $R^5$ is absent;

L is selected from -O-, -NH-, substituted or unsubstituted branched or straight chain $C_1$-$C_6$ alkylene, substituted or unsubstituted branched or straight chain $C_1$-$C_6$ alkyleneoxy, substituted

or unsubstituted saturated or unsaturated $C_3$-$C_6$ cycloalkylene, substituted or unsubstituted saturated or unsaturated $C_3$-$C_6$ cycloalkyleneoxy, substituted or unsubstituted branched or straight chain $C_1$-$C_6$ alkylenethio, substituted or unsubstituted saturated or unsaturated 3 to 6-membered oxacycloalkylene, substituted or unsubstituted saturated or unsaturated 3 to 6-membered azacycloalkylene, substituted or unsubstituted saturated or unsaturated 3 to 6-membered thiacycloalkylene, substituted or unsubstituted unsubstituted saturated or unsaturated 3- to 6-membered oxacycloalkyleneoxy, substituted or unsubstituted saturated or unsaturated 3-to

6-membered azacycloalkyleneoxy, substituted or unsubstituted saturated or unsaturated 3- to 6-membered thiacycloalkyleneoxy; wherein the "substituted" refers to selectively having 1 to 4 substituents selected from deuterium, halogen, cyano, hydroxyl, carboxyl, carbonyl, sulfonyl, amino, $C_1$-$C_4$ alkyl, $C_1$-$C_4$ hydroxyalkyl, $C_3$-$C_6$ cycloalkyl, $C_1$-$C_4$ alkoxy, $C_3$-$C_6$ cycloalkoxy, 4 to 8-membered heterocycloalkyl containing 1 to 3 heteroatoms selected from N, O and S, and 5 to 10 membered heteroaryl containing 1 to 3 heteroatoms selected from N, O and S.

**2.** The macrocyclic imidazo [1,2-b] pyridazine compound represented by formula I according to claim 1, or an isotopically labeled compound, an optical isomer, a geometric isomer,

a tautomer, an isomers mixture, a pharmaceutically acceptable salt, a prodrug, or a metabolite thereof, wherein,

preferably, $R_1$, $R^2$ and $R^3$ are each independently selected from hydrogen, deuterium, halogen, cyano, hydroxyl, amino, substituted or unsubstituted saturated or unsaturated $C_1$-$C_4$ alkyl, substituted or unsubstituted saturated or unsaturated $C_3$-$C_6$ cycloalkyl, substituted or unsubstituted saturated or unsaturated $C_1$-$C_4$ alkoxy, substituted or unsubstituted saturated or unsaturated $C_3$-$C_6$ cycloalkoxy, or $R^1$ and $R^2$ together with the N and C atoms to which they are connected form a substituted or unsubstituted 4- to 8- membered heterocycloalkyl containing 1 to 3 heteroatoms selected from N, O and S, or a substituted or unsubstituted 5- to 10- membered heteroaryl containing 1 to 3 heteroatoms selected from N, O and S, or $R^2$ and $R^3$ together with the C atom to which they are connected form a substituted or unsubstituted saturated or unsaturated $C_3$-$C_6$ cycloalkyl; wherein the "substituted" refers to selectively having 1 to 3 substituents selected from deuterium, hydroxyl, halogen, cyano, sulfonyl, amino, $C_1$-$C_3$ alkyl, $C_3$-$C_6$ cycloalkyl, $C_1$-$C_3$ alkoxy and $C_3$-$C_6$ cycloalkoxy;

more preferably, $R_1$, $R^2$ and $R^3$ are each independently selected from hydrogen, deuterium, halogen, cyano, hydroxyl, amino, substituted or unsubstituted saturated or unsaturated $C_1$-$C_3$ alkyl, substituted or unsubstituted saturated or unsaturated $C_5$-$C_6$ cycloalkyl, or $R^1$ and $R^2$ together with the N and C atoms to which they are connected form a substituted or unsubstituted 4- to 7- membered heterocycloalkyl containing 1 to 3 heteroatoms selected from N, O and S, or $R^2$ and $R^3$ together with the C atom to which they are connected form a

substituted or unsubstituted saturated or unsaturated $C_5$-$C_6$ cycloalkyl; wherein the "substituted" refers to selectively having 1 to 3 substituents selected from deuterium, hydroxyl, halogen, cyano, sulfonyl, amino, $C_1$-$C_3$ alkyl and $C_5$-$C_6$ cycloalkyl;

more preferably, $R_1$, $R^2$ and $R^3$ are each independently selected from hydrogen, deuterium, halogen, substituted or unsubstituted saturated or unsaturated $C_1$-$C_3$ alkyl, or $R^1$ and $R^2$ together with the N and C atoms to which they are connected form a substituted or unsubstituted 4-to 6-membered heterocycloalkyl containing 1 or 2 heteroatoms selected from N, O and S, or $R^2$ and $R^3$ together with the C atom to which they are connected form a substituted or unsubstituted saturated or unsaturated $C_5$-$C_6$ cycloalkyl; wherein the "substituted" refers to selectively having 1 to 2 substituents selected from deuterium, hydroxyl and halogen;

preferably, $R_4$ is selected from hydrogen, deuterium, halogen, cyano, hydroxyl, amino, saturated or unsaturated $C_1$-$C_4$ alkyl, saturated or unsaturated $C_5$-$C_6$ cycloalkyl, saturated or unsaturated $C_1$-$C_4$ alkoxy, and saturated or unsaturated $C_5$-$C_6$ cycloalkoxy;

more preferably, $R_4$ is selected from hydrogen, deuterium, halogen, cyano, hydroxyl, amino, saturated or unsaturated $C_1$-$C_3$ alkyl, and saturated or unsaturated $C_1$-$C_3$ alkoxy;

more preferably, $R_4$ is selected from hydrogen, deuterium, halogen, saturated or unsaturated $C_1$-$C_3$ alkyl, and saturated or unsaturated $C_1$-$C_3$ alkoxy;

more preferably, $R_4$ is selected from hydrogen, deuterium and halogen;

Preferably, $R^5$ is selected from hydrogen, deuterium, halogen, cyano, hydroxyl, amino, saturated or unsaturated $C_1$-$C_4$ alkyl, saturated or unsaturated $C_5$-$C_6$ cycloalkyl, substituted or unsaturated $C_1$-$C_4$ alkoxy, and saturated or unsaturated $C_5$-$C_6$ cycloalkoxy, or $R^5$ is absent;

more preferably, $R^5$ is selected from hydrogen, deuterium, halogen, cyano, hydroxyl, amino, saturated or unsaturated $C_1$-$C_3$ alkyl, and saturated or unsaturated $C_1$-$C_3$ alkoxy, or $R^5$ is absent; more preferably, $R^5$ is selected from hydrogen, deuterium, halogen, saturated or

unsaturated $C_1$-$C_3$ alkyl, and substituted or unsaturated $C_1$-$C_3$ alkoxy, or $R^5$ is absent;

more preferably, $R^5$ is selected from hydrogen, deuterium and halogen, or $R^5$ is absent.

**3.** The macrocyclic imidazo [1,2-b] pyridazine compound represented by formula I according to claim 1, or an isotopically labeled compound, an optical isomer, a geometric isomer, a tautomer, an isomers mixture, a pharmaceutically acceptable salt, a prodrug, or a metabolite thereof, wherein,

L is selected from -O-, -NH-, substituted or unsubstituted branched or straight chain $C_1$-$C_4$ alkylene, substituted or unsubstituted branched or straight chain $C_1$-$C_4$ alkyleneoxy, substituted or unsubstituted saturated or unsaturated $C_4$-$C_6$ cycloalkylene, substituted or unsubstituted saturated or unsaturated $C_4$-$C_6$ cycloalkyleneoxy, substituted or unsubstituted branched or

straight chain $C_1$-$C_4$ alkylenethio, substituted or unsubstituted saturated or unsaturated 4 to 6-membered oxacycloalkylene, substituted or unsubstituted saturated or unsaturated 4 to 6-membered azacycloalkylene, substituted or unsubstituted saturated or unsaturated 4 to 6-membered thiacycloalkylene, substituted or unsubstituted unsubstituted saturated or unsaturated 4- to 6-membered oxacycloalkyleneoxy, substituted or unsubstituted saturated or unsaturated 4- to 6-membered azacycloalkyleneoxy, substituted or unsubstituted saturated or unsaturated 4- to 6-memberedthiacycloalkyleneoxy; wherein the "substituted" refers to selectively having 1 to 3 substituents selected from deuterium, halogen, cyano, hydroxyl, carbonyl, amino, $C_1$-$C_4$ alkyl, $C_1$-$C_4$ hydroxyalkyl and $C_3$-$C_6$ cycloalkyl;

more preferably, L is selected from -O-, -NH-, substituted or unsubstituted branched or straight chain $C_1$-$C_3$ alkylene, substituted or unsubstituted branched or straight chain $C_1$-$C_4$ alkyleneoxy, substituted or unsubstituted saturated or unsaturated $C_5$-$C_6$ cycloalkylene, substituted or unsubstituted saturated or unsaturated $C_5$-$C_6$ cycloalkyleneoxy, substituted or unsubstituted saturated or unsaturated 5 to 6-membered oxacycloalkylene, substituted or unsubstituted saturated or unsaturated 5 to 6-membered azacycloalkylene, substituted or unsubstituted unsubstituted saturated or unsaturated 5 to 6-membered oxacycloalkyleneoxy, substituted or unsubstituted saturated or unsaturated 5 to 6-membered azacycloalkyleneoxy; wherein the "substituted" refers to selectively having 1 to 3 substituents selected from deuterium and halogen;
more preferably, L is selected from substituted or unsubstituted branched or straight chain $C_1$-$C_3$ alkylene, substituted or unsubstituted branched or straight chain $C_1$-$C_3$ alkyleneoxy, substituted or unsubstituted saturated or unsaturated $C_5$-$C_6$ cycloalkylene, substituted or unsubstituted saturated or unsaturated $C_5$-$C_6$ cycloalkyleneoxy, substituted or unsubstituted saturated or unsaturated 5 to 6-membered oxacycloalkylene, substituted or unsubstituted saturated or unsaturated 5 to 6-membered azacycloalkylene, substituted or unsubstituted unsubstituted saturated or unsaturated 5 to 6-membered oxacycloalkyleneoxy, substituted or unsubstituted saturated or unsaturated 5 to 6-membered azacycloalkyleneoxy; wherein the "substituted" refers to selectively having 1 to 2 substituents selected from deuterium and halogen;
more preferably, L is selected from $-CH_2O-$, $-CH_2CH_2O-$, $-CH_2CH_2CH_2O-$, $-CH_2CH(CH_3)O-$, $-CH(CH_3)CH_2O-$,

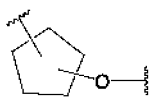 , 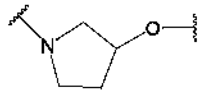 , 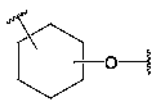

and

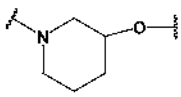 .

**4.** The macrocyclic imidazo [1,2-b] pyridazine compound represented by formula I according to claim 1, or an isotopically labeled compound, an optical isomer, a geometric isomer, a tautomer, an isomers mixture, a pharmaceutically acceptable salt, a prodrug, or a metabolite thereof, wherein, the compound represented by formula I, or an isotopically labeled compound, an optical isomer, a geometric isomer, a tautomer, an isomers mixture, a pharmaceutically acceptable salt, a prodrug, or a metabolite thereof, is represented by Formula I-1, Formula I-2, Formula I-3, Formula I-4, Formula I-5 or Formula I-6;

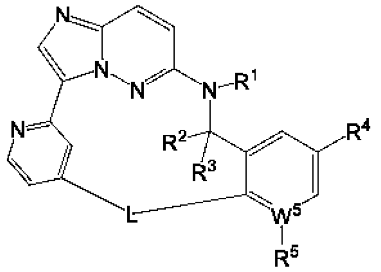

Formula I-1

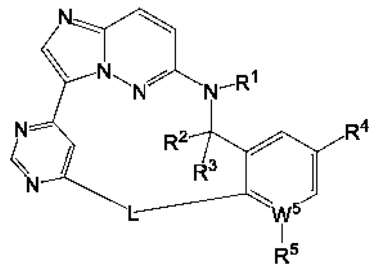

Formula I-2

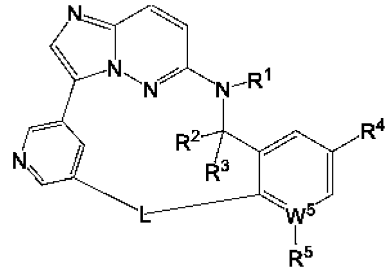

Formula I-3

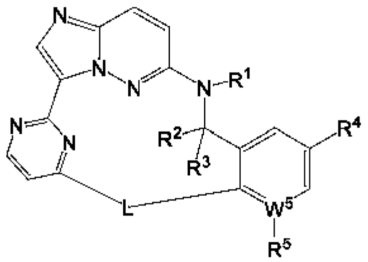

Formula I-4

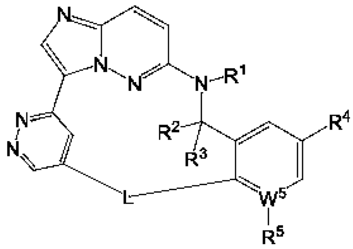

Formula I-5

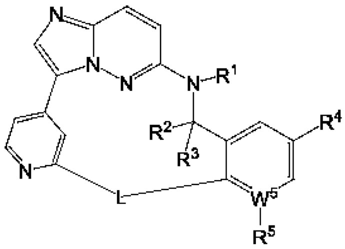

Formula I-6

wherein, the substituents $W^5$, $R^1$, $R^2$, $R^3$, $R_4$, $R^5$ and L are as defined in claim 1.

**5.** The macrocyclic imidazo [1,2-b] pyridazine compound represented by formula I according to claim 1, or an isotopically labeled compound, an optical isomer, a geometric isomer, a tautomer, an isomers mixture, a pharmaceutically acceptable salt, a prodrug, or a metabolite thereof, wherein, the compound represented by formula I, or an isotopically labeled compound, an optical isomer, a geometric isomer, a tautomer, an isomers mixture, a pharmaceutically acceptable salt, a prodrug, or a metabolite thereof, is represented by Formula I-1-1, Formula I-2-1, Formula I-3-1, Formula I-4-1, Formula I-5-1 or Formula I-6-1;

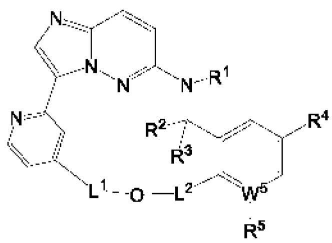

Formula I-1-1

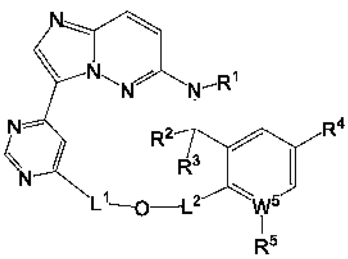

Formula I-2-1

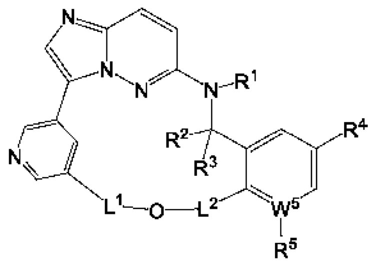

Formula I-3-1

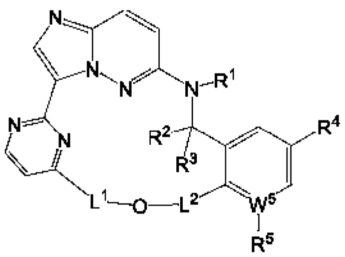

Formula I-4-1

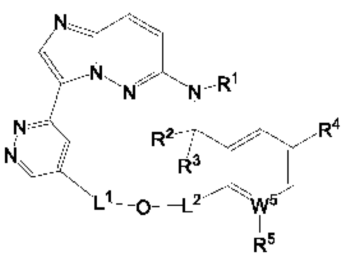

Formula I-5-1

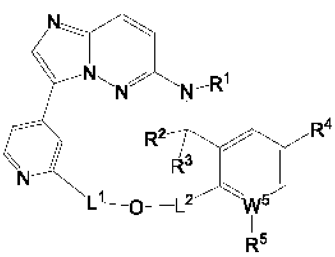

Formula I-6-1

wherein, the substituents $W^5$, $R^1$, $R^2$, $R_3$, $R^4$ and $R^4$ are as defined in claim 1;

$L_{1\ and}$ $L^2$ are independently selected from a chemical bond, substituted or unsubstituted branched or straight chain $C_1$-$C_3$ alkylene, substituted or unsubstituted saturated or unsaturated -$C_3$-$C_6$ - cycloalkylene, substituted or unsubstituted saturated or unsaturated 3- to 6-membered heterocyclic alkylene ring containing 1 or 2 heteroatoms selected

from O and N; wherein the "substituted" refers to selectively having 1 to 2 substituents selected from deuterium and halogen, and $L^1$ and $L^2$ are not simultaneously chemical bonds;
preferably, $L^1$ and $L^2$ are independently selected from chemical bond, methylene, ethylene, propylene, isopropylene, cyclopropylene, cyclobutylene, cyclopentylene, cyclohexylene, halogenated methylene, halogenated ethylene, halogenated propylene, halogenated isopropylene, halogenated cyclopropylene, halogenated cyclobutylene, halogenated cyclopentylene, halogenated cyclohexylene, oxiranylene, oxetanylene, tetrahydrofuranylene, tetrahydropyranylene, pyrrolidinylene, piperidinylene, halogenated oxiranylene, halogenated oxetanylene, halogenated tetrahydrofuranylene, halogenated tetrahydropyranylene, halogenated pyrrolidinylene, halogenated piperidinylene, and $L^1$ and $L_2$ are not simultaneously chemical bonds.

**6.** The macrocyclic imidazo [1,2-b] pyridazine compound represented by formula I according to any one of claims 1-5, or an isotopically labeled compound, an optical isomer, a geometric isomer, a tautomer, an isomers mixture, a pharmaceutically acceptable salt, a prodrug, or a metabolite thereof, wherein the compound is selected from:

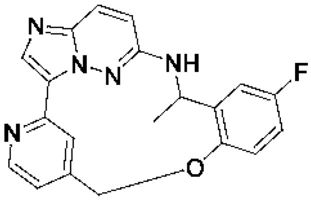

I-1

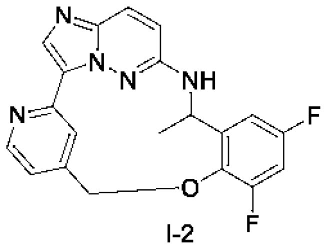

I-2

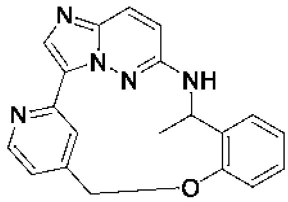

I-3

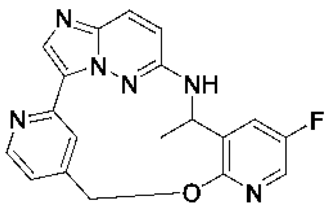

I-4

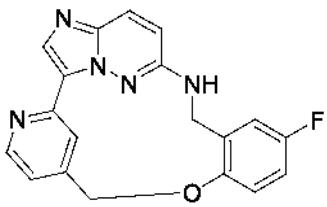

I-5

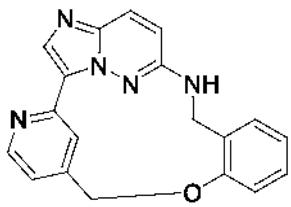

I-6

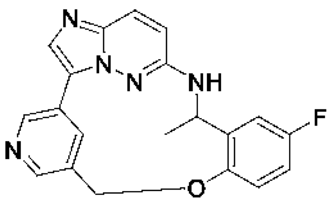

I-7

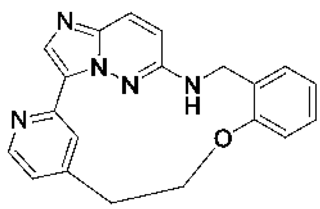

I-8

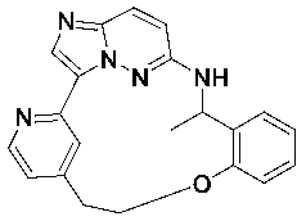

I-9

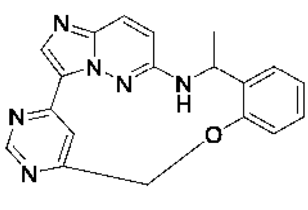

I-10

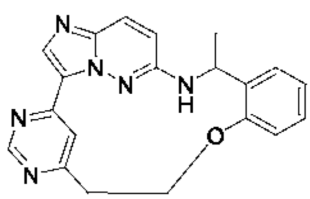

I-11

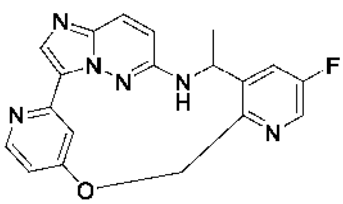

I-12

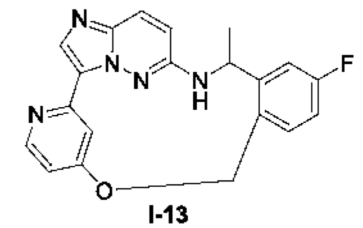
I-13

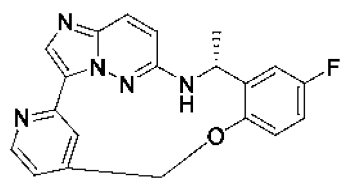
I-14

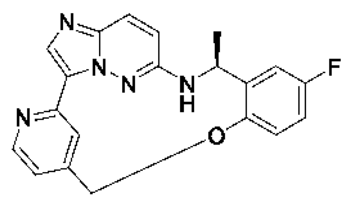
I-15

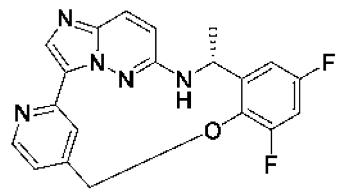
I-16

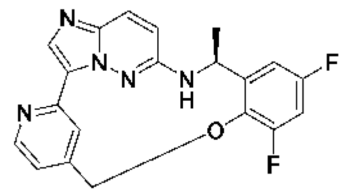
I-17

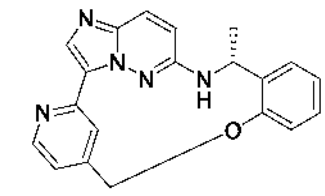
I-18

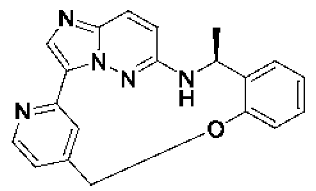
I-19

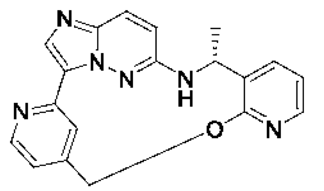
I-20

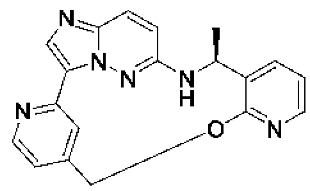
I-21

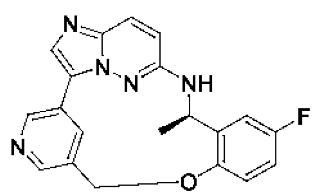
I-22

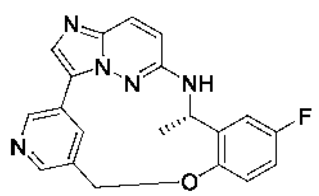
I-23

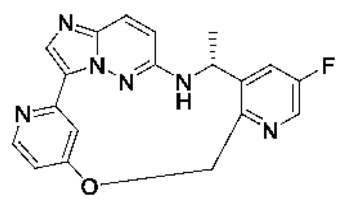
I-24

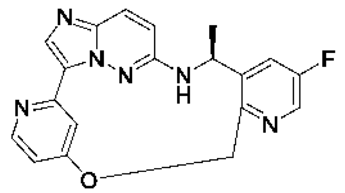
I-25

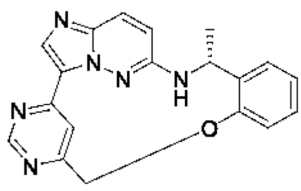
I-26

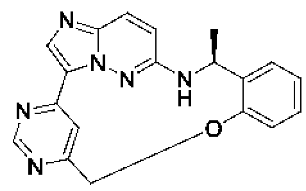
I-27

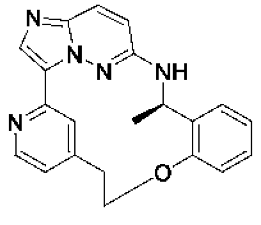
I-28

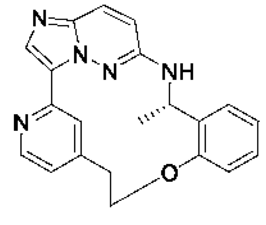
I-29

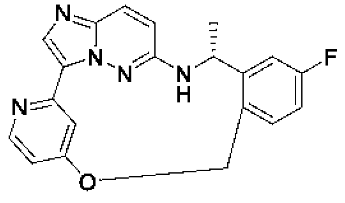
I-30

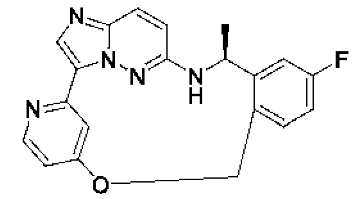
I-31

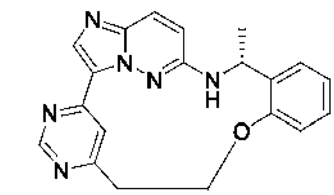
I-32

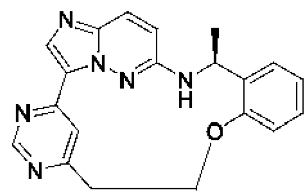
I-33

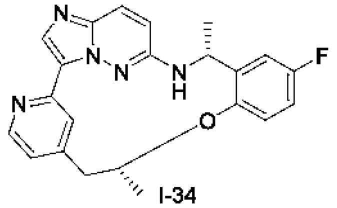
I-34

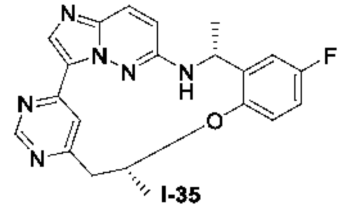
I-35

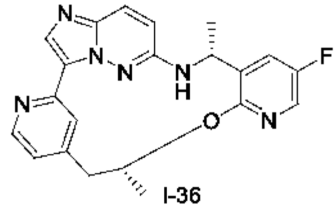
I-36

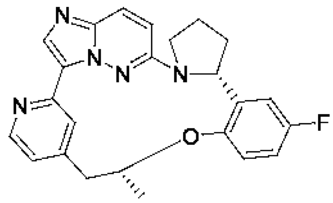
I-37

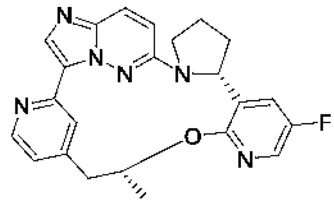
I-38

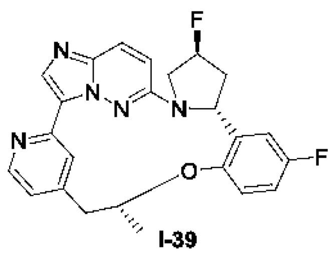
I-39

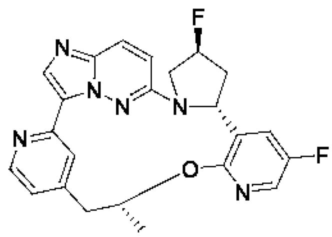
I-40

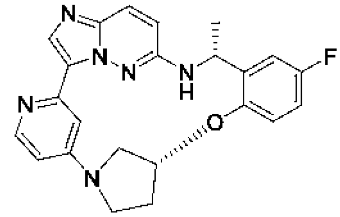
I-41

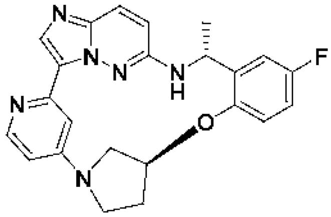
I-42

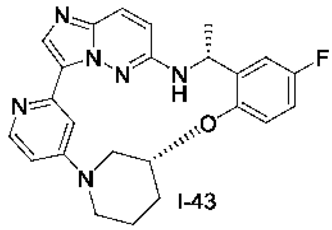
I-43

I-43

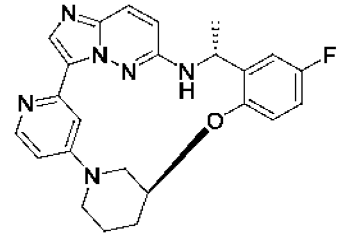
I-44

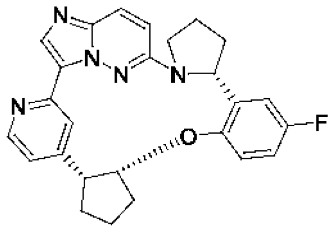
I-45

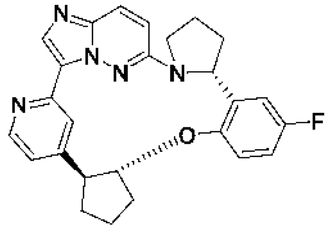
I-46

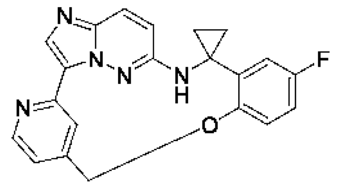
I-47

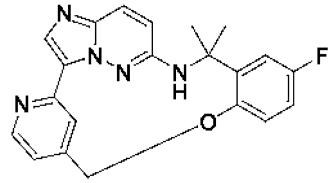
I-48

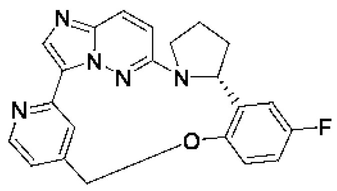

I-49

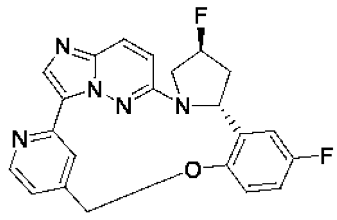

I-50

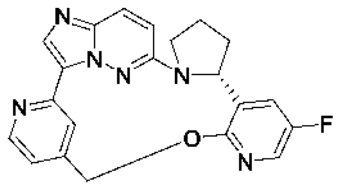

I-51

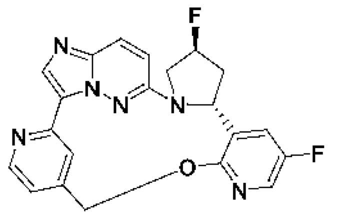

I-52

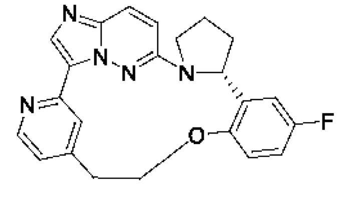

I-53

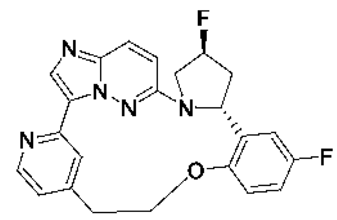

I-54

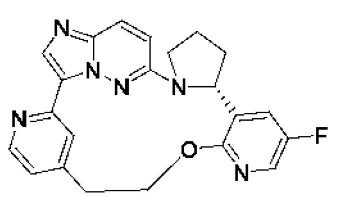

I-55

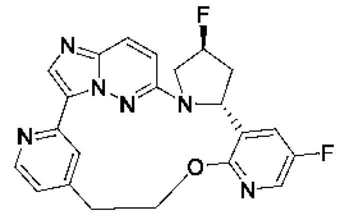

I-56

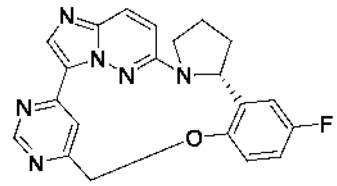

I-57

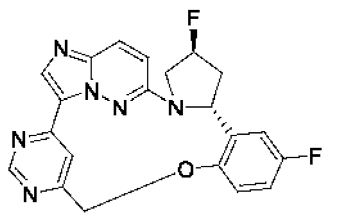

I-58

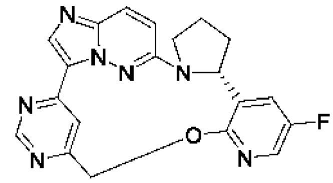

I-59

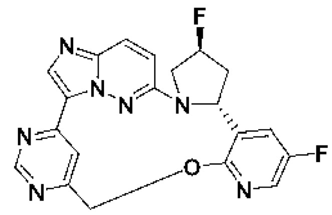

I-60

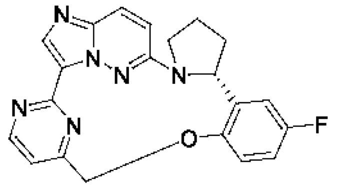

I-61

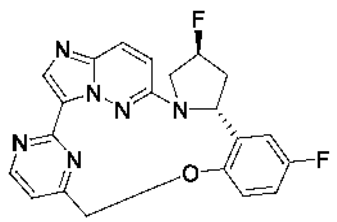

I-62

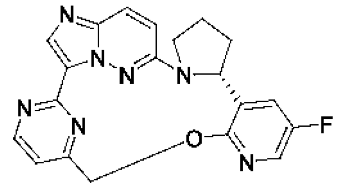

I-63

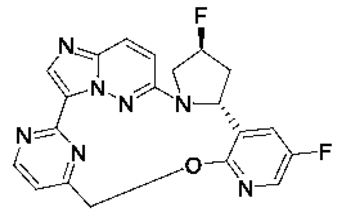

I-64

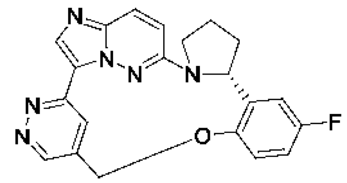

I-65

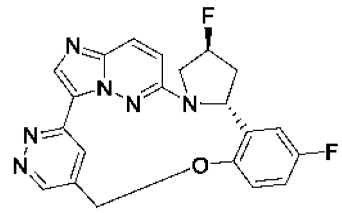

I-66

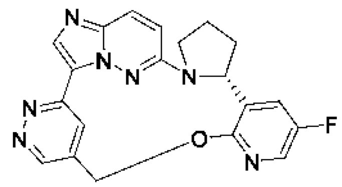

I-67

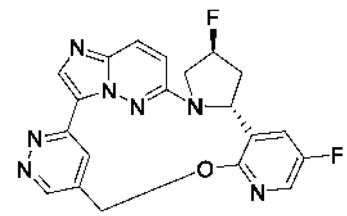

I-68

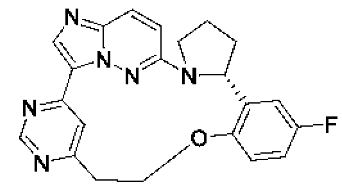

I-69

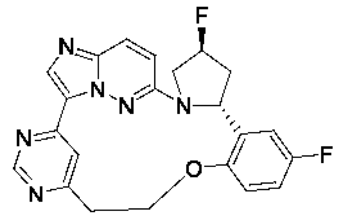

I-70

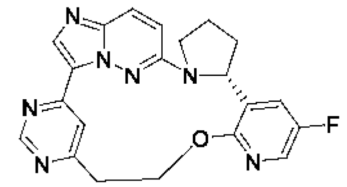

I-71

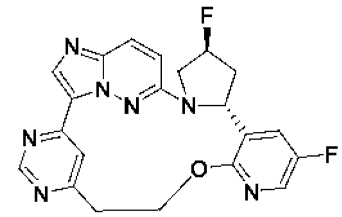

I-72

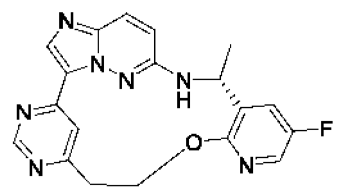

I-73

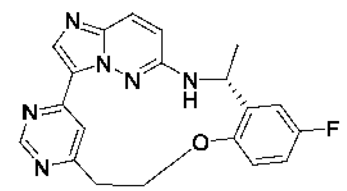

I-74

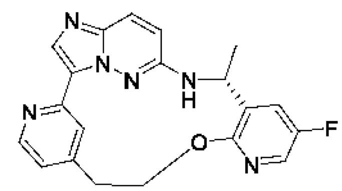

I-75

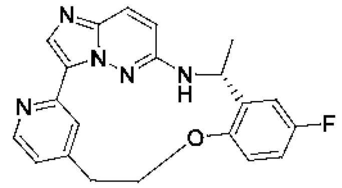

I-76

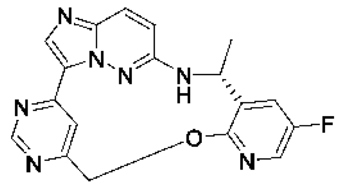

I-77

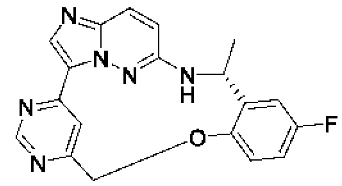

I-78

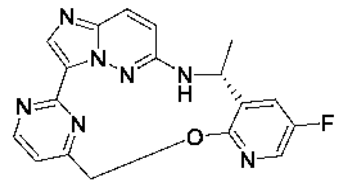

I-79

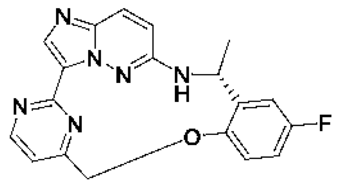

I-80

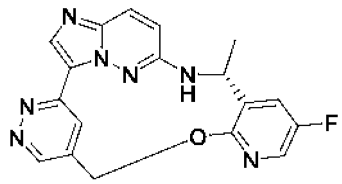

I-81

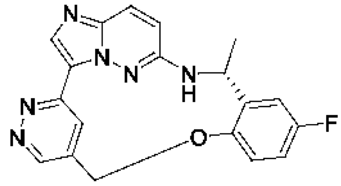

I-82

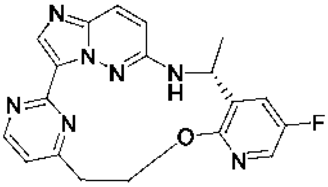

I-83

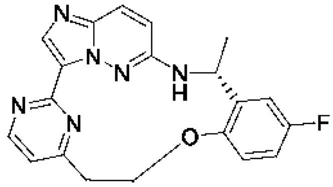

I-84

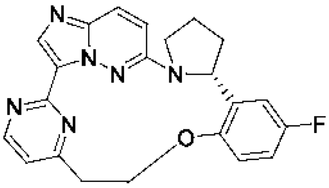

I-85

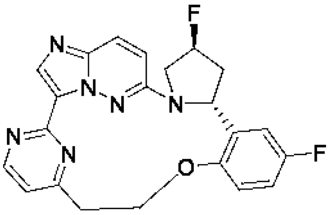

I-86

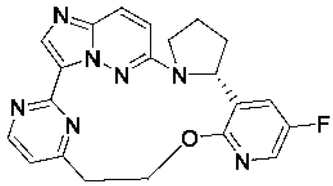

I-87

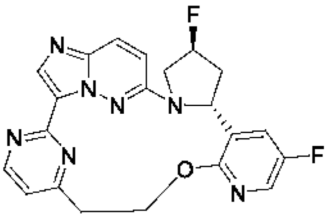

I-88

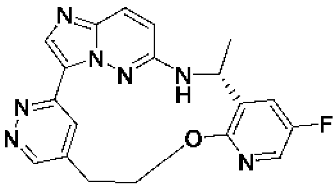

I-89

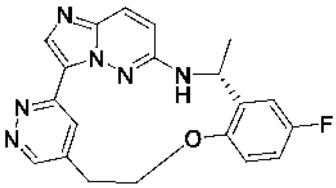

I-90

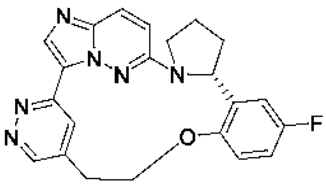

I-91

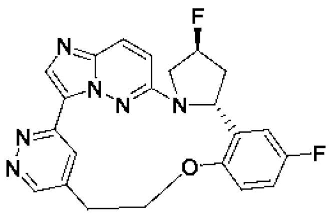

I-92

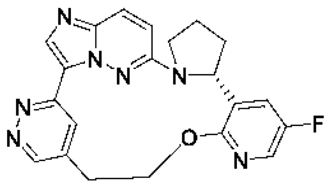

I-93

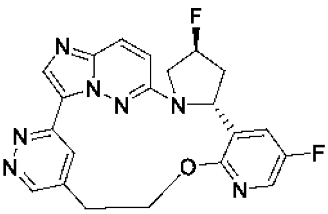

I-94

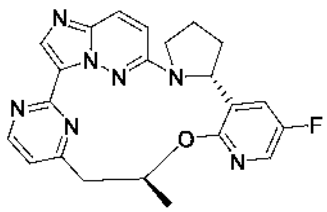

I-95

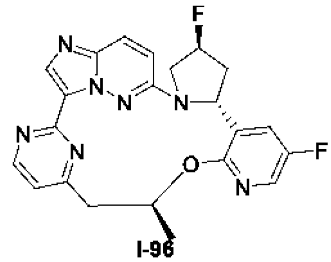

I-96

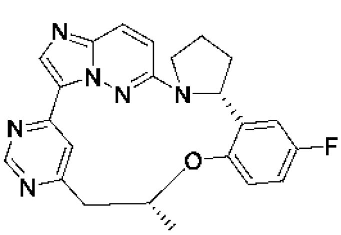

I-97

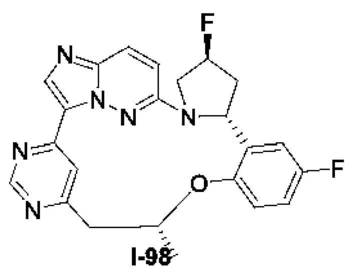

I-98

**7.** A pharmaceutical composition, comprising a therapeutically effective amount of the compound according to any one

of claims 1- 6, or an isotopically labeled compound, an optical isomer, a geometric isomer, a tautomer, an isomers mixture, a pharmaceutically acceptable salt, a prodrug, or a metabolite thereof, and pharmaceutically acceptable carriers.

**8.** A use of the compound represented by formula I according to any one of claims 1- 6, or an isotopically labeled compound, an optical isomer, a geometric isomer, a tautomer, an isomers mixture, a pharmaceutically acceptable salt, a prodrug, or a metabolite thereof in the preparation of a drug as a TRK kinase inhibitor for the treatment or prevention of diseases or disorders mediated by TRK or TRK mutations in a subject in need thereof;
preferably, the disease or disorder mediated by TRK or TRK mutations is selected from one or more of cancers, neurodegenerative diseases, inflammation, and pain;
more preferably, the disease or disorder mediated by TRK or TRK mutations is selected from surgical pain, inflammatory pain, neuropathic pain, Alzheimer's disease, Parkinson's disease, multiple sclerosis, colon cancer, thyroid cancer, lung cancer, prostate cancer, ovarian cancer, breast cancer, salivary gland cancer, pancreatic cancer, melanoma, salivary tumor, cholangiocarcinoma, stromal tumor, brain tumor and malignant blood disease.

**9.** A kit, comprising the compound represented by formula I according to any one of claims 1- 6, or an isotopically labeled compound, an optical isomer, a geometric isomer, a tautomer, an isomers mixture, a pharmaceutically acceptable salt, a prodrug, or a metabolite thereof, or the pharmaceutical composition according to the present application, and a container and instructions for use.

**10.** A method for treating diseases or disorders mediated by TRK or TRK mutations, comprising a step of administering to a subject in need thereof an effective amount of the compound represented by formula I according to any one of claims 1- 6, or an isotopically labeled compound, an optical isomer, a geometric isomer, a tautomer, an isomers mixture, a pharmaceutically acceptable salt, a prodrug, or a metabolite thereof, or the pharmaceutical composition according to claim 7.

## INTERNATIONAL SEARCH REPORT

International application No. **PCT/CN2023/116671**

**A. CLASSIFICATION OF SUBJECT MATTER**

C07D 498/22(2006.01)i；C07D471/22(2006.01)i；C07D487/22(2006.01)i；C07D515/22(2006.01)i；A61K31/5025(2006.01)i；A61P25/00(2006.01)i；A61P29/00(2006.01)i；A61P35/00(2006.01)i

According to International Patent Classification (IPC) or to both national classification and IPC

**B. FIELDS SEARCHED**

Minimum documentation searched (classification system followed by classification symbols)

IPC:C07D498/-,C07D471/-,C07D487/-,C07D515/-,A61K,A61P

Documentation searched other than minimum documentation to the extent that such documents are included in the fields searched

Electronic data base consulted during the international search (name of data base and, where practicable, search terms used)

CNKI, CNABS, CNTXT, ENTXT, DWPI, VEN, REGISTRY, CAPLUS, MARPAT, 大环, 咪唑, 哒嗪, 肿瘤, 癌症, 疼痛, 神经, 原肌球蛋白受体激酶, kinase, macrocyclic, imidazole, pyridazine, tumor, cancer, pain, nerve, TRK, 根据式I进行的结构式检索, search according to structural formula I.

**C. DOCUMENTS CONSIDERED TO BE RELEVANT**

| Category* | Citation of document, with indication, where appropriate, of the relevant passages | Relevant to claim No. |
|---|---|---|
| A | CN 106687464 A (ONCODESIGN S.A.) 17 May 2017 (2017-05-17)<br>entire document, in particular claim 8, embodiments 1-14 | 1-10 |
| A | CN 105209040 A (ONCODESIGN S.A.) 30 December 2015 (2015-12-30)<br>entire document, in particular claim 9 | 1-10 |
| A | CN 105228625 A (ONCODESIGN S.A.) 06 January 2016 (2016-01-06)<br>entire document, in particular claim 9 | 1-10 |
| A | CN 105143232 A (IPSEN PHARMA S.A.S.; ONCODESIGN S.A.) 09 December 2015 (2015-12-09)<br>entire document, in particular claim 7 | 1-10 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 November 2023** | **24 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/116671**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **10**
because they relate to subject matter not required to be searched by this Authority, namely:

Claim 10 relates to a method for treatment of the human or animal body (PCT Rule 39.1(iv)). Nevertheless, a search is still conducted on the basis of the technical subject matter of the use of the compound/composition in the preparation of a drug for treating TRK/TRK mutation-mediated diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/116671**

| Patent document cited in search report | Publication date (day/month/year) | Patent family member(s) | Publication date (day/month/year) |
|---|---|---|---|
| CN 106687464 A | 17 May 2017 | SA 517381087 B1 | 09 February 2021 |
| | | TW 201619168 A | 01 June 2016 |
| | | TWI 709563 B | 11 November 2020 |
| | | SI 3194407 T1 | 31 March 2020 |
| | | WO 2016042087 A1 | 24 March 2016 |
| | | CA 2958782 A1 | 24 March 2016 |
| | | EP 3194407 A1 | 26 July 2017 |
| | | EP 3194407 B1 | 23 October 2019 |
| | | HRP 20192334 T1 | 20 March 2020 |
| | | LT 3194407 T | 27 January 2020 |
| | | HUE 048518 T2 | 28 July 2020 |
| | | KR 20170048596 A | 08 May 2017 |
| | | KR 102563829 B1 | 03 August 2023 |
| | | MX 2017003469 A | 08 May 2017 |
| | | UA 121315 C2 | 12 May 2020 |
| | | PT 3194407 T | 20 January 2020 |
| | | EA 201790609 A1 | 31 July 2017 |
| | | EA 032872 B1 | 31 July 2019 |
| | | ES 2763344 T3 | 28 May 2020 |
| | | SG 11201701116 XA | 30 March 2017 |
| | | BR 112017004035 A2 | 05 December 2017 |
| | | BR 112017004035 A8 | 09 May 2023 |
| | | AU 2015316799 A1 | 27 April 2017 |
| | | AU 2015316799 B2 | 11 July 2019 |
| | | ZA 201701331 B | 29 July 2020 |
| | | IL 250544 A0 | 30 March 2017 |
| | | IL 250544 B | 30 June 2020 |
| | | JP 2017528481 A | 28 September 2017 |
| | | JP 6736545 B2 | 05 August 2020 |
| | | MY 186523 A | 24 July 2021 |
| | | DK 3194407 T3 | 27 January 2020 |
| | | PL 3194407 T3 | 30 April 2020 |
| | | US 2019127390 A1 | 02 May 2019 |
| | | US 10676486 B2 | 09 June 2020 |
| CN 105209040 A | 30 December 2015 | EA 201591794 A1 | 30 December 2015 |
| | | HK 1219057 A1 | 24 March 2017 |
| | | CA 2906262 A1 | 18 September 2014 |
| | | US 2016024113 A1 | 28 January 2016 |
| | | US 9586975 B2 | 07 March 2017 |
| | | JP 2016510797 A | 11 April 2016 |
| | | BR 112015022650 A2 | 18 July 2017 |
| | | BR 112015022650 A8 | 23 January 2018 |
| | | AU 2014230125 A1 | 29 October 2015 |
| | | WO 2014140313 A1 | 18 September 2014 |
| | | IL 241325 A0 | 30 November 2015 |
| | | KR 20150133767 A | 30 November 2015 |
| | | MX 2015012528 A | 15 April 2016 |
| | | SG 11201507493 SA | 29 October 2015 |
| CN 105228625 A | 06 January 2016 | AU 2014230111 A1 | 29 October 2015 |
| | | WO 2014140299 A1 | 18 September 2014 |

Form PCT/ISA/210 (patent family annex) (July 2022)

**INTERNATIONAL SEARCH REPORT**
**Information on patent family members**

International application No.
**PCT/CN2023/116671**

| Patent document cited in search report | Publication date (day/month/year) | Patent family member(s) | Publication date (day/month/year) |
|---|---|---|---|
| | | IL 241250 A0 | 30 November 2015 |
| | | SG 11201507594 YA | 29 October 2015 |
| | | EA 201591773 A1 | 29 January 2016 |
| | | BR 112015022982 A2 | 18 July 2017 |
| | | JP 2016510796 A | 11 April 2016 |
| | | KR 20150133765 A | 30 November 2015 |
| | | CA 2906257 A1 | 18 September 2014 |
| | | US 2016024114 A1 | 28 January 2016 |
| | | HK 1218263 A1 | 10 February 2017 |
| | | MX 2015012526 A | 26 April 2016 |
| CN 105143232 A | 09 December 2015 | BR 112015019873 A2 | 18 July 2017 |
| | | AU 2014230485 A1 | 24 September 2015 |
| | | TW 201444850 A | 01 December 2014 |
| | | MX 2015011359 A | 16 December 2015 |
| | | DK 2970333 T3 | 21 August 2017 |
| | | PL 2970333 T3 | 29 December 2017 |
| | | KR 20150129016 A | 18 November 2015 |
| | | ZA 201506327 B | 22 February 2017 |
| | | US 2016031905 A1 | 04 February 2016 |
| | | WO 2014140235 A1 | 18 September 2014 |
| | | HK 1219481 A1 | 07 April 2017 |
| | | PT 2970333 T | 01 August 2017 |
| | | CA 2904462 A1 | 18 September 2014 |
| | | JP 2016510793 A | 11 April 2016 |
| | | ES 2635021 T3 | 02 October 2017 |
| | | EP 2970333 A1 | 20 January 2016 |
| | | EP 2970333 B1 | 03 May 2017 |
| | | EA 201591698 A1 | 29 February 2016 |
| | | SG 11201506357 RA | 29 September 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211090622
- CN 102264736 A
- CN 104520300 A
- CN 112110938
- US 2016221948 A
- WO 2016196244 A
- WO 201048314 A
- WO 2017004342 A


### Non-patent literature cited in the description


- *Journal of Medicinal Chemistry*, 1974, vol. 17 (7), 708-15
- *Tetrahedron Asymmetry*, 2006, vol. 17 (2), 268-274
- *Tetrahedron Asymmetry*, 2004, vol. 15 (3), 481-488